(19) Europäisches Patentamt
European Patent Office
Office européen des brevets

(11) EP 1 862 555 A1

(12) **EUROPEAN PATENT APPLICATION**

(43) Date of publication:
**05.12.2007 Bulletin 2007/49**

(51) Int Cl.:
***C12Q 1/68*** *(2006.01)* ***C12N 15/10*** *(2006.01)*

(21) Application number: **06011026.9**

(22) Date of filing: **29.05.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Klinikum der Universität Regensburg
93053 Regensburg (DE)**

(72) Inventor: **The designation of the inventor has not yet been filed**

(74) Representative: **Vossius & Partner
Siebertstrasse 4
81675 München (DE)**

(54) **Means and methods for diagnosing cancer or a corresponding predisposition**

(57) The present invention relates to an in vitro method of diagnosing a predisposition of cancer or cancer comprising determining the methylation status of at least one nucleic acid molecule having a nucleotide sequence selected from any one of KLF11 (SEQ ID NO: 1), PLA2G7 (SEQ ID NO: 2), PFC (SEQ ID NO: 3) and MAFB (SEQ ID NO: 4), an allelic variant, a portion or the complementary strand of any one of said nucleotide sequences, said method preferably further comprising determining the methylation status of a nucleic acid molecule having a nucleotide sequence selected from any one of SEQ ID NOs: 5-111, an allelic variant, a portion or the complementary strand of any of said nucleotide sequences. Further, the present invention relates to a kit comprising a polypeptide capable of binding methylated DNA and probes, primers and/or aptamers specific for at least one nucleic acid molecule having a nucleotide sequences of any one of SEQ ID NOs: 1-111, an allelic variant, a portion or the complementary strand of any of said nucleotide sequences. A diagnostic composition and uses of at least one nucleic acid molecule having a nucleotde sequence of any one of SED ID NOs: 1-111, an allelic variant, a portion or the complementary strand of any of said nucleotide sequences is also envisaged.



Printed by Jouve, 75001 PARIS (FR)

SEQUENCE LISTING

<110> Klinikum der Universität Regensburg

<120> MEANS AND METHODS FOR DIAGNOSING CANCER OR A PREDISPOSITION THEREFOR

<130> L1904 EP S3

<160> 202

<170> PatentIn version 3.3

<210> 1
<211> 575
<212> DNA
<213> homo sapiens

<400> 1
ttaaaaaagg aggattcctc actgcggcct ctgccccttg cactgagcgc caacccgcat 60

cacagctcag caaaggcgcg cgcagtgaac agacggagga ggggtggctc ctcgctgagc 120

tgaaggctgc ggcgacgaaa ataaacagca tggttcctgc tctccacgaa cctggcgctg 180

cagacgtggg caaaccacac caagagacgc gagcaaccga aatataggga gaaaaaaaaa 240

ggtgccctgg acagcgggct agatgtctcc tgcaggctcg aggacttccc ggaggggggcg 300

gcgttttctc ggggcctggg aagaatgggc gggtttcagc gggcgccttc tgggtgactg 360

cccgtttcgg cttcccctga ccccgtaaca gagcggaaga ggcccagaga cgccagagga 420

ggggcgtcag cgggacactc aggccaataa gctctgactc ccgctgtctg caaaaatgac 480

cggatgcagg aatgtcacct agaaccggcc agctttccga cctctccggt caaggacgca 540

ttcctaagag acgaagggag aatgacagaa gttaa 575

<210> 2
<211> 413
<212> DNA
<213> homo sapiens

<400> 2
ttaacttagc ccgccgcgga gagagccagg caatgcctgg gtggtgtcgt gctcaccagc 60

tcctttccgc agacctgatg ccgatcagat ttactccctg ctgaattcac cccccagtca 120

ggccggtttt ccttgtttca ccgtgttctc tgaatacact gaattactcc gagcatctat 180

agttggcaat gccctgcgca ttttccgcac tccgatacac tttacactga acaccaattc 240

ggaagttgtg tggaccgtgc tccagattgg aaggcctgcc actgtgctgg tgtcatttct 300

ccctggaaat gacaatgaag gacaatcagg aatagcactt aggtctgagg agaaatggag 360

ctagaaggag ccgtttcata gctcagtgta acacagagtt atttggggtt taa 413

<210> 3
<211> 814
<212> DNA
<213> homo sapiens

<400> 3
ttaagtgttg aatagatgtt cacacgaaag gaataccttc tgagtctgtt ccctcactca 60

gatgtcctct ttctaccagc cctcctccac cctagatgct cagactcagc tttagcatac 120

agcaggcgct aataagtgtt gaatgaatga acaaattacc agaggaaagt gtctggattt 180

gtcttggggg ctcctccggg cggatactga ctctagcttc tttgcccctc ctcagctcag 240

agcgcaacac aggcacttcc tcggcccgcc ccccgcctcg ccgagctatt tgtcgctggt 300

aattgtggca ttggagctgg gctgtcggga gtgggtgggt gcaggtgctc gggcggcagg 360

gtgccagcct cggccacgcc tctgccgttc atcctcgact gacgcggagt cggagtcggc 420

cggcagaggc tcctccaggg ttccggctgg tgggaatcta gggaggtcca ggagacgggg 480

agacaacgaa caagcacgcc ggccaatcaa gaagtgcctg ccaggacaag tagccaatca 540

ggaaacccgt aacgcggagc tctgcagagg aacgtgccgg gcggccctga ggctctaagg 600

gggctgcgca gggcccagac attggggtta tggcagcggc ggggagaggc tgggactgca 660

cactcgcttt cactcctgcc aacctgcgac cacagtggaa aatactgttt tccgcaacag 720

gctgctgtgt cttggcccgt gctgtgctgt ttgccctatg agatgctatc accctacttt 780

tggggaaggg gataggttgt ttttcctcct ttaa 814

<210> 4
<211> 482
<212> DNA
<213> homo sapiens

<400> 4
ttaaatgctg gaagagcgcg tctacaaccc tcttcgagaa gcgtgctctc cgcagaaatg 60

agtcggccgc ctggagagag agcctgggcg gtgccgctgc gcagcccctg ccagtagctg 120

ggggttgggg actcgcacct tgtaaatgtc ctcgtcttgt ttgaacgcag tgagagcaca 180

ctcgtttcca gatcactcgg gaccgggtgt ctcggatctg tgcagactat gtatggctcc 240

ggcctcaggc ggccagggcg ggacaagcac gcctgactct cttggtcccg actggactag 300

agctggtagc accctccctc agggtcctaa gagacctgtg gctcccagag tgtttctgcc 360

actgtggact tctgcgcggg caccaagatg gacaggccaa gctgtgaccc actccacccc 420

cgcccatacg tcccctcggc tctgcctgac actggccgtc gaagacttcc gtggcccatt 480

aa 482

<210> 5
<211> 1208
<212> DNA
<213> homo sapiens

<400> 5
ttaaatcgta cagagaataa agaccttgtg aagtctatgg tctccctaga tcagatttag 60

aaaccacaaa gatcaaagtg acaagtaccc gtccaaaatt ttctggatga ctttctttag 120

aaaaggggct tctggatcaa gacaaatttc cttcccgttc ttcagggagg ctctgaagga 180

aagaaaaaga agatacactc atgagatacc aaggttgaag acccaggctg cgggatttct 240

ctcttgccaa ggtcacagcg gacacagcag cacagaactt acaccacttc caccttggag 300

cactgtgggc ctatggcgaa cacttgcaga ttactgatca ttttgggatg aactccttgc 360

gtggtctgta aacaaacgca acgcagctct ctcaacacag cagcggcagg accagctggg 420

gaagaaagag agacaccctt tatagggcag gttgctggga gagttccctg gaacgcagcg 480

accccgcaga ggctgggatg cacctctgtg cccgagtgcg agtgcgtccc gcgcgccatg 540

cgctctcacc gctggcgatg ggccctggct gcgtcagcag cagcagcagc accaacagcg 600

cgcacaagga gctcgaagga ccggggacac gggccgcgcg gctggacagg aggctcatag 660

tggtcaagag agcgctgcga gcggtcgcgg gttcctgaac tgggtggagg agcggagatt 720

ggaggagcga agattggagg atccggagca ctgtggcttc ctcgtgcctt ctgcactcct 780

tttatctaca ctcatctctc cccactgaca ggaaactcaa gctttgggat gctggggaaa 840

ttccctgaac tcatggggca gtgtggaaag aagaggttgg gggagggggg ccagacaatg 900

ggaactggtg gggagggtgg ccaggaggag ctgcgtgcac ctctaccgag agggacgttg 960

taaccctgct ccgcggtgga gggagctgtg gctccagcta gaagggagta gctattcagc 1020

aggattcagc agggcctcat ggcctgtgcc agagcgggct agggatgccg gcttttctga 1080

ggcacgcagc cctctgggag atgctcctcc tctccttcta gagagaaaag gtggaaccaa 1140

agtaacagga gccttgattt gagaaaaatt tctgaaagtc aatcctttct aattattatt 1200

actattaa 1208

<210> 6
<211> 1096
<212> DNA
<213> homo sapiens

<400> 6
ttaaaaaagt ttcattttag aatctcttta gattttcaga aaagttgcaa ggatagtgca 60

gactttcttt ctttccctgt ccccttctt cccccttcc ttctctcctt cctttcttgt 120

```
ttcttctgcc ctttctctca ctgtctttcc atacttccct cttgctgcct tcacttttcc    180
ctttttcagc ttctctgcct ctcggtggct gcttccctcc gctttcccgc aacttccgtg    240
ggtccttctc attgccccct cacgactcct agtctcccca tcccttatcc aaaggagggc    300
ccctagagtt tccagcccct gccgcctacc cccaagccct ctgcgacttg atcgaggatc    360
gcctgctgaa atgcccacga aatcacctcg attcggaggt ttcctaacaa cccacagcct    420
tccccttcct ctctcagctc atcccgatcc tcatccggga ccgcggcgca ctgcgcacat    480
cgcgccccaa atgcttgcac acccggggct ggaaggtcgc ctggtccctc tgagggtcat    540
ctaggccaac ttctctaact ccagacagta cttttcttag aagttttcct agattttggc    600
ttaaatatgg atttcagatc aacaagggct aatcttttag tgtgagtatt cccgtgtaaa    660
acttactttt cttttgccaa atctggcaac ccgacaccag ggacctgttg aatccgtgct    720
tagtagagac caacgtgggc atcagggggt ccgctaactg agccaagcgc aggggccaga    780
aacgtctgca ggcgcctcct ggatatccca tctgcctttc tgaatgctac tctgctttcg    840
taaaagtgac tgcttcttgt cttctacccc ggcatttctc aaactcctag cgcggaggtt    900
ttataaacct agcgctgccc gctagttgtg gccgccttct gaaagtttct gtgcgggtta    960
cctgtcggct ggcccggcct ccatgggctt ccttggcctg gaacaaagcc ctgacgggcg   1020
gcccgctttt agcgccaggg ctgggcggcc tcggatgcac gccgaggact agcctaggag   1080
aggctgctgt gtttaa                                                   1096
```

<210> 7
<211> 1137
<212> DNA
<213> homo sapiens

<400> 7

```
ttaaaagtct ctggataact tttccaagcg ggtggaatgg gagtagggtg aggaaagggt     60
gctttgtctc agaaccagtc ctttagttcc tgactctgtg tggatgagag gctcctccag    120
aggtcgctgt tccccaggaa tccattacta gcgtccgcac cgcgaggcca gagctcccac    180
tgttcccctg gctttgcttc tatgaggctt acgcggctct gcatctgccc agaggactca    240
gggacgcggc aatatttccg cccccacgag ggtacttacg gcctcgccag ttaccctgtc    300
tgggactcag aagggttaca cgtgaaggtg agcgcatgtg catgtctatg gtacacacag    360
gtcgctatgg ctctgcaccc tccctcctgc gctgccccaa ttcagggccc ggaagggcgt    420
cctgttcccg aggtgccact gtgccagcag gcggcgtgta attggtagga gggaggctgc    480
cgctggagta ttcttggctg tccctcagtg cgatccctgc atgaactcct tttccatgct    540
```

acagtcacag accctcaacc caggcgcacc ctcacccatc ctcattcccc actagggtct 600

cgctgttgac cccgagcagg tttgtcgtcc ccgaccaggt cgcaaggctt cagactctgg 660

ggggaagtcg cgggcctgga tgataagcct tccaggcagt cctgggtcca ctctgtggac 720

accaagcatg aggtctggag gggaggacac aggacacagg atgcaagaca ggcgcccgtc 780

actcagggtc gcaatccgcc atacagagcc actgttgtcc agaggacaga cacatgagga 840

cagagaaaga gaggctcggc ccagctggcc gagaaaccca caagagcgca cttagacatg 900

aagttctcct tcccacatac ctggaagcgt cccccactgc ccctggccgc ctctgctctc 960

aagagcgtgg agccttccct accactccgg gaactctgtg atcttcagga ccggcgggtt 1020

tcaagtggcg ctgttttcgg tctctcctga ggtcgcaacc ctcttcccac gaacaacagc 1080

ccacaggctc cgcagctcgc cttggtctca acaaccgcgc agtacttggg tgcttaa 1137

<210> 8
<211> 646
<212> DNA
<213> homo sapiens

<400> 8
ttaaggacaa tactttcccc tggcagccat gcgcgccgag cagagtcagc ccgggaggct 60

cttttcccac cgcccgaggg aagcccgaga gtcccgcagg ctggcgcggt gggagtgggg 120

cggtggtaac tgcgcgctgt gcgaatagag acgtcgaggc aagcgcgacg gcctcgagtg 180

gctagggaaa gaatccaggg gcgtgcaccc agtgatttcc aatctctgaa accagtgatt 240

tccaacttgt tcagccaaga aactctcagg cgtgaaatca cgttaccgcc tagtgggtaa 300

aagcaaaggc gtggggtaca cagataactt gtttggttca aatccagcct cccgcacgtg 360

ctggccgggc ggaggcggac aattgctcca cctctggaag cgcagagcgc agtccaccaa 420

atggaggcaa ataacgtgtc catctcactg cccggcggtg aggatcaggg gtgggccaag 480

gccgaaggtt ggtaaaccca gcgctcattt tatcagcaga cggactccct ctctatatat 540

ccaaaggtct gcacaaatgt tcctgacaag ctatggaaaa tgtttggaat tccaactcct 600

gaatcactag ccctttcccc acagcttatc tttctccctc ggttaa 646

<210> 9
<211> 1166
<212> DNA
<213> homo sapiens

<400> 9
ttaagagtca tatgagtcta aaggaatgaa cgaggtacct caaccaggta catttcagta 60

cgacctaaat ggtgcccagc ccgggttcct ccttgcacca aaagcgaagc acatctgggg 120

gtttttcccg gcgggacgcg ccagctggag cgcggcggcg ggaggcgccc agcgccaaca 180
cccagctcct ccccgtctgc tgcggccccg cacagcctcc ttaccgatga tgggcttctt 240
ggcggtgtcg ccgtggggtc tagacagctc gaggctcgcc gccccgcaga gtagcaggcc 300
cagcacgcac agcaggcagc ccggactggc catggcgctc gccgcctccc gccgcctttc 360
aaaagctctg cgggcgggcg ggggctgcgg cagggacacc tcgcggcagc gcactcgcgt 420
gagactcggg tgggcggtgc tccgcagcgt gggcctatgg gaaatgcagt cccggcgcgc 480
gctaggcggc cgtggggcgg cggtgggagg ctctgggagt gcagccggcg ctgcgcgccc 540
gccccattgc tctgtccccg gtccgcgacc gcgaccgcga ccccgacccc cacgggccgc 600
ctaagcggag actctggaaa cgcctggggc gggtaccggg tggtctccgc gtccttccct 660
ttcaactgtt acgtcgatgt ggacttcagt cacccgtaca aagctgggct ggccaaccca 720
ggtcctcgag aggggaggtt gggtgtcccc ggccgagttt ggtaaccagg cagcggggag 780
gctggcaggg gattctagaa ggcgcctgag aactgcgcgc caaccgcacc gccccettga 840
aggtcggggc cctatctccg ggaatggtga caacgttcgc tgtacggaca gcctgtgccc 900
ctgaatgtga atgtccacac ttgctatttg gggggtcgcg gcatctgtag gaagtgaaga 960
cgggacggcg ataggaacac acgcacaggc gcatcactag ggtcagaact gggccaactc 1020
cgggcgtcta ctttgaagcg ggcacgccca ggccacgcct gcaaacggcg tcagcttcct 1080
ctgatcaacc gcggtcagcg ccatccaccg accaccacta agcctatcca ccggggcaga 1140
aaagatgact ggttcctatt cgttaa 1166

<210> 10
<211> 928
<212> DNA
<213> homo sapiens

<400> 10
ttaaatctct ttcctgggct aagctgggag tgcgccccaa gtctgaagct ccggcttcag 60
ttgcgaattc cccatcctaa gattgaaggg tgggggtgg cttcccaatt tctccatcac 120
caagctctgg gctcgccttt ccctggcatt tttgcccctc agctgggtct ccgactttcc 180
tcttgggagg cgaccgggtt cgtctggatg cagagtcccg gccttcccgc aaccctcctg 240
gagagggggc ttccggaaat gcgccgcgtc ggggaggctc ccacggtttt cctgtgacat 300
gggcagggtg cgaaccccgc gccagtcttt cccctgcaaa gcctcgcagc agccaggcaa 360
ggagggcgtg gggaaacagg gagacacgca cagggcgcgc gggtgggacc cagcccttcg 420
aggtaagact tcgaggaaat gcgccggcaa cagtgacgga tgcgagatcc gaacgccgac 480

ctccgcacgc gcctctccct cggtagttcc gagaaaggga caagccggct gcagcgggga 540

gcggttgggc cggttcctcg gacgccccag cactctggaa ctggctttgg agccccttct 600

ccctgtgttc ccatccacat tatgacacct actatgtgga cccacgctgg gtgagttcct 660

gttccaccca gtcccctacc tgcaccccca agcagctgca cagcccccac tcccttccag 720

tctgcagccc agccccttc cccccaaccc ccgccaagga tccaactgtg tgtcctctct 780

cccagtctct cccagtgtac agtgtccggg cttatggaca gatttcacta gctcagccta 840

aggtatagcc ctaccccctc caacaccatg gacagcctga taccccagat tagctcagaa 900

atggataggg ggacagattg ggcattaa 928

<210> 11
<211> 1098
<212> DNA
<213> homo sapiens

<400> 11
ttaacgaact acgccagaag aatgggcgta gggcccacag cccctcacac aacccaggcc 60

aagctccctc gtacactcag accaggagaa gaaacagaca cgcaatacac aagccccgct 120

cccttggagc ctacacttgg gcgaacacac cactgctgtt tgcagtatcc cgagacaggc 180

ccggcaggcg tgagctaaga ggcctcgggc ccgaacttgg gatttgaggc tctctcacat 240

cgccggtccc agcggcagcg cctgaggcac agccaagacc atccgaggtc cccgggccgc 300

gcgcaggtag cagacttctt cagaactggg ggagctaaag agagagtggg cttagagccc 360

aggcctccct ggatcccaga tcctcgcctc cgcctacagc caagcccctc agcttttgcc 420

cagagctgct gacttcctcg ccgcctccct gcgcaatggg acctagagat aagccccaag 480

cccgggtgct agtccaggca gagtcgggtg ctagtccagg cagagtccag tgctagtccg 540

cggcctcaac agagctgtcg gctgggctcc ggaagcccac ccggaacctg atggaggcag 600

cgaattcctc agagcagagg acactgcgga gaggctcgaa tccacttccc acattgtgcg 660

ggaccgagcg cgcagggcgc tctcgcgtgg cctccttagc tcgggttcag ccgaggctct 720

cacgtacact tcgttacccc ttccccccgc cgcagctctg gtccttcctt ccactggctc 780

tcttctctgg cctatggaaa caacgaacta cttttcgcct ttcaaggccc gggtcccaag 840

ccacctcctc tgtgaagccg ccaacaccct ggtagctgcc tctcagtgtt ttgcaactgg 900

cacggccctg tccctcggtc cgcttctact agcgctgggg gtgctgcgag tcttgcccac 960

tagactagga gctccttgag ggcaggaacc gaatctgatt cgtttccatt ttcccagccc 1020

ccctgcgcgg cctggcgcag aaaagacgcg caataaacat ttgctgaatt gaattgcgtt 1080

ttctccttat tttattaa 1098

<210> 12

<211> 721
<212> DNA
<213> homo sapiens

<400> 12
ttaatatttt ctgctagctc tgcttgtcat aactgcaact cggaatagaa caacctctct 60

cccgtgcata cgcaccccca actctcgccc tatcctccca tccacagtca tcactgacct 120

gtagacccgg aggagcaggc ttccgggggt cggtccaggt tggggataca gggaggggtc 180

ctcgcgttcc gcacaacctg tagtgcttgg gtctgacttc ggcagccctc gggtacagtc 240

tgccgggcta gattcccgtt gcttctccgc gtctctccgc gggccgctct agcgccggga 300

agggggcgcc gcaggtgact gcccgcggca ctgggacggc tgacagattt atggagactt 360

cacagcatct ggctggggtc cgcgagggca gggcggagtc cggtcgatcc gatgctgggg 420

tctaaagctt ccccagccac acgcctgggc cgagtcgccc ccagagtccc cggcccctgc 480

tccccttggg cgggtgtaca caaggatccg gccagcgccg agtgtactgg ttcctcccac 540

ccagtgtagc cccctccttc acactctcgg cccggcttcc agtctcccac tgagtaaata 600

tttatccaga gagcgagtcg cccctcggag gaggcctcga cgcggctagg gaaaggcttt 660

ttggcttctc cctggcctga gccagctgca ggccttactg gtgttttggt tttggtctta 720

a 721

<210> 13
<211> 507
<212> DNA
<213> homo sapiens

<400> 13
ttaaaagaaa agcagaaatc cccgcaactg ctcaacctct cgtgactgcc tgtgttctgg 60

ggttctgaga gggaccgtgc gctgcctggg gaagcaatgc aagtctccat agcctgcaca 120

gagcacaatt tgaagagtcg gaatggtgag gaccgacttc tgagcaagca gagctccacc 180

gcccccaatg tggtgaacgc agcccgggcc aaattccgca cggtcgctat catcgcgcgc 240

agcctgggga cgttcacgcc tcagcatcac atttctctca aagagtccac cgcaaagcag 300

actggcatga aatataggta tgcacgtaag attccctctg tgcggggtat ccaggttcta 360

tacaggtgca gtggagacag tccatcggtt ttgaaaagtg atttactcat cagtgggttg 420

ctcactttag aattctcagg tggttctaaa atagctcttt agggggatga ataaacccag 480

ggtggttgtg tgtgtaaaga tttttaa 507

<210> 14
<211> 537
<212> DNA
<213> homo sapiens

<400> 14
ttaaatcgct cggagtttct ggagatcttc ccttcatacc ttccgctggc gcctagggtc 60

ttctcggcca cattggggat aaactgcttg taggctaaag gggtcagctt tttggggtgc 120

ctcctcttcc cgaacccccct gcccggtccg cacgccagtc ccgagcatac cagcagcgag 180

gagacgagga ctagcagcag acatctcgcc agcagcagca tctcgcccat ggaactgatg 240

acttccgagc tgtccccgtg cgggtccgtg cgcgagtgcg cgcggcgggt gtgtgcgtgt 300

gcgctctctc ttgcgctttc ccttcctcgc tccggctcgc ccgctcgctc tctccctcgc 360

tggctgcctc gctctttctc ttcctatata accttgcccg ccgcggctgc gggggactct 420

ccaccgctcc ccacccagac aggggctgga atggcaggct gccggccgct gataacggaa 480

cacatcggag ttgggtcgcg agacagcaat caaaagacaa aaagagtctg attttaa 537

<210> 15
<211> 592
<212> DNA
<213> homo sapiens

<400> 15
ttaaaccgac cgaaggcgtg ttttgtggag tggcttcgaa agcggccgag gcagccgaag 60

ccgctttcga agttagcaaa caaagcgtct ccccagtaac cgcccggttc cagacgaggc 120

tcctgccact caggccgcgg agtcgcagcg tcgcccagcc cccggctcct cctccctgcg 180

tctctcctcc ctccgctccc ggctccccag cggataggag ccaccaatcc cagccacaga 240

caacccttca aacgaaatag cgaggcggcc ccctctaacg ggcggcgggg gcgctggccc 300

cctccctgcg ccacatctgt cccgcaccgg gcgcagcagc tgattcatct gcagccaggt 360

ccgggaagga actgtgctcc cggcagttgc agggcagcgt ccgggcgggt ggcttgtccc 420

caggaccgcg ctccccccga gccgttttag gtgtgtgttc tcttctgtcc cgagcagtca 480

gcaaaccaca gaagaaaaaa atcgtaaggt tgctgccttc ttagagagac gctttttttc 540

cctgttgatt cattttagtc ctgtaattgg aaaaaccacc caggcttttt aa 592

<210> 16
<211> 1003
<212> DNA
<213> homo sapiens

<400> 16
ttaacctctc cattgcatca tccccatccc ggtgggtaca gaagcgcagc aaccccgcgg 60

tgctcccgga agacaccgcg tctccaacat ttacacggag atgctgctcg gctcaggtcg 120

ggtccgtacg cttcccgagg tgcgtactac gcgagccaca cacatcacta ccgatattcc 180

tgagaaagtc ggtgccacag taagtcagct gcaagcgtgg ggtggccggt gacgcgctgt 240

gcccgtgaat acgcccattg tgcgggtcag acgcagccag ggcgcagcgt tcaggaggcg 300

ggtgggcagc cgaactttca ctggccacga gcgcttcacc ttctagaatt tgccagggac 360

taggcatcaa ctcccagtga gaaaatcaca tgtgaagttg gaagacacat gctgcccttc 420

acttttctac ggagttcaca tcagtttcag caatccgcgt tctcagttcg ctttgcccgg 480

gcgagtgggt tccatcatca cctgaaccaa aaacgcacca gggcgcgcgt gcccaggcac 540

caaggccaga gaaaccgcac gtttcgagtc cccagttgct gagaaacaat cacagccctc 600

ctcttttctc ccccttctgc aggttccgac ctgaatctga aagtgcttct aaaccccatg 660

cttcacagaa cgttcggagg gggaggcgct ccgtgggggc tgctgcgtgc ccatgtagcc 720

gaactgactc gaataaaagc gtgtccacgt ctacatcaca catctgtgta aatgcagaag 780

cctgcggatg cgcggggctg gccagacttc acagtgtgca gacacatggg ctcatgcacg 840

cgcacgcgcg cgctcacaca cacaccccaa attacctccg ggatggagaa agccgctgcg 900

gattggccag cagagccgtc actcacgccc agggggcagg gagacttcgc aaagctgttt 960

cctttttttt ttttcctcct ctcctcccta ccgtttcatt taa 1003

<210> 17
<211> 2181
<212> DNA
<213> homo sapiens

<400> 17
ttaatgccca ctgcaagggg agcagaaccc gagctggggg gcagagtcca gcgctgggag 60

cctgcggcca ggggcgccga tcgcccccca gcgcagagtg tggatcccag tgcctggaga 120

gagcagctcc ctttccttag gacgcgggga gcgcactgcc cccagcccag ggcccaggac 180

ccagagccag accccaaacg cagagtctca ggtccggagc ccgatgccca gatggcggag 240

accggagcgc acagccagga accgggttcc caaagtctgg ttacccgcgc ctggggcgct 300

tctggaagcg ctgcggacca gcttcgccaa caagtgcaat ccgcgctccc cggcggcccg 360

agtggggagg ggctggggct gcgggcctct agctgggccc tgatgacagc atgggggagg 420

gggagcctgg cgtggaaccc gagtcccggt aaaaaaatac accagagcag agctcccaaa 480

ataaacccga gcccgcgcca gctgcagggc gcattctggg gctgaggagc tgcgtcccct 540

```
cccagagggg cgccacgggt cccttcccaa gagacttatg acccctcctc cggggtaacc     600
cttccccgt cccccgcata cccgtgcatt gcttttaccg atttgggttt cttcttgggc     660
gcgaggttgt cgtagaaaat cttggcttcc tcccagaccg gggccgcagc ggtctccgct     720
cctggcccgg gctcgcgggg ctcctggggc tcccggggct gcccaggttc catgctccgg     780
ctctgacccg gcccggccag agcaggcggc ggcgtagacg cggaggtggc tggggctggg     840
ctgcaggagg gaggcgagtc ccggcgaggg aagggggcgg gcagcggcga ggaggctgtg     900
cgtgtgagct gggctccccg cccgccgggc gggggaggt cacactggcc ctctcggtcg     960
cccgtgtgtc gctgccgctg ctgctgctac tgctgtacgg tatgagggat agctgggctc    1020
tccttccccg accgacccac gccagcagcc ggttgggtgg tgagggggc tctctggctc    1080
tctgggtttg tgggctgcag cggattgggg attgcgaaaa tgatgttctt cgcaggcgct    1140
gcagggcgcg gagacaggag aggcctgaca actttttttt tcccccagga tagtgatccc    1200
caaaggacaa aatgaaaagc tagccgctcc cctcccaaaa gctagctgcc catctttcct    1260
ttaggagcta ggaccctggg atcctgacga tgtccccttc cctccgaggc cagtccatcc    1320
ggcagacggg caggaggtga tgcatggatg gctcctctca ctagcagaag cctgctccca    1380
gcgggagctc agtgaagggc ccaacccggc agctcctcca caccacctag gaggggccct    1440
tgaagtccat gttgagacca ggctcgggtg gtctcaggtc gagaccttgg cctggggtgg    1500
ggacccaggc cagagagact cccagggact ctgaacccga attcttgctg gccctttggt    1560
ggtcagcaga gggcgcgcat tcctaggccc ccacctgccc agcttggggt gatttgttat    1620
aggccatgaa ggggctggat agagggaggg cagaagaggg gcctcggaac taccatcctg    1680
gaattccagg gtgttgtccc tcttgatgca gagctctgta ctagtctcag ggtgcagaca    1740
caaatgtcac ccatcccaag gtacaggcca ggaagcagac agcctctctc agaggtgtgc    1800
atgggtgggt gggagaagca ggagaaaaca ctggcttagc taggagggcc tctgactggg    1860
gaggtgaggg ggctacagaa tggagtgatc ccctcagcct ccaccaggtc ctgggaggaa    1920
taaaccaacc cttgtgtggg aggaagggaa aaatcataat aaaatgttag gattgggga     1980
agtgtgttta gacttacttc tagaagattg tttccagaga tggcccaaaa acctcttcca    2040
tcctgcctgt cctttggaaa tgtgactttg acattcctcc cattcagagg tggagtccag    2100
tctgagctgg ccctgtgact tgctttgacc aattgaatgg agtagaagtg gtgctctgtg    2160
gtttccaagc ttacgtctta a                                              2181
```

<210> 18
<211> 786
<212> DNA

<213> homo sapiens

<400> 18
ttaacctctc acaaagcctt caactcacaa accgcgatcc ttggaaacca tcctccaaag 60

cagtgcttgg aggcctctaa ggcccccgga ccaactcccg ctggaagaag cctgcaggga 120

ctcgggaatc acgggaacct ttcccgtcgg ttccgggcct ggagggccag gaagagccgc 180

gcgtccgcct ttcgtcccgc caggaactcc ccataggaca cgacaccgca ggaacaagcg 240

tcctgggagc ccctgggatc ttggctgtcg tctctaggga ccctacaccg tgaaatgata 300

gaggcgaggt tccttgggtt ccgcaagtcg acgaaaatag ctcgtggaga aggcgcgtcc 360

tgcaactgca gttcgcaagc tctcagggcg ccccgccagc tgggggccag attgggtgac 420

actcccctcg acgcagcctc cggagcggcg cgcactctcc agaggccagc aggactgcgc 480

tctctaccgc agaacctgct ccagctaggt gttctctccc catctcgccg tcgctctgcc 540

ccctcactct ctctggacct cagagccggt tctctccttc ctcctcccgc gctttccgtc 600

cggggatcgc aacctccagc ccgtgggcaa cgcgttcagc ccaagacccc acagtttttc 660

ccaacgaccc ccctaccaag ggcttctctt gtttctgctc tcctgccccg cacttcccaa 720

aggtgagaat ctcccaggga ctgctggaca gagaaggcct gggcaggggc agtagccgat 780

atttaa 786

<210> 19
<211> 715
<212> DNA
<213> homo sapiens

<400> 19
ttaaaaagac tgctatggcc cgagtccctc tgctgtgccg ggcactgtgc tgggcatgta 60

acaggcatat tcttctgatc tttacaactc tcccatgagg caggcactat cgttagccca 120

ttttacagat gtggccatag aggcccagag aggagaaggg gcttacctaa ggctatagac 180

tgttggtatc tggagataaa cccgggatgg tgctcactaa actaccttgg gtgtcagtcc 240

tgcttcaaga ctccagagag ataaagagag atgacctcag agacaaagag actcagaccc 300

agccagaggc ccaatggaca gtgggagggg tgggtggaag aaggctggtc tctgtctgac 360

caagcccccc cagaataacg caggctgccc ccctaggtgg aaacaatgac acaatcagct 420

cccaatacca agggcctgac atcacaaggg gaggggaagg cagctgaggt tgtgggggga 480

ggtgccccgc cccttggcag gcccctacag ccaatggaac ggccctggaa gagacccggg 540

tcgcctccgg agcttcaaaa acatgtgagg agggaagagt gtgcagacgg aacttcagcc 600

gctgcctctg ttctcagcgt cagtgccgcc actgcccccg ccagagccca ccggccagca 660

tgtcctctgc tcacttcaac cgaggccctg cctacgggct gtcagccgag gttaa 715

<210> 20
<211> 501
<212> DNA
<213> homo sapiens

<400> 20
ttaaaaaagg atactccgag ggaagagagc aagggcggtg cgccgccaag gaccaactag 60

cggcggagct tcgatcttgc ctaggcgcgg agagctccca acctgggctg gaaccttgcc 120

cagcacaggt cagttcgtct ttctctgctc ttctttggct cggcttcgaa gtccattcat 180

gagcaaggaa aagtggaggc agcgagccac ctgcagacgc aatgcagttc catggacttt 240

cttttcacgc gcgcgcccag aatggactgg ggacctttgg gacgggatgg gacgggtaca 300

cctggatgcc ttttcgcggg gttcacggtg tagggagatt agaggcattg ggtgatgaga 360

gcgcagggat agtggaaata ccatgtgctg ctccgggaag cttttcccag tgtttgagtg 420

acgtgagcgt gtggcgggaa gaagctgcgt ttggctgcgg gacagtggcg gctccagcat 480

ttccatatag aaggggctta a 501

<210> 21
<211> 873
<212> DNA
<213> homo sapiens

<400> 21
ttccagtcca tgccttttcc tccctcggga accttccagt gtctcccact gtcctcgcga 60

ccccgtggcc ctcacctggg tcctgggctg gcagcggctg ctcccgatac gccccgtatt 120

gccggtagga ggctccgtag gtatattccg acttgggcga gtaagcgccc gtgcctgcaa 180

gcccattgag attgaattgg tggtggtagg tgtaggggtt caccgtctgg ccatagggct 240

ggcccgagta gtaatcgtgc tggggagcgc tgtagtagcc caggtcagtg acagaagact 300

ttaagccgca agcaggcgga gggtactgcc cactccggcc tccccctccc gcgcacttgt 360

actttatctg caggatctcc acacctcagg acagcgagaa ggcgctttcg cagcgccctc 420

tgctaagagt cgggacgggc tctgcccgag tgtagccaca ccgcccccag cctcaactcg 480

ccttttcctt atttcatggg catccgttct ctctggctgt cccacatttg ctctgcacta 540

gtcagtcctt tctgacgctg tcaaaaccag tttccccagc actctgtctc gggaaaggcg 600

tcccttccta cctgcagctc gtgtagcctc gacagccccc ccttgcagaa aagttcggcc 660

agaaagctgg gcgtagaggg gcctgggatg tctgccaagc tccggcgtgc tgagtggtac 720

tctcggtagc ctagggaggc gcccaactcg ggcgcccagc ggacgcgatg gaacactctg 780

gaggcgtact tgagggtctg ggtcatggtc tggttcaggg tgctcgcgaa agaaagcgct 840

tctcctgagc atcatatctc aacccctatt taa 873

<210> 22
<211> 1778
<212> DNA
<213> homo sapiens

<400> 22
ttaaaaaagt ttcattttag aatctcttta gattttcaga aaagttgcaa ggatagtgca 60

gactttcttt ctttccctgt ccccttcttt ccccccttcc ttctctcctt cctttcttgt 120

ttcttctgcc ctttctctca ctgtctttcc atacttccct cttgctgcct tcacttttcc 180

ctttttcagc ttctctgcct ctcggtggct gcttccctcc gctttcccgc aacttccgtg 240

ggtccttctc attgcccccct cacgactcct agtctcccca tcccttatcc aaaggagggc 300

ccctagagtt tccagcccct gccgcctacc cccaagccct ctgcgacttg atcgaggatc 360

gcctgctgaa atgcccacga aatcacctcg attcggaggt ttcctaacaa cccacagcct 420

tccccttcct ctctcagctc atcccgatcc tcatccggga ccgcggcgca ctgcgcacat 480

cgcgccccaa atgcttgcac acccggggct ggaaggtcgc ctggtccctc tgagggtcat 540

ctaggccaac ttctctaact ccagacagta cttttcttag aagttttcct agattttggc 600

ttccagtcca tgccttttcc tccctcggga accttccagt gtctcccact gtcctcgcga 660

ccccgtggcc ctcacctggg tcctgggctg gcagcggctg ctcccgatac gccccgtatt 720

gccggtagga ggctccgtag gtatattccg acttgggcga gtaagcgccc gtgcctgcaa 780

gcccattgag attgaattgg tggtggtagg tgtaggggtt caccgtctgg ccatagggct 840

ggcccgagta gtaatcgtgc tggggagcgc tgtagtagcc caggtcagtg acagaagact 900

cgggcagggt aggcgagtcc ttggagcccg catggcagct aagggagctg gagatgtcgg 960

tgaggatgct gctgagcttg cgatcgaagg agccactcat cctggcgggc gctgcacctc 1020

cccagggctg gcctccgcag aggacaggaa cggaccggag tgcgagagag gcggaagaga 1080

cgaggcaggg gtgtgtgtcc agaaggcgaa gggaggaccc ggccgcgtct gggggggaggg 1140

ggaggccgag aggcgcttac accattgcct gccgtcaggc ggtcgctgcg cgcctctcct 1200

cgcgtcccaa gccacaatca aatgctgcca gctccgcccg gccagccggt tatagactca 1260

atagtggagt cgccgaggga ggctcctacg cgctgattgg ctgcaagtct tgccttccag 1320

gcaaaacacg cttttgctct ctcgctccta tgctctcctc tctctctctc tctccctttt 1380

ccttctctcc tccctcctct ctcttctttt tccccagtct ttgttgttac tcaaaacaca 1440

cacacacaca cacacacaca cacacaccct cgctactctt gtagctccag cccagataag 1500

ttctctgaag gaggggcgtg gaggtggagg ggaagggttg ctctgtggag cccccaggat 1560

ctgtcagtct gtttctctgg ctgggggatg agagatgggc ttaggtgagg tgggcaagga 1620

ggggagtttg cccattgtgt gtttgtggaa agagatggtc taaggtgggg tatggttgtg 1680

tgtgtgtgca cacgtgtgca cgcactgtgc aaatgacagg cacacaaatg tacccaaagt 1740

ggtgaggact cacagcccct gaaaatggtt tctcttaa 1778

<210> 23
<211> 658
<212> DNA
<213> homo sapiens

<400> 23
ttaatttact aaactgtttg agcagccgta gattcccttc acaatccggt aaaatctata 60

attactcctc cccttcccca aaatgtactt attttcccat tcaatatcgg agcttctcgg 120

acttcctgaa gttagtccca ggataaagat cctctttctc ctccccttg ccccgcccca 180

cgacctcgct tcccagtaat tgctgggatc ccgattctct ggaggcattc cgggcggatt 240

gattgagtgt aattcaactg agttatgatt acagctgctg gttcccctgt gccgcagacg 300

ggtgtgtagg gagggtgggg cacgtggaga tgggcaagat gtctggagag agcagtgggg 360

ctgttgtgca cgtttttgct agaggtgagg gttgagagct agggaggaaa caattccgag 420

cccaccccgc tgggacccac gggctgaggt cctccaggaa gcactggggc tcagctttcc 480

ctatgtcagt acggatggag gacacacagg aggaaggctg gtggctctgc cggaacattc 540

tgagggcctt gttctctgcc tacgctggga cctgcagtcc catctcccat ttggcctcag 600

ccagcgtggt ttattgtacc caaatcccgc ttgtttcctc cttatgcagc agttttaa 658

<210> 24
<211> 650
<212> DNA
<213> homo sapiens

<400> 24
ttaaaccagc cgtcatagcg ctgcacttcc cagggcagtg agagtgcgtc ctgactgcct 60

gtgggcacag agaagggcct cctcagcact acgctcccag ctacccctcc cgagcaacga 120

aggccttggc cagtccttcc ccagctcgcg gagcgcccct aaccggaccc aagtgtcggg 180

ccgcactggg aagtttccca tcccgctgag ctgcacggcg aaatgacctt ccagtctcag 240

ggagccgctt gccgcacctc tccccgcccc acctcccagg gatcctgggg aacaccccgc 300

cgctagagga gcctgatact tgcccgatgg acccagggat ccagtcagaa gagctcccag 360

gagcatcagt gcctctggtc ttgcacggag catgccaacc ctgcagcctg cggggtgagg 420

gtgggggtgg taggtggtat gcgaaagcca ctgttagggc gtcctctatg cctcccctct 480

tgttcctaca gctagtactg gaggaggagc accagctgtt tccacttctg gaaggtagct 540

gttagaagca tcaccgagga ccttcatcca aacgccactc tttccaggac aattggcagc 600

tctggaggca ttgacactgt tccccatccg ccaccccatc agagagttaa 650

<210> 25
<211> 321
<212> DNA
<213> homo sapiens

<400> 25
ttaaataagc ccagagtacc tgtgcggggt gctgtactct gcttcaaacg caaagtagga 60

aatcctgcac tcgactatgc aaacgcttgc cgctagccgc atccttctat cgccccccaa 120

ctcaagaccc gaaatggcca actcttcacc gagggggcgt cccatcgctg ccttgccttg 180

gcccgcccct tcgccaagct caatcccttt tccccagaaa aacctgtgcc tgtcctcaga 240

cccggcccca cggctggaag tcaaaaccca ccccggagcc acgcggctgg agcacgtttt 300

tacacctgct cggccgctta a 321

<210> 26
<211> 696
<212> DNA
<213> homo sapiens

<400> 26
ttaaggaggg gcgaccctcg gggaggcagc aacacttgca gcctctgagc tagtctgaaa 60

gctcccttgt tcccaggact tgcggggttt tgaagcttta tgtcctgacc ccaagctctg 120

gggagctctc ctgtctccct gccctaggtc tgttcctgtg tctgaggctc ccggtgtctc 180

ctagctcatc ccccctttc tcttcccgcc ctcatcacac acacacacac acacacacac 240

acacacacac acacacacac acagcggcag gaaggagggg ccgaacgatc ccgggagctg 300

ctcgggaaag gagagcaaag tgagggacgg ggtcctgccc taccaacgcg cttccgaggc 360

tgggaaacca tgtggacgct gggatggggg cggtggcggt tttgcatgaa cgatcggttc 420

ctcaggttct tgctcctgct gtcaagatac aagcagcaca cctcacaacc acccatgccc 480

tcagtctgct cagagtcggg ggttcccctc tttgatcacc ccactccgcc caccccaggg 540

gactggtgaa cagcacagag gccaggaatc ggagacatct gcgagagagg cggagcaatc 600

ctggcagagt tcagccggcg ctgaagaagc cgcaagaaaa cgtggaacaa gacctctgtc 660

tagtgaaggt catttatgtg caagctgagg gtttaa 696

<210> 27

<211> 629
<212> DNA
<213> homo sapiens

<400> 27
ttaagaggca gcctcgaccc cgcctctgcc ttctgaccca tccctaaaga tggccgggca 60

cccctacagg acccccagaa gccgacctct ccctccacgt gcgccgccgc ctcgcccctc 120

cccgcagtcc tccaggtctt cttcctgtcc ccaaccgtcg gggaccccca agctccgtcc 180

tccgcttcct gctctcttta tcccgccccc gctccttctg ggccaacgcc gccagtgact 240

attcccaggg atccctcaga gctgaggacg ccgcggccgc atccccattt gggaccttgc 300

cccgactccg acctccggat ctccagctcc accaaacctc gaagccacca tccgccccca 360

aactgccgct gcgccaggct ttcggagggg agtcgtggca acagccatta ggatgcacta 420

agcagggcac ttcctgagcc caaggacacc gaggggcatt cactgagtga atggggatgt 480

gctgctaata actaacacta attgaccacc tgccgtgtgc ccatcctctt cgaacatgca 540

acttccttgt tccttatctt gttactgtct ctttctctct gccagaatcc aatcctcaca 600

agggcaggga ctggggtctg ttttgttaa 629

<210> 28
<211> 1081
<212> DNA
<213> homo sapiens

<400> 28
ttaagacact catctggtgt ctgagtctgc aggtgacact gcttaggtac ggagcgcgga 60

gtcggagcgg ggacgcgccg ggctgcagct ctgggatgca atccgcctgc ctcgcaagct 120

gctcgccgct gcgctggcag cgcttacgct gagctcggag gatcccatta ggacttgccc 180

ggggatgtgc acctgccgtg cgctccgagg tcagtggcag gtctgcaggc agccgaggga 240

gcggtgaagc cggaagacct gtctctgccc atgtgcaagg cagtgatttc gggtgagaga 300

gcgggtgggt gagtctcttt agagttggaa agaaggcaaa atcttgggtt tctcctcggc 360

ggagggacag gggactcccc cagttgggtc ttgcacttcc cttacctctc ccaccccgcg 420

tgcgccccgg cccagtgccg aagccccact tcaaagatgc tctgggagac cgggatcctg 480

gctgctcagc gggaaggcag caggcaagtg gtctaacgtt ggcggcggtg gcggtggcgg 540

cggcggcggc accgacagta aagcggggag caagagaatt tgaaaagaga catccgctcc 600

ctctcacacg ctgaggggga ggcattcgcg tttccccaaa tgagaaagaa ccacaagaaa 660

tcatgaagta aaaactttgg aaagctgcca ctggagatgt tgcacaaaaa cctggaatct 720

tttaggagtc tcctcccagt cgttgaaggt ttggggagca gctgatacag caaggagggc 780

tcttgcaagg attcaaggta acagtgcagg gtttcggtga tttttctccc cttttcattg 840

tcctttttgg tgtgtccgtt ggtaaaggga ccattttgct ctcatacaac cctcttgccc 900

tggtgcctgg cagtggcagg cagagggaat gatgccacct ctcctagttt ctgtcttgtg 960

gagttgagca atggactttg gggttgagct agagattgct ctggtattgc ctcctggtgt 1020

tgcatggctg tcagagggtt tgggctccct gcctgaccta tattgctttt gcctggttta 1080

a 1081

<210> 29
<211> 1039
<212> DNA
<213> homo sapiens

<400> 29
ttaatcgcag atgattccat gagtttgaga cccactgtcc taacactgtg atcctccaag 60

tgtgatccca ggaccagagc aacatcacta tctgggaatt tgttagaaat gcaaactctc 120

aggccccacc ccaaacctac tgaatcggca gctctgggga tggagcctga cagtccttta 180

tccctgcagg cgattcagat gcttgctaag gtttcattag gtaggtcggg gatgcggccc 240

aagaatgttc atttcttgac gtttccaggg gctgctggtg gtggtcgtgg tctgagaacc 300

acacttagag aatcacgagc agaaaaaaat ctgatctgtg acacacagct ggttgggctg 360

acagagcaaa gcctcaaacc caccatttct tctcttcaac cacagaccac caaatctgca 420

agtattacat ctctgctcct ccccaggagc tgcccacaag caccccctac ttccaagacc 480

cctgttcttg tctcaacccg cccttctatc agggtcccta cactccactt ctgcccagct 540

cctgtccagc tgtgagttca cttgaggcct ctcccgtgaa atctgccctc tcggtctgag 600

gtcagggaac tcctgtaaga cgcaaaaggc ccacggttct gagccagtgg cctccgaagg 660

ccggaatccg ctagccccgg gtctggcaga acgtcacttg ctgcgcccta ggcctcttcc 720

cgccacgctt agagagaggg actggaggct cagaaggggg aagagaaggt aacgcggcag 780

ctgctgggtg gggacggtga ctgtgcccct agatcgctgc ctcctgggtc ctgaatcttg 840

ctccccgctc cgtagagttc ccgaaacccc gaaggctctc aagcaccgca agccactcct 900

aacctccccc acgccattcc ccaagggcac tgtcccccga ggctgggcgg gcgggaactg 960

tcatgggatt cgctcgggcg gagggcgctg ggccaggacg tgggcgggcg cggggtggcc 1020

gccgctcccc caagcttaa 1039

<210> 30

<211> 965
<212> DNA
<213> homo sapiens

<400> 30
ttaactgaca gagtggttga aagaagtctg gaaatgagag aagaggggtc agaatgtaaa 60

agaggaatcc tggttccctt ccacgggggt cccgaggtgc tttgaggagg gagaaagagg 120

gcgtcccctc tggggagccc actctccggg cttctactga cctggtctcc gcctcaccgg 180

cctcttgcgg ccgctgcaga agcgcacttt gctgaacacc ccgaggacgt gcctctcgca 240

cagggagcgc ccgtctttgc tggggctgga gcggcgcttg gaggccgaca ctcggtcgct 300

gttggactcc ctcgcctgcc gcttctgccg gatcaaggag ctggctatcg ccgcagccat 360

agctgctcag cgagggcctc aggccccagc ctctactgcg ccctccggct tgcgctccgc 420

cggggcgagg gcaggacctg ggcggccagg gaaagggcag tcgcggggag gcagtgctaa 480

aatttgagga ggctgcagta tcgaaaaccc ggcgctcaca aggttagtca aagtctgggc 540

agtggcgaca aaatgtgtga aaatccagat gtaaacttcc ccaacctctg gcggccgggg 600

ggcggggcgg ggcggtccca ggccctcttg cgaagtagac gtttgcaccc caaacttgca 660

ccccaaggcg atcggcgtcc aaggggcagt ggggagttta gtcacactgc gttcggggta 720

ccaagtggaa ggggaagaac gatgcccaaa ataacaagac gtgcctctgt tggagaggcg 780

caagcgttgt aaggtgtcca aagtatacct acacatacat acatagaaaa cccgtttaca 840

aagcagagtc tggacccagg cgggtagcgc gcccccggta gaaaatacta aaaagtgaat 900

aaaacgttcc tttagaaaac aagccaccaa ccgcacgaga gaaggagagg aaggcagcaa 960

tttaa 965

<210> 31
<211> 435
<212> DNA
<213> homo sapiens

<400> 31
ttaagaaaaa aaaatccact tttcatagcg tgatggccga agcggaattt ctcctaggct 60

tcttcaaagc ccaagaccgg tgatgcagaa tggactctgc cagcttcgga gacttgatcc 120

aggtcaggga tcccaggttc gcagccgggg gatttccacg aaaacagcgc ctcctgtcgt 180

ggagagggaa agctgaattt tcccgcaggc gcctccgggg agcaccgcca ggcgatactg 240

gattgaactc cgcacgcatc ccagtcaggg atttaggttc ttagagccga ggccaagatg 300

gaaggagaga tggccgttcg tccagctccc agcctctctc catctcagtg gccgcattgc 360

aagataccta acaaatttag gaggtgaggt tctagtgtac ccagatgcca gccagtcact 420

ttccctgagg tttaa 435

<210> 32
<211> 1166
<212> DNA
<213> homo sapiens

<400> 32
ttaagagtca tatgagtcta aaggaatgaa cgaggtacct caaccaggta catttcagta 60

cgacctaaat ggtgcccagc ccgggttcct ccttgcacca aaagcgaagc acatctgggg 120

gtttttcccg gcgggacgcg ccagctggag cgcggcggcg ggaggcgccc agcgccaaca 180

cccagctcct ccccgtctgc tgcggccccg cacagcctcc ttaccgatga tgggcttctt 240

ggcggtgtcg ccgtggggtc tagacagctc gaggctcgcc gccccgcaga gtagcaggcc 300

cagcacgcac agcaggcagc ccggactggc catggcgctc gccgcctccc gccgcctttc 360

aaaagctctg cgggcgggcg ggggctgcgg cagggacacc tcgcggcagc gcactcgcgt 420

gagactcggg tgggcggtgc tccgcagcgt gggcctatgg gaaatgcagt cccggcgcgc 480

gctaggcggc cgtggggcgg cggtgggagg ctctgggagt gcagccggcg ctgcgcgccc 540

gccccattgc tctgtccccg gtccgcgacc gcgaccgcga ccccgacccc cacgggccgc 600

ctaagcggag actctggaaa cgcctggggc gggtaccggg tggtctccgc gtccttccct 660

ttcaactgtt acgtcgatgt ggacttcagt cacccgtaca aagctgggct ggccaaccca 720

ggtcctcgag aggggaggtt gggtgtcccc ggccgagttt ggtaaccagg cagcggggag 780

gctggcaggg gattctagaa ggcgcctgag aactgcgcgc caaccgcacc gccccccttga 840

aggtcggggc cctatctccg ggaatggtga caacgttcgc tgtacggaca gcctgtgccc 900

ctgaatgtga atgtccacac ttgctatttg gggggtcgcg gcatctgtag gaagtgaaga 960

cgggacggcg ataggaacac acgcacaggc gcatcactag ggtcagaact gggccaactc 1020

cgggcgtcta ctttgaagcg ggcacgccca ggccacgcct gcaaacggcg tcagcttcct 1080

ctgatcaacc gcggtcagcg ccatccaccg accaccacta agcctatcca ccggggcaga 1140

aaagatgact ggttcctatt cgttaa 1166

<210> 33
<211> 662
<212> DNA
<213> homo sapiens

<400> 33
ttaaccagtt tctgaaacat gttttggtca atgcccctta gcgcattgtg gtccaaaaac 60

aactgctcca ggagcaccat cttatccagc agcgcacctg gaagatgcgt gattttgttg 120

cgcgacagcc tcagggtttt cagttttatc aggtcactga aggtgccggg ggcaacggcg 180

gaaatgtggc tgtcggagat catgaggcgc tgcaggacgg tcatgccgct gaagctctgg 240

ctctgcagga cgccgcggcc cattccgaag agcaggatgt gcgtgaggtt ggtgggcagg 300

cctagcgcgg agatgcgcgc cacgtcgccc cccgagcact gcgcggcgtc ccggaagaca 360

cacttgcaag ctggcggaca ggggaagggc tgggcgcgca gaagcccgag caccgcgcac 420

agtagagtcc ccctcagcat gtctgaaaaa gcaaccgtgg gagtgtggtc aacacacagg 480

agcgttcgcg cctgtactga accctgggat cctgcacttt gtgcagaggc acaatccttt 540

cgccagaatt ctcactccct cttttcttag gactacagat ttccctacgt ggtgaaaaga 600

cactcattct acactgcgtg gcctcatgct tttgctttat tgattttcca ctctcagttt 660

aa 662

\

<210> 34
<211> 303
<212> DNA
<213> homo sapiens

<400> 34
ttaacgcctc cacgccgtgc caggcgtcgg atggcgggct tggtgatgcc ctgaatgttg 60

tcgcgcagaa ctttgcggtg gcgcttagcg cctcctttgc ccagaccctt cccgcctttg 120

ccgcgcccag acatgtcttg tactaaacaa aatgcaacac ggcaagcctc gcagcagtac 180

tttatatagt aataaatcgg acctgattgg gaactctcca caccagctgc atccggtcgg 240

ccgattcaca gaccttgcgc cctgcccacg acttcaggaa aaaatggaaa aggggggtact 300

taa 303

<210> 35
<211> 1293
<212> DNA
<213> homo sapiens

<400> 35
ttaaaaggct tttgtttata acagatcagg gagcgggcca cttagccaaa tgtctctcag 60

aaaaaaataa gacgcatggg ggactgggag aatcgttctg ggagcccgcg gccgtgcctg 120

gagtgcgcgg ctaccagtgg cgaccgagtg gcgccggcag gactgtgagt tggtgtgccc 180

aggccgggct gggctgatgc ggacacggga ggcagcaggc actcggggga aacgtttata 240

cttcaaggcg ttgtaaaacg gggaatgtat ttgaagcaaa cgggctttgc gtttgacaga 300

aaactggcca cccttccaag ctgcacgccg gtcttgagca gagttgaatt gtggctctca 360

gaagcagtgc attcgttgcg tgccctcctt cctccagcaa agcccctgac cttggggagg 420

gcctgtgtcc ccgttcggag ttcacaagag aggagacagg gctccccgac attggcctct 480

ctaagggagg atgactgccc cctttccctc cccgaagttc attgtgaaga gcctgcaacc 540

cccagaatta gcgctggcct tgttggccct ccaagccctg gaccaaaggc cctggggcct 600

gccgcttgcc cctctcccgg agctggcctc agctttggtg gggaggccgg ggctccagtg 660

agcccccggg cgaggctcca acgagtggcg tgggtgttcc ccgcaggcaa ccttggctcc 720

actagtctga ccgccggtcc accttgtcta tagtgacgct acctttcccg aggctgaacg 780

gggtcctcgg tctatgtgaa gtgagagctg ccctccctaa atgtcgttgt cctctcaagg 840

cggccccagc ggctgcacat ctggctgtgc gggggcccct tggacttggc tggcagccgc 900

ctgcgaaaca ccgcttctca gcggtctgca gttcctttcc tccctgcgcc caagatgggc 960

ctgacaaggg cccgggatca gaccagaacc tctgggggct gaagacctct ccccaccccc 1020

atcttacaaa gtgtgtgggg cttgcggaga gtggccttgg atcctaaggc gccaaggtct 1080

cagactctga agtggaaggg aaatgcgcga agtaggagtc ttttgtgtct gtgcagaagg 1140

aggttctccg gccgccggcc cttgttgcac gtctggatag acccccttatt gcattggtgg 1200

tagactcccc atttgccccc tagatttcct ttcaccttgg cttggaggtg cacagtgcaa 1260

cccggaggag cgcccgggga agacagcttt taa 1293

<210> 36
<211> 785
<212> DNA
<213> homo sapiens

<400> 36
ttaaatcagc tttatgatat agcgaaaact tagagttagc tttgcccatc atcccatcgt 60

cctaaagcaa cacctgtttc ccttcttccc ttttctggtg atggtttggg gtgtgcattt 120

ggaaggggga actatctggg ttcagccata aagagcattg agaaggaata ggggtaagac 180

ccctggccta cgggtagagg ccagtgactg agactctcat tgcgggaggg ggcgattagt 240

cgcaaagccg cccattccag gatttggaga tgcagctcca gcacaccctt ctttctctgt 300

ctgcccttag cctctccagg atctcctaac ggaggcccct tggtgggtcg ggagcctcta 360

ccgaccacta gtgggaaggg cacgaagatc ttgctataca gagtaggact gggaggggac 420

agcgcgtagt cgcagagtca gggaggggac ctcaccacct gtctcctccc tgaggtctta 480

gaacagatac aagaaattcc aggcgaaggt cccacaggtg agcgtgtgcc tgcgagagcc 540

gtggccgctg catagggagg gccccgcgga gtggggcggc agctgtggga gctgtaggtg 600

tccccattag cccgggagtc tccgcccccg gaggaacctg ggccttgcgg gtgagagtcc 660

cgagcgaacg cgtcagcccg ggtgtgctct ctacggtcca ggggggccag ggccttcttc 720

gggtgggccc gccgcccctc ccgcaggccg cacgccgctc ctccggggtc gcagggctca 780

gttaa 785

<210> 37
<211> 363
<212> DNA
<213> homo sapiens

<400> 37
ttaaattgag atttccagtc acgttgatgt ggaatgggat cgttgttctt cccgagggtt 60

tgataaggat gctcaacttc acctgtcaga aagcacggga gctgggcctg gcccgcgggg 120

agcgtaagct cgctcacacg cggtccaact caggcaaatg cagcatgccc gacctgctct 180

gccgccatcc ctccgaaagc gataatttcc ttgggctacc gaatccatca gagtagtcat 240

ttgtcccctc gccggcggtg cggggagcgt aacatttgag tagcctaact ctcacagcgc 300

gcactccagc attgttttgc tggaagattg ttactgtggt aagcggctgg tagcctgtgt 360

taa 363

<210> 38
<211> 454
<212> DNA
<213> homo sapiens

<400> 38
ttaaaaaaaa aaaaaaaaaa aaaagaccgg gggtgttgtt ttcttcgtgt gcacgtaaat 60

ttcctcccgg agccgcgttg aaagctggca gttggctttt acacttgccc agcagccgaa 120

ttagagcaac cgcctgtttc tttcgcccac gcagggagtc atatagccca gaaaaaactt 180

cccgggactt ttagcctgtc ccaaggaaaa aaaaattgga aacataccca catgcaaaca 240

gctggaagag ctaaaatcag aaaccactaa ccgccgcggg agcgcagtgc acgaaagggt 300

ttagttcagt ttatttcaag acacttcatt tagtagctct ttggcgtgtc gtatatttgc 360

tacccagcgc ctactccgtg ggtctgccca gggtcttgct gtgagagggc agtggcgttt 420

gtcagagatg tgactcacat gatgagagcg ttaa 454

<210> 39
<211> 624
<212> DNA
<213> homo sapiens

<400> 39
ttaacctaat ttcaaaggcg gggacttgat tccttttgtt catcaagagt ttgcccgcct 60

gcaagacccc agcgcctttg tagtccccac cctccttcac tgctctcaac ttctccccag 120

```
gctccttgga ttcgcacctc ccaggcgact gtttgttagt ttggggtgtg tgtgtgtgtg     180
tgtgtgccca tgtgtgcacg cgcgcgcgac aggggcggta cctatccctc caggtatgca     240
aggtccactc accttgacct gactctctgt catccccaac gaataggcca aacgagccct     300
ctcgggcccc gccaagtatt ttgtttgttc gaaagtcttc tccagggcga agatctgctg     360
tccggaaaaa gtgggtctcg tgtgttttct cttcccgtct ttgtccaaca aaatggatcc     420
ttgatctgtg agaaccaata aacaacgaga gaggggaaa aacaatcggt tacaagcggc      480
tgcactaggg ggaaaaaacg aactcgaaaa acaatgtttt gtggggaaag aaagctcagt     540
ctgtggcgga caccagaagt gtagtggcgc ttccagacta acggcacgtc tccctttgct     600
tttttacatc cacctgtttt ttaa                                            624
```

```
<210>  40
<211>  878
<212>  DNA
<213>  homo sapiens

<400>  40
ttaaaggcag ttgccagatc ctcccagagg gcagcctccc ggctactgtt ccctcgggcg      60
ggcttgcatc cggcacagcc ctgagctggc gggaatcggg ggcggtgggt ttcggcccca     120
gacgcggcca acccggctct ccccgcaatc tccctgtgga gatggagctg gaggcccagc     180
caatgagact ccccctcatt agcatagcac agtgacggca cgggggtgcc ttggggacaa     240
ccaatggggg cgagctaatt agcggattgc ggggctccag acccttctct gggaggaggg     300
gggccagggt gcttggagtg ttccactatg ccacgagctc cctcgctttg attgccttca     360
gtccaggtgg gcgggatgaa aaaattgcac gtttctggaa agggaatccc cactctcagc     420
cccaggaaaa tcccattttt ttctcacatt ctgaaagaag ggaggtgttt ccctgaactc     480
ggggaaaaag aagggccacc ttaggccgct gaagagaagc atttagatga cagggaaagc     540
acatggaaac gcactgagga ttttacagtg gtgagttgct gttactgctg tcgctgctac     600
tagcagcgtt gtgggcttcc aggtggcctg gcctcagcct tcggtcccct cctattagtg     660
gttcacacgg aggaagaagt gagcccgaga gaacagggtc tgtttttcag taagaaccac     720
tgcttacatg aaccctgcta cacaagctat ggttctattt gcgcttaccg ttctttcctt     780
caaaaactga ttgcatcaga gtatatacag accgaatacc aaccacctga cagcctgggc     840
cggaggataa ccatatggct tctcctgccc ttacttaa                             878
```

```
<210>  41
<211>  424
<212>  DNA
```

<213> homo sapiens

<400> 41
ttaagggcag agagaaaggt gacaaaattt gaaataaagt gagccactga agacaggaat 60

gggcaaactc cagctagcct ggaactatcc cggtttcctc tgtgaaccag cctaggggct 120

tcccctcggt ccgcccctgc acggaaggac gtgcgtgaca ctgggctgcg tcattggcag 180

aactgcccgc gcccagaaaa cgagctggag tggagaacag aatgcaattg cagacaatct 240

tcctctggtt ccctggggag gcctgctctg ggcagagaga tcccagggga ctgtgcggga 300

aggagctaaa gcaatagccc aaaagcgcca gggaatgcac ttgaagtttc agacgttttt 360

cttttctttg aaactaagtt tccagcccac caggaaaaac tgcctcgcga cctagtgatt 420

ttaa 424

<210> 42
<211> 681
<212> DNA
<213> homo sapiens

<400> 42
ttaaattcga gaaacattta ttgagaacct gttctatgta cagcactgtg ctaggtccca 60

tagggagagt aagtcattta ggattgcact ctaaaatctg ggcatattgg tgcaaaggga 120

ggtgaacagc aggcgtaggt gagtgtcaga ggacctgacc caagctggga ccctcctgca 180

ggtaaaacgg tgggtagtga gaagctctag gaaggggcg cttccgaaca cgcgcgtcga 240

ggagggcgtt ccaggactct gagggagcag cccagctgga ccgaggccgc gtcgttcctg 300

ggcttactat tcccagaccc ggactcccga ttccggagtc acggcccagg acgcgaaaag 360

actctacact ggcaccacgc tcctccttag gcgggccgtc agtcccgggt gcgggctgcg 420

ctggaggctg aggtgggagc gacatggtgt ggaggggcaa gaaatgtcgg cactagacgc 480

gccaagaagg agattctacg agcaattccc ccctcgggcc attgtgttgc tgtttattag 540

cccctgggag ggcgtcagga caaaaggaac cctcctccct tcttagtact taggcccaag 600

gtcgggtgtg ggagccggcg cgctgctttc taggcaggca ctgaagctac ggcagccacg 660

caaataggta tcagccgtta a 681

<210> 43
<211> 636
<212> DNA
<213> homo sapiens

<400> 43
ttaacaagca aagggaaaga aaagtgcagg gaaaaaactg ggacggaaga ggtgagagta 60

tgcggtgtgg tggactgcag gagaccacaa acttcaacag actggtccct aagagaagtc 120

ggcagaaagg tagacaaagc agtctccgcc gcacacccaa gagggccgag gcaggtccag 180

gacagccgga ccttggggca gctcagtaat actcacgcat ggaagagtcc cagccggtgg 240

ggcggggtcc ctagaaagcg gcgacaactt tacagactgg accccaggta agggaactcc 300

ctcccagcgt tggaacgctg caaaccagct gcttggacgc aggcagcccc cgcctagaga 360

atcaagaaaa ggtgtccagg cttcgggctg ccagactcag acccagactc cgacgcagtt 420

ccagtgccag agctaaagct agagccagag cctgatactt cagcacccat ccctagcctc 480

cattaccgcg gagctgagca gacgtggcag cgcacggcga tgacgtcagc tacgcgaccg 540

cggtctgccc cagccgcgag tgcgtccccg gaatcagccg gcacgacaac ccctaccatt 600

tgcacctgcg cagtggggcg cgccttctcc tcttaa 636

<210> 44
<211> 459
<212> DNA
<213> homo sapiens

<400> 44
ttaaaaccag ctgaactttc cctgccaggt gcggcagggt gggggtgggg gaggatgaaa 60

aacgaagtcc cccggggcat ttacagtttc caatggatca ccttagtatg cgtgttgcaa 120

ttataccagg gatgcacccg agagcttttc tttgtgcggc cctttcatct ggatcacttt 180

gtgttttgaa caagtgaggc tcgcctttgt gcccccagaa tttcaagtac agagtcacac 240

acggaacaac aatggcacgc taatcctcgc gctctaaatg ggctcacttg tgggtctgac 300

aattcgccca gggaaacgag aagagacgaa gcgagggcgc gagctagtgt cgcctgagac 360

gaggaagagg acagctgcag aactaatttg gattacattc cttgcagaaa ttctgaaaag 420

ggcttttcgg catagctcaa ctcggagccg cacagttaa 459

<210> 45
<211> 690
<212> DNA
<213> homo sapiens

<400> 45
ttaagccact agaagctcac cgtgagtcca ttcacagggc cagagttcca agtgtgcggc 60

gtgggcgggg cctgcagaaa gggacggggc ctgcagaaag ggacggggct ggcaaaggcc 120

tggtctacct caccgatccg ggtcgcgggg gctcctaggg ggttccttcc acagctggtg 180

cgcgtgcgca agagtgcgtc gaggttgttc cgcggcaatt tatgaaactc ggagccggca 240

gtgagttgag ggttcggcga ggtcagggat tcctagttgc agcgtgatta ccaacgccag 300

ctcccttcac tgtccaagac agcgattccg cggctcctct gagaactggc gcttgtgagt 360

gactgaacgg gccactggag aggagcggtg ctcacgggta tcgttctcgt cagcgggcgg 420

tggggacgcg gggtcttgga attgggaatc acgcttgagg gcgtccatgc ggaacgtgca 480

gcttacctca gcttgcttgc agttacccag tttccacgct gccctccaag gtctgtgccg 540

gaaaacctgg aggagggcgc gagctgacgg gagggatgga aacgtgggct gcggaagtaa 600

gacaagcgtt gagagcagcg acagagggcg agggggtggg cggcgggacg gctggggtca 660

ggatccagct ccgctaaagc aaggatttaa 690

<210> 46
<211> 1015
<212> DNA
<213> homo sapiens

<400> 46
ttaatcacag taacattttt ctcagaaatg aaaaaatcca cctgagattc atgtgaaata 60

tcaagggaca ctctctccca tggtcatatt ttagacctcg tcattttcta gcaaatattc 120

ctccataata atttgcagca ctactctcca acaactttga cttgtatttc caccttggtc 180

ccaccagcat gctagcatca gtaatattag aacctcgcca atacctacag tccactgaga 240

cgatcacctt tttactcttt caactcctcg tgtactcaca gcctgccaag cacatttctt 300

gtcaggagag gacagctcac ttctcctaat gtctcacttc ccaaaattgt gtcccatgaa 360

aatttctagc tagatgaagg cagagaaaga aaatatttga tttcttaggc tttatggaag 420

cgataggaat ggcaatagtg gtttggaatg gagactgcac gcaccagtca acagggtgtg 480

tcccgaataa gcagtgggta aaggacattt actgaaatgc tatctccaac tccacttcag 540

gaaagcttgc tgcagagtta gagtgatata gtccaaatgc tctgtttgag tcgcgggccc 600

tttgtctcac acgggcgagt ccggggaatt ctgggaagtg gagtccacac gctctgtgga 660

ggctcgagca cttccggtcc ccaactgtga cgttgctatt tcatttacca cagtagtttg 720

agtcatttcc acatcttgcg agtccttccg aacgagtctc ctttccttgg ggctcgcaac 780

cacccaatga tcgattcagg agaaaagaag cctgggcgga gagcagaggt ttggaggggc 840

gcgggcgggg attgccgttc cagtcagctg agccttctgg cgtcgggggg ctctgctagg 900

gtctcaggga gtgggattgg cgcggcccgg tgtgccctac tttgacctcg cttagtccgc 960

ctccctcctc cctacgtgtg cagccccttt cagtgtggtg tggagtgtca cttaa 1015

<210> 47
<211> 638
<212> DNA
<213> homo sapiens

<400> 47
ttaaaacgtt tttcctccta aggccattca gtcgaaccaa ttttccgaag tgattcatag 60

agctgaattc tgccaaggca gctctttcac ttttagccac taaagcaccg cgtggaaccg 120

ggttttgtgt ttttctaaaa taaagagaag gattatcaga gaggagaggg agggggcctg 180

aagggggacg ggcagagact tagtgtggcc agaggtacaa gcagagggaa gaaaagaccc 240

ttttctagac aggccccaag taagttcgga ggaattcagc gttttgtgcc tggcgcacaa 300

aagcagcgcg ctccgcgctt ggtttggagg ggctgaattc atctgtttgc agaccattcc 360

acctgcgtat cacaacaaac tgctcaaaga cgctccagca ctttcaacag gccgggtgct 420

cttggcgaca acaaaagtgg tcgaggctga gggttcttac ggaggggggt ggtggtggct 480

agcaagagag cgagcgagag aattaggttt aggaaagggg gaggactggg tgcaagggtt 540

ttttgaaaag ttttggctgg ttttgttcgc gggacaatta tatcttgcta gccagctttt 600

gcaagaccac gagctttgta gccgcaatca cagtttaa 638

<210> 48
<211> 761
<212> DNA
<213> homo sapiens

<400> 48
ttaaccccac agataactca gaacaccgac ctcccaaaag accatctagc atcgattcca 60

ccaattccca aatcacctat cgtcacagac tactccaatc actgccccgc aagtacacca 120

tctctgaccg ccaaaacacc ctgtctcatc acagctctca ccgcggcgct ggcctcgggc 180

tcggacacag ccatctccag tggtcgcccg gcctctttgg tcgtttccgc acttctcctc 240

cggtccacgg tctctggctt tgtcagaatc tggacgccgc tggagaccga catggcggct 300

acccggaggg cggccttagc gggtccggca gccgcgatgg cggcgccctt cggtaatagc 360

gcacgggcgc ggccatcttg gcaaacactc agccgtacag cggaagtgcg gggacttctg 420

ggacaattag atcgggaggt ggaagctggc gagggcgtta cttgcaggat gcaggagtga 480

tgcgatcaga gccagccgga accgagttcc gttacgcact acaggactga cctgggcctg 540

acaacccact gccggagttc ggatcgcatc actgccggaa gatattttac agaatttgga 600

attgcttttc cgaatacatg tcagtgcggc cagcggctag aagtccggga atcacaacgc 660

ttagtgcgga ctttagaaag gcaggatgcg agaaattgga agcataacgg agaaaagcct 720

gatcgaacaa ataacattat tttataggga gaggacgtta a 761

<210> 49
<211> 796
<212> DNA

<213> homo sapiens

<400> 49
ttaagaagcc agaatggcgg gctgagggga gaaatgcccg catgttacaa ctctgcaggt 60

gtacacacgt ggacaccata tgtacccatg aattccgcct ttggaagtga acttgattgc 120

agcctatttt cgtctggagg cggtggattc ccggtaggag gcgccttcag tcctcggctc 180

gatcctgtga cgccagccag ctgcctatcg gcttctcagt gtttgaactt caaagggctg 240

gacgctcacc caagaagggg accccggcct gacctctctc ggaattcaaa aaaatctaag 300

gctcagagag ggaatgggag ctggctcttc cctctgtgtt tagcatccac gtttttggc 360

ggtctggctg aaaccagccc accctagttc gggcgccaga gcaacgcagt tccgagggca 420

gatctccaag gggcggaggc agagccgcgg gtgagaggct tccgcgccga cttcgggttt 480

gcactccccg gagccagaac tccgtggcgg ctccgggcct ccgtggatcc gagcagagac 540

tggctgcccg cctgccactc gcaggctggg aaaacgccag ccggtgcctc gccgggaaag 600

ttctcggcaa agctggggat gagccctcag cagggcaggc tcccggtgca gcgcgccccc 660

atccattgcc cgcgcgcaag aacccggaat gggccccacc tgggccggga ctcgggcgtg 720

cgatcccgag ggcgccctgg ggaccacgca tcgggtgggg accctggccg agggcgtgag 780

ctgactcagt ttttaa 796

<210> 50
<211> 469
<212> DNA
<213> homo sapiens

<400> 50
ttaaacagac gcacacgaca gatattctca cagaaaagca gtcagccaag cccagaaccc 60

atagatctag gtccacactc acgcctctgc atgctcccca gaaagacgga tgcatcgccc 120

gcaggtagag acgggctcac caatcccact caaaacccgc tgccggctta cagctcgcaa 180

gccgcccaca gtgccagccc catgccaagc atcactaggg gctctagaat aggcaggggc 240

cttgttctca cttcctttgt gttgctttta ttttagttgt tttgtttgtt gtttttctta 300

gagttatcac ctgcccctct gtctccctac ccagccccca agcctcccct ccaagcctcc 360

actgcgcatg tgccgtgcat ccctgcccct tctgggtacc cgtaggcgct cccgggcgct 420

ggcggcggcg gaggagaggg agcagcgtca cgggcgcccg gcccgttaa 469

<210> 51
<211> 382
<212> DNA
<213> homo sapiens

<400> 51
ttaaacacta ccacccatgg aatgcaaatt gactttgtcc ctccacaaac tggtcatttc 60

tcatcacgaa gccatgacca gcatgacctt tacatgtgga tcaacagctc tcggtccttt 120

gacagccagt tcctttgact tggccacttg atagtgacca ttcaacctaa ggaggtgtga 180

cccagcggtg gtacgaggtg gctggacctc agttctaacg gtgacaactt cagctaaagc 240

acgtttgttc tggctgtccg tcctctgctt gtgtaacttt tatttcctca gtgtgaacag 300

cgctgcactt gagggacagg aagggggtgc ttccatagct gtcattgcta tggcgcaagt 360

ttacttttac tcttcaattt aa 382

<210> 52
<211> 423
<212> DNA
<213> homo sapiens

<400> 52
ttaagattcc agtgcgtttt gtaaaaacca atcataatgc taataatgac tgaaccatcg 60

aaaagcaaag ttatagacag cgcctgcaga cgctttcggg tccggagccg gggagggaac 120

ttgtgtattt tacaactgcg atgcctatcg ggaatgtggc aatcgccgag gtggggcctg 180

acagtaatct tgacaaatgt gtgaagtgtc agctcctcct gcgctcctta gcgccgggtt 240

ttgtgcagtt ttgtaccgta tataccgaga aacttgggag caggggaaaa atgatttcga 300

cacacccagc tacagtacct ggttcgttag cggactgttg gaaagagaga agaagcggga 360

atgggagcgg gaagaacgtt gcgtaattag actcccaatt attggcgaga gcggccgctt 420

taa 423

<210> 53
<211> 911
<212> DNA
<213> homo sapiens

<400> 53
ttaaaggcgt tcaggtcttt cttctaaatc gtcccattca agagcaagtg tccaagagaa 60

agagtgacag cagccaagag cttggaatag gccaagctcc cagtagttgt ggtggctcat 120

tgcctgagga catagtgtgg tgggggagg aaccttgaga aacccatggc ctgtcccgtc 180

acaaaagagt ttgttgacgc cctgctcccc tccagctact cccaacacac caggcagggg 240

ctggctcttg ggcatcatca tttccaacat ctgcctgggc tgggagaccc gtagcaaatg 300

ttgatgatca aaggaacaaa ccaacactgc ggttatggtt tccctcggga gctgtttttc 360

ctccgggccc acgcctcttg tacagacaat aaaaaaggaa cacggtttct cagaggttca 420

gaggttccac caaagcctct ttccttttac aaacctggcc cctgtgccct aagcctttgt 480

tctgagacta ggagacctac agatggagaa gggggagctc agctcaggac tgagggtgac 540

ctgttcccag gggatctagc atgggggaca gtgggacaag agcaaagatt cccctgccag 600

tgatgggtcc caggtggtct ggagaaatct aggagtggct gctgaaggcg gggcctccca 660

ggggtccgca aagcaagtta ccagggacag tagggagcac ggcccgcccc acactggcgc 720

agatgctccg ggcgcccctg ggtggcgctg caggctcagc tgtcgtggat cctcggtgtt 780

agcatctccc tggcgagcgc ctcggagcca gcgcacagcg atctctacca gggtgtggac 840

ggagggaatg ccattcggag cctgcgctgg ctcagggcgc ccgcgtggca ctaggagcca 900

acaggattta a 911

<210> 54
<211> 723
<212> DNA
<213> homo sapiens

<400> 54
ttaagaccat ttccaacact gtttatacaa aggccctaaa aatgggtcct tctaagacac 60

ttccaggggt caagcaagaa gagactatga aagtcttgat cacagctaac attcataggc 120

aaaaatgtcc tcagctttct cttttttcct ttcttccaag ttggctactg tctataaact 180

ccagaaatta gtagtcaaag catcccctaa cccttgagcc tgtgggaata caggaatttc 240

aacccgagca cgtcagtccc caaggcctcc tttactcttg gagaaatccc tcacaacaaa 300

ggacaaggga gaagcaaggg ggaggggcgc ccagtggact tggctggaag aagtgagcgg 360

gctggggttg gaagagtaac tcgggctgcg ggctgaacgc agtcggcaac cgcggaagag 420

cagcatctcc cctgcgcctg tggatacgcc agtccaggga tggcgagtgc tttctcctcc 480

ccagcttctc cctcgctctt cgaggtgact cgtgggaccc tgcgtcctag tgctgggtgt 540

gaatcggcta tttcacaccc agttcttcct cccctccgcc acacgcagtc acattcctgg 600

agctattcca agctgcctcc gctaagcacc gaataagcgg accctgcctg gaaacttgag 660

cgaagctgaa ctgcgccgaa ctccaccgtc cagtgacccg agccagtgtg gacgcccttt 720

taa 723

<210> 55
<211> 782
<212> DNA
<213> homo sapiens

<400> 55
ttaatcctgt gacttcgcat cctgcgcatc gaagaccttt atttgctttg cacgtctctt 60

ttcttccccg ctaagcaacc acgtgccttg aaatgggaaa gggatacaga tgttcggctg 120

ggcttccccg ggtgggtccc tgaaatgcgt cctggttagc acatggggtg ggagcacctt 180
gcccgagcct tacctctcta ccctctcttc gccggtccgc aggaggctct gacgccagcc 240
aggtacccaa tggcaagagg atgaggacgg cgttcactag cacgcaactc ctggagctgg 300
agagagaatt ctcttccaac atgtacctgt ctcgactccg gaggattgaa atcgccactt 360
acctgaacct gtcggagaag caggtgaaaa tctggtttca gaaccgccga gtgaagcaca 420
agaaggaggg gaagggcacg cagaggaaca gtcacgcggg ctgcaagtgc gtcgggagcc 480
aggtgcacta cgcgcgctcc gaggatgagg actccctgtc gccggcctca gccaacgatg 540
acaaggagat ttccccctta tgagggaggg cctcctccct cacatccccc gctcctggca 600
gaccaggcaa cgccaaggcg tggggcaccc aggggccaga atccttgctc attgcagtgg 660
tcccatctgg agaagaaacg aacctggaag ttctggaatg gacaagccgg gacctgaccc 720
ttcgcgtctc cttcttgacc tgtttatcta ggactccaac ttgaagttac agattttttt 780
aa 782

<210> 56
<211> 751
<212> DNA
<213> homo sapiens

<400> 56
ttaaacaaac ttcaggagga attactgtgg ctttttcagg gaggctagag ctttctctgg 60
gatgagggca aaaaatgaaa tgagtggaaa aatcatctaa cagtgcaaac tccacactac 120
cctgtaaaac acagaagagc caccgtagga gagatttgca ggcaaagtaa aaccactaac 180
tgcacgatcc agtcttcttt taggtgcctc gtctgtattt acttatttca ctggagtgac 240
cacccggtaa catagtagaa acattatttc cattttacag atgaggaaac tgagggacag 300
tagattcagg acgatgcctc gagtctcacc cagctggtgg gcggtagggc tgggatctga 360
acaggctggc tggctccggc gaacctcggc tcttccctac tctgctctta tgttgcgtgg 420
aaactaggcc aggatcaggg aactcctgat cttcaccctg aggagaagca aagagaagac 480
agccctgagg cgcccccactc tggggcagcg tgtgtgaccc aggccttcgg accgagaagg 540
gagaaaggaa atacgtcctt ccctatcctg aagcggaggg cgttccaggt ttcgcaggta 600
gtgctgcagc cgcgcaagga ccgggcgagg ctgcgcccct gagcccaagt ctccactcac 660
cggccttccc ttgccccctc acctgcacgc gggtactttt agccctgggc aagccttgag 720
ccaagcgccg agaccaaacc cgggaaatta a 751

<210> 57

<211> 761
<212> DNA
<213> homo sapiens

<400> 57
ttaatttcat cgtcccataa atgaggaaat atcagttccc atactaaatt tttatccctg 60

gctgataaat atgacgggaa attttcgttg ggtcctcgta aaagggaacg gtttcgatca 120

aactggtggg gaaagactaa tgcagataat acaaacagag tggccataaa gtctcgcccg 180

tttcccgcgg gatgggcggt gggacaaatg gctccctgga ttgactgctg cccggactgt 240

cccaagtctg ggcctaaac agcctaacct tgccctggat aacccccatg ctgaggccac 300

ggtgaccctg catatgaaaa cacaaaacca tttcaaatct cccctggccg gtcctgcaag 360

caaatcatac actccacatt tccttccagt tttccggagg ttcaggaacc cctgcgtctt 420

ctctctccac ccctggatgg ggaaattcgg cggatttctt tctgcggtct tctagccgca 480

gcgggcttgg gcctccttag aaacggtcta gcctcctcac tcctccactc cagggcgctg 540

ggctccagtc ctttccccgc caaactccag cccttcggaa agtctgaacc ctaggctctg 600

tccaaggaaa aatgtggggc gacaggaaag acaagttgtt ctccagattc cgtttcctga 660

ggaaggggtg gagagtttag ttcgctttgg aagaaggcga tggagaaacg gaaataactg 720

tttgaaacat acggttcccc ttatttcttc cttttattta a 761

<210> 58
<211> 502
<212> DNA
<213> homo sapiens

<400> 58
ttaaattagt tttcattact tttttttttt tcttcagctt cagctctccc tcagcgaggg 60

aggaggctgt gggctgcgga ctgagtgctg gaatgaggag taattgagct tcagctgagc 120

cggacgtagc tttctcctcc tggtgtcatt gctgcagcct ccagtgccgg gtccctagtt 180

cctcagctgc ctatcttccc ggtgcaacat cgcctgtaaa gacagcaaag ccaccgcaga 240

agttgcccgg cagaagactc cggaggcatt ggctcagtaa cttttcacgt cattttctgc 300

tcgggagccc cttctagcct ctccgcgcag cctttcccac cgcaaatcac cagtgctcat 360

ggggcaggcg gagaggagct tgcagcattg agcggaaccg gacttgagcc cgtgatgtcc 420

ggcaccaaat tggaggactc cccccttgt cgcaactggt catctgcttc ggagctgaat 480

gaaactcaag agccttttt aa 502

<210> 59
<211> 716
<212> DNA

<213> homo sapiens

<400> 59
ttaaattcca acatacacgg gtggtcctgc cctggataaa actcaggaag caaaatcaac 60

cttcgagaga ggtgaggtag gaagtctgag cattgccgac gggccggagc aaatcgctcc 120

cgggctagac cctgcctaca ccgcggcggg gacaggtgga ggtttcaacc cctgtttggc 180

aacctcgggc gcagccaggc cccgcccaga aatttccggg acacgccccg gaagtactga 240

gagggacttc cgccccgagg cgaggcctcg gagctggttg ctgctcacag gcagagaaac 300

gctggtatgc atttgggtgt cgcttgggtg gatgttgcac agctgcaacc cctcagacct 360

cgtctctccg cgcctgcctg ggtggaatgt gtgcaggaac tccaggagag gcaacccatc 420

ctgggacggt ggggattgta tccaaatgca gtcttccagg aaagggtttt tcctcgagat 480

ttagtgccgc aggtgcccag agttgggact acttcccttt cagttacaac cctggttttt 540

actgccgtct tcagagcttc attgagtgcc aactacaagt gtgcttctgg taccaattgg 600

tatcactgcc caccagatct tcagctctct ttttcaaaca aaagggggaa aatgcaaata 660

attggaaata aatgttcggt gctctaaatt caggagaggc ttcttccacc acttaa 716

<210> 60
<211> 658
<212> DNA
<213> homo sapiens

<400> 60
ttaatttact aaactgtttg agcagccgta gattcccttc acaatccggt aaaatctata 60

attactcctc cccttcccca aaatgtactt attttcccat tcaatatcgg agcttctcgg 120

acttcctgaa gttagtccca ggataaagat cctctttctc ctccccccttg ccccgcccca 180

cgacctcgct tcccagtaat tgctgggatc ccgattctct ggaggcattc cgggcggatt 240

gattgagtgt aattcaactg agttatgatt acagctgctg gttcccctgt gccgcagacg 300

ggtgtgtagg gagggtgggg cacgtggaga tgggcaagat gtctggagag agcagtgggg 360

ctgttgtgca cgtttttgct agaggtgagg gttgagagct agggaggaaa caattccgag 420

cccaccccgc tgggacccac gggctgaggt cctccaggaa gcactggggc tcagctttcc 480

ctatgtcagt acggatggag gacacacagg aggaaggctg gtggctctgc cggaacattc 540

tgagggcctt gttctctgcc tacgctggga cctgcagtcc catctcccat ttggcctcag 600

ccagcgtggt ttattgtacc caaatcccgc ttgtttcctc cttatgcagc agttttaa 658

<210> 61
<211> 324
<212> DNA

<213> homo sapiens

<400> 61
ttaattcggg tgccgaccaa gtcctccggg gtccgcaaag gctggcatcc caccaccaac 60

gcgcgtgact agggtcttct gttttgcgcg tcttccagtt ccactgaggg ccgagacttt 120

gtctttgcgg ccccagtact tgcttagttc cgagagtgcg gtttgcactc agtaagtagc 180

cacttactga gtccaatcga ttattggaaa cctaattttt catcactgct tctcccacaa 240

gaagctctag gactgactcc tcaaagacca aaactggaat tagcaatccc gctgtttacc 300

cggaggcccg gtcaaatgtc ttaa 324

<210> 62
<211> 1265
<212> DNA
<213> homo sapiens

<400> 62
ttaatgggcc tttgcgtcct ccttccctga gcctcctttt attccagact tctcagtgtg 60

agtctgtgcg tccctccgac gatctcaggg agtggggtgc cttcatctgc ctgttccctg 120

ttcctcaggc tgacgctccc gctgtcctcc ccgcctcccc tcactccttt tctccctccc 180

ttcctccttg tggggaggct cttggccagg gtccctgagc ccgggcgggt gctggcagag 240

gacgcagaag ggtgaggtc acgtctccct tgagccccga gccgctggct tttcagagcc 300

tcgccacaag ccggcggcca gagccccaga ccacacagac cgtgcgctcc tccgccctcc 360

cggcgccgcc ggcctcgccc atgtctcagt acgcccctag cccggacttc aagagggctt 420

tggacagcag tcccgaggcc aacactgaag atgacaagac cgaggaggac gtgcccatgc 480

ccaagaacta cctgtggctc accatcgtct cgtgtttttg ccctgcgtac cccatcaaca 540

tcgtggcttt ggtcttttcc atcatggtga gtgaatcacg gccagaggca gcctgggagg 600

agagacccgg gcggctttga gcccctgcag gggagtccgc gcgctctctg cggctccctt 660

cctcacggcc cggcccgcgc taggtgttct ttgtcctcgc acctcctcct cacctttctc 720

gggctctcag agctctcccc gcaatcatca gcacctcctc tgcactcctc gtggtactca 780

gagccctgat caagcttccc ccaggctagc tttcctcttc tttccagctc ccagggtgcg 840

tttcctctcc aacccgggga agttcttccg tggactttgc tgactcctct gaccttccta 900

ggcacttgcc cggggcttct caaccctctt ttctagagcc ccagtgcgcg ccaccctagc 960

gagcgcagta agctcatacc ccgagcatgc aggctctacg ttcctttccc tgccgctccg 1020

ggggctcctg ctctccagcg cccaggactg tctctatctc agcctgtgct cccttctctc 1080

tttgctgcgc ccaagggcac cgcttccgcc actctccggg gggtccccag gcgattcctg 1140

atgccccctc cttgatcccg tttccgcgct ttggcacggc acgctctgtc caggcaacag 1200

tttcctctcg cttcttccta cacccaactt cctctccttg cctccctccg gcgcccctt 1260

tttaa 1265

<210> 63
<211> 325
<212> DNA
<213> homo sapiens

<400> 63
ttaaaaggca ggcaattctg gcacaggcta cagcaggaat gagccttgag gccatgatgc 60

taagtgaagt cagtccatca caaaaggaca aatgggactg tggcattccg cttctatggg 120

gtgcggagag tggttagatt cctccagaca gaaagagcac gcttggctgc cagggccggg 180

gagagcgggg agaggggagc gggagttatc atttcacgga cgcgatgttt cgtccccttt 240

gatgaagcgt tctggagatg ggcagtggcg acactcgccc atatacgcaa cggctgagtg 300

cactccatgc caccgaactg gttaa 325

<210> 64
<211> 1038
<212> DNA
<213> homo sapiens

<400> 64
ttaaaatcat acttttgaac ctaagcaacg cctgcgcagg tctcaggagt ccgcgtcaaa 60

tccctgtatt ttcgagaact cgatttttcc gcgaatcaat gtctgctttt tggataccgt 120

agatcagggt gtaaagattg agtgtgatga caatttctcg gaagataggg acgtctgagc 180

tctggaagta ccatctctct tctctatccc taccccctcc tccagccgcc tcctttcctg 240

gcgtggctct tttcccggga gctttccagt tagaggctgc acacctgtcc gggcgtgtcc 300

cgggatttgg gtagcaggtg ggggcggctg aggcgacatg gaggggatgg cgggttcagg 360

gcctgggaac cccgggggtg aacttcctca ggtgacgtct gcggccggag gcccgcaccg 420

agtgcacgtg tttcacacac gcgcgcgcgc acacacgcac cagagcgcgg cgaggagact 480

gtccccctcc attgcgtttc acagcggctc cgggaccagt gtggcctgtg agccttttgt 540

ccatccgggc gtgggagcct ggcctgggaa cagggcaggc cgttgcgtat gcctcggacc 600

gggagcttcg gtccctttgg cggggtcttt ggcctctagg ggcctatctg ggtttccttc 660

ttggtttggt aagagcagca gtagcgccag gtacgccttg gagactgtag gcggaatcgc 720

tccagctcct gctctctccc gcgccccagc cccgggcgga gggaacaggt gggaaccgcg 780

ggctggaagg agggagacgt cccctacctc cactccggaa gcctcggggt aacgtctccc 840

attgctctag cggagttcag atctccaatt ccagtttcaa ctggaaccct caccgggtct 900

gtctgctcag ttaggagggg gtgcgtttgc attgattttt tgtggcatct ggaaatttgc 960

ggtgtttcag gaataaattt tagcatgttt ttcttttctg ttagccactg ccctccgttt 1020

ctgcattttg ttttttaa 1038

<210> 65
<211> 738
<212> DNA
<213> homo sapiens

<400> 65
ttaaaaatgc gtaacagtaa tattttcaca atgttttcca acttataaag tgcttttcca 60

accattttct catttgatct ttacacacca gactgtacat agagagcaca ccaatgaatg 120

tccctatttt cagatgagga aacaggctaa atggggtaga gatgcttagg caattctgca 180

caccatgcca gaagtagaac gaaattcaaa tccagtgctt tgcagggtca ccaccaccct 240

gccttatgaa aatggattcc taggaggagc gttttgcaaa aattccactg cttcctccaa 300

cctctgccac tgaaagggac tctttggctt aggtaacaaa caccctttcg aaacatgacc 360

tactgttcct tccaccggct taccaggaag ggtcacggcc aaaataaaca cctctgggag 420

gccggcacat ggcataaatt gaagggtaaa cctcagcaat ggggaacttc ggagggccct 480

caggcatcca ctctacccgg gggccgccag ccgcacgcag cctcggattc gccctgcaca 540

tctgccgccc agcaggtacc atgcgccagc cactccgccg ggcacaccgc agaccctgag 600

ctaaggcagg gtcactgaca cgcgggagtc ctcttttccc agtcgcggat gcatcaaatt 660

cctttcattc acatcacaca cacaggatat caactgaaag ctacacccca cagtcccagt 720

ttcaggaggt gggattaa 738

<210> 66
<211> 315
<212> DNA
<213> homo sapiens

<400> 66
ttaaaggtga ggtgtcgaga cgcgcccctg gacagacaac gcccgaaggt ccaggccact 60

acgaagtcca cggaggggca gctttcccct acgtgtaggc ccgcgaacaa agaactaccg 120

ttttctgatt ttttttggcc gatgttcgtc atgcagaacc ttgaatcccg atgaagattt 180

ggaggccttt tgtatcctca acatcagagg tcatgaagtc agttaccttc tgcctttcaa 240

gctacaactt tttccacttc tctcatcact ccaagcacca gctttctcat cctgcttatt 300

tcacagccat tttaa 315

<210> 67
<211> 547
<212> DNA
<213> homo sapiens

<400> 67
ttaaggtgaa gtgaggtcac tggatggccc tcctcctatc tgactggtgt cctgatgggg 60

acgcggacac ccacacagga acggacccgg ggagaacaac acatcgtctc tgcgccaggg 120

agagaggcgt ccggggaacc cagccgtgcc cacgcattta tctccgcctt ccggctcaga 180

attgtgagga aacccgcttc tgttgtgcaa gtcccgggtc tgtggtgctc ggccatggcg 240

cccccagaac tgcatcccca ccttctgctc tgagccaccc atcccctctg gaagggctga 300

gcttctctcc ctttccacct tctgttctga gccacccgtc cctccagaa gggctgagct 360

tctctctctt tcactgccta tgctctggga aactttcaga atccccaggc tgtttctgga 420

atagtctggg atggtcattt tcggtcctca cagaaccggg ggtttggaaa gtaggtgaag 480

ccatatgggt tgcagagaat ctggcccagc ccctcactag tccctgtgct tgttagttat 540

tctttaa 547

<210> 68
<211> 827
<212> DNA
<213> homo sapiens

<400> 68
ttaaggctgg ggatggggtg aggtggggtg gagttgatcg atttgggccc agggtggctg 60

atgaccttat tggacccgat tctgtgatcc ccatcccgag agctgcacgg aggcggagac 120

tgtggctgcc gtgcaggtgt cccggcccag ccatctgtat ggggtggggg tggggtgaaa 180

gtcttggact aatggtgggg tgcttgcctg tgatctctct cacgttttcc tttgaggcct 240

ccactcggaa taagccgccg gcgagggagc ggcccattcg tgggaaacgc gcccaccgct 300

tgggttcttt cctaaggcct ccccaccaac aaggctgaaa ttccaaggcc ctggaagatg 360

aggcaaagct agcattgcca gagtcggggg agaggggagg atgctgggta aagtgcccac 420

ccggggtggt ctaaagtcgt caccccacag agcagattct gctgctaacc agagcagaga 480

gccgcacctt cccagcggcg ggccccagtg cgcacagcgt gactttgcct cccccaggct 540

cagcaagtcg ttgacgatct ttagcagtcc cgcatcccag gtcaataaac tggaaacgga 600

tagaatgaga atcatggatg agagataaag ggcagcgggg tgatgcgcat ttcttgtagt 660

gcctccaaaa aaccgtggtc tctcgccgct aataaaacac ccttgtggtg aggcctttgg 720

tctggtctca actgcagaga tgttttgtct taggtaattc gagcccccaa atccagcacc 780

ttgggattat attatacaca cacttatatt ctttctgtat tttttaa 827

<210> 69
<211> 748
<212> DNA
<213> homo sapiens

<400> 69
ttaagatgct caacactcaa atcggggtat tgatctccac ggaagcccca aaccctcgcc 60

atcgagagac ccccatggcc cggggtgatg gctgtggggc ttggtgctcc cagagagctc 120

agtggctaca gaatgggtgg ggattctgcg tgtctcccgg agcctgaacc cctttcctgg 180

ttatggccgg tagctgtctc cagggctaac gtgggcagcg caggggggcg gaaaccgggt 240

tttagccaaa tgcctcgaca tcgccgcgcc tccgcctcct cgtcgctgaa agaaatgtcg 300

gggtttcatc agagctaggg agcgacagtc gggaacagcg agtctgccga agccggctgt 360

tgtgtgaggg tgtgagacgg cggggcggtg aggggccacc gcggcttggg ggatagtgcg 420

tgtggggttg accgtgtgtc tgcttgagag gctgtgaaga tatgggggc agatatggga 480

gaaatgctcg ggcctgaagt ccccagccca ccgtgctcaa gagtagcgga cgttttgcca 540

ccatccttgt ctgtgctact gtctgctgca gcttccgtgc cccgttctcc tggagcaggc 600

aagacctgga gtgaggtgct tgggtgcgct cgagagagct tccccctgct ccacctgtcc 660

cgcggtgcgc gcaggccaac gcgtcgggca gtgggcttca agcgctggtt tagccacaaa 720

agaccagaag taaagagttc cggcttaa 748

<210> 70
<211> 679
<212> DNA
<213> homo sapiens

<400> 70
ttaaaggcat catcgcagcc attagaggga aaagttgcgg cgccgcgagc ccggcgaagt 60

cctcagcccg ccccgcttgc ccactcccca cttcccgagc cggctccgtg tttagggagg 120

gcagtgatca cgcaagccgg agcggcgggc tgacgttgga cgagctgcca ggtagctgaa 180

agcaggcagc caggcagccg agacacttcc cagcgattcc agcctgggct ccgcaggaag 240

cctcgctgaa tcccagccag ctggttctaa ccttccagaa tcgcaatccc ttctccccac 300

agccagccct cgccaagcaa gcagcaggat gtttgcagtg tcgcgcccag ggctctgaga 360

ctgagcctgc catccactcg cacgcctttc tttcagggct tttcggctgt tggctacact 420

gatgtgaccc ccctcccttt ttggaatgat ggggatcttt ttggtgtatg ttggatttgt 480

tttcttttcc gttttatatg tacaacaagg gctttcttct caaggtaagt cttgctcaat 540

ggcaaatata catttgcttg cgtgtgtgtg tgtttcctac actcaaaaag aatggacagg 600

cagatagaat aagcttccta gcaagtaaca gtgggcttag gctgtataaa tagtgttctg 660

tttgacgatt ttttttaaa 679

<210> 71
<211> 659
<212> DNA
<213> homo sapiens

<400> 71
ttaattaggt cgactccctt ccttcgccgc tccctcctgt ccccactcag cctccctaca 60

cctatgcggc gagcctcggg ccaagacctc cgccctggct ccgccagtcc tgcgggggcc 120

ggcatgcacc agacctcaaa agggatcttc acgggaattc cgcatagcca gatcccaagc 180

ctccagggag gccaccagcc ctcccccatc ttcttcttcc tcctccgtct cctttcctct 240

tccctccgcc tctacgtatt tctttctatt cgtttctttt cgtttggtga ggtgccaccc 300

gccacagccc tgtcccctag ggagaggcct ggactaactt tgccgtcctc gtggccttag 360

cccggactgt gagtctcgag cggcttccga gaggcagctc gggtgtcttc gcagagacag 420

atgaggtggg ctggcgattc cctagcctca gatggctctg ccaccagcca gctctgtgat 480

cctgggtaag tctttcaacc tctctgagtc ttgacgttgg taaaaggcag gagatttaga 540

gaaggtgttg ggagactaaa tcttttcatt cgtttgagaa agggagtcgc ctgtgttggg 600

acctaaggag gaagtgaggt tcttgagaat tctagatctg aaatggccgt ttgttttaa 659

<210> 72
<211> 367
<212> DNA
<213> homo sapiens

<400> 72
ttaaaaaacc aaaagtctct ctcttcctct ccctctccca gcaacagcat tagggagata 60

ctccccaccg caaaatacac ccactcaccc actggcgaat gttgcactat cattccagga 120

gtggggggag gttgctgcga cagctttagg ctcctctccg cgttattggg tcgtcgtagg 180

tgcgctgtgg gctgctgcgg gctctgagac ccggccacag gactcgcggc tcacgacctg 240

agtgtaccta gataggttct cagaggctac tgggaaaatc gcaaatacag gacgcctgta 300

ctgcaaagct cccctaaaac ctcatcagtc ttcctctctt gactcttggg cgtcccagga 360

attttaa 367

<210> 73
<211> 640
<212> DNA

<213> homo sapiens

<400> 73
ttaaatcaac cctaactggc cagaagaaac cagaccatgg acggtcagat ccctcccatc 60

cgccctctcg cccctttcac ctttgccccc cttctcctct tccgacacag tctcaaaccc 120

aaagtgcgtt tccgctgccc gcttctcttg ggacaagagc cgagcactta gtaggtcccc 180

gggccaagag ctacgaagcc cgaggagctg gcagccccgc atcgcccgcg accacccacg 240

gccgcaatag ctccatagag cacagctccc gggggccgcc atcttggtag tcgagtgaca 300

acggccagag agtacgcctt gttgcgtcac tccacgaacg acggcggctg gccggcaaca 360

ttattagccg aacgcgccct gaaatctgat acacagttgt gccggcatta tctctaggat 420

ccaaagggtg gtctctaagt atcggaaatc ccttcctctc tctcttctca aattgaattt 480

acaattcacc aggaatcagg ggatctcagg ggagccccgg gtcacctctt agcggccatg 540

tctctgaatc cggctaggca gaaaagtact aactcgcaag atatccagga actcttgttg 600

tcttgtgcag aagggggaaa ttgaggcatg gtgtggttaa 640

<210> 74
<211> 1074
<212> DNA
<213> homo sapiens

<400> 74
ttaagcagcg gaaaacctgg aggagcccag ggagctccga gccttgctcc ccaggcgctg 60

tccagagttc tgccgcgtcc caaatctccg cagggaaacc cgcgccctcc tcagcctact 120

agaccctttc acagggtccc ttctgtttta ttgccgagga gaggagcttt gcttcccgga 180

gatccaaagg gaaccgctcc gagtttctct ctccctctcc cgttcgtcac ctctcctttg 240

ttttcgtggc aatgcgaagt gcttctgcaa gtctctgggt ccccaggcag agcgggcgga 300

tgatattcag caccacgctc cttgcagttg ctttgtcgcc agaaaaagcg accttgtctg 360

atggcctctt tccgtctggg tttcttctcc ctcgtcgtcg tcctcttcct cctcctttct 420

ctcgagctga ttctctttca ttgctatgac ctcattgggg tttaggaggt tgacggagga 480

ggaaaaagcc taatgtattt gtagatccag gaccagtact cttggctggc ctttgatccg 540

ccccgccccc ggaccgcagc aacagaaatc caggcgaccc ctacctccgc ttctccccgt 600

ggctcctttc tgggcttcta tccagcggag aggggaggcg atgcctacgt caacgcgggc 660

tcattctgag aaaaactttc tttagcttgt acgcggggta gggggagcag tgagcaaaga 720

aagacggaaa ccccaggggg gtggaaaaag ccagactcct aatgtcacta ggactctcaa 780

ggcccctcac caaggaactc ccctgtctag gagagctaga ttcaagcctg cttacaagga 840

atgtccactc ccggccatca cagcctgtca ccttccgtgt cgtagggcga agaagcagtt 900

gatgggggcc acacctccac ggtgattttg ttcctctcca gccacagacc cttgctttcc 960

cctaggagcg tgaccacacg ttctgggagg catagctaga cccaactgag ctgggattat 1020

atattctcaa caaaattcaa gagatgtgta tgcattagaa caggcattat ttaa 1074

<210> 75
<211> 1099
<212> DNA
<213> homo sapiens

<400> 75
ttaaacttcg gacggctggt gaattgtgcg gcgggcgcgg ggccctggga ggcagccccc 60

tcctgggtcg ctgcccgcgg gataaagcaa tttccaagca cccgcgatat ctccccgctc 120

cccgcaggag aagcggggag taaacgcccc tcaagtgtgc acaagcaaag agcgggtttc 180

cctgtaactt ttcttgtagt tttgaaagaa agcggcccgg ctgcctttca ggtctcttac 240

tatcgaaaaa gatcagcccc cattttgttc aggcggcggg gaggccggga cgcgatgaga 300

gatttacaag gtgtcctttc aaaaagaatt cccagtggag acgaggctga aacgtcttct 360

ttacaattac aaccaaaata attagaaaag cgcaaagtac attttggaac gattgggcaa 420

aaacgaaatc tagccgcaga aatgttttct ctgcggcctc agtcaccaaa ctaattagtc 480

caagaaatct tctggtcttt acaactttct cagagtccgg aactcccttt gctaacattg 540

caactagacc attttttcag aggatgaata ttttttacag aaattgcgaa tgcagttgtg 600

tgccatttgg gaaccctgcc tgtgtttgcg ggggagggag agagcttcag tgtgaggacc 660

tgcacccttt gtggagagct ggggaaggga gatgtttgct gttctgagtt gtttttccca 720

cctagaggga taatatgtaa aaattattcc cacccaaaag gtgtgtgttt ctccagctct 780

cccactggtt ctgagagagt aaactcaaac ccaaaccctg attctaggcc taggtttcca 840

agccattata attgggtgtt tggaagtcaa aagataaaat tgtatttgaa tgtctgtctg 900

cgcaatttat ggtaataatg aggcctaatg aggttgttag aaagataaaa tgttatttac 960

caaaaaacct gatgggataa tttgacttgc tgtgttttac tactgattat aaaaagaata 1020

tcgattgcaa ataaatcagc gcctctaaat gcctgcaaac agctagtgtt tgctccctcc 1080

agatcaaagt caaacttaa 1099

<210> 76
<211> 868
<212> DNA
<213> homo sapiens

<400> 76

ttaaaatctt cagcctgcag tgggatctaa ctaccttccc ttccgtcccc agtgcctcct 60

gatgcttgca atctcattcc caattcacca gcgtgcgtga atgcagactc cagatccagc 120

gcagacgcca agaattcgct caggggcacc agctaaatag caaacagatg ccatgtcaaa 180

catgtgaaag cctcagcgac tagggtaccg ccgggaaaac cagttccagc ccaggaattt 240

ctgggtggaa acactggccg ccgaattgtt ttattctctt tgcttgattc gagtcggttc 300

tgaaagcagg tgtcccctct cccccggctt caggaaggga aaccccggcc ccaggcagtt 360

gcagagctaa gaaaaaccat cccgccaggc cccttcttgt ggtaacgcgg tcctaaccct 420

gctctgcccc acaaccccat ttcacccggg agacgcagac gccaggctcc ctagcacttt 480

gtcgcctctt cctccagcct ggcttcctga gctctggaaa agttccgggc ctccggacgt 540

tgtttccatc ttcgccttcg ctgcctgggc cgcatgcctc ctgggcttat ttaggaagcg 600

aagttagcgc cacacatttg gctccagaaa tttgttctga cggcgcggcc gacgccctgc 660

agggtcattt ctgaacgcat tcccaaatat cctggtcctc aagcccttac cacccccctag 720

cacacgaagc cccagctggc ggtggagcag gtgtgttgct ggtagctcat cgctggcttg 780

gagatagagg tgcttggtac ctgtcccctt tccgacaaca cctgaaaccg tatcggggca 840

cacccctcat ccaaatatcc acgtttaa 868

<210> 77
<211> 946
<212> DNA
<213> homo sapiens

<400> 77
ttaaatccag cttatttcgg cggcagctgg actattcctt tgcttatttg gggatgggtg 60

aggggcatac cctttaggaa aacgtttcca aatgatgtaa taatttctct ctcatctctg 120

gtcttgctcc agacttggcc tcttcctcgc cactctgtcc cggcctcccg cccccgcagg 180

ctcccgtgcg gacttagcag ttcgcgccgt agccctcctg gtttttttacc gcgccttggt 240

cgcgcggccc ccggtgcggg cctcgtcgct ccgacagccc tacagctgtc cctacggccc 300

gcaccgacag ctcgtccccg ccacctccct ccgggctgta gggacgtcga gatccgaatc 360

ctcacagcgc ctgacccggg gccgtgttgg gtacgggtac ggcgagagcc ttccgagccg 420

cctccaaaac tctccccgcg gacctcagca gtccctccac gcgtcggggc cctgctggcc 480

ggccatgccc tctgaggaga cgcggaaaag agcgaggcat ataggaaggt ggggcgtttt 540

atccgcctcg gttttgaccc atctgccatg tagtctcccc aggcaccgag ggaaaggacg 600

acggaagccc gccgcccgga tcgctggcgg gagagagggc agcccgcacg ggagaaggag 660

gagccccgcg gggtctccgc tgggggtggt aattggaatg caggcctggg ggtctctgtc 720

cccctccttg tctcctaaaa tgcttccggc aagctctggc gaaccccgaa gctgtaggaa 780

aaaggtattt tattttgtgt ttggtagggt cggggtaaag gttttccctg ggacttcctc 840

tcctggggct cctccggggt gcgtggggta atgtcccagc gcaggtcgag ttgcagatcc 900

gagtccccgg ggttgttgac tgtccttctt gcaggacatt tcttaa 946

<210> 78
<211> 576
<212> DNA
<213> homo sapiens

<400> 78
ttaatggggc cggggagggg ccgccgctgc cgagaactga gggcgacaga acaatcccac 60

ctcttgtgag gagcaggtct gccattctgc ttgtcagttc tcaccttccc ctcctccacg 120

gcaaagagtc cctttcacac ccttctgagt gtgcgggtgc gcccagtttt acaagtcctc 180

tgtcctgcca gggtagtggg agaagcacgc gatggggcta gcgcccagtc ctgcgggggc 240

aggcgccaag ctcccgggag tccgcaagtc gcttcctata ttggcaagct tcaaatgaga 300

cattagaatc tccggcaacc ttccttcaaa cctgaaccgg ggaagagaat aagccgccct 360

atggccaagg cgcaattgcg tacccgtgtg tacaggtcca tgccctgtcc cctacactct 420

aattttctca aagaaaattg ttttgccaaa agtaaccctg agaaatgcag acgctccaaa 480

cgccctctcc acttcggatg aagccagcat ctagtagggt ctccggccct caagaaggtg 540

aggcaaagag atgaattgtc agacagctgc ttttaa 576

<210> 79
<211> 556
<212> DNA
<213> homo sapiens

<400> 79
ttaaagaagg ccttgtcccc cagcaacaac actcttgccc ttgtccccc agtaccaaca 60

ctcttgcgct ccccatttcc cggctgcact cttctcattc tccccttccg agaggcccgg 120

ccaccggctc gggagctggg ccggtcgctt tgggagcgga tgaggaaggt taggagaagc 180

agcgagatag atcccaattt tacaattcta ttttctttcg gtagggtctc ggcgtcctgg 240

gccacgttga gagcgaacgt gggccgagcg gaggacacag agtaaaaagc gacgcccgct 300

gtatacataa atccgcaccc gctgcccgcc cgggtactgc ctgctctggc ttccgctctc 360

ttccgaggct gggcaagtcc aaaagttccc gaaaggggg ttcaaagagg ggcgctcagt 420

gcacgtgatt tcgtttcatc tagggtgttg ggggtgggga gattcctcac tggatttggt 480

ttctccagca gaactgtctc atcctccaca ttcatgccgc tctagcggtt ccgagtgaaa 540

gaggcaagag gtttaa 556

<210> 80
<211> 836
<212> DNA
<213> homo sapiens

<400> 80
ttaaaacact attgtttgcc aaaattccat caggtgctaa ggcatacatt atacaaattt 60

agatagttcc aacattacaa gatgttcatt gtaatcactc atacccaaat tcaacatgta 120

aaactcccaa gcccgaaacc atcacctcct cctcgttagt tttcagataa ggtgttattt 180

attctataaa gggtaaccta aaccaggaat ttctagggaa acacgaaggg gatgtttaca 240

tcaaactgtc tacaaggcag gacgaggcag tctgatcact tatcagattc cttctctatg 300

acatcaagga acatggagaa gttttccttc tacgctggtg aagtcatggt cctcttatct 360

atctgatgat acctaggaaa cactagcctc caaccgcagc ccctactgca aaaaggcagt 420

cagtgcccat tgccggggtc ttcccaagcg gagcacctca aaagccgcac cgcgcagtag 480

aggtctccag gggcccccac tcgcccccag tgactacgga gcggcctcta cctcccgccg 540

gcgcctggcc acttctcctc ccccgcagtc tccgcttgat ttccatgtga ctgcaggcaa 600

gaatagccca gcctttgtgc ctctgcaaag aacgcaaaca aaaatgtaca ccatcaccac 660

ccaacaaacg cgcaactccg caagacacaa agcgtctccc aaacatcgct tccctggtaa 720

catccggcat cctggggctg ccaaatcgca gacaaagacg cccatcagcg cggggggtga 780

ggagactaag ggagagacaa gaccaacgtg ctcccaccaa cgggagcggc ggttaa 836

<210> 81
<211> 686
<212> DNA
<213> homo sapiens

<400> 81
ttaaaaacat atcagcatcg cagtgagact tgatctttga aagagagaca cagaaggaca 60

accgaaaaga gagcagcttt ctcaagaggt aaataagttt gttgaaatct gataggcttc 120

atcccatttc cttcaacaac tgagtacact atttgctaac tgtactaaaa aacgttttgc 180

acattttttc ttattatttg ctcacagctt ttgactacca gagaatgttt cggatacagg 240

actaattcct gtggatgaca gaatgcgacc gttgaatggg acattgggtg tcatctggtc 300

caaatcttga ccccttttca ttttccccga aagaaagact tgcggctcgg aaccgagggc 360

ggcatgccca aggtcacaga aagggtggtc taatgttttg gttcctgcag cccagagaac 420

aagctatccc atcccacagg agccttggcg aaaagctccc cggccggcgc aaggccagac 480

ttctcgcctc cactgtgttg attgcggttg ccagggcagc ggcgcattgc tccccgcctc 540

gtcctggtga cgtcacagag caatgtccaa tgggaaagca gctcggtgtc acgcacctcc 600

tatcccggcg ggcaccgcct gcgccgcggc gagtgaggcg tcgtccgtac tggaggctag 660

ctcttgtcgc ggccgcggcg agttaa 686

<210> 82
<211> 536
<212> DNA
<213> homo sapiens

<400> 82
ttaaaaaacc gcgaaacagg ctagcgccta cttcccacat cccccagcga aacaagccct 60

ccctcctcct ccagccaccc cctctcgggg agtcccacga cccggggacg gaacacccca 120

acccagcccc gcgtcacccg cactgagagc gcgccaactg ctaagcggcg ttcgccgctt 180

ctctgctgcc ttttgcgggg gtagggggat gtctcggcta aacaatcgct gcctacatga 240

tccgaaggtg tgcccacttt ggtgtaagag gtattgccta actcaaactc aaggtgctga 300

aagaaaaggg tcacaggatt agctagtcgg cagtgtaaga acatcgttgt ccagaggcag 360

ggcaagggca cgacaaaggg aactcttcgg tccttccaag cgttctccct ttatagagta 420

tgccgtgctg tgaaacagtg ttaggaacta acttatgggg agcaatttag tagcaaccac 480

acgcagctaa ttgcagatag gattgctcag ggctggtgct tctggaaaga gattaa 536

<210> 83
<211> 1186
<212> DNA
<213> homo sapiens

<400> 83
ttaagtggcc ctgatgatgt taccaagccc ggtcacctcc accccttttct cagtcaaaga 60

cattttgaat ctggagcagc agcaccagca cttccatggt gcgcacttgc aggcggactt 120

ggagcaccac ttccactctg cgccctgcat gctggccgcc gctgagggga cgcaattttc 180

tgacggaggg gaggaggacg aggaagacga gggcgagaaa ttgtcctatt tgaactcact 240

agccgcagca gacggccacg gggattcagg gctgtgtccc cagggctatg tccacacggt 300

cctgcgagac tcgtgcagcg agcccaagga acatgaagag gagcccgagg tcgtgaggga 360

ccggagccaa agtgagtaga gggggaaaga aaacgcaccc gcacacctgg agcaatggca 420

gagcgcacac aaacagccca cagaccttgc cagcgcagct gcccatccct ttaccctttg 480

gctggggcca tgcctccgcc ctggtcaccg acagctccac aaagcgcagg tacctgcaag 540

ggagccgggg tggagagaca aggacgaggt gaccctgaca actactcccc ccaaaagggg 600

gaaatgaggg aagggaaggt ggttactgct gccctgatgc tgagaggcga cgtgggctca 660

gcgcctgggt cctcataact ggcagtattt ttgctcttcc ccccgcccca aaaggctctt 720

cccccaaaaa gggcaggagc ctggaccttt ttcgttttcc ctctagggtg agatgagttc 780

tcttagccca gggcagccac cccttcttct ccgaaaagac agcccaagca gcctgtgtct 840

ctcgcacccg caggggcagg aactttggtg gaggccagct gggcggtttc ctcccttctc 900

tgcttggagc tcagcgtcct tgaacccgtg gcactcggta gagagagagg agatgatcgg 960

aaagtgcgtg ggaacaatgt ctattccgcg cgaccatagc tctcacatcc ctaaggcgcc 1020

agcctttttt gaaaatccgt aacgttttgc tttgtgtccc aggctgcggg cctaatagaa 1080

aacgcgccga acttgagtcc gagatcttgg ctgctcacat cggatgggaa gtgaattgat 1140

agggagatgg gataatcaaa taaagtcccc aaggaagtgt atttaa 1186

<210> 84
<211> 249
<212> DNA
<213> homo sapiens

<400> 84
ttaaacaatt cctcggttgc tgcggcacct gcgcttcggc agcctgacga actgtacctc 60

cggccctcag ccgattccct cttactttcg gaagatgaat aagcctccaa gctttcaccc 120

cccgaaatca acccaaatca gctccgcctc cccctcaaat cagagaaagt gtttggaaaa 180

tggagtcaca tccatcaccg aagattccca atccggaaac ccagagctca gcaacccaga 240

tagcattaa 249

<210> 85
<211> 887
<212> DNA
<213> homo sapiens

<400> 85
ttaaagcagg ctcagtcagg ttactgagtg cctgttgctc catcacacga attcccaagt 60

tgtgggactc accttcaggg gtgtcttggt ggagcacctc ttgggaggtg atctgacctg 120

ttggttcagg agcactgcgc gtattcactc tggcgctcct ttgtgcaaca ggagaccaga 180

ggactcccac ggtcccactg gctccttcca cgtaatgtgc cccgctggct ctctcgcctc 240

ccccaacccg ggggtctgcg tcccctgcgc cccagtccct ctttgtgcag ccagtagctg 300

cagagctctt cgtcttgtat tcatggcaga tgcacagcga gtgactgaca cgttccaggg 360

acggcgaatg ggaaagagcg cgggcggccg ggggagggtg tgtagtggca gacggccgct 420

gggattatga atgggggctg gggggccggc gagtgtgggg ttcaaaccag gattttcggt 480

```
ctctggcttt ggataggcac gcagggctgt gcgtaatcct tcagctctgg tggtaaggga    540
gatgcagtga gctcccggct gggagagagg gttattttgc aaaactcccc tggtaccgtg    600
cccgcggaat attgcaggag gtctgtgagg taggtggggg aaaaaataac ctgggagggc    660
aatcagggag aggccagacg actggggacg gcgtgctttg caccctagaa ttgacaggtg    720
gtggcctcag cagttacaac ctactacaca gcatctcgtc cgtgcgatac ggtcacattg    780
ttattccccg cacccggctg aggagcgcag cgcggcagac caagtggcac cggcgagctg    840
tctctcttcc gggggcggg ggtggacagt agggagaggg ggtttaa                   887


<210>  86
<211>  382
<212>  DNA
<213>  homo sapiens

<400>  86
ttaagaagat gctgatggat caaatttgaa actgtggatc cactaaacgc ctttttagaa     60
ggtttttttt ttttctttca aaatagcaag ccttaggagc tcaactccag agccaattca    120
cttcgcgtga agtcggcact ccttgggagt agaggtcccc tctcaccggc tcccaccctc    180
cagaggggag gatttctggg gagcccgacg atccgctcgc ttttagcctt tctccttgca    240
acgcgcaaat ctctccctag ttgggctcaa gtagagagga tcaccaactg ctcatcccag    300
cggacactct gcaaaaaact ctaatggctt gtgcgcgggt ccacgctggt cgctagcggc    360
gccaacttta ctcattcgtt aa                                             382


<210>  87
<211>  1023
<212>  DNA
<213>  homo sapiens

<400>  87
ttaactccct ttacagagtt ctaaatgggg cgggggtagg agagcaatgg aagggaaata     60
aacgattact ccagaaaaca acaatttgag tttatttcca ggaaacaggg cctccacaac    120
aacgtgagaa actgtcaggt acaaagtaag ttatttgcgt cctgcggctt cagccaggag    180
cgtggctgtc gcttttcccg tcttctccat caccctctgg ccaccactgc gtctccatct    240
gcaccgcgaa ccccagcagc agatgcgctc gccagccaag cgaagctggg taggttggcc    300
agaaaagttg tcaggactgg cgggaggtag acaagtgctc gggagcccgc tccccagcgt    360
ccacctggtg aaacttcaga gcccccagag gagagaacga aagaagcaac caacctacac    420
gaaggataac aaaactggag gggaacagag ggcgggaccg ggagagccag ggttcacgga    480
cttctctgag cggcttgacc gagaaccccc agggctacag cgagacgctc cacttgtctc    540
```

cttttgggtt ctccggcaga aggcagacat ttacagtccg cttccagccc caggccggat 600

ccggggagcg cggggccaga gccccaggcc ggatccgggg agcgcggggc cagcctggcg 660

gtggttttgc ccgaggagcg ggctcccacg cgcgagtcag gcttcctcca ccaggcgccc 720

agacctcgat gcccatgaac gctcagagtg ggtccttaga accgcatttc aacacatctt 780

acagggctcc gccaacacac acacagaaca tgctccaagc agtgcccgag cgcacagggc 840

aagcccggga gcaacccaga gctgggggag gagaaaaggg gactccggga gggacgggga 900

agtagttaga caatagtcag gggaggttat tcccgggaca aaaagcacct ggcaaagcgc 960

gccgaggtca gtcccttcga atctccacat ttctgacaat ggcttcccag ggaatttgtt 1020

taa 1023

<210> 88
<211> 536
<212> DNA
<213> homo sapiens

<400> 88
ttaagtagga aacaaaagca ccaggatttt cacacagaga cacagataaa cacaccttat 60

tcatccaaga caaagaaaga aaacttggct ttacctgcag ttgtaggtcc ggatctcttt 120

gttgtctcgc ttgcacttga ttgccacgaa gatcatagtt acaaagagga tgcccgcaat 180

ggagcccagg gcaataatga aaatcaagga caagttcaca gagcccattg actcttgggc 240

atcgagagca ggggacaagt agattaggac gagagcagag gcagagagag atgtcttgcc 300

gtggtcgtga gccaccacga taagctcata ggaggacttg gagctctccc cgaaggtgcg 360

ggtggttctg acttcgccat tgacctggtc tatttcaaag aagccgcggt cgccctcggt 420

catgtcgtag gtgactcggc cattttcgcc ctcatcgtag tcttctgcct tgacaacagt 480

caccaggtag cctatgccag agttgcgggg tatgtagacc tcggcagtgc cgttaa 536

<210> 89
<211> 546
<212> DNA
<213> homo sapiens

<400> 89
ttaaaatagc acatcaacgt ccgcagcaac tccccgggaa ggaaaatatg tccggggagg 60

tggcgctgga gaggagtagg ggaaagggag gggagctcgc ccgcccgggt gctgatcagg 120

cctcacaaaa gccgctcgaa tcgccttgtt atcataccaa aaggtctggg caggcccaaa 180

ggagcccctt tctctcggtt ttagcaactt cgccggcaac tgtctcgcaa tcttgagaac 240

aaacacagct ccacaatggc cggcgttttc tcggaatgca aatgttgcgg gctccgcaaa 300

gctgggtttc tgttaccgag cctaatgccg gcattgtgaa actcgcgtgc aaacaccaag 360

attgacatgg gcgtcacgtg atggattagg gctccaggaa agttttcaaa gaggcgacaa 420

tggggggctc agactgatct gatatctccc acagtttagc gggaaagaaa atcaggatat 480

attctcatat gtaataggat tatcttgtcc atttcaagtt ctatacagca tgtaatttat 540

ttttaa 546

<210> 90
<211> 680
<212> DNA
<213> homo sapiens

<400> 90
ttaaagacag ggtacataga ttcaagaggt tgcgctttga ggataaaaag ctgggccctg 60

tgcagttttc caagacaacc tctgggcgcc gctgtcgacc aaaaggaagt gaaggcttct 120

caattctgca ggagcgtcag gcagggtgcc ttcctgcttt gtccggtgca ctgagcacag 180

acgctgctcc tgttcaccct cgagcgccta actaataagt cctaagctca aatcacagaa 240

gtccagggtg ctgccatttc tttttgaaaa acatcccaga ggaaagaagc tccgcggaga 300

gttcctgaaa tgcagagagc cagagaagcc gaaatgatga aaagtcaggt actgtttccc 360

ttcctgctgt ctttgttctg cggggccatc tcccagcaga tccgatacac gattccagag 420

gagctagcca acggctcacg ggtggggaaa cttgccaagg atctggggct cagtgtccgg 480

gagttgccaa ctcgaaaact gcgggttagt gcagaggatt atttcaacgt tagtttggag 540

agcggggatt tgttagtgaa cggtaggata gatcgagaga agatttgcgg aaggaaactt 600

gagtgtgcac tagaattcga aacggtcgct gaaaacccaa tgaatgtttt ccacgtggtt 660

gttgtaatcc aagatattaa 680

<210> 91
<211> 667
<212> DNA
<213> homo sapiens

<400> 91
ttaatggttt gcttgcttgc cgggagttgt agttcaccgc tcgcccgttt gccgggaaca 60

cccggagggc gagactacag ctcccaggag tgcacggggc tcctcggcag tacgcgtgtg 120

cggcgccagg agaggacagc tccagtgctg atgttggagc cggttagcga accccaagag 180

tgcagagtgt ggagcgtgga gcgccgggac tgtgcacgct tgaccggaag cccagaccag 240

tgcggtccta gccagagaga aaggacattt gccaacaatg agacacgaag cgcccatgca 300

ggtgggtcca taaagaaaga gtggcctcgg gaggaattga aagagatgct ttccccttg 360

ctccgcgcac aagcagttca acgtaaggaa aggtctaggc ggtcacccgc ggagatgcgg 420

ctctgtgaca tgaccctggg aacagagttg ggtttcttta cgctctgtgt ggctgtgatt 480

ggggctgtct tgatgaggga tcgggctatt cgcaatcttt ctaggctctg taaagaggat 540

aagagtgtct gtctaaaatg aaaacccaag caaacaaaag tcagaggagc atgcaatctc 600

gtgttggcat tgatctcctc tcttggcatc cggggtttga gatcgcggtg aaaactcacg 660

gatttaa 667

<210> 92
<211> 681
<212> DNA
<213> homo sapiens

<400> 92
ttaaattcga gaaacattta ttgagaacct gttctatgta cagcactgtg ctaggtccca 60

tagggagagt aagtcattta ggattgcact ctaaaatctg ggcatattgg tgcaaaggga 120

ggtgaacagc aggcgtaggt gagtgtcaga ggacctgacc caagctggga ccctcctgca 180

ggtaaaacgg tgggtagtga gaagctctag gaagggggcg cttccgaaca cgcgcgtcga 240

ggagggcgtt ccaggactct gagggagcag cccagctgga ccgaggccgc gtcgttcctg 300

ggcttactat tcccagaccc ggactcccga ttccggagtc acggcccagg acgcgaaaag 360

actctacact ggcaccacgc tcctccttag gcgggccgtc agtcccgggt gcgggctgcg 420

ctggaggctg aggtgggagc gacatggtgt ggagggggcaa gaaatgtcgg cactagacgc 480

gccaagaagg agattctacg agcaattccc ccctcgggcc attgtgttgc tgtttattag 540

cccctgggag ggcgtcagga caaaaggaac cctcctccct tcttagtact taggcccaag 600

gtcgggtgtg ggagccggcg cgctgctttc taggcaggca ctgaagctac ggcagccacg 660

caaataggta tcagccgtta a 681

<210> 93
<211> 283
<212> DNA
<213> homo sapiens

<400> 93
ttaagaatct tcctgaaaaa actaatacat atgtattaca tgctaacaca cgcatacatt 60

cttgcgtaga attgtacata actcaggagt gtctagagtg aaaagaccgt atcgcactcc 120

ctcccacggc cgcggctctc ccacggccgc ggctcccccg cggtaacctt cggtcagggt 180

gtgctctgcc gcggttcttt ccagtctctg cgcttttaca tgcacacgca cagattgcga 240

gtggaatctc ctgggctttt caaaagaatg ttttcaacat taa 283

<210> 94
<211> 1098
<212> DNA
<213> homo sapiens

<400> 94
ttaatgttct tcgtctattg ctagagaaaa tgtctttcct tggaggaggc attggcacga 60

gttactataa actccctctg aatctcaaga cttctgggac gccgattccg ctcctggcct 120

ggggcaaggc gtgggagctt ggaagccagc gctgcgctcc ccgtgggaag cgatcgtctc 180

ctctgtcaac tcgcgcctgg gcacttagcc cctcccgttt cagggcgccg cctccccgga 240

tggcaaacac tataaagtgg cggcgaataa ggttcctcct gctgctctcg gtttagtcca 300

agatcagcga tatcacgcgt cccccggagc atcgcgtgca ggagccatgg cgcgggagct 360

ataccacgaa gagttcgccc gggcgggcaa gcaggcgggg ctgcaggtct ggaggattga 420

gaagctggag ctggtgcccg tgccccagag cgctcacggc gacttctacg tcggggatgc 480

ctacctggtg ctgcacacgg ccaagacgag ccgaggcttc acctaccacc tgcacttctg 540

gctcggtaag ggacggcggg cggcgggacc ccgacgcacc aaggccggcg aggggagggc 600

gtaggggtct gagatttgca ggcgtgggag taaaggggac cgcaaactga gctagccact 660

cgcgccccca cggggctgcc tttggggccg gccactttct ccctaccgaa gtcaactcgc 720

cggctgcaaa cgcggggctc aggcgttgtc cgcaattgac ttacctcgca ggctttgacc 780

tttgccaggt gtagttcctg gactttggag ctggtgactg gcttcttccc ctttcaccgt 840

caagtatcca aacgccactc ttgtccaggg ccgctgcctc agcgcacacc gtctctggcc 900

aagccccgct caccttccga caaactcagt cctgctttcc ctgtcgggat ggagaggaag 960

tcataaagtc tgggggcatc tgccgccgct aggaagaagt ccttttcttt ttcttcacac 1020

attattttta tttgttgctt tcgggcactg gcttattttc tctatgattg cgggaattat 1080

tgttctgggc ttctttaa 1098

<210> 95
<211> 1260
<212> DNA
<213> homo sapiens

<400> 95
ttaaattctg ggcaagattt tacagaaatg cagcttcttt tttctaattt agttgtgtgg 60

tgtgtgtgtg taaagtgggg gaggttccac ttggtgggga aaggagaatt tgccattgct 120

gctcgtctac tcaggactgt ttctgttgtt gttgtgtttc agcatcagag agtgagagtg 180

tattgcagca atctgactat ttggaagact gttccttgaa tttcccaatt caaaagcctc 240

ggtagagctg agggatgctt gatacgtcaa cacagaccac aaaaggcagg gcttttctaa 300

agagattata attatatcta ccttttgggt acaggaggtg aatggaagga agggattctg 360

gagcagatat cccaaaagaa gaatcccgaa gcagaactcc tcgcacaagg ttatctaaat 420

ctccttgaca ggtgcacagg cagagaaggc atttggccct tgaagtaaca tttacttgag 480

aggttgggac aattctgtca cgcttaggac aagccagctg accctgagcc caggagcacc 540

ctaggactgc agcacagaaa atacaccagc tggccggtcg cccctccttt gttccattcc 600

cgggggattg gagtagcgtt ggagtcaccg acgccatccc ctcccgcctc tggcgtgcat 660

ggagcatgcg cttccttcct cacttcctct gcaggaggga gcgagagtaa agctacgccc 720

tggcgcgcag tctccgcgtc acaggaactt cagcacccac agggcggaca gcgctcccct 780

ctacctggag acttgactcc cgcgcgcccc aaccctgctt atcccttgac cgtcgagtgt 840

cagagatcct gcagccgccc agtcccggcc cctctcccgc cccacaccca ccctcctggc 900

tcttcctgtt tttactcctc cttttcattc ataacaaaag ctacagctcc aggagcccag 960

cgccgggctg tgacccaagc cgagcgtgga agaatggggt tcctcgggac cggcacttgg 1020

attctggtgt tagtgctccc gattcaagct ttccccaaac ctggaggaag ccaaggtatg 1080

tgaacacttt tcttcttcct accttccttt tatttcgcca cgaaaaggta aagtttggca 1140

taatacgtga gctgtaaatc accctgacgc gttttctgat caaatcatat ccatgaatac 1200

ggacagagaa atcagttcga atttagagac agaagacaga ttttttttct tcacttttaa 1260

<210> 96
<211> 359
<212> DNA
<213> homo sapiens

<400> 96
ttaaaagcag acgaatgcct ttttgaaagc acgcattgtg tgcgtttcta gaggagccgt 60

gggcttggga atgaacggca ggcttccccg ttcggtggtt tccagtggga aatcactccg 120

gaaagggaac ccttcggtat cgatttattg tttttgtctt ccaaaccatt atctctttga 180

ccgaaatact gtctgagaag ttccgcaggg acaaagtcag gtaaactgga ccgggcagat 240

ctttggcaag atggtgataa cggggatgct gttttcagtg tctgtttgtg atggaagcat 300

tgttgggtaa tagcaatttc tgatcggtag agcatgctgg ttctaaaaaa ataagttaa 359

<210> 97
<211> 556
<212> DNA
<213> homo sapiens

<400> 97
ttaaaaagga aaaaaaaatg tatctatgaa ccaaaggccc ttttgcctgg gttcgttgat 60

tccaaaggct gccctctctt ggaagatctt gtcttgcatc ccgttctggg ccaccagcca 120

ggagtactac tgacgggcgc tgctccgcat tgtttcactg acttgtgatg ggtccgtgtt 180

cctcagatcc cctcccagcc tgcctaccct cgctcagagc ttccctccct cctggattcc 240

tctcggagct tttttcagaa gcccatttgc agccgaaagc tgagatgtgc aggatgatac 300

gcagcgtgtt gaaattttta tgaatgaact ttgccgaatg aatttctctg tgtgtgtgca 360

agctaataaa tctgtgaatg aagctgagaa tagctgtaat tgactgacgg tctcagaggt 420

gcttggtttt tatccaatca ggaggcagct tgatgactca tacaccataa atatactaaa 480

ctagtagccg agagaaataa agctagcggg agaggaggga cgtattttat tttgtggtat 540

ctgatgaatg agttaa 556

<210> 98
<211> 512
<212> DNA
<213> homo sapiens

<400> 98
ttaaaagctc aagccttttt ccttctacct cttacaacac cagcgagcat tccactgggg 60

gaggggtcct tcgctccgcc acctggccta tttgttggag ttacttacga tgtcttcacc 120

cagaaaagag gtccgagggt atgttagtct ccggtacccc tccttccacc aacgtcctta 180

cacctaactg atccaattca aaactgcaac aaacccctag cacctgtgag cgtacagaat 240

acaccgcggc caaactcgat gtgctctaca atgcacatcc tacatacgag aaatactggt 300

ttcaattagg cgaagtagga aaggatggcg gcgagtacct atggtgcaga gcgctcagga 360

tctccgaggt ttgctgcgag gggctaccca gcaggagggg caaactctct gcgaagctga 420

attgtatcca tcatactgta gccttgtagc ttctcctctt ctggccacca actttccgaa 480

aggctaccga atcacgcttg taatccgttt aa 512

<210> 99
<211> 445
<212> DNA
<213> homo sapiens

<400> 99
ttaagcggga ggacgcgcgg gacctgtgag ccctcccgga ggtggacccc cttcccagca 60

cctctgcgag agatccgggg acctgtgggc agctggcgga ggggagactc agcagacgga 120

ccctctccgt tgcctgcctc ccaaaatgga gccaggcttc caacttccgc tgccttcgga 180

catagggacc cagttcccag gagcggggag gtagggttgg ggctggggca tttggattgt 240

aattgggagc tctgccgtac gccagggcgg ttccaaactc taaaccgttc ccaccgtcag 300

ggagacctac agtttcgggg gaccaccctg gtctggcctt gtatatagga aatgctgctg 360

aactgaatag aaaggaactt gggagatttg aaacagtgct cgggttttcg ctaggaccgg 420

tttgggcttt gtacaggtta tttaa 445

<210> 100
<211> 1059
<212> DNA
<213> homo sapiens

<400> 100
ttaattcgcc aggtaaaagg aaccccatca gctcgcatcc ctcaccccca tcctgagcac 60

tgagtgtttg cgagaaactc gccctgcgcc agcaactctg ggctccggtc tcagctcccg 120

cgtagacgcg gaggagagat tggcggcccc cgctggggaa attgagcccg gcgccggtcc 180

gggacgtgca agaggggaa agggggagat cccagacctc ggtttcccga gggcttccct 240

ggggagccac tcgcacctct gtgtctccgc tcccggcggc agacctcgcg gcgcttgctc 300

aagagcgggt atccgaaagc acgtcccgaa cacggagaag cgccctccgt ctcctgcctc 360

gtgacctgcg ccctttccct ccctcaccgc atcagatgga ggaccctggc tcctttcgcc 420

cctggcaaaa tgctctagcc aacactgggt caacagcctt agtttccaga tctgtaacct 480

gaacatcagg agcgggttcc tttccttcta cctgtgtctg aggtcctctg gtcggggact 540

gcgcgcgtct tcccaggtca gggaggaggg gtcggggcga cctgagaaag acacaacgaa 600

gcgaatggag cccctgaatg gggaaagggg agtggacacc aggaggcccc tgcagaaatg 660

caggaccgat ccactcgcga gctgacaatg ccacgcgcta gacttggcct gacggtggca 720

acgcttggca cctgggggat aggggccttc tcctggcctc agtccccaaa ttctgcgcag 780

aatagaacgg ctggcatctc aagtgctctt ttttcacccg gatctctaca gacacaagtt 840

ccctcactga gctgcgcaga ggtggatccg cacaggaggg aatatcgcgt aggaattcct 900

gcaaatataa atctcagcag caaagcagtc cagattgact ggaacacacc acccccagcc 960

accaccatcc aagttttccc tggttctcgg agttggaaac agtttctggg cttttggcgc 1020

cccctctgga tacagatgaa aactgcagtc catttttaa 1059

<210> 101
<211> 580
<212> DNA
<213> homo sapiens

<400> 101
ttaaactacc tccactctcc agcatttcgt gcacatagtt ttaggatagg gtttcccaaa 60

ctggcatggc ctggagcaag acgctcagga agacattcac atttcttgta gagtgaaaca 120

agtgtgggga atgctccgtt cccttctccc caaggtgctt cacagcgctc acgaacatct 180

gaagagctct cagaagtggg aggcaaaggc atctctttca cccaggactt ccagcatctt 240

gcctacacca cgctgtttgc agcggctctg cagcatactg cagcgctgtt cataacgccc 300

atgtgggatc accgcttaga gaagcaaaga aagcactttg gcagaagacg gggtttccac 360

cctcctcggc atcaaccagt gtctgggacg ggaactggcg gccgctgcgg cctggcttcc 420

cagctgccgc ccgggtctgc ccctcccaca ctgcagcccc gtacacccct cgctgtgtgt 480

gacctttgga aaagttccat ctctccaagc ttcatgtttc tcatttgtat aaaaggaaaa 540

aggtatgaac accccccttg cagggtgtta ggaagattaa 580

<210> 102
<211> 565
<212> DNA
<213> homo sapiens

<400> 102
ttaagccagt gccttgcagc ctaggcgcgg ggcttttcca ccggttctca gttccgcttt 60

agtccagaga gaagacacta cgcttcgggg ttcaggtgag agccagagga agctggggtg 120

ctcgtggaag aaggggacgc aggaggaagc gaagagtaga gaccctggat gcgtcccggc 180

cgacgcaggt ggtgggccag gagagcccgc agggcggaag agaaactgac cacttaggaa 240

ggggcgccga ggccacgggg gaggtgaggc agcgttaggt gtggtgagca tgccctggag 300

ggactccatt tatttctacc tcggttacta cctccaggcc ccagaacctc cctggcctgg 360

tctggcggtt tgggtttgca gaactgcccg ccagagagcg taggcgaggg tgaaggagtc 420

cgggagaact cttggaaaga gactacttta tgggtagagg gagtagccaa agtgaagctc 480

ccccagcagc tgggctgcac cggcgcccag cctggccacg ccactgggga gctggacccc 540

tggggctcct cactctcctg tttaa 565

<210> 103
<211> 957
<212> DNA
<213> homo sapiens

<400> 103
ttaagggaac tccaagtttc gctttcttgc cctctgcggg cttggaaagg ccgccctcca 60

tcctctgcca ccccatttca acccccgttt ccacctcccc gctgccccgc gcccaacaga 120

tctccggctc cgcacccgct ggggctgcta aactgttgtt gtattcccgc cgagggcgcc 180

attgaggtgc agattgggac ctgccggctc tggactgccg cccccggtgt aggcgctgat 240

gaaaggcccg ggcgagcgcc agggtcgcct ctggagccag ccgagctgca tttatgccag 300

cgtcattacc acgctaagtc gcttcattgc atgtcaatgc tccggcgggg ccagaacccc 360

gggacagcag cgccaggcct ggggttggag gtcggagatc caggcctgac gtgggggca 420

taaaaagggc gtcgcgggga agagggagta tttgtgtgag agacagagtc acaaagagaa 480

agagacagtg aggggccaga acgactctct tttctccgat tgtcaatgcc cagtgggagc 540

cgggagccca acaggcccag cccatcagat tcggcccctc cgggccccaa atccgctcgc 600

cccacccgag atccaggcct ccagccactt gcctaactgt gagcccgcaa gagccaggcc 660

cgcggctccc tccttcctcc tcctgcggca gtctcgcggc tttcaaacct tagtcgaacc 720

cacagaaggc ccagtcccag gccaaaccta ctcaacaggc accttctcac ggcctaggaa 780

ttctgcagcg aaattcactg gaatttgagg agaaaaccca aagactgctc cgaaaggact 840

cccccagtct tcagcctcca aataagagag ttaggaaggg cctttagccc acagggtcag 900

gaagggaaag gtgtggattc ccgggatttg tctctggtat gggatttgca tccttaa 957

<210> 104
<211> 529
<212> DNA
<213> homo sapiens

<400> 104
ttaaaggcgc cgggcttagc cgggaagtgt atggcgaccc agaacacgga gcggagaagg 60

cctcagggga cacgaggcta ccgccttagt cctcgggcct gcgctccgag cggttagggt 120

gcgtacggat gggctccggg atgttagtgg agaggtgaca ggagtctacc atcccacggc 180

caagcgtgtg aggcctccgg cgggcggggg tcccccccgg ggcagggcag ggggagagtc 240

aggcatgtct ctaacgggct ccctagtaga accgctagga aagtggcctt caaccctagg 300

cacctttgtg gctttcaggt agttgggcga ctaaaaattc acttttgcct gctgtgaaat 360

gggacaggtg gttgtcaggt tagagactgt gcccacgaag tctcgtttgg cgggccggga 420

ctgacgtgga cccgggccct cctcggaagc ttgggaacct gtgagcccct cagctcctcg 480

cctcactttg cctgtttgaa aaaaggagtt gtttgagagg aagagttaa 529

<210> 105
<211> 814
<212> DNA
<213> homo sapiens

<400> 105
ttaatacatt tcaaaggata aaattagaac acagggaatc aacttcctgt ttacagaaat 60

gacttcgcag aaaactctga aattactcca caagtgggtg ggcggctacc ctgagaaaat 120

tcatgtcatc gatccgcaac tgacacagtg gagtctcccg cactgcagat ctcatctcaa 180

agccagagct ggcctcaaac tgcttttcca agcctggact gcgagggctg ccgacccggg 240

cccaccccgc acaggcgctc agaatgttcg atacgtgcgc ctccttacac ccctgcctca 300

ccaggccgag ctcatggaaa tccgtgggct tcgaccctcc gccgcacgtt gtcccttcac 360

actggtccta tagagccgat cctaattccc atggcctgac agtacaaccg catggagatg 420

cacgtctcta agacgcctca ccactgccta agaagcaatc aaggtgacgc gcccaccgga 480

aacggaagta gtcgactcga ggtgtgggac tacctttccc agaaccctcc gcagcgcagt 540

ccgatcctag gggcgatggt gtcgtaaatg cgcgtccacc tcgagtcacg cccactgcga 600

ggcggcggga ggcggcatgc aaggtgtttc tgagttgaaa agaatgtctt tactcagtac 660

taacatattc gcgttgtagc aaacctgact atattctcaa acaagcccat catcccatca 720

ccagggatat aaagcattat tgattttctg gtgtaaaagc caactaaata tttatttcaa 780

aagtagttca tattacatgt aaagaatggt ttaa 814

<210> 106
<211> 804
<212> DNA
<213> homo sapiens

<400> 106
ttaaaaatcc aactaagcta ctttggaaag ggtgattcat aaactctgca gcatctctca 60

aaactccttc gtgacaggca gctgtcatgg aatgtggcca gagcctgaga caagattatc 120

aaatcagctg tgatattctg tgagagatgg agctacttag ggccgcatag attcggaatc 180

cagccaacta tggaattcca agaccctcgc tccctcgctt tccgaagtca aatctaacat 240

ggatttggcc ccaacagtgg gcaaggtcgg ggcatgaggt caggaaagga gaaacatcgg 300

ttttcgatct aataatggct gggctggagc ttgttccctg tgtccaaagg gcgtgcgtac 360

tagcacaagc gttgagaaag gtctggaccc gatgtctgca ggaagtgtca ggacctccct 420

gttctgggga ccttcccagc ccacaccagg tccgtgctct ctagattccc ccaacctcgc 480

tccacaaccc ggaaggagct caggccggac ttggcgtgag ggtccctgcg gggctgggtc 540

acctacccgt ccgcgatcac cccacagggg ctgttagtaa tctccctgct tcctaggccg 600

ttgagaagca ctcaggtcac ccactctcgg tgaccagagc ggtcagtctt tccccgccat 660

gaaatgtgag ttcgggtggc agccgccgcg cccctgcagt gtcccctgca catctccggt 720

ctcggtctcg cctttctcag ctccgcgccg ccttttcctg ggcgccgcag gactcataga 780

agtgaggcgg ggccacagat ttaa 804

<210> 107
<211> 631
<212> DNA
<213> homo sapiens

<400> 107
ttaacagcgt cactggttct gcccgttaga taacagtacc acctctcgtc ctagtcgagc 60

taaccaaaaa tgtgtccaga cattgccaga ggaccgctgg ggggtgaaat ctctcctttt 120

ttcttccctc tggttgagat cctctggctg cgtcacattt tcaccgggcc tcctatgtaa 180

actccctgta gacggagccg agtgtcttag tcatcgcgga gccccaggtg ctcagcacgg 240

cgccaggtac gtggtgggcg cctaatattt ggcagaggaa taaatgacgt catgctgctg 300

tgtgccgggc gctgtactcg gaagtgggtt gttattggta tggtagttgc aatgcgaggc 360

tgtcacatcc ttcacttgtc gcattgtagt cccgctccgg ctgtattgtc atttcctgga 420

ccctctgtga gcgtgaggac cctttcaggc ctgtccacgc ctggccccgc agctcatggc 480

ccctggtctc gttccagctc attgtagttg ctccgtcact tatagaatga atgaatgaat 540

gcaacacagg gcagtctgtg tgcaaaggag tgctttggga tcaagcagag catcacacca 600

gaagtgacag ttgaaaggag tcaggcttta a 631

<210> 108
<211> 628
<212> DNA
<213> homo sapiens

<400> 108
ttaatttatc atgtgacgtt ttatttcttt tgagtttagg gatccacagc ctgagacccg 60

gcgagcctcg ctgcccttgc gagagccatg gattcgcatt tgcgcagagt cggggtctgt 120

tgctacgggt cccctgggct cctttgaatc gctttggcag ctccctcggt ttagagctac 180

agggttttct ccctcccctg agactctaac gcgtgcacca actcgcgcct agagggcgtg 240

tatgacccca gctcagttct aagccgaggg gctgctgccg gacattctag ggtctgatct 300

ggcgggctcg aagggaggac agtcactgcg acccggaaag ctccgcgctt acgacgccca 360

cccagaaaat ccggatcaca tttcccgtgg tccaccgcgc atctaggagg ttacctgcgt 420

ccaggcggtg acttgcgagg gaccaccttt cccagggtcc acggcgcatt taggtggtgg 480

cttgctctgg actacatttc ccagggtcca ccgcggatct aggaggaggc ttgagatgca 540

gcctcccagc ttcgaggcta ccacctgccc gaattggtgc tttggcagag tctctggtgc 600

ccgggtgaat gggttcaggg tctcttaa 628

<210> 109

<211> 889
<212> DNA
<213> homo sapiens

<400> 109
ttaaaaaaaa aaaaaggcac gcataacagt gaatgcaaaa catccgcaat ttcagggggt 60

cggagacccc tgcgcaccca cctaaggggt ccatgcacct gggctatgag gctggaagca 120

gaaagggggc gccagaaagc gcacgccgcg agggccggcg ggaaatgtag tctcacgccg 180

gtaaagccac agagcgacga tgaggcgaga cgtctgcgtt cttctcaggc ctgagaccca 240

accggcccgg gcaccagctc ctgctggacc ccagagcgcc tggaaagctc cggaacctct 300

cacctgccgc ctcctcgggt ccaagtgcca ccgcggccgc gccccggca gcccagggcg 360

cgcttccacc acggtaccgg tggattcgcc gtgcgcagcc ggaagatggc gcagacgcac 420

aaagcacacc gatgctgcgc catgataggg ccggcgccgc agtgtcttcc gggaaacata 480

gtctttaggc gtaaaggcag cagcccggcc ttgaagccgg atctcgcgat gtttcagggt 540

gagccggacg caggcgtgcc tgcgcagtgc gcggaggagt gctgttcgtg tgttcgagtc 600

cctgggttgc ttcctggggt ctgtggtgct gggtgtgcta tctgcgtgtg attctctagc 660

gagagattgt gggcgagtga ccgagtgggc aaggggccgt cactgtgtgt gcgtgatttt 720

gacagtgtgt ggtggtagct tctgactccg cgtgggtcgt tgaatgtatg actgggaccg 780

tttagcggtg gatacacaac tgtgtgcggc tgtggaagat tgcctcatcc gttacgttcc 840

catttccgtg gaacaacagt tttaccagaa ggtttcgaag gaaccttaa 889

<210> 110
<211> 785
<212> DNA
<213> homo sapiens

<400> 110
ttaaaagtcc ttaccgcaag gtggattccc gcccggggag cctcccaacc tcgcccccgg 60

cccctgaagc gcagcgccgc agccccagtc ccggcggggg aggccgcgtc ctgtactggg 120

tcctgggacc cttgagaccc cacacttcta ataattcagc cccacccttt tcctccttga 180

tccgggcctt cctactcccc aggcctcccc ttcgcagcca tcgctactcc cgacccccgg 240

gtgtggggac gtgacttctt ctgtcttagg acattcagga ttcttcgtcc caaattccat 300

cccactaaaa atgtgctctt ggcggaggtg ggcaccttat tgtactcccc tgcctatgga 360

tgtgtgggga aagatggtga aagagtggca ggaaatagaa aaattttgaa aaagaagcat 420

gagggagatc cataagcaga cggcagagag tagctgagag atttacagag acagtgaatg 480

aagccgagaa tgtagcaggg ggagaaaagt aactggagaa atgtagagaa aagctgggtc 540

tccagagaga tgaagatgaa gcaagattgg gagaggggca gaaaggggga ggctcctcag 600

acagagtagg ggagaggagg agggatttgg agccagggca gacagagcag catggtgctg 660

ggacagcaag agggggcagc tgatgacaag gagagcagag ggagaaccac agcatgggga 720

ggcctgggat ccgggggggc cagagagtgg ggacaagaga gtgtagcagg gaagaggaaa 780

tttaa 785

<210> 111
<211> 205
<212> DNA
<213> homo sapiens

<400> 111
ttaagctccg agaatccccc ctccccgcct gtaagtgtct ttggggtggt ggaagcggag 60

acctctgcca cactgggggc tgcctgatgt ccggtccggg aaggatgccc tgctgtgttt 120

gctcgggaca ggtaagagtt tgtggacctc gatggtctcc aaaaaattct cccctagtgg 180

tccagagtag gagggaaagg gttaa 205

<210> 112
<211> 4598
<212> DNA
<213> Homo sapiens

<220>
<221> CDS
<222> (851)..(1933)

<400> 112
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccag tgaattttaa cgttgcagga 420

caggatgtgg tgcccgatgt gactagctct ttgctgcagg ccgtcctatc ctctggttcc 480

gataagagac ccagaactcc ggccccccac cgcccaccgc cacccccata catatgtggt 540

acgcaagtaa gagtgcctgc gcatgcccca tgtgccccac caagagtttt gcatcccata 600

caagtcccca aagtggagaa ccgaaccaat tcttcgcggg cagaacaaaa gcttctgcac 660

acgtctccac tcgaatttgg agccggccgg cgtgtgcaaa agaggtgaat cgaacgaaag 720

```
acccgtgtgt aaagccgcgt ttccaaaatg tataaaaccg agagcatctg gccaatgtgc     780

atcagttgtg gtcagcagca aaatcaagtg aatcatctca gtgcaactaa aggggggatc     840

cgatctcaat atg aag tta tgc ata tta ctg gcc gtc gtg gcc ttt gtt        889
           Met Lys Leu Cys Ile Leu Leu Ala Val Val Ala Phe Val
           1               5                   10

ggc ctc tcg ctc ggg aga tct cca tgg ccc ggg gta cct act agc acg       937
Gly Leu Ser Leu Gly Arg Ser Pro Trp Pro Gly Val Pro Thr Ser Thr
    15                  20                  25

gag agc ggg aag agg atg gat tgc ccg gcc ctc ccc ccc gga tgg aag       985
Glu Ser Gly Lys Arg Met Asp Cys Pro Ala Leu Pro Pro Gly Trp Lys
30                  35                  40                  45

aag gag gaa gtg atc cga aaa tct ggg cta agt gct ggc aag agc gat      1033
Lys Glu Glu Val Ile Arg Lys Ser Gly Leu Ser Ala Gly Lys Ser Asp
                50                  55                  60

gtc tac tac ttc agt cca agt ggt aag aag ttc aga agc aag cct cag      1081
Val Tyr Tyr Phe Ser Pro Ser Gly Lys Lys Phe Arg Ser Lys Pro Gln
            65                  70                  75

ttg gca agg tac ctg gga aat act gtt gat ctc agc agt ttt gac ttc      1129
Leu Ala Arg Tyr Leu Gly Asn Thr Val Asp Leu Ser Ser Phe Asp Phe
        80                  85                  90

aga act gga aag atg atg cct agt aaa tta cag aag aac aaa cag aga      1177
Arg Thr Gly Lys Met Met Pro Ser Lys Leu Gln Lys Asn Lys Gln Arg
    95                  100                 105

ctg cga aac gat cct ctg gcg gcc gcg gat ccc atc gaa ggt cgt ggt      1225
Leu Arg Asn Asp Pro Leu Ala Ala Ala Asp Pro Ile Glu Gly Arg Gly
110                 115                 120                 125

ggt ggt ggt ggt gat ccc aaa tct tgt gac aaa cct cac aca tgc cca      1273
Gly Gly Gly Gly Asp Pro Lys Ser Cys Asp Lys Pro His Thr Cys Pro
                130                 135                 140

ctg tgc cca gca cct gaa ctc ctg ggg gga ccg tca gtc ttc ctc ttc      1321
Leu Cys Pro Ala Pro Glu Leu Leu Gly Gly Pro Ser Val Phe Leu Phe
            145                 150                 155

ccc cca aaa ccc aag gac acc ctc atg atc tcc cgg acc cct gag gtc      1369
Pro Pro Lys Pro Lys Asp Thr Leu Met Ile Ser Arg Thr Pro Glu Val
        160                 165                 170

aca tgc gtg gtg gtg gac gtg agc cac gaa gac cct gag gtc aag ttc      1417
Thr Cys Val Val Val Asp Val Ser His Glu Asp Pro Glu Val Lys Phe
    175                 180                 185

aac tgg tac gtg gac ggc gtg gag gtg cat aat gcc aag aca aag ccg      1465
Asn Trp Tyr Val Asp Gly Val Glu Val His Asn Ala Lys Thr Lys Pro
190                 195                 200                 205

cgg gag gag cag tac aac agc acg tac cgt gtg gtc agc gtc ctc acc      1513
Arg Glu Glu Gln Tyr Asn Ser Thr Tyr Arg Val Val Ser Val Leu Thr
                210                 215                 220
```

```
gtc ctg cac cag gac tgg ctg aat ggc aag gag tac aag tgc aag gtc      1561
Val Leu His Gln Asp Trp Leu Asn Gly Lys Glu Tyr Lys Cys Lys Val
            225                 230                 235

tcc aac aaa gcc ctc cca gcc ccc atc gag aaa acc atc tcc aaa gcc      1609
Ser Asn Lys Ala Leu Pro Ala Pro Ile Glu Lys Thr Ile Ser Lys Ala
        240                 245                 250

aaa ggg cag ccc cga gaa cca cag gtg tac acc ctg ccc cca tcc cgg      1657
Lys Gly Gln Pro Arg Glu Pro Gln Val Tyr Thr Leu Pro Pro Ser Arg
    255                 260                 265

gat gag ctg acc aag aac cag gtc agc ctg acc tgc cta gtc aaa ggc      1705
Asp Glu Leu Thr Lys Asn Gln Val Ser Leu Thr Cys Leu Val Lys Gly
270                 275                 280                 285

ttc tat ccc agc gac atc gcc gtg gag tgg gag agc aat ggg cag ccg      1753
Phe Tyr Pro Ser Asp Ile Ala Val Glu Trp Glu Ser Asn Gly Gln Pro
                290                 295                 300

gag aac aac tac aag gcc acg cct ccc gtg ctg gac tcc gac ggc tcc      1801
Glu Asn Asn Tyr Lys Ala Thr Pro Pro Val Leu Asp Ser Asp Gly Ser
            305                 310                 315

ttc ttc ctc tac agc aag ctc acc gtg gac aag agc agg tgg cag cag      1849
Phe Phe Leu Tyr Ser Lys Leu Thr Val Asp Lys Ser Arg Trp Gln Gln
        320                 325                 330

ggg aac gtc ttc tca tgc tcc gtg atg cat gag gct ctg cac aac cac      1897
Gly Asn Val Phe Ser Cys Ser Val Met His Glu Ala Leu His Asn His
    335                 340                 345

tac acg cag aag agc ctc tcc ctg tct ccg ggt aaa tgagctagag           1943
Tyr Thr Gln Lys Ser Leu Ser Leu Ser Pro Gly Lys
350                 355                 360

ggcccgcggt tcgaaggtaa gcctatccct aaccctctcc tcggtctcga ttctacgcgt    2003

accggtcatc atcaccatca ccattgagtt taaacccgct gatcagcctc gactgtgcct    2063

tctaaggcct gagctcgctg atcagcctcg atcgaggatc cagacatgat aagatacatt    2123

gatgagtttg gacaaaccac aactagaatg cagtgaaaaa aatgctttat ttgtgaaatt    2183

tgtgatgcta ttgctttatt tgtaaccatt ataagctgca ataaacaagt taacaacaac    2243

aattgcattc attttatgtt tcaggttcag ggggaggtgt gggaggtttt ttaaagcaag    2303

taaaacctct acaaatgtgg tatggctgat tatgatcagt cgacctgcag gcatgcaagc    2363

ttggcgtaat catggtcata gctgtttcct gtgtgaaatt gttatccgct cacaattcca    2423

cacaacatac gagccggaag cataaagtgt aaagcctggg gtgcctaatg agtgagctaa    2483

ctcacattaa ttgcgttgcg ctcactgccc gctttccagt cgggaaacct gtcgtgccag    2543

ctgcattaat gaatcggcca acgcgcgggg agaggcggtt tgcgtattgg gcgctcttcc    2603

gcttcctcgc tcactgactc gctgcgctcg gtcgttcggc tgcggcgagc ggtatcagct    2663
```

```
cactcaaagg cggtaatacg gttatccaca gaatcagggg ataacgcagg aaagaacatg   2723
tgagcaaaag gccagcaaaa ggccaggaac cgtaaaaagg ccgcgttgct ggcgtttttc   2783
cataggctcc gcccccctga cgagcatcac aaaaatcgac gctcaagtca gaggtggcga   2843
aacccgacag gactataaag ataccaggcg tttccccctg gaagctccct cgtgcgctct   2903
cctgttccga ccctgccgct taccggatac ctgtccgcct ttctcccttc gggaagcgtg   2963
gcgctttctc atagctcacg ctgtaggtat ctcagttcgg tgtaggtcgt tcgctccaag   3023
ctgggctgtg tgcacgaacc ccccgttcag cccgaccgct gcgccttatc cggtaactat   3083
cgtcttgagt ccaacccggt aagacacgac ttatcgccac tggcagcagc cactggtaac   3143
aggattagca gagcgaggta tgtaggcggt gctacagagt tcttgaagtg gtggcctaac   3203
tacggctaca ctagaaggac agtatttggt atctgcgctc tgctgaagcc agttaccttc   3263
ggaaaaagag ttggtagctc ttgatccggc aaacaaacca ccgctggtag cggtggtttt   3323
tttgtttgca agcagcagat tacgcgcaga aaaaaaggat ctcaagaaga tcctttgatc   3383
ttttctacgg ggtctgacgc tcagtggaac gaaaactcac gttaagggat tttggtcatg   3443
agattatcaa aaaggatctt cacctagatc cttttaaatt aaaaatgaag ttttaaatca   3503
atctaaagta tatatgagta aacttggtct gacagttacc aatgcttaat cagtgaggca   3563
cctatctcag cgatctgtct atttcgttca tccatagttg cctgactccc cgtcgtgtag   3623
ataactacga tacgggaggg cttaccatct ggccccagtg ctgcaatgat accgcgagac   3683
ccacgctcac cggctccaga tttatcagca ataaaccagc cagccggaag ggccgagcgc   3743
agaagtggtc ctgcaacttt atccgcctcc atccagtcta ttaattgttg ccgggaagct   3803
agagtaagta gttcgccagt taatagtttg cgcaacgttg ttgccattgc tacaggcatc   3863
gtggtgtcac gctcgtcgtt tggtatggct tcattcagct ccggttccca acgatcaagg   3923
cgagttacat gatcccccat gttgtgcaaa aaagcggtta gctccttcgg tcctccgatc   3983
gttgtcagaa gtaagttggc cgcagtgtta tcactcatgg ttatggcagc actgcataat   4043
tctcttactg tcatgccatc cgtaagatgc ttttctgtga ctggtgagta ctcaaccaag   4103
tcattctgag aatagtgtat gcggcgaccg agttgctctt gcccggcgtc aatacgggat   4163
aataccgcgc cacatagcag aactttaaaa gtgctcatca ttggaaaacg ttcttcgggg   4223
cgaaaactct caaggatctt accgctgttg agatccagtt cgatgtaacc cactcgtgca   4283
cccaactgat cttcagcatc ttttactttc accagcgttt ctgggtgagc aaaaacagga   4343
aggcaaaatg ccgcaaaaaa gggaataagg gcgacacgga aatgttgaat actcatactc   4403
ttcctttttc aatattattg aagcatttat cagggttatt gtctcatgag cggatacata   4463
```

tttgaatgta tttagaaaaa taaacaaata ggggttccgc gcacatttcc ccgaaaagtg 4523

ccacctgacg tctaagaaac cattattatc atgacattaa cctataaaaa taggcgtatc 4583

acgaggccct ttcgt 4598

<210> 113
<211> 361
<212> PRT
<213> Homo sapiens

<400> 113

```
Met Lys Leu Cys Ile Leu Leu Ala Val Val Ala Phe Val Gly Leu Ser
1               5                   10                  15

Leu Gly Arg Ser Pro Trp Pro Gly Val Pro Thr Ser Thr Glu Ser Gly
            20                  25                  30

Lys Arg Met Asp Cys Pro Ala Leu Pro Pro Gly Trp Lys Lys Glu Glu
        35                  40                  45

Val Ile Arg Lys Ser Gly Leu Ser Ala Gly Lys Ser Asp Val Tyr Tyr
    50                  55                  60

Phe Ser Pro Ser Gly Lys Lys Phe Arg Ser Lys Pro Gln Leu Ala Arg
65                  70                  75                  80

Tyr Leu Gly Asn Thr Val Asp Leu Ser Ser Phe Asp Phe Arg Thr Gly
                85                  90                  95

Lys Met Met Pro Ser Lys Leu Gln Lys Asn Lys Gln Arg Leu Arg Asn
            100                 105                 110

Asp Pro Leu Ala Ala Ala Asp Pro Ile Glu Gly Arg Gly Gly Gly Gly
        115                 120                 125

Gly Asp Pro Lys Ser Cys Asp Lys Pro His Thr Cys Pro Leu Cys Pro
    130                 135                 140

Ala Pro Glu Leu Leu Gly Gly Pro Ser Val Phe Leu Phe Pro Pro Lys
145                 150                 155                 160

Pro Lys Asp Thr Leu Met Ile Ser Arg Thr Pro Glu Val Thr Cys Val
                165                 170                 175

Val Val Asp Val Ser His Glu Asp Pro Glu Val Lys Phe Asn Trp Tyr
```

```
            180                     185                     190

Val Asp Gly Val Glu Val His Asn Ala Lys Thr Lys Pro Arg Glu Glu
        195                     200                     205

Gln Tyr Asn Ser Thr Tyr Arg Val Val Ser Val Leu Thr Val Leu His
    210                     215                     220

Gln Asp Trp Leu Asn Gly Lys Glu Tyr Lys Cys Lys Val Ser Asn Lys
225                     230                     235                     240

Ala Leu Pro Ala Pro Ile Glu Lys Thr Ile Ser Lys Ala Lys Gly Gln
                245                     250                     255

Pro Arg Glu Pro Gln Val Tyr Thr Leu Pro Pro Ser Arg Asp Glu Leu
            260                     265                     270

Thr Lys Asn Gln Val Ser Leu Thr Cys Leu Val Lys Gly Phe Tyr Pro
        275                     280                     285

Ser Asp Ile Ala Val Glu Trp Glu Ser Asn Gly Gln Pro Glu Asn Asn
    290                     295                     300

Tyr Lys Ala Thr Pro Pro Val Leu Asp Ser Asp Gly Ser Phe Phe Leu
305                     310                     315                     320

Tyr Ser Lys Leu Thr Val Asp Lys Ser Arg Trp Gln Gln Gly Asn Val
                325                     330                     335

Phe Ser Cys Ser Val Met His Glu Ala Leu His Asn His Tyr Thr Gln
            340                     345                     350

Lys Ser Leu Ser Leu Ser Pro Gly Lys
        355                     360


<210>  114
<211>  361
<212>  PRT
<213>  Homo sapiens

<400>  114

Met Lys Leu Cys Ile Leu Leu Ala Val Val Ala Phe Val Gly Leu Ser
1               5                   10                  15

Leu Gly Arg Ser Pro Trp Pro Gly Val Pro Thr Ser Thr Glu Ser Gly
            20                  25                  30
```

```
Lys Arg Met Asp Cys Pro Ala Leu Pro Pro Gly Trp Lys Lys Glu Glu
        35                  40                  45

Val Ile Arg Lys Ser Gly Leu Ser Ala Gly Lys Ser Asp Val Tyr Tyr
    50                  55                  60

Phe Ser Pro Ser Gly Lys Lys Phe Arg Ser Lys Pro Gln Leu Ala Arg
65                  70                  75                  80

Tyr Leu Gly Asn Thr Val Asp Leu Ser Ser Phe Asp Phe Arg Thr Gly
                85                  90                  95

Lys Met Met Pro Ser Lys Leu Gln Lys Asn Lys Gln Arg Leu Arg Asn
            100                 105                 110

Asp Pro Leu Ala Ala Ala Asp Pro Ile Glu Gly Arg Gly Gly Gly Gly
        115                 120                 125

Gly Asp Pro Lys Ser Cys Asp Lys Pro His Thr Cys Pro Leu Cys Pro
    130                 135                 140

Ala Pro Glu Leu Leu Gly Gly Pro Ser Val Phe Leu Phe Pro Pro Lys
145                 150                 155                 160

Pro Lys Asp Thr Leu Met Ile Ser Arg Thr Pro Glu Val Thr Cys Val
                165                 170                 175

Val Val Asp Val Ser His Glu Asp Pro Glu Val Lys Phe Asn Trp Tyr
            180                 185                 190

Val Asp Gly Val Glu Val His Asn Ala Lys Thr Lys Pro Arg Glu Glu
        195                 200                 205

Gln Tyr Asn Ser Thr Tyr Arg Val Val Ser Val Leu Thr Val Leu His
    210                 215                 220

Gln Asp Trp Leu Asn Gly Lys Glu Tyr Lys Cys Lys Val Ser Asn Lys
225                 230                 235                 240

Ala Leu Pro Ala Pro Ile Glu Lys Thr Ile Ser Lys Ala Lys Gly Gln
                245                 250                 255

Pro Arg Glu Pro Gln Val Tyr Thr Leu Pro Pro Ser Arg Asp Glu Leu
            260                 265                 270
```

```
Thr Lys Asn Gln Val Ser Leu Thr Cys Leu Val Lys Gly Phe Tyr Pro
        275                 280                 285

Ser Asp Ile Ala Val Glu Trp Glu Ser Asn Gly Gln Pro Glu Asn Asn
    290                 295                 300

Tyr Lys Ala Thr Pro Pro Val Leu Asp Ser Asp Gly Ser Phe Phe Leu
305                 310                 315                 320

Tyr Ser Lys Leu Thr Val Asp Lys Ser Arg Trp Gln Gln Gly Asn Val
                325                 330                 335

Phe Ser Cys Ser Val Met His Glu Ala Leu His Asn His Tyr Thr Gln
            340                 345                 350

Lys Ser Leu Ser Leu Ser Pro Gly Lys
        355                 360


<210>  115
<211>  28
<212>  DNA
<213>  artificial sequence

<220>
<223>  Primer: MBD2

<400>  115
agatgctagc acggagagcg ggaagagg                                          28


<210>  116
<211>  36
<212>  DNA
<213>  artificial sequence

<220>
<223>  Primer: MBD2

<400>  116
atcacgcggc cgccagagga tcgtttcgca gtctcg                                 36


<210>  117
<211>  20
<212>  DNA
<213>  artificial sequence

<220>
<223>  Primer: CBX6

<400>  117
aagcttccgc cattgctctg                                                   20
```

<210> 118
<211> 18
<212> DNA
<213> artificial sequence

<220>
<223> Primer: CBX6

<400> 118
tccgttcctg gacagccc 18

<210> 119
<211> 21
<212> DNA
<213> artificial sequence

<220>
<223> Primer: CDKN2B

<400> 119
ggctcagctt cattaccctc c 21

<210> 120
<211> 19
<212> DNA
<213> artificial sequence

<220>
<223> Primer: CDKN2B

<400> 120
aaagcccgga gctaacgac 19

<210> 121
<211> 22
<212> DNA
<213> artificial sequence

<220>
<223> Primer: CHI3L1

<400> 121
atcaccctag tggctcttct gc 22

<210> 122
<211> 26
<212> DNA
<213> artificial sequence

<220>
<223> Primer: CHI3L1

<400> 122
cttttatggg aactgagcta tgtgtc 26

<210> 123
<211> 21
<212> DNA
<213> artificial sequence

<220>
<223> Primer: COL14A1

<400> 123
agacgcaatg cagttccatg g 21

<210> 124
<211> 21
<212> DNA
<213> artificial sequence

<220>
<223> Primer: COL14A1

<400> 124
atctccctac accgtgaacc c 21

<210> 125
<211> 26
<212> DNA
<213> artificial sequence

<220>
<223> Primer: CYP1B1

<400> 125
tgttgaatcc gtgcttagta gagacc 26

<210> 126
<211> 27
<212> DNA
<213> artificial sequence

<220>
<223> Primer: CYP1B1

<400> 126
cagagtagca ttcagaaagg cagatgg 27

<210> 127
<211> 22
<212> DNA
<213> artificial sequence

<220>
<223> Primer: CYP27B1

<400> 127
catccgttct ctctggctgt cc 22

<210> 128
<211> 22
<212> DNA
<213> artificial sequence

<220>
<223> Primer: CYP27B1

<400> 128
ctgtcgaggc tacacgagct gc 22

<210> 129
<211> 23
<212> DNA
<213> artificial sequence

<220>
<223> Primer: DMRT2

<400> 129
cacgtttttg ctagaggtga ggg 23

<210> 130
<211> 24
<212> DNA
<213> artificial sequence

<220>
<223> Primer: DMRT2

<400> 130
tcctccatcc gtactgacat aggg 24

<210> 131
<211> 19
<212> DNA
<213> artificial sequence

<220>
<223> Primer: ESR1

<400> 131
gactgcactt gctcccgtc 19

<210> 132
<211> 21
<212> DNA
<213> artificial sequence

<220>
<223> Primer: ESR1

<400> 132
aagagcacag cccgaggtta g 21

<210> 133
<211> 22
<212> DNA
<213> artificial sequence

<220>
<223> Primer: FARP1

<400> 133
gctccgtaga gttcccgaaa cc 22

<210> 134
<211> 22
<212> DNA
<213> artificial sequence

<220>
<223> Primer: FARP1

<400> 134
agcgaatccc atgacagttc cc 22

<210> 135
<211> 27
<212> DNA
<213> artificial sequence

<220>
<223> Primer: FNBP1

<400> 135
atccaaaggt ctgcacaaat gttcctg 27

<210> 136
<211> 24
<212> DNA
<213> artificial sequence

<220>
<223> Primer: FNBP1

<400> 136
cgagggagaa agataagctg tggg 24

<210> 137
<211> 24
<212> DNA
<213> artificial sequence

<220>
<223> Primer: HOXD10

<400> 137
tctatagtga cgctaccttt cccg 24

<210> 138
<211> 25
<212> DNA
<213> artificial sequence

<220>
<223> Primer: HOXD10

<400> 138
cttgagagga caacgacatt taggg 25

<210> 139
<211> 23
<212> DNA
<213> artificial sequence

<220>
<223> Primer: JUN

<400> 139
aggagttagt gtgacagggt cgc 23

<210> 140
<211> 25
<212> DNA
<213> artificial sequence

<220>
<223> Primer: JUN

<400> 140
ccaaatcgca ctcttatatc ctggc 25

<210> 141
<211> 19
<212> DNA
<213> artificial sequence

<220>
<223> Primer: JUN(p)

<400> 141
attggctcgc gtcgctctc 19

<210> 142
<211> 21
<212> DNA
<213> artificial sequence

<220>
<223> Primer: JUN(p)

<400> 142
ggagcattac ctcatcccgt g 21

<210> 143
<211> 28
<212> DNA
<213> artificial sequence

<220>
<223> Primer: KLF5

<400> 143
agacacttca tttagtagct ctttggcg 28

<210> 144
<211> 20
<212> DNA
<213> artificial sequence

<220>
<223> Primer: KLF5

<400> 144
gccctctcac agcaagaccc 20

<210> 145
<211> 21
<212> DNA
<213> artificial sequence

<220>
<223> Primer: KLF11

<400> 145
gacagcgggc tagatgtctc c 21

<210> 146
<211> 19
<212> DNA
<213> artificial sequence

<220>
<223> Primer: KLF11

<400> 146
gtcaggggaa gccgaaacg 19

<210> 147
<211> 23
<212> DNA
<213> artificial sequence

<220>
<223> Primer: KLF11(p)

<400> 147
gttgaggcct ctaggtgggt ctc 23

<210> 148
<211> 23
<212> DNA
<213> artificial sequence

<220>
<223> Primer: KLF11(p)

<400> 148
ccacgcttat aggaacctcc tgc 23

<210> 149
<211> 26
<212> DNA
<213> artificial sequence

<220>
<223> Primer: LDLR

<400> 149
gggtacaaat aatcactcca tccctg 26

<210> 150
<211> 22
<212> DNA
<213> artificial sequence

<220>
<223> Primer: LDLR

<400> 150
taaatccctc agactcctcc cg 22

<210> 151
<211> 23
<212> DNA
<213> artificial sequence

<220>
<223> Primer: MAFB

<400> 151
tgtgcagact atgtatggct ccg 23

<210> 152
<211> 21
<212> DNA
<213> artificial sequence

<220>
<223> Primer: MAFB

<400> 152
aaacactctg ggagccacag g 21

<210> 153
<211> 22
<212> DNA
<213> artificial sequence

<220>
<223> Primer: MAFB (p)

<400> 153
tcgaggtgtg tcttctgttc gg 22

<210> 154
<211> 21
<212> DNA
<213> artificial sequence

<220>
<223> Primer: MAFB (p)

<400> 154
gacctgctca agttcgacgt g 21

<210> 155
<211> 27
<212> DNA
<213> artificial sequence

<220>
<223> Primer: MLF1

<400> 155
aaatctgata ggcttcatcc catttcc 27

<210> 156
<211> 25
<212> DNA
<213> artificial sequence

<220>
<223> Primer: MLF1

<400> 156
gtcctgtatc cgaaacattc tctgg 25

<210> 157
<211> 26
<212> DNA
<213> artificial sequence

<220>
<223> Primer: PAX9

<400> 157
ctctgcttgt cataactgca actcgg 26

<210> 158
<211> 25
<212> DNA
<213> artificial sequence

<220>
<223> Primer: PAX9

<400> 158
tgatgactgt ggatgggagg atagg 25

<210> 159
<211> 22
<212> DNA
<213> artificial sequence

<220>
<223> Primer: PDE4B

<400> 159
caggaggtct gtgaggtagg tg 22

<210> 160
<211> 26
<212> DNA
<213> artificial sequence

<220>
<223> Primer: PDE4B

<400> 160
tgtgtagtag gttgtaactg ctgagg 26

<210> 161
<211> 22
<212> DNA
<213> artificial sequence

<220>
<223> Primer: PFC

<400> 161
cgttacgggt ttcctgattg gc 22

<210> 162
<211> 22
<212> DNA
<213> artificial sequence

<220>
<223> Primer: PFC

<400> 162
ggaatctagg gaggtccagg ag 22

<210> 163
<211> 22
<212> DNA
<213> artificial sequence

<220>
<223> Primer: PLA2G7

<400> 163
gtgctggtgt catttctccc tg 22

<210> 164
<211> 24
<212> DNA
<213> artificial sequence

<220>
<223> Primer: PLA2G7

<400> 164
tctagctcca tttctcctca gacc 24

<210> 165
<211> 20
<212> DNA
<213> artificial sequence

<220>
<223> Primer: RAB3C

<400> 165
tgagggatcg ggctattcgc 20

<210> 166
<211> 22
<212> DNA
<213> artificial sequence

<220>
<223> Primer: RAB3C

<400> 166
gccaagagag gagatcaatg cc 22

<210> 167
<211> 23
<212> DNA
<213> artificial sequence

<220>
<223> Primer: RAX

<400> 167
catggacacc cgtgaattcc gag 23

<210> 168
<211> 21
<212> DNA
<213> artificial sequence

<220>
<223> Primer: RAX

<400> 168
aggtaaagcg cccaggttga g 21

<210> 169
<211> 22
<212> DNA
<213> artificial sequence

<220>
<223> Primer: RGMA

<400> 169
aaagaccgta tcgcactccc tc 22

<210> 170
<211> 21
<212> DNA
<213> artificial sequence

<220>
<223> Primer: RGMA

<400> 170
cgcagagact ggaaagaacc g 21

<210> 171
<211> 23
<212> DNA
<213> artificial sequence

<220>
<223> Primer: RPIB9

<400> 171
aaagactcta cactggcacc acg 23

<210> 172
<211> 21
<212> DNA
<213> artificial sequence

<220>
<223> Primer: RPIB9

<400> 172
tagtgccgac atttcttgcc c 21

<210> 173
<211> 27
<212> DNA
<213> artificial sequence

<220>
<223> Primer: RPP30

<400> 173
agcttctaag ttactatcag cccttcc 27

<210> 174
<211> 25
<212> DNA
<213> artificial sequence

<220>
<223> Primer: RPP30

<400> 174
gtattgttcc aacactccca cgtcc 25

<210> 175
<211> 21
<212> DNA
<213> artificial sequence

<220>
<223> Primer: SETBP1

<400> 175
tgtgcgtttc tagaggagcc g 21

<210> 176
<211> 22
<212> DNA
<213> artificial sequence

<220>
<223> Primer: SETBP1

<400> 176
aaatcgatac cgaagggttc cc 22

<210> 177
<211> 23
<212> DNA
<213> artificial sequence

<220>
<223> Primer: SLITRK3

<400> 177
tacctcttac aacaccagcg agc 23

<210> 178
<211> 26
<212> DNA
<213> artificial sequence

<220>
<223> Primer: SLITRK3

<400> 178
ggatcagtta ggtgtaagga cgttgg 26

<210> 179
<211> 23
<212> DNA
<213> artificial sequence

<220>
<223> Primer: SNRNP

<400> 179
tacatcaggg tgattgcagt tcc 23

<210> 180
<211> 24
<212> DNA
<213> artificial sequence

<220>
<223> Primer: SNRNP

<400> 180
taccgatcac ttcacgtacc ttcg 24

<210> 181
<211> 20
<212> DNA
<213> artificial sequence

<220>
<223> Primer: SSIAH2

<400> 181
ctgagacact ccgctccagc 20

<210> 182
<211> 24
<212> DNA
<213> artificial sequence

<220>
<223> Primer: SSIAH2

<400> 182
tgttattggc tgtctctgca cctc 24

<210> 183
<211> 21
<212> DNA
<213> artificial sequence

<220>
<223> Primer: TGIF

<400> 183
gtccgggaag gaactgtgct c 21

<210> 184
<211> 23
<212> DNA
<213> artificial sequence

<220>
<223> Primer: TGIF

<400> 184
ctgctcggga cagaagagaa cac 23

<210> 185
<211> 23
<212> DNA
<213> artificial sequence

<220>
<223> Primer: TLR2

<400> 185
tgtgtttcag gtgatgtgag gtc 23

<210> 186
<211> 20
<212> DNA
<213> artificial sequence

<220>
<223> Primer: TLR2

<400> 186
cgaatcgaga cgctagaggc 20

<210> 187
<211> 25
<212> DNA
<213> artificial sequence

<220>
<223> Primer: ZFP36L1

<400> 187
aaacattgtc ccgagactca cttcc 25

<210> 188
<211> 20
<212> DNA
<213> artificial sequence

<220>
<223> Primer: ZFP36L1

<400> 188
gtctgtccag cggcattacc 20

<210> 189
<211> 22
<212> DNA
<213> artificial sequence

<220>
<223> Primer: ZNF516

<400> 189
caggtgatga tggaacccac tc 22

<210> 190
<211> 22
<212> DNA
<213> artificial sequence

<220>
<223> Primer: ZNF516

<400> 190
tgctgccctt cacttttcta cg 22

<210> 191
<211> 22
<212> DNA
<213> artificial sequence

<220>
<223> Primer: ZNF516 (p)

<400> 191
ccctcagtgt ggcagaactt tg 22

<210> 192
<211> 18
<212> DNA
<213> artificial sequence

<220>
<223> Primer: ZNF516 (p)

<400> 192
cccagcctgg aaatggtc 18

<210> 193
<211> 18
<212> DNA
<213> artificial sequence

<220>
<223> Primer: MAFB

<400> 193
gagccggaga gagagacg 18

<210> 194
<211> 21
<212> DNA
<213> artificial sequence

<220>
<223> Primer: MAFB

<400> 194
aggagtctcc agatggcctt g 21

<210> 195
<211> 20
<212> DNA
<213> artificial sequence

<220>
<223> Primer: JUN

<400> 195
cggcggtaaa gaccagaagg 20

<210> 196
<211> 19
<212> DNA
<213> artificial sequence

<220>
<223> Primer: JUN

<400> 196
cgcccaagtt caacaaccg 19

<210> 197
<211> 22
<212> DNA
<213> artificial sequence

<220>
<223> Primer: KLF11

<400> 197
acctacttca aaagttccca cc 22

<210> 198
<211> 21
<212> DNA
<213> artificial sequence

<220>
<223> Primer: KLF11

<400> 198
catgaaacgt cggtcacaca c 21

<210> 199
<211> 25
<212> DNA
<213> artificial sequence

<220>
<223> Primer: SSIAH2

<400> 199
gtttcagcac tacaaggcta aacgg 25

<210> 200
<211> 20
<212> DNA
<213> artificial sequence

<220>
<223> Primer: SSIAH2

<400> 200
aagctgcctt gctctggagc 20

<210> 201
<211> 24
<212> DNA
<213> artificial sequence

<220>
<223> Primer: ZNF516

<400> 201
gttctgaagt tcataccacc tccg 24

<210> 202
<211> 21
<212> DNA
<213> artificial sequence

<220>
<223> Primer: ZNF516

<400> 202
tcagaggcac tgtctggacg g 21

## Description

The present invention relates to an in vitro method of diagnosing a predisposition of cancer or cancer comprising determining the methylation status of at least one nucleic acid molecule having a nucleotide sequence selected from any one of KLF11 (SEQ ID NO: 1), PLA2G7 (SEQ ID NO: 2), PFC (SEQ ID NO: 3) and MAFB (SEQ ID NO: 4), an allelic variant, a portion or the complementary strand of any of said nucleotide sequences, said method preferably further comprising determining the methylation status of a nucleic acid molecule having a nucleotide sequence selected from any one of SEQ ID NOs: 5-111, an allelic variant, a portion or the complementary strand of any of said nucleotide sequences. Further, the present invention relates to a kit comprising a polypeptide capable of binding methylated DNA and probes, primers and/or aptamers specific for at least one nucleic acid molecule having a nucleotide sequence of any one SEQ ID NOs: 1-111, an allelic variant, a portion or the complementary strand of any of said nucleotide sequences. A diagnostic composition and uses of at least one nucleic acid molecule having a nucleotide sequence of any one of SED ID NOs: 1-111, an allelic variant, a portion or the complementary strand of any of said nucleotide sequences is also envisaged.

The transcriptional state of a gene is largely determined by its local epigenetic code. Pathological changes in chromatin structure or DNA methylation that lead to abnormal expression or repression of genes are now being recognized as important contributing factors in developmental diseases, cancer and aging (1-5).

In cancer the epigenetic silencing of tumor-suppressor genes appears to be a common, non-random and tumor-type specific event that often coincides with the aberrant methylation of CpG dinucleotides in so called CpG islands. These regions of high CpG content are mostly un-methylated in normal cells and frequently contain gene promoters and transcription start sites (2;6-8).

Hypermethylation of CpG islands appears to be a tumor-type specific event (9;10) and current efforts are concentrating on finding ways to exploit the diagnostic and therapeutic implications of the abnormalities (11;12). A comprehensive knowledge of the methylation profile of a given tumor may provide important information for risk assessment, diagnosis, monitoring and treatment (6;13).

In view of the foregoing, it is evident that methylation of CpG dinucleotides is an important epigenetic mechanism for controlling transcriptional activity of a cell. Generally, methylation of CpG dinucleotides correlates with transcriptional inactivity. Yet, during normal or degenerated differentiation processes the methylation pattern of genloci may change. Accordingly, the reversal of normal methylation patterns during tumorigenesis can lead to an abnormal repression (or activation) of genes, for instance, tumor suppressor genes or oncogenes, respectively, and, thus, leading to tumorigenesis.

At present, many techniques are known which are used for the detection of the CpG methylation of single known candidate gene loci (14). Commonly used assays rely on two basic approaches to distinguish methylated and unmethylated DNA: digestion with methylation-sensitive restriction enzymes or bisulfite treatment of DNA. Methods allowing the analysis of the CpG methylation throughout the genome are less well established. In most techniques, e.g. Restriction Landmark Genomic Scanning (RLGS) (32) or Methylated CpG Island Amplification (MCA) (33;34), methylation-sensitive restriction enzymes are used as a component of the method. Here, a major disadvantage is that the analyses merely inform on the methylation status of the cytosine residues which have been recognized by the methylation-sensitive restriction enzymes used. Also, the selection of the restriction enzymes automatically limits the number of detectable sequences - a global analysis of CpG methylation is therefore not achieved.

So far, some markers which are subject to methylation and, thus, being potentially of clinical interest when assessing a predisposition of cancer or when diagnosing cancer have been suggested. However, many of the markers suggested in the prior art could not satisfy the clinicians' expectations. A reason could be that the way these markers have been identified was not based on techniques which allow the reliable detection of methylated DNA. Another explanation could be that the identification of relevant marker genes requires means and methods to detect hypermethylation on a genome-wide level which has so far not been sufficiently approached by the prior art.

Namely, the investigation of aberrant CpG-island methylation has primarily taken a candidate gene approach. However, as stated above, a reliable genome-wide survey of the methylation status may lead to the identification of reliable markers.

Thus, there is still a need for further means and methods for diagnosing cancer or a predisposition of cancer. Accordingly, the technical problem underlying the present invention is to comply with the needs described above.

The solution to this technical problem is achieved by providing the embodiments characterized in the claims.

In a first aspect the present invention relates to an in vitro method of diagnosing cancer or a predisposition of cancer comprising determining the methylation status of at least one nucleic acid molecule having a nucleotide sequence selected from any one of KLF11 (SEQ ID NO: 1), PLA2G7 (SEQ ID NO: 2), PFC (SEQ ID NO: 3) and MAFB (SEQ ID NO: 4), an allelic variant, a portion or the complementary strand of any of said nucleotide sequences.

In a further aspect, the method of the present invention further comprises determining the methylation status of at least one nucleic acid molecule having a nucleotide sequence selected from any one of SEQ ID NOs: 5-16, 17-48 or 49-111, an allelic variant, a portion or the complementary strand of any of said nucleotide sequences.

The nucleic acid molecules having a nucleotide sequence of any one of SEQ ID NOs: 1-111 are shown in Table 1.

Table 1

| SEQ ID NO.: | GENE_SYMBOL | GENE_NAME | GB_ACC | CPG_ISLAND_POSITION |
|---|---|---|---|---|
| 1 | **KLF11** | Kruppel-like factor 11 | NM_003597 | chr2:10,133,187-10,133,761 |
| 2 | **PLA2G7** | phospholipase A2, group VII (platelet-activating factor acetylhydrolase, plasma) | NM_005084 | chr6:46,811,258-46,811,670 |
| 3 | **PFC** | properdin P factor, complement | NM_002621 | chrX:47,239,036-47,239,849 |
| 4 | **MAFB** | v-maf musculoaponeurotic fibrosarcoma oncogene homolog B (avian) | NM_005461 | chr20:38,753,131-38,753,612 |
| 5 | **CXCL5** | chemokine (C-X-C motif) ligand 5 | NM_002994 | chr4:75,228,676-75,229,883 |
| 6 | **CYP1B1** | cytochrome P450, family 1, subfamily B, polypeptide 1 | NM_000104 | chr2:38,212,244-38,212,739 |
| 7 | **FOXD2** | forkhead box D2 | NM_004474 | chr1:47,610,135-47,611,271 |
| 8 | **FNBP1** | formin binding protein 1 | NM_015033 | chr9:129,885,382-129,886,027 |
| 9 | **GDNF** | glial cell derived neurotrophic factor | NM_000514 | chr5:37,874,055-37,874,661 |
| 10 | **HCN4** | hyperpolarization activated cyclic nucleotide-gated potassium channel 4 | NM_005477 | chr15:71,448,505-71,449,432 |
| 11 | **LBX1** | ladybird homeobox homolog 1 (Drosophila) | NM_006562 | chr10:102,972,867-102,973,964 |
| 12 | **PAX9** | paired box gene 9 | NM_006194 | chr14:36,197,382-36,198,102 |
| 13 | **KCNAB1** | potassium voltage-gated channel, shaker-related subfamily, beta member 1 | NM_172159 | chr3:157,492,303-157,492,809 |
| 14 | **SHH** | sonic hedgehog homolog (Drosophila) | NM_000193 | chr7:155,104,072-155,104,608 |
| 15 | TGIF | TGFB-induced factor (TALE family homeobox) | NM_174886 | chr18:3,438,088-3,438,679 |
| 16 | **ZNF516** | zinc finger protein 516 | XM_496278 | chr18:72,333,079-72,334,081 |
| 17 | **NT5C1A** | 5'-nucleotidase, cytosolic IA | NM_032526 | chr1:39,806,031-39,808,211 |

| | | | | |
|---|---|---|---|---|
| 18 | **BMP4** | bone morphogenetic protein 4 | NM_130850 | chr14:53,492,075-53,492,860 |
| 19 | **CNN1** | calponin 1, basic, smooth muscle | NM_001299 | chr19:11,510,084-11,510,798 |
| 20 | **COL14A1** | collagen, type XIV, alpha 1 (undulin) | NM_021110 | chr8:121,206,633-121,207,133 |
| 21 | **CYP27B1** | cytochrome P450, family 27, subfamily B, polypeptide 1 | NM_000785 | chr12:56,446,586-56,447,158 |
| 22 | **DLX3** | distal-less homeo box 3 | NM_005220 | chr17:45,426,362-45,428,139 |
| 23 | **DMRT2** | doublesex and mab-3 related transcription factor 2 | NM_181872 | chr9:1,037,501-1,038,158 |
| 24 | **DUOX2** | dual oxidase 2 | NM_014080 | chr15:43,192,509-43,193,158 |
| 25 | **ELAVL2** | ELAV (embryonic lethal, abnormal vision, Drosophila)-like 2 (Hu antigen B) | NM_004432 | chr9:23,812,342-23,812,662 |
| 26 | **EPHA10** | EPH receptor A10 | NM_173641 | chr1:37,900,221-37,900,916 |
| 27 | **ERAS** | ES cell expressed Ras | NM_181532 | chrX:48,441,919-48,442,547 |
| 28 | **FAM5B** | family with sequence similarity 5, member B | NM_021165 | chr1:173,871,729-173,872,809 |
| 29 | **FARP1** | FERM, RhoGEF (ARHGEF) and pleckstrin domain protein 1 (chondrocyte-derived) | NM_001001715 | chr13:97,591,780-97,592,818 |
| 30 | **FGF12** | fibroblast growth factor 12 | NM_021032 | chr3:193,608,355-193,609,319 |
| 31 | **FOXA1** | forkhead box A1 | NM_004496 | chr14:37,136,518-37,136,952 |
| 32 | **GGH** | gamma-glutamyl hydrolase (conjugase, folylpolygammaglutamyl hydrolase) | NM_003878 | chr8:64,113,549-64,114,714 |
| 33 | **GP5** | glycoprotein V (platelet) | NM_004488 | chr3:195,599,869-195,600,530 |
| 34 | **HIST1H4L** | histone 1, H4l | NM_003546 | chr6:27,949,134-27,949,436 |
| 35 | **HOXD10** | homeo box D10 | NM_002148 | chr2:176,802,125-176,803,417 |
| 36 | **FLJ42461** | hypothetical protein | NM_198501 | chr17:4,433,626-4,434,410 |
| 37 | **KIAA1024** | hypothetical protein | NM_015206 | chr15:77,512,398-77,512,760 |
| 38 | **KLF5** | Kruppel-like factor 5 | NM_001730 | chr13:72,532,295-72,532,748 |
| 39 | **NKX6-1** | NK6 transcription factor related, locus 1 (Drosophila) | NM_006168 | chr4:85,773,752-85,774,375 |
| 40 | **KCNT2** | potassium channel, subfamily T, | NM_198503 | chr1:193,309,524-193,310,401 |

| | | | | |
|---|---|---|---|---|
| | | member 2 | | |
| 41 | **RAB38** | RAB38, member RAS oncogene family | NM_022337 | chr11:87,547,487-87,547,910 |
| 42 | **RAX** | retina and anterior neural fold homeobox | NM_013435 | chr18:55,090,094-55,091,041 |
| 43 | **TMEM39A** | transmembrane protein 39A | NM_018266 | chr3:120,664,657-120,665,292 |
| 44 | **ERBB4** | v-erb-a erythroblastic leukemia viral oncogene homolog 4 (avian) | NM_005235 | chr2:213,226,500-213,226,958 |
| 45 | **ZNF215** | zinc finger protein 215 | NM_013250 | chr11:6,904,139-6,904,828 |
| 46 | **ZNF222** | zinc finger protein 222 | NM_013360 | chr19:49,220,643-49,221,657 |
| 47 | **ZNF312** | zinc finger protein 312 | NM_018008 | chr3:62,335,092-62,335,729 |
| 48 | **ZFP37** | zinc finger protein 37 homolog (mouse) | NM_003408 | chr9:112,898,231-112,898,991 |
| 49 | **C1orf126** | chromosome 1 open reading frame 126 | NM_182534 | chr1:15,224,137-15,224,932 |
| 50 | **C16orf45** | chromosome 16 open reading frame 45 | NM_033201 | chr16:15,435,452-15,435,920 |
| 51 | **C20orf39** | chromosome 20 open reading frame 39 | NM_024893 | chr20:24,590,574-24,590,955 |
| 52 | **EPAS1** | endothelial PAS domain protein 1 | NM_001430 | chr2:46,438,273-46,438,695 |
| 53 | **FADS3** | fatty acid desaturase 3 | NM_021727 | chr11:61,394,612-61,395,522 |
| 54 | **GPM6A** | glycoprotein M6A | NM_201592 | chr4:177,297,242-177,297,964 |
| 55 | **GSH2** | GS homeobox 2 | NM_133267 | chr4:54,808,455-54,809,236 |
| 56 | **HELT** | Hey-like transcriptional repressor | NM_001029887 | chr4:186,311,865-186,312,615 |
| 57 | **HOXC10** | homeo box C10 | NM_017409 | chr12:52,663,260-52,664,020 |
| 58 | **HCRTR2** | hypocretin (orexin) receptor 2 | NM_001526 | chr6:55,146,931-55,147,432 |
| 59 | **CR611340** | hypothetical gene | CR611340 | chr6:28,237,275-28,237,990 |
| 60 | **AK055761** | hypothetical gene | AK055761 | chr9:1,037,501-1,038,158 |
| 61 | **BC026095** | hypothetical gene | BC026095 | chr11:59,079,888-59,080,211 |
| 62 | **AF086288** | hypothetical gene | AF086288 | chr12:118,493,994-118,495,258 |
| 63 | **AY358245** | hypothetical gene | AY358245 | chr15:91,058,353-91,058,677 |
| 64 | **FLJ13192** | hypothetical gene FLJ13192 | XM_378507 | chr15:34,959,477-34,960,514 |

| | | | | |
|---|---|---|---|---|
| 65 | **FLJ36633** | hypothetical genes | AK093952 | chr8:110,725,073-110,725,810 |
| 66 | **FLJ20972** | hypothetical protein FLJ20972 | NM_025030 | chr1:42,598,446-42,598,760 |
| 67 | **FLJ40542** | hypothetical protein FLJ40542 | AK097861 | chr22:16,634,834-16,635,380 |
| 68 | **FLJ42262** | hypothetical protein FLJ42262 | AK124256 | chr8:65,445,948-65,446,774 |
| 69 | **KIAA1465** | hypothetical protein KIAA1465 | NM_020851 | chr15:72,208,753-72,209,500 |
| 70 | **MGC33530** | hypothetical protein LOC222008 | NM_182546 | chr7:54,384,188-54,384,866 |
| 71 | **LOC282992** | hypothetical protein LOC282992 | AK096698 | chr10:102,986,973-102,987,631 |
| 72 | **FLJ10159** | hypothetical protein LOC55084 | NM_018013 | chr6:107,915,839-107,916,205 |
| 73 | **MGC4767** | hypothetical protein LOC84274 | NM_032314 | chr12:119,429,383-119,430,022 |
| 74 | **MGC42090** | hypothetical protein MGC42090 | NM_152774 | chr7:19,585,846-19,586,919 |
| 75 | **IRX1** | iroquois homeobox protein 1 | NM_024337 | chr5:3,650,419-3,651,517 |
| 76 | **FLJ90650** | laeverin | NM_173800 | chr5:115,324,982-115,325,849 |
| 77 | **LHX4** | LIM homeobox 4 | NM_033343 | chr1:176,933,893-176,934,838 |
| 78 | **LHX9** | LIM homeobox 9 | NM_001014434 | chr1:194,617,002-194,617,577 |
| 79 | **LMX1A** | LIM homeobox transcription factor 1, alpha | NM_177398 | chr1:162,055,037-162,055,592 |
| 80 | **MPDZ** | multiple PDZ domain protein | NM_003829 | chr9:13,267,880-13,268,715 |
| 81 | **MLF1** | myeloid leukemia factor 1 | NM_022443 | chr3:159,771,055-159,771,740 |
| 82 | **NTN4** | netrin 4 | NM_021229 | chr12:94,687,424-94,687,959 |
| 83 | **NKX2-3** | NK2 transcription factor related, locus 3 (Drosophila) | NM_145285 | chr10:101,282,866-101,284,051 |
| 84 | **OTP** | orthopedia homolog (Drosophila) | NM_032109 | chr5:76,969,190-76,969,438 |
| 85 | **PDE4B** | phosphodiesterase 4B, cAMP-specific (phosphodiesterase E4 dunce homolog, Drosophila) | NM_001037341 | chr1:65,969,706-65,970,592 |
| 86 | **PLD5** | phospholipase D family, member 5 | NM_152666 | chr1:239,012,625-239,013,006 |
| 87 | **PTPRK** | protein tyrosine phosphatase, receptor type, K | NM_002844 | chr6:128,881,695-128,882,717 |
| 88 | **PCDH19** | protocadherin 19 | NM_020766 | chrX:99,467,500-99,468,035 |
| 89 | **PCDH8** | protocadherin 8 | NM_032949 | chr13:52,323,619-52,324,164 |
| 90 | **PCDHGB1** | protocadherin gamma subfamily B, 2 | NM_018922 | chr5:140,709,703-140,710,382 |

| | | | | |
|---|---|---|---|---|
| 91 | **RAB3C** | RAB3C, member RAS oncogene family | NM_138453 | chr5:57,914,516-57,915,182 |
| 92 | **RPIB9** | Rap2-binding protein 9 | NM_138290 | chr7:86,901,392-86,902,072 |
| 93 | **RGMA** | RGM domain family, member A | NM_020211 | chr15:91,433,939-91,434,221 |
| 94 | **SCIN** | scinderin | NM_033128 | chr7:12,383,307-12,384,404 |
| 95 | **SCG3** | secretogranin III | NM_013243 | chr15:49,760,252-49,761,511 |
| 96 | **SETBP1** | SET binding protein 1 | NM_015559 | chr18:40,515,105-40,515,463 |
| 97 | **SHC4** | SHC (Src homology 2 domain containing) family, member 4 | NM_203349 | chr15:47,043,030-47,043,585 |
| 98 | **SLITRK3** | SLIT and NTRK-like family, member 3 | NM_014926 | chr3:166,395,732-166,396,243 |
| 99 | **SP5** | Sp5 transcription factor | NM_001003845 | chr2:171,399,393-171,399,837 |
| 100 | **SOX9** | SRY (sex determining region Y)-box 9 (campomelic dysplasia, autosomal sex-reversal) | NM_000346 | chr17:67,625,497-67,626,555 |
| 101 | **TIRAP** | toll-interleukin 1 receptor (TIR) domain containing adaptor protein | NM_052887 | chr11:125,657,284-125,657,863 |
| 102 | **TCF2** | transcription factor 2, hepatic; LF-B3; variant hepatic nuclear factor | NM_006481 | chr17:33,179,423-33,179,987 |
| 103 | **TFAP2A** | transcription factor AP-2 alpha | AK092393/NM_003220 | chr6:10,525,393-10,526,349 |
| 104 | **TFAP2C** | transcription factor AP-2 gamma (activating enhancer binding protein 2 gamma) | NM_003222 | chr20:54,633,902-54,634,430 |
| 105 | **ZNF229** | zinc finger protein 229 | NM_014518 | chr19:49,644,036-49,644,849 |
| 106 | **LOC389549** | zinc finger protein 312-like | NM_001024613 | chr7:121,533,481-121,534,284 |
| 107 | **ZNF483** | zinc finger protein 483 | NM_133464 | chr9:111,367,383-111,368,013 |
| 108 | **ZNF565** | zinc finger protein 565 | NM_152477 | chr19:41,427,804-41,428,431 |
| 109 | **ZNF582** | zinc finger protein 582 | NM_144690 | chr19:61,596,320-61,597,208 |
| 110 | **ZNF610** | zinc finger protein 610 | NM_173530 | chr19:57,531,642-57,532,426 |
| 111 | **ZNF629** | zinc finger protein 629 | XM_034819 | chr16:30,705,032-30,705,236 |

Table 1 also mentions the terms "Gene Symbol (Gene_Symbol)", the "Gene Name (Gene_Name)", the "GenBank Accession Number (GB_ACC)", and the "CPG Island Position (CPG_Island_Position)". In the context of the present invention, the terms "Gene Symbol", "Gene Name", "GenBank Accession Number" or "CPG Island Position" may be used in the alternative to the respective SEQ ID NOs so as to designate the nucleic acid molecule whose methylation status is determined by employing the methods of the present invention. The nucleic acid molecules having the nucleotide sequence as shown in SEQ ID NOs:1-111 represent promoter regions, regions within the open reading frame, regions of exons or introns. The skilled person knows how to determine which nucleotide sequence shown in SEQ ID NOs: 1-111 may be, for example, a promoter sequence, exon sequence, intron sequence, etc.
The CpG Island Position refers to the May 2004 human reference sequence (NCBI Build 35) that was produced by the International Human Genome Sequencing Consortium (www.genome.ucsc.edu).

Before the present invention is described in detail, it is to be understood that this invention is not limited to the particular methodology, protocols, bacteria, vectors, and reagents etc. described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.
Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).
Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.
It must be noted that as used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.
Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

It is known that the methylation of CpG islands is associated with transcriptional repression and, in cancer, leads to the abnormal silencing of tumor-suppressor genes. Since aberrant hypermethylation may be used as a marker for disease, a sensitive method for the global detection of DNA methylation events is of particular importance. Therefore, a novel and robust technique, called methyl-CpG immunoprecipitation (MCIp), which allows the unbiased genome wide profiling of CpG-methylation in limited DNA samples, was developed. The approach is based on a recombinant, antibody-like protein that efficiently binds native CpG-methylated DNA. In combination with DNA microarrays, in particular CpG island microarrays the technique was used to identify over one hundred genes with aberrantly methylated CpG islands in three myeloid leukemia cell lines. Interestingly, within all hypermethylation targets, genes involved in transcriptional regulation were significantly over-represented. More than half of the identified genes were absent in microarray expression studies in either leukemia or normal monocytes, indicating that hypermethylation in cancer may be largely independent of the transcriptional status of the affected gene. Most individually tested genes were also hypermethylated in primary blast cells from AML patients, suggesting that the approach can identify novel potential disease markers. Notably, myeloid leukemia cell lines and blast cells from AML patients have been used exemplary to demonstrate the usefulness and value of the technique. The technique, however, may prove useful for genome wide comparative methylation analysis, not only in the diagnosis of a predisposition of malignancies or in the diagnosis of malignancies.

In particular, by applying a novel and simple technique that allows the generation of unbiased, genome-wide profiles of normal or aberrant CpG-island methylation, markers have been identified which are subject to aberrant methylation in cancerous cells. These markers appear to be promising in diagnosing cancer or a predisposition of cancer. The procedure which has been applied to identify these markers is independent of methylation-sensitive restriction endonucleases or bisulfite-treatment, which are of limited use for genome-wide profiling strategies (14). In combination with appropriate DNA microarrays, MClp-enriched DNA fragments can be used to detect hypermethylation events on a genome wide scale. The efficacy of this system was tested by the profiling of three leukemia cell lines using a 12K CpG island microarray which led to the identification of many novel genes that are hypermethylated in human myeloid leukemia. However, the system is not limited to the profiling of leukemia cell lines, but can be applied to the diagnosis of cancer or a predisposition of cancer in general as further explained herein. Accordingly, the novel profiling technique provides a powerful tool to identify changes in DNA methylation as well as novel marker nucleic acid molecules, including genes or fragments thereof, which are of potential diagnostic or prognostic value when determining the methylation status as described herein.

As explained herein, the methods of the present invention is preferably useful for diagnosing cancer or a predisposition of cancer.
Briefly, by applying the central technique, called MCIp (methyl-CpG immunoprecipitation) which consists of the binding of methylated DNA fragments to the bivalent, antibody-like fusion protein MBD-$F_c$ (a methyl-binding domain fused to an $F_c$-tail) in an immunoprecipitation-like approach, enriched methylated DNA fragments can be efficiently detected on single gene level or throughout the genome. The power of these techniques was demonstrated by the identification of a large number of genes that are affected by aberrant hypermethylation, e.g. in myeloid leukemias. Accordingly, nucleotide sequences of genes, allelic variants or portions thereof that are affected by aberrant methylation, in particular, by hypermethylation, can be used for diagnosing cancer or a predisposition of cancer as described herein. Of course, the technique described herein is also applicable in diagnosing cancer or a predisposition of cancer when hypomethylation of one or more genes, preferably those described herein in Table 1 may be determined.

"Cancer" when used herein means a group of diseases characterized by uncontrolled growth and spread of abnormal cells. If the spread is not controlled, it can result in death. Cancer, when used herein, includes, but is not limited to malignant neoplasms, tumors, malignant tumors, tumorous diseases, metastatic tumors, carcinomas, sarcomas, lymphomas etc. Non-limiting examples of cancer are cancer of the skin, breast, brain, cervical carcinomas, testicular carcinomas, head and neck, lung, mediastinum, gastrointestinal tract, pancreas, liver, kidney, genitourinary system (including kidney, urethra, bladder, prostate, urethra, penis and testis), gynaecological system (including cervix, vagina, vulva, uterine body, gestational trophoblastic, ovarian, fallopian tube cancer), breast, endocrine system (including thyroid, parathyroid, adrenal cortex, pancreatic cancer), childhood (including retinoblastoma, Wilm's tumor, neurofibromatoses, neuroblastoma, Ewing's sarcoma family of tumors, rhabdomyosarcoma, a lymphoma, comprising non-Hodgkin's lymphomas, cutaneous T-cell lymphomas, primary central nervous system lymphoma, and Hodgkin's disease), unknown primary site or metastatic cancer, a sarcoma of the soft tissue and bone, a mesothelioma, a melanoma, a neoplasm of the central nervous system, a lymphoma, a leukemia, a paraneoplastic syndrome, a peritoneal carcinomastosis, a immunosuppression-related malignancy and/or metastatic cancer etc.

Preferably, the cancer or the predisposition of cancer to be diagnosed by the methods of the present invention is a leukemia. More specifically, said leukemia may be selected from the group consisting of acute leukemias (including acute myelocytic leukemia) such as acute myelogenous leukemia (AML), chronic myelogenous leukemia, lymphocytic leukemias (including B-cell leukemia or T-cell leukemia), a lymphoma (including T-cell lymphoma or B-cell lymphoma such as, inter alia, Burkitt's lymphoma, B-cell derived chronic lymphatic leukemia (B-CLL), Hodgkin's lymphoma and non-Hodgkin's lymphoma), plasma cell neoplasms, myelodysplastic syndromes, multiple myeloma, mixed leukemia, hairy cell leukemia, aplastic anemia, myelodysplastic syndrome and polycythemia vera.

The efficacy and usefulness of the techniques described herein was exemplary used for profiling of three leukemia cell lines and could identify a large number of gene fragments that are likely to be methylated in neoplastic cells. Interestingly, most genes that were identified as hypermethylated in leukemia cell lines showed extremely low or undetectable mRNA expression levels in microarray experiments. A comparison with published expression profiles for human bone-marrow, CD33 positive bone marrow cells, as well as mature myeloid cells (http://symatlas.gnf.org/SymAtlas/) indicates that a large proportion of these genes may not be significantly transcribed in myeloid cell types. A hypothetical (so far unknown) targeting mechanism may therefore induce CpG methylation of genes independent of their transcriptional status during cellular differentiation. Although such genes may not have a significant suppressor role in tumor development and/or progression, they may still serve as valuable biomarkers, provided that the targeting mechanism is specific for the disease.

Acute leukemia is characterized by a block of differentiation of early progenitors, which leads to the accumulation of immature cells in bone marrow and blood. The frequent mutation or down-regulation of a relatively small number of transcription factors in AML patients suggests that the inactivation of transcriptional regulators may be critically involved into the malignant transformation process. The methylation profiling of leukemia cell lines preferentially identified genes that are involved in transcriptional regulation. Half of the listed genes with an assigned molecular function (46/89) are involved in DNA-binding and transcriptional regulation, which represents a significant over representation. Aberrant hypermethylation of these transcription factor genes may lead to their epigenetic down-regulation and likely contributes to the observed differentiation arrest in leukemia cells. This observation is in line with a previous study from Rush et al. (40) that investigated the methylation status of a large set of CpG islands in AML patients using RLGS and also found that a large proportion of the known methylated promoters (4/11) corresponded to genes involved in transcriptional regulation.

The list of hypermethylation targets contains several transcription factor genes, including *MAFB, JUN* and *KLF11* which are highly expressed in normal myeloid cells. A good tumor suppressor candidate e.g. is represented by the bZip transcription factor MAFB, which is expressed specifically in the myeloid lineage of the hemopoietic system. Its expression is up-regulated successively during myeloid differentiation from multipotent progenitors to macrophages suggesting an essential role of *MAFB* in early myeloid and monocytic differentiation (41).

In summary, the data provided validate the experimental and possibly diagnostic potential of the MCIp technique. The recombinant MBD-$F_c$ protein and its application in DNA methylation analysis may represent an important step towards genome-wide CpG methylation profiling not only in cancer diagnostics. Accordingly, the identification of those hypermethylation and/or hypomethylation targets that are relevant in cancer development and/or progression appears to be possible by applying the techniques described herein. Once identified, these targets can be used for diagnosing cancer or a predisposition of cancer by determining their methylation status as described herein. In certain embodiments, hypermethalytion is determined. However, also hypomethylation can be determined.

The term "predisposition of cancer" encompasses a state of a subject of being predisposed to, having a tendency, prevalence, inclination or susceptibility to cancer. Moreover, said term also encompasses that a subject is at a risk of acquiring cancer. Namely, if the methylation status of at least one of the nucleic acid molecules having a nucleotide sequence of any one of SEQ ID NOs:1-111 an allelic variant, a portion or the complementary strand of any of said nucleotide sequences is aberrant, i.e. hyper-or hypomethylated, preferably the methylation status is hypermethylated, which can be determined by the means and methods of the present invention, this may be indicative that a subject has a predisposition of cancer or that a subject has cancer.

When used herein, the term "methylation status" encompasses the status of a nucleic acid molecule, preferably DNA or genomic DNA, as regards its methylation. DNA methyl transferases (DNMTs) can catalyse the transfer of a methyl group from the methyl donor S-adenosylmethionine to the cytosine ring, and thereby produce the base 5-methylcytosine. Specific cytosine residues are modified in mammals, which precede a guanosine residue in the DNA sequence (CpG dinucleotide) (Singal, Blood 93 (1999), 4059-4070); Robertson, Nat. Rev. Genet. 1 (2000), 11-19; Ng, Curr. Opin. Genet. Dev. (2000), 158-163; Razin, EMBO J. 17 (1998), 4905-4908). Thus, methylation of DNA leads to methylated DNA. The term "methylated DNA" encompasses methylated DNA, more preferably, CpG methylated DNA including hemi-methylated or DNA methylated at both strands or single-stranded, methylated DNA. The most important example to date is methylated cytosine that occurs mostly in the context of the dinucleotide CpG, but also in the context of CpNpG- and CpNpN-sequences. However, in principle, other naturally occurring nucleotides may also be methylated.
DNA demethylases can counteract the action of DNA methyltransferases. Accordingly, they can remove methyl groups from methylated nucleotides. For some diagnostic purposes, it is also interesting to determine whether a nucleic acid molecule is hypomethylated. The techniques described herein which employ a polypeptide being capable of binding methylated DNA are also suitable to isolate, enrich and/or purify hypomethylated DNA. Specifically, when employing the polypeptide being capable of binding methylated DNA, the skilled person may not retrieve the DNA bound by said polypeptide, but the DNA not bound by said polypeptide. Accordingly, hypomethylated DNA may be isolated, purified and/or enriched and could be used fur a further analysis as described herein.

In the context of the methods of diagnosing cancer or a predisposition of cancer, the methylation status of at least one of the nucleic acid molecules having a nucleotide sequence of any one of SEQ ID NOs: 1-111, an allelic variant, a portion or the complementary strand of any of said nucleotide sequences is determined, wherein in comparison to a healthy subject an aberrant methylation status, i.e. hypomethylation and/or hypermethylation of at least one of the nucleic acid molecules having the nucleotide sequence of any one of SEQ ID NOs: 1-111 may be indicative of cancer or a predisposition of cancer. In the methods of diagnosis of the present invention, preferably hypermethylation and/or hypomethylation, more preferably, hypermethylation, of at least one of the nucleic acid molecules having the nucleotide sequence of any one of SEQ ID NOs: 1-111, an allelic variant, a portion or the complementary strand of any of said nucleotide sequences is determined.

Preferably, the methylation status of at least one of the nucleic acid molecules having the nucleotide sequence of any one of SEQ ID NOs: 49-111, an allelic variant, a portion or the complementary strand of any of said nucleotide sequences, more preferably the methylation status of at least one of the nucleic acid molecules having the nucleotide sequence of any one of SEQ ID NOs: 17-48, an allelic variant, a portion or the complementary strand of any of said nucleotide sequences, even more preferably the methylation status of at least one of the nucleic acid molecules having the nucleotide sequence of any one of SEQ ID NOs: 5-16, an allelic variant, a portion or the complementary strand of any of said nucleotide sequences and most preferably the methylation status of at least one of the nucleic acid molecules having the nucleotide sequence of any one of SEQ ID NOs: 1-4, an allelic variant, a portion or the complementary strand of any of said nucleotide sequences is determined. Of course, as described herein below in more detail, it would be possible to determine the methylation status of all nucleic acid molecules having the nucleotide sequence of any one of SEQ ID NOs: 1-11, an allelic variant, a portion or the complementary strand of any of said nucleotide sequences, for example, by employing a DNA microarray. However, as stated before, it is preferred to determine the methylation status of one or more of the nucleic acid molecules having the nucleotide sequence of any one of SEQ ID NOs: 1-111, an allelic variant, a portion or the complementary strand of any of said nucleotide sequences.

The term "nucleic acid molecule" when used herein encompasses any nucleic acid molecule having a nucleotide sequence of bases comprising purine- and pyrimidine bases which are comprised by said nucleic acid molecule, whereby said bases represent the primary structure of a nucleic acid molecule. When used herein in the context of nucleotide sequences, the term "having" is interchangeable with the term "comprising". Nucleic acid sequences include DNA, cDNA, genomic DNA, RNA, synthetic forms, for example, PNA, and mixed polymers, both sense and antisense strands, or may contain non-natural or derivatized nucleotide bases, as will be readily appreciated by those skilled in the art. For example, the polynucleotide can be composed of single- and double-stranded DNA, DNA that is a mixture of single-and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. The polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, the term "nucleic acid molecules" embraces chemically, enzymatically, or metabolically modified forms.

The term "nucleic acid molecule" also embraces an "allelic variant" of a nucleic acid molecule whose methylation status can be determined by employing the methods of diagnosis of the present invention. When used herein an "allelic variant" includes naturally occurring variants of the nucleic acid molecule whose methylation status is determined by employing the methods of the present invention. Accordingly, an "allelic variant" refers to one of several alternate forms of a nucleotide sequence occupying a given locus on a chromosome of an organism. (Genes II, Lewin, B., ed., John Wiley & Sons, New York (1985)). Allelic variants may thus vary at the nucleic acid level. Alternatively, non-naturally occurring variants may be produced by mutagenesis techniques or by direct synthesis. Non-naturally occurring variants are also embraced by the term "allelic variant". Preferably, an allelic variant of a nucleic acid molecule as described herein is at least 70, 75, 80, 85, 90, 91, 92, 93, 94, 95, 96, 97, 98 or 99% identical to a nucleotide sequence of any one of SEQ ID NOs: 1-111 or a portion thereof.

The term "portion" encompasses any portion of the nucleotide sequence of a nucleic acid molecule of any one of SEQ ID NOs: 1-111 or an allelic variant thereof as far as it is suitable to determine the methylation status. Preferably, said portion comprises at least 8, 9, 10, 11, 12, 13, 14, 15, 18, 20, 21, 25, 30, 35, 40, 45, 50, 60, 70, 80, 90, 100, 110, 120, 150, 170, 180, 190, 200, 300, 350, 400, 450, 500 nucleotides in length.

Preferably, the method of the present invention comprises the steps of

(a) isolating DNA from cells of a sample obtained from a subject;
(b) contacting said DNA with a polypeptide capable of binding methylated DNA;
(c) optionally amplifying said DNA bound by said polypeptide capable of binding methylated DNA; and
(d) determining the methylation status of said DNA.

In accordance with the preferred embodiment of the method of diagnosis of the present invention, the first method step comprises the isolation of DNA from cells of a sample of a subject.

Isolation of DNA from cells of an individual can, e.g., be effected by isolating cells from a subject, and isolating the genomic DNA of said cells. Such cells can be collected from body fluids, skin, hair, biopsies and other sources. Collection and analysis of cells from bodily fluids such as biopsies, blood, urine and cerebrospinal fluid is well known to the art; see for example, Rodak, "Haematology: Clinical Principles & Applications" second ed., WB Saunders Co, 2002; Brunzel, "Fundamentals of Urine and Body Fluids Analysis", WB Saunders Co, 1994; Herndon and Brumback (Ed.), "Cerebrospinal Fluid", Kluwer Academic Pub., 1989. In addition, methods for DNA isolation are well described in the art; see, for example, Sambrook et al., "Molecular Cloning: A Laboratory Manual", 3rd edition, Cold Spring Harbor Laboratory, 2001.

Typically, cells are lysed and DNA is isolated using basic well-established protocols (Sambrook et al., "Molecular Cloning: A Laboratory Manual", 3rd edition, Cold Spring Harbor Laboratory, 2001; Current Protocols in Molecular Biology, Ausubel et al., eds. John Wiley and Sons, Inc. , NY, 1999) or commercial kits (e. g. those available from QIAGEN (Valencia, CA) or Promega (Madison,WI)). Once isolated, it is preferred that the DNA is free of DNA processing enzymes and contaminating salts and the entire genome is equally represented.

For example, genomic DNA is prepared by using a kit known in the art, for example, using the Blood and Cell Culture Midi Kit (Qiagen). The quality of the genomic DNA-preparation is optionally controlled by agarose gel electrophoresis and DNA concentration may, for example, be determined by UV spectrophotometry.

Quantitation of DNA is, for example, done by using PicoGreen dsDNA Quantitation Reagent (Molecular Probes).

In accordance with the present invention by the term "sample" is intended any biological sample obtained from an individual, cell line, tissue culture, or other source containing polynucleotides (including but not-limited to genomic DNA, spliced and/or unspliced mRNA) or portions thereof. Biological samples include body fluids (such as blood, sera, plasma, urine, synovial fluid and spinal fluid) and tissue sources. Methods for obtaining tissue biopsies and body fluids from mammals are well known in the art. A biological sample which comprises genomic DNA is preferred.

In the context of the present invention the term "subject" means an individual. Preferably, the subject is a vertebrate, even more preferred a mammal, particularly preferred a human.

In accordance with the preferred embodiment of the method of diagnosis of the present invention, the further method step comprises contacting said DNA with a polypeptide capable of binding methylated DNA. Preferably, said polypeptide capable of binding methylated DNA is a bifunctional polypeptide comprising the DNA-binding domain of a protein belonging to the family of Methyl-CpG binding proteins (MBDs) and an Fc portion of an antibody.

The term "contacting" includes every technique which causes that a polypeptide which is capable of binding methylated DNA as is described herein is brought into contact by any known means and methods in the art with a sample comprising methylated and/or unmethylated DNA, preferably genomic DNA isolated from cells of a subject.

Examples of such binding polypeptides are given herein below and comprise, inter alia and preferably, a polypeptide belonging to the Methyl-DNA binding protein (MBD) family, and most preferably a bifunctional polypeptide comprising the DNA-binding domain of a protein belonging to the family of Methyl-CpG binding proteins (MBDs) and an Fc portion of an antibody. Said DNA-binding domain is described herein below. Optionally, said bifunctional polypeptide comprises a polypeptide linker which is described herein below. Accordingly, said bifunctional polypeptide is preferably characterized by the amino acid sequence shown in SEQ ID NO: 113 (Figure 11) which is encoded by the nucleotide sequence shown in SEQ ID NO: 112 (Figure 11)

The term "polypeptide capable of binding methylated DNA" encompasses any polypeptide which can bind methylated DNA as described herein. The capability of binding methylated DNA can be tested by methods known in the art. The term "polypeptide" when used herein means a peptide, a protein, or a polypeptide which are used interchangeable and which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. As mentioned the terms polypeptide and protein are often used interchangeably herein. The term polypeptide also refers to, and does not exclude, modifications of the polypeptide. Modifications include glycosylation, acetylation, acylation, phosphorylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formulation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination; see, for instance, PROTEINS - STRUCTURE AND MOLECULAR PROPERTIES, 2nd Ed., T. E. Creighton, W. H. Freeman and Company, New York (1993); POST-TRANSLATIONAL COVALENT MODIFICATION OF PROTEINS, B. C. Johnson, Ed., Academic Press, New York (1983), pgs. 1-12; Seifter, Meth. Enzymol. 182 (1990); 626-646, Rattan, Ann. NY Acad. Sci. 663 (1992); 48-62. Preferably, the term "polypeptide" encompasses a polypepetide capable of binding methylated DNA. Said term also encompasses a bifunctional polypeptide which is capable of binding metylated DNA and it encompasses an anti-methylated DNA antibody. Such polypeptides are described herein and are employed in the method of the present invention for detecting methylated DNA.

A "bifunctional polypeptide" means that a polypeptide has, in addition to binding to methylated DNA, preferably to CpG methylated DNA, due to an Fc portion of an antibody which is part of the said bifunctional polypeptide, further capabilities. For example, said Fc portion preferably offers the possibility to conjugate, link or covalently couple (a) compound(s) or moieties to said Fc portion. As used herein, the term "covalently coupled" means that the specified compounds or moieties are either directly covalently bonded to one another, or else are indirectly covalently joined to one another through an intervening moiety or moieties, such as a bridge, spacer, or linkage moiety or moieties. Furthermore, said Fc portion may be used to couple said bifunctional polypeptide to a container as is described herein. A preferred bifunctional polypeptide is characterized by the amino acid sequence shown in SEQ ID NO: 113 (Figure 11). Further preferred bifunctional polypeptides are described herein below.

The utility of naturally occurring methyl CpG binding (MBD) proteins to separate methylated and unmethylated DNA fragments are known for more than a decade. Already in 1994, the laboratory of A. Bird developed a method for enriching methylated DNA fragments by means of affinity chromatography using recombinant MeCP2 (35). The technique has been used, improved and combined with further techniques by other laboratories (36;37). A disadvantage of MeCP2-affinity chromatography is the large amount of genomic DNA required (50-100 μg) and the relatively time-consuming procedure. Also, a recent report by Klose et al. (38) demonstrated that MeCP2 requires an A/T run adjacent to the methylated CpG dinucleotide for efficient DNA binding, suggesting that MeCP2-affinity chromatography will be biased towards certain CpG motifs. No binding requirements or preferences of MBD2 were detected in this and previous studies. However, for example, the high methyl-CpG affinity of MBD2 (39) combined with the bivalent, antibody-like structure of the recombinant MBD-$F_c$ polypeptide largely is believed to increase its binding capacity, enabling the efficient retention of DNA fragments in dependence on their methylation degree. The properties of the recombinant MBD-$F_c$ polypeptide allow its application in small-scale assays requiring only little amounts of DNA. This may actually permit the profiling of DNA methylation of candidate genes from very limited cell numbers including biopsy samples or cells collected by laser-mediated microdissection. The MCIp approach presented in this report requires only little amounts of genomic DNA for a complete genome wide methylation profile when, for example, optionally combined with an unspecific LM-PCR amplification step (The latter step could be omitted if sufficient starting material is available). An unmethylated DNA fragment may be 200- to 500-fold depleted and up to 80% of a highly methylated fragment may be recovered in the high-salt MCIp fraction, demonstrating the high affinity of our recombinant polypeptide. In addition to enzymatic restriction, the DNA may also be fragmented by sonication, resulting in a similar enrichment of methylated fragments in the high salt fraction. A recent publication by Weber et al. (31) describes a related approach using a 5-methyl cytosine (5mC) antibody and a denaturing step before the immunoprecipitation of DNA fragments. Their analysis revealed only a small set of promoters being methylated differentially in a normal and a transformed cell line, suggesting that aberrant methylation of CpG island promoters in malignancy might be less frequent than previously hypothesized. In contrast to their observations, the proteins being capable of binding methylated DNA which are described herein and which may be employed in certain preferred methods for diagnosing cancer or a predisposition of cancer of the present application, detect a much higher percentage of differentially methylated genes, much in line with previous estimates, using the same CpG island microarray platform.

Without being bound by theory, it is believed that the nascent bifunctional polypeptide comprising an methyl-DNA-binding domain and an Fc portion of an antibody is folded within a host cell such that preferably two polypeptides are joined at their Fc portion in a manner similar or, preferably, identical to the constant region of an antibody, resulting in a bifunctional polypeptide as described herein.

It was surprisingly found that said bifunctional polypeptide, preferably behaving as an antibody-like protein can preferably bind CpG methylated DNA in an antibody-like manner. That means, the bifunctional polypeptide has a high affinity and high avidity to its "antigen" which is preferably methylated DNA that is preferably methylated at CpG dinucleotides. Again, without being bound by theory, the high affinity and avidity of the bifunctional polypeptide employed in the method of the present invention for detecting methylated DNA for its "antigen" is caused by the unique structure of said bifunctional polypeptide. This is because, it is assumed that the constant regions form disulfide-bonds between immunoglobulin heavy chains of the constant regions of each of two polypeptide molecules of said bifunctional polypeptide. Accordingly, preferably an antibody-like structure is formed which closely resembles the structure of an antibody.

Moreover, without being bound by theory it is assumed that this antibody-like structure lends, for example, stability on the bifunctional polypeptide employed in the method of the present invention for detecting methylated DNA. This is because, it is described in the art that proteins fused to a constant region of an antibody may confer a higher stability and half-life of the said protein. In addition, it is believed that the antibody-like structure caused by the intermolecular interaction of the constant regions brings the methyl-DNA-binding domain of one polypeptide of the bifunctional polypeptide used in accordance with the method of the present invention for detecting methylated DNA in close proximity to the methyl-DNA-binding domain of another polypeptide of the present invention employed in the method of the present invention. This allows bivalent interactions between the methyl-DNA-binding protein(s) and methylated DNA. Accordingly, the bifunctional polypeptide described herein is preferably capable of binding to its antigen via two methyl DNA-binding domains which are part of said bifunctional polypeptide. The high affinity binding of the bifunctional polypeptide is, inter alia, also achieved by using preferably methyl-DNA-binding domains of proteins instead of the full-length methyl-DNA-binding protein containing domains for the interaction with other proteins that may, however, disturb or interfere the unique applicability as described herein which are known to specifically bind to methylated DNA, preferably, CpG methylated DNA, rather than to unmethylated DNA. The preferred use of the methyl-DNA-binding domain, moreover, is believed to guarantee that indeed methylated DNA is bound since the detection is direct and not indirect. Most prior art methods can only indirectly detect methylated DNA by PCR.

These properties award the preferred bifunctional polypeptide to be a reliable and easy applicable diagnostic tool which can be employed in the method of the present invention for binding methylated DNA. Yet, it can also be employed in methods for, inter alia, isolating, purifying enriching methylated DNA even if said DNA is only present in very small amounts, e.g., about more than 10 ng, less than 10 ng, less than 7.5 ng, less than 5 ng, less than 2.5 ng, less than 1000 pg, less than 500 pg, less than 250 pg or about 150 pg as described herein. Accordingly, due to its antibody-like structure the bifunctional polypeptide described herein is a robust molecule rendering it to be applicable, for instance, for various applications including multi-step procedures in a single tube assay as is described herein and in the appended Examples.

In the following, preferred polypeptides capable of binding methylated DNA are described. Accordingly, a polypeptide used in the methods of the present invention for detecting methylated DNA is preferably selected from the group consisting of

(a) a polypeptide belonging to the Methyl-DNA binding protein (MBD) family;
(b) a fragment of the polypeptide of (a), wherein said fragment is capable of binding methylated DNA;
(c) a variant of the polypeptide of (a) or the fragment of (b), wherein in said variant one or more amino acid residues are substituted compared to the polypeptide of (a) or the fragment of (b), and wherein said variant is capable of binding methylated DNA;
(d) a polypeptide which is an anti-methylated DNA antibody or fragment thereof; and
(e) a polypeptide which is at least 70% identical to a polypeptide of any one of (a) to (c) and which is capable of binding methylated DNA.

Of course, it is envisaged that the herein described polypeptides which are capable of detecting methylated DNA are encoded by a nucleic acid molecule. The polynucleotide of the present invention encoding a polypeptide which is capable of binding methylated DNA and which is employed in the method of the present invention is preferably composed of any polyribonucleotide or polydeoxribonucleotide, which may be unmodified RNA or DNA or modified RNA or DNA.

In an alternative, but also preferred embodiment, a bifunctional polypeptide (i.e. the MBD protein to be preferably employed in the methods and kits provided herein) capable of binding methylated DNA which is employed in the method of the present invention is encoded by a nucleic acid molecule comprising a nucleotide sequence of the present invention described hereinabove is selected from the group consisting of:

(a) a nucleic acid sequence having the nucleotide sequence shown in SEQ ID NO: 112 (Figure 11);
(b) a nucleic acid sequence having a nucleotide sequence encoding a polypeptide having the amino acid sequence shown in SEQ ID: NO 113 (Figure 11);
(c) a nucleic acid sequence having a nucleotide sequence encoding a fragment of a polypeptide having the amino acid sequence shown in SEQ ID: NO 113 (Figure 11), wherein said fragment comprises at least amino acids 130 to 361 of said polypeptide and which is capable of binding methylated DNA;
(d) a nucleic acid sequence having a nucleotide sequence encoding a variant of a polypeptide encoded by a polynucleotide of any one of (a) to (c), wherein in said variant one or more amino acid residues are substituted compared to said polypeptide, and said variant is capable of binding methylated DNA;
(e) a nucleic acid sequence having a nucleotide sequence which hybridizes with a nucleic acid sequence of any one of (a) to (d) and which is at least 65% identical to the nucleotide sequence of the nucleic acid molecule of (a) and which encodes a polypeptide being capable of binding methylated DNA;
(f) a nucleic acid molecule encoding a polypeptide which is at least 65% identified to a polypeptide encoded by a nucleic acid molecule of (b) and which is capable of binding methylated DNA; and
(g) a nucleic acid sequence having a nucleotide sequence being degenerate to the nucleotide sequence of the polynucleotide of any one of (a) to (f);

or the complementary strand of such a polynucleotide.

The above embodiments relate, accordingly, e.g. to the use of a "MBD-Fc" molecule in the kits and methods provided herein.

As described above, a fragment of a bifunctional polypeptide employed in the method of the present invention for binding methylated DNA which has the amino acid sequence shown in SEQ ID: NO 113 (Figure 11) comprises at least amino acids 130 to 361 of the amino acid sequence shown in SEQ ID: NO 113 (Figure 11). That means that said fragment may comprise in addition to amino acids 130 to 361 which represent the Fc portion, one or more amino acids such that said fragment is capable of binding methylated DNA, preferably, CpG methylated DNA, rather than unmethylated DNA. Accordingly, it is envisaged that said fragment comprises more preferably, at least amino acids 116 to 361 of the amino acid sequence shown in SEQ ID: NO 113 (Figure 11). Even more preferably, said fragment may comprise at least amino acids 29 to 115 and 130 to 361 of the amino acid sequence shown in SEQ ID: NO 113 (Figure 11). In a most preferred embodiment, said fragment may comprise at least amino acids 29 to 361. It is generally preferred that the fragments of the polypeptide described herein are able to bind to methylated DNA, preferably to CpG methylated DNA, rather than unmethylated DNA. This ability can be tested by methods known in the art or preferably by those methods described in the appended Examples.

The present invention preferably also relates to methods, wherein nucleic acid sequences which hybridize to the nucleic acid sequence encoding a polypeptide which is capable of binding methylated DNA are employed. Said hybridizing nucleic acids encode a polypeptide which is capable of binding methylated DNA: Moreover, in the methods of the present invention, nucleic acids are employed which hybridize to the sequences shown in SEQ ID NO: 112 or fragments or variants thereof as described herein (Figure 11) and which are at least 65% identical to the nucleic acid sequence shown in SEQ ID NO: 112 (Figure 11) and which preferably encode a bifunctional polypeptide being capable of binding methylated DNA, preferably CpG methylated DNA, rather than unmethylated DNA, wherein said plypeptide is employed in the method of the present invention for detecting methylated DNA. Furthermore, the present invention preferably relates to methods in which nucleic acid sequences encoding a polypeptide are employed which are at least 65%, more preferably 70%, 75%, 80%, 85%, 90%, more preferably 99% identical to a polypeptide as described herein which is capable of binding methylated DNA. It is also preferably envisaged that in the methods of the present invention polypeptides are employed which are at least 65%, more preferably 70%, 75%, 80%, 85%, 90%, more preferably 99% identical to the polypeptide shown in SEQ ID NO: 113. The term "hybridizes" as used in accordance with the present invention preferably relates to hybridizations under stringent conditions. The term "hybridizing sequences" preferably refers to sequences which display a sequence identity of at least 65%, even more preferably at least 70%, particularly preferred at least 80%, more particularly preferred at least 90%, even more particularly preferred at least 95% and most preferably at least 97, 98% or 99% identity with a nucleic acid sequence as described above encoding a polypeptide which is capable of binding methylated DNA or a bifunctional polypeptide which is able to bind to methylated DNA, preferably CpG methylated DNA, rather than unmethylated DNA, wherein said polypeptide capable of binding methylated DNA or said bifunctional polypeptide is employed in the method of the present invention for detecting methylated DNA. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1 xSSC, 0.1 % SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences as described herein. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analyzis) or between one nucleic acid sequence present in solution and another nucleic acid sequence immobilized on a solid support (e.g., membranes, filters, chips, pins or glass slides to which, e.g., cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementarity between nucleic acid strands has significant effects on the efficiency and strength of hybridization between nucleic acid strands. This is of particular importance in amplification reactions, which depend upon binding between nucleic acids strands.

Moreover, the present invention also relates to methods employing nucleic acid molecules the sequence of which is degenerate in comparison with the sequence of an above-described nucleic acid molecule, wherein such degenerate nucleic acid molecules encode a polypeptide which is capable of binding methylated DNA or which encode a bifunctional polypeptide as described herein and which is employed in the method of the present invention for binding methylated DNA. When used in accordance with the present invention the term "being degenerate as a result of the genetic code" means that due to the redundancy of the genetic code different nucleotide sequences code for the same amino acid.

Of course, the present invention also envisages the complementary strand to the aforementioned and below mentioned nucleic acid molecules if they may be in a single-stranded form.

Preferably, the nucleic acid molecule encoding a polypeptide which is capable of binding methylated DNA or a bifunctional polypeptide capable of binding methylated DNA and which is/are employed in the method of the present invention may be any type of nucleic acid, e.g. DNA, genomicDNA, cDNA, RNA or PNA (peptide nucleic acid).

For the purposes of the present invention, a peptide nucleic acid (PNA) is a polyamide type of DNA analog and the monomeric units for adenine, guanine, thymine and cytosine are available commercially (Perceptive Biosystems). Certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

The DNA may, for example, be genomic DNA or cDNA. The RNA may be, e.g., mRNA. The nucleic acid molecule may be natural, synthetic or semisynthetic or it may be a derivative, such as peptide nucleic acid (Nielsen, Science 254 (1991), 1497-1500) or phosphorothioates. Furthermore, the nucleic acid molecule may be a recombinantly produced chimeric nucleic acid molecule comprising any of the aforementioned nucleic acid molecules either alone or in combination.

The nucleic acid molecule encoding a polypeptide described herein which is employed in the method of the present invention for binding methylated DNA is envisaged to be contained in a vector (e.g. a plasmid, cosmid, virus, bacteriophage) which may be transformed into a host cell (a prokaryotic or eukaryotic cell) so as to, inter alia, produce a polypeptide of the present invention which is employed in the method of the present invention. A polypeptide of the invention which is employed in the method of the present invention may be produced by microbiological methods or by transgenic mammals. It is also envisaged that a polypeptide of the invention is recovered from transgenic plants. Alternatively, a polypeptide of the invention may be produced synthetically or semi-synthetically.

For example, chemical synthesis, such as the solid phase procedure described by Houghton Proc. Natl. Acad. Sci. USA (82) (1985), 5131-5135, can be used. Another method is in vitro translation of mRNA. A preferred method involves the recombinant production of protein in host cells as described above. For example, nucleotide acid sequences comprising all or a portion of any one of the nucleotide sequences according to the invention can be synthesized by PCR, inserted into an expression vector, and a host cell transformed with the expression vector. Thereafter, the host cell is cultured to produce the desired polypeptide, which is isolated and purified. Protein isolation and purification can be achieved by any one of several known techniques; for example and without limitation, ion exchange chromatography, gel filtration chromatography and affinity chromatography, high pressure liquid chromatography (HPLC), reversed phase HPLC, preparative disc gel electrophoresis. In addition, cell-free translation systems can be used to produce a polypeptide of the present invention. Suitable cell-free expression systems for use in accordance with the present invention include rabbit reticulocyte lysate, wheat germ extract, canine pancreatic microsomal membranes, E. coli S30 extract, and coupled transcription/translation systems such as the TNT-system (Promega). These systems allow the expression of recombinant polypeptides or peptides upon the addition of cloning vectors, DNA fragments, or RNA sequences containing coding regions and appropriate promoter elements. As mentioned supra, protein isolation/purification techniques may require modification of the proteins of the present invention using conventional methods. For example, a histidine tag can be added to the protein to allow purification on a nickel column. Other modifications may cause higher or lower activity, permit higher levels of protein production, or simplify purification of the protein. After production of a polypeptide which is employed in the method of the present invention it may be modified by pegylation, derivatization and the like.

The term "polypeptide belonging to the Methyl-DNA binding protein (MBD)" encompasses a polypeptide which has preferably the structural and/or functional characteristics of the methyl-DNA-binding domain (MBD) of a protein of the MBD family which comprises the proteins MeCP2, MBD1, MBD2, MBD3 and MBD4. Said term also encompasses polypeptides with the capability of binding methylated DNA, including, inter alia, antibodies raised against methylated DNA. Preferably, said antibody is an anti-5-methylcysteine antibody or fragment thereof. Preferably, said fragment is a Fab, $F(ab')_2$, Fv or scFv fragment. The methyl-DNA-binding activity can be tested by methods known in the art. It is preferred that a polypeptide described herein binds methylated DNA either as a monomer or dimer or multivalent molecule as described elsewhere herein. It is preferably capable of binding to highly methylated DNA or low methylated DNA. Preferably, it can bind single methylated CpG pairs. MeCP2, MBD1, MBD2, MBD3 and MBD4 constitute a family of vertebrate proteins that share the methyl-CpG-binding domain. The MBD protein family comprises two subgroups based upon sequences of the known MBDs. The methyl-DNA-binding domain of MBD4 is most similar to that of MeCP2 in primary sequence, while the methyl-DNA-binding domain of MBD1, MBD2 and MBD3 are more similar to each other than to those of either MBD4 or MeCP2. However, the methyl-DNA-binding domains within each protein appear to be related evolutionarily based on the presence of an intron located at a conserved position within all five genes of MeCP2, MBD1, MBD2, MBD3 and MBD4. Yet, the sequence similarity between the members of the MBD family is largely limited to their methyl-DNA-binding domain, although MBD2 and MBD3 are similar and share about 70% of overall identity over most of their length. The greatest divergence occurs at the C-terminus, where MBD3 has 12 consecutive glutamic acid residues.

A protein belonging to the MBD family or fragment thereof, preferably a methyl-DNA-binding domain, useful in accordance with the methods of the present invention can, for example, be identified by using sequence comparisons and/or alignments by employing means and methods known in the art, preferably those described herein and comparing and/or aligning (a) known MBD(s) to/with a sequence suspected to be an MBD.

For example, when a position in both of the two compared sequences is occupied by the same base or amino acid monomer subunit (for instance, if a position in each of the two DNA molecules is occupied by adenine, or a position in each of two polypeptides is occupied by a lysine), then the respective molecules are identical at that position. The percentage identity between two sequences is a function of the number of matching or identical positions shared by the two sequences divided by the number of positions compared x 100. For instance, if 6 of 10 of the positions in two sequences are matched or are identical, then the two sequences are 60% identical. By way of example, the DNA sequences CTGACT and CAGGTT share 50% homology (3 of the 6 total positions are matched). Generally, a comparison is made when two sequences are aligned to give maximum homology and/or identity. Such alignment can be provided using, for instance, the method of Needleman, J. Mol Biol. 48 (1970): 443-453, implemented conveniently by computer programs such as the Align program (DNAstar, Inc.). Homologous sequences share identical or similar amino acid residues, where similar residues are conservative substitutions for, or "allowed point mutations" of, corresponding amino acid residues in an aligned reference sequence. In this regard, a "conservative substitution" of a residue in a reference sequence are those substitutions that are physically or functionally similar to the corresponding reference residues, e. g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: Atlas of Protein Sequence and Structure, 5: Suppl. 3, chapter 22: 354-352, Nat. Biomed. Res. Foundation, Washington, D. C. (1978).

Preferably, a fragment of a polypeptide described herein and employed in the method of the present invention which is capable of binding methylated DNA, preferably, a methyl-DNA-binding domain or fragment thereof of a polypeptide employed in the method of the present invention, has preferably the structural and/or functional characteristics of a protein belonging to the MBD-family as described herein. Preferably, a fragment of a methyl-DNA-binding protein described herein is able to bind methylated DNA, preferably CpG methylated DNA.

The methyl-DNA-binding domain or fragment thereof of a polypeptide of the present invention which is employed in the method of the present invention is preferably of insect origin, nematode origin, fish origin, amphibian origin, more preferably of vertebrate origin, even more preferably of mammal origin, most preferably of mouse and particularly preferred of human origin.

Preferably, the methyl-DNA-binding domain or fragment thereof of a polypeptide of the present invention which is employed in the method of the present invention possesses a unique alpha-helix/beta-strand sandwich structure with characteristic loops as is shown in Figure 1 of Ballester and Wolffe, Eur. J. Biochem. 268 (2001), 1-6 and is able to bind methylated DNA.

More preferably, the protein belonging to the MBD family or fragment thereof of a polypeptide of the present invention which is employed in the method of the persent invention comprises at least 50, more preferably at least 60, even more preferably at least 70 or at least 80 amino acid residues of the MBDs shown in Figure 1 of Ballester and Wolffe (2001), loc. cit. and is able to bind methylated DNA.

Even more preferably, the methyl-DNA-binding domain or fragment or variant thereof of a polypeptide of the present invention employed in the method of the present invention shares preferably 50 %, 60%, 70%, 80% or 90%, more preferably 95% or 97%, even more preferably 98% and most preferably 99% identity on amino acid level to the MBDs shown in Figure 1 of Ballester and Wolffe (2001), loc. cit. and is able to bind methylated DNA. Means and methods for determining the identity of sequences, for example, amino acid sequences are described elsewhere herein.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (e.g., at least 65% identity, preferably, at least 70-95% identity, more preferably at least 95%, 96%, 97%, 98% or 99% identity), when compared and aligned for maximum correspondence over a window of comparison, or over a designated region as measured using a sequence comparison algorithm as known in the art, or by manual alignment and visual inspection. Sequences having, for example, 65% to 95% or greater sequence identity are considered to be substantially identical. Such a definition also applies to the complement of a test sequence. Preferably the described identity exists over a region that is at least about 232 amino acids or 696 nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art. Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul Nucl. Acids Res. 25 (1977), 3389-3402). The BLASTN program for nucleic acid sequences uses as defaults a word length (W) of 11, an expectation (E) of 10, M=5, N=4, and a comparison of both strands. For amino acid sequences, the BLASTP program uses as defaults a word length (W) of 3, and an expectation (E) of 10. The BLOSUM62 scoring matrix (Henikoff Proc. Natl. Acad. Sci., USA, 89, (1989), 10915) uses alignments (B) of 50, expectation (E) of 10, M=5, N=4, and a comparison of both strands.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity} \times \%\text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.
Most preferably, the methyl-DNA-binding domain or fragment or variant thereof of a polypeptide of the present invention employed in the method of the present invention comprises the methyl-DNA-binding domain of the MBD proteins shown in Figure 1 of Ballester and Wolffe (2001), loc. cit. or the methyl-DNA-binding domain of the MBD proteins described in Hendrich and Tweedy, Trends Genet. 19 (2003), 269-77 and is able to bind methylated DNA.
In a particular preferred embodiment of the invention, the methyl-DNA-binding domain of a polypeptide employed in the method of the present invention is that of human MBD2. In a more particular preferred embodiment, the methyl-DNA-binding domain is that of human MBD2 comprising amino acids 144 to 230 of the amino acid sequence having Genbank accession number NM_003927. In a most particular preferred embodiment, the methyl-DNA-binding domain of a polypeptide employed in the method of the present invention comprises the amino acid sequence from position 29 to 115 of the amino acid sequence shown in SEQ ID NO:113 (Figure 11).

A "variant" of a polypeptide of the present invention which is capable of binding methylated DNA and which is employed in the method of the present invention encompasses a polypeptide wherein one or more amino acid residues are substituted, preferably conservatively substituted compared to said polypeptide and wherein said variant is preferably able to bind to methylated DNA, preferably CpG methylated DNA. Such variants include deletions, insertions, inversions, repeats, and substitutions selected according to general rules known in the art so as have no effect on the activity of a polypeptide of the present invention. For example, guidance concerning how to make phenotypically silent amino acid substitutions is provided in Bowie, Science 247: (1990) 1306-1310, wherein the authors indicate that there are two main strategies for studying the tolerance of an amino acid sequence to change.
The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein. The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244: (1989) 1081-1085.) The resulting mutant molecules can then be tested for biological activity.
As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved.
The invention encompasses polypeptides having a lower degree of identity but having sufficient similarity so as to perform one or more of the functions performed by a polypeptide as described herein which is employed in the method of the present invention. Similarity is determined by conserved amino acid substitution. Such substitutions are those that substitute a given amino acid in a polypeptide by another amino acid of like characteristics (e.g., chemical properties). According to Cunningham et al. above, such conservative substitutions are likely to be phenotypically silent. Additional guidance concerning which amino acid changes are likely to be phenotypically silent is found in Bowie, Science 247: (1990) 1306-1310.
Tolerated conservative amino acid substitutions of the present invention involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

In addition, the present invention also encompasses the conservative substitutions provided in the Table below.

**Table IV**

| For Amino Aeid | Code | Replace with anv of: |
|---|---|---|
| Alanine | A D-Ala, | Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gin, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-As |
| Glutamic Acid | E | D-Glu, D-Asp, Asp, Asn, D-Asn, Gln, D-Gln |
| Glycine | G | Ala, D-Ala, Pro, D-Pro, β-Ala, Acp |
| Isoleucine | | D-Ile, Val, D-Val, Leu, D-Leu, Met, D-Met |
| Leucine | L | D-Leu, Val, D-Val, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-1-thioazolidine-4-carboxylic acid, D - or L-1-oxazolidine-4-carboxylic acid |
| Serine | S | D-Ser, Thr, D-Thr, allo-Thr, Met, D-Met, Met(O), D-Met(0), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-ile, Met, D-Met |

Aside from the uses described above, such amino acid substitutions may also increase protein or peptide stability. The invention encompasses amino acid substitutions that contain, for example, one or more non-peptide bonds (which replace the peptide bonds) in the protein or peptide sequence. Also included are substitutions that include amino acid residues other than naturally occurring L-amino acids, e.g., D-amino acids or non-naturally occurring or synthetic amino acids, e.g., β or γ amino acids.

Both identity and similarity can be readily calculated by reference to the following publications: Computational Molecular Biology, Lesk, A.M., ed., Oxford University Press, New York, 1988; Biocomputing: Infoliuaties and Genome Projects, Smith, DM., ed., Academic Press, New York, 1993; Informafies Computer Analyzis of Sequence Data, Part 1, Griffin, A.M., and Griffin, H.G., eds., Humana Press,New Jersey, 1994; Sequence Analyzis in Molecular Biology, von Heinje, G., Academie Press, 1987; and Sequence Analyzis Primer, Gribskov, M. and Devereux, eds., M Stockton Press, New York, 1991.

As mentioned herein above, a polypeptide to be used for binding methylated DNA also encompasses preferably an anti-methylated DNA antibody which is preferably an anti-5-methylcytosine antibody or a Fab, $F(ab')_2$, Fv or scFv fragment thereof. Preferably, said anti-5-methylcytosine antibody specifically binds to methylated DNA, preferably CpG-methylated DNA. The term "specifically" in this context means that said antibody reacts with CpG-methylated DNA, but not with unmethylated DNA and/or DNA methylated at other nucleotides than cytosine and/or DNA methylated at other positions than the C5 atom of cytosine.

Whether the antibody specifically reacts as defined herein above can easily be tested, inter alia, by comparing the binding reaction of said antibody with CpG-methylated DNA and with unmethylated DNA and/or DNA methylated at other nucleotides than cytosine and/or DNA methylated at other positions than the C5 atom of cytosine.

The antibody of the present invention can be, for example, polyclonal or monoclonal. The term "antibody" also comprises derivatives or fragments thereof which still retain the binding specificity such as a Fab, $F(ab')_2$, Fv or scFv fragment. Techniques for the production of antibodies are well known in the art and described, e.g. in Harlow and Lane "Antibodies, A Laboratory Manual", CSH Press, Cold Spring Harbor, 1988. The present invention furthermore includes chimeric, single chain and humanized antibodies, as well as antibody fragments as mentioned above; see also, for example, Harlow and Lane, loc. cit.. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies to polypeptide(s) of this invention. Also, transgenic animals may be used to express humanized antibodies to polypeptides of this invention. Most preferably, the anti-methylated DNA antibody of this invention is a monoclonal antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Techniques describing the production of single chain antibodies (e.g., US Patent 4,946,778) can be adapted to produce single chain antibodies to immunogenic polypeptides as described above. Accordingly, in context of the present invention, the term "antibody molecule" relates to full immunoglobulin molecules as well as to parts of such immunoglobulin molecules. Furthermore, the term relates, as discussed above, to modified and/or altered antibody molecules, like chimeric and humanized antibodies. The term also relates to monoclonal or polyclonal antibodies as well as to recombinantly or synthetically generated/synthesized antibodies. The term also relates to intact antibodies as well as to antibody fragments thereof, like, separated light and heavy chains, Fab, Fab/c, Fv, Fab', F(ab')2. The term "antibody molecule" also comprises bifunctional antibodies and antibody constructs, like single chain Fvs (scFv) or antibody-fusion proteins. It is also envisaged in context of this invention that the term "antibody" comprises antibody constructs which may be expressed in cells, e.g. antibody constructs which may be transfected and/or transduced via, inter alia, viruses or vectors. Of course, the antibody of the present invention can be coupled, linked or conjugated to detectable substances.

Examples of detectable substances include various enzymes, prosthetic groups, fluorescent materials, luminescent materials, bioluminescent materials, radioactive materials, positron emitting metals using various positron emission tomographies, and non-radioactive paramagnetic metal ions. The detectable substance may be coupled or conjugated either directly to an Fc portion of an antibody (or fragment thereof) or indirectly, through an intermediate (such as, for example, a linker known in the art) using techniques known in the art. See, for example, U.S. Patent No. 4,741,900 for metal ions which can be conjugated to an Fc portion of antibodies for use as diagnostics according to the present invention. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a lurninescent material includes luminol; examples of bioluminescent materials include luciferase, luciferin, and aequorin; and examples of suitable radioactive material include $^{125}I$, $^{131}I$,or $^{99}Tc$. Techniques for conjugating coupling or linked compounds to the Fc portion are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy"', in Monoclonal Antibodies For Cancer Detection And Therapy, Baldwin et al. (eds.), pp. 303-16 (Academic Press 1985), and Thorpe, Immunol. Rev., 119-158.

In a preferred embodiment of the present invention, a polypeptide as described herein which is used in the method of the present invention is fused at its N- and/or C-terminus to a heterologous polypeptide for detecting methylated DNA which is preferably selected from the group consisting of a HA-tag, myc6-tag, FLAG-tag, STREP-tag, STREP II-tag, TAP-tag, HAT-tag, chitin-binding domain (CBD), maltose-binding protein, His6-tag, Glutathione-S-transferase (GST) tag, Intein-tag, Streptavidin-binding protein (SBP) tag and a Fc-portion of an antibody. A "tag" is an amino acid sequence which is homologous or heterologous to an amino acid sequence sequence to which it is fused. Said tag may, inter alia, facilitate purification of a protein or facilitate detection of said protein to which it is fused. The fusion refers to a co-linear linkage and results in a translation fusion. In an also further preferred embodiment a polypeptide of the present invention which is capable of binding methylated DNA is fused to a heterologous polypeptide and optionally comprises an additional linker between the N- and/or C-terminus of said polypeptide and said heterologous polypeptide. Said linker is preferably a flexible linker. Preferably, it comprises plural, hydrophilic, peptide-bonded amino acids.

Optionally, the linker comprises a protease cleavage site which allows to cut off the heterologous polypeptide fused to a polypeptide of the present invention, if desirable. Protease cleavage sites are, for example, a thrombin cleavage site.

Preferably, said linker comprises a plurality of glycine, alanine, aspartate, glutamate, proline, isoleucine and/or arginine residues. It is further preferred that said polypeptide linker comprises a plurality of consecutive copies of an amino acid sequence. Usually, the polypeptide linker comprises 1 to 20, preferably 1 to 19, 1 to 18, 1 to 17, 1 to 16 or 1 to 15 amino acids although polypeptide linkers of more than 20 amino acids may work as well.

Preferably, said Fc protein of an antibody comprises preferably at least a portion of the constant region of an immunoglobulin heavy chain molecule. The Fc region is preferably limited to the constant domain hinge region and the $C_H2$ and $C_H3$ domains. The Fc region in a polypeptide of the present invention which is capable of binding methylated DNA and which is employed in the method of the present invention can also be limited to a portion of the hinge region, the portion being capable of forming intermolecular disulfide bridges, and the $C_H2$ and $C_H3$ domains, or functional equivalents thereof.
Alternatively, it is also preferred that the Fc portion comprises at least so many $C_H$ regions which are required such that a polypeptide of the present invention capable of binding methylated DNA has still the properties of a polypeptide described hereinabove, in particular the properties of the polypeptide used in the appended Examples.
In another alternative, it is also preferred that said constant region may contain one or more amino acid substitutions when compared to constant regions known in the art. Preferably it contains 1 to 100, 1 to 90, 1 to 80, 1 to 70, 1 to 60, 1 to 50, 1-40, 1 to 30 or 1 to 20, more preferably 1 to 10, even more preferably 1 to 9, 1 to 8, 1 to 7 or 1 to 6 and most preferably 1 to 5, 1-4, 1 to 3 or 2 or 1 substitution(s). The comparison is preferably done as is known in the art or, more preferably, as described elsewhere herein.
Alternatively, said constant region comprises preferably at least the $C_H1$ region, more preferably the $C_H1$ and $C_H2$ regions and most preferably the $C_H1$, $C_H2$ and $C_H3$ region. As is known in the art, the constant region of an antibody contains two immunoglobulin heavy chains which harbour three characteristic immunoglobulin domains composed of about 110 amino acids, wherein the two immunoglobulin heavy chains are covalently linked via disulfide bonds.
It is also envisaged that the constant region could preferably be of chicken or duck origin. Yet, preferably, the constant region is of the IgM, IgA, IgD or IgE isotype and more preferably it is of the IgG isotype, most preferably of the IgG1 isotype. Preferably, the aforementioned isotypes are of vertebrate origin, more preferably of mammal origin, even more preferably of mouse, rat, goat, horse, donkey, camel or chimpanzee origin and most preferably of human origin. Preferably, said IgG isotype is of class IgG1, IgG2, IgG3, IgG4 and said IgA isotype is of class IgA1, IgA2.

In a further preferred embodiment of the present invention a polypeptide used in the method of the present invention is a fusion protein between the methyl-DNA binding domain of the MBD2 protein and the Fc portion of an antibody as disclosed herein. Optionally the preferred fusion protein comprises a linker polypeptide as described herein, wherein said linker polypeptide is preferably located between the methyl-DNA binding domain of MBD2 and the Fc portion of an antibody.

In accordance with the above described preferred embodiment of the present invention, the further method step comprises optionally amplifying the DNA bound by the polypeptide capable of binding methylated DNA.

The term "amplifying" refers to any method that allows the generation of a multitude of identical or essentially identical (i.e. at least 95% more preferred at least 98%, even more preferred at least 99% and most preferred at least 99.5% such as 99.9% identical) nucleic acid molecules or parts thereof. Such methods are well established in the art and include, but are not limited to NASBA, LM-PCR, PCR, such as conventional or real-time PCR including either single or multiplex conventional or real-time PCR using preferably gene-specific primers.; see Sambrook et al., "Molecular Cloning: A Laboratory Manual", 3rd edition, Cold Spring Harbor Laboratory, 2001. Various PCR techniques, including real-time PCR are reviewed, for example, by Ding, J. Biochem. Mol. Biol. 37 (2004), 1-10.

As mentioned above, a variety of amplification methods are known in the art, all of which are expected to be useful for amplifying DNA bound by a polypeptide described herein which is capable of binding methylated DNA. It is preferred that the amplification in step (c) is effected by PCR. PCR is a powerful technique used to amplify DNA millions of fold, by repeated replication of a template, in a short period of time. The process utilizes sets of specific in vitro synthesized oligonucleotides to prime DNA synthesis. The design of the primers is dependent upon the sequences of the DNA that is desired to be analyzed. It is known that the length of a primer results from different parameters (Gillam (1979), Gene 8, 81-97; Innis (1990), PCR Protocols: A guide to methods and applications, Academic Press, San Diego, USA). Preferably, the primer should only hybridize or bind to a specific region of a target nucleotide sequence. The length of a primer that statistically hybridizes only to one region of a target nucleotide sequence can be calculated by the following formula: $(1/4)^x$ (whereby x is the length of the primer). For example a hepta- or octanucleotide would be sufficient to bind statistically only once on a sequence of 37 kb. However, it is known that a primer exactly matching to a complementary template strand must be at least 9 base pairs in length, otherwise no stable-double strand can be generated (Goulian (1973), Biochemistry 12, 2893-2901). It is also envisaged that computer-based algorithms can be used to design primers capable of amplifying the nucleic acid molecules of the invention. Preferably, the primers of the invention are at least 10 nucleotides in length, more preferred at least 12 nucleotides in length, even more preferred at least 15 nucleotides in length, particularly preferred at least 18 nucleotides in length, even more particularly preferred at least 20 nucleotides in length and most preferably at least 25 nucleotides in length. The invention, however, can also be carried out with primers which are shorter or longer.
The PCR technique is carried out through many cycles (usually 20 - 50) of melting the template at high temperature, allowing the primers to anneal to complimentary sequences within the template and then replicating the template with DNA polymerase. The process has been automated with the use of thermostable DNA polymerases isolated from bacteria that grow in thermal vents in the ocean or hot springs. During the first round of replication a single copy of DNA is converted to two copies and so on resulting in an exponential increase in the number of copies of the sequences targeted by the primers. After just 20 cycles a single copy of DNA is amplified over 2,000,000 fold.

In a particular preferred embodiment, the contacting step as described above is carried out insofar as the protein being capable of binding methylated DNA as described herein is coated onto the walls of preferably a PCR-cycler compatible reaction container, preferably a tube and used to selectively capture methylated DNA and/or DNA-fragments from preferably a genomic DNA mixture. The term "container" encompasses any container which is commonly used and/or suitable for scientific and/or diagnostic purposes. Preferably, said container is composed of the following materials: polystyrene, polyvinyl chloride or polypropylene or the like, more preferably it is composed of polycarbonate. It is also preferred that polystyrene, polyvinyl chloride, polypropylene or polycarbonate is thermocycler-compatible, i.e. it is preferably heat-stable and/or durable at different temperatures for different time intervals. It is moreover preferred that polystyrene, polyvinyl chloride, polypropylene or polycarbonate are inert to chemical and/or biological agents used in connection with the method of the present invention.

It is also a preferred embodiment that the specific DNA and/or DNA-fragment (e.g. a CpG island promoter of a specific gene) which is bound by the polypeptide capable of binding methylated DNA may be, if deemed necessary by the skilled person, amplified in the same container which is used for contacting DNA with said polypeptide capable of binding methylated DNA. Briefly, in a first step preferably a methyl-CpG-binding polypeptide is preferably added into a coatable PCR-vessel, for example, TopYield Strips from Nunc. In doing so, the polypeptide is preferably coated onto the inner surface of said vessel by techniques known in the art and described herein. In a next step, blocking reagents, e.g., about 5% milk powder are added into the coated PCR vessel. In a further step, preferably DNA-fragments of interest (for example, methylated and/or unmethylated DNA-fragments (the term "CpG-methylation low" used in Figure 1 A and B comprises and particularly refers to unmethylated DNA)) are added into the coated and blocked PCR vessel. It is believed that the methyl-CpG-binding polypeptide binds specifically to methylated DNA, if present. In a following step, the coated and blocked PCR vessel containing preferably DNA-fragments is incubated and then washed to remove unbound DNA-fragments. Afterwards, if a genome-wide approach for profiling the methylation status of DNA from a subject is envisaged, LM-PCR is employed to amplify, preferably in a representative manner, the DNA bound by the polypeptide capable of binding methylated DNA. Alternatively, a gene-specific PCR may be employed, if specific regions within said DNA should be amplified. If so, a PCR mix including preferably gene-specific primers or, but also preferred, at least two, three, four, five, six, seven etc. pairs of primers for, e.g., multiplex PCR for the gene or genlocus or genloci of interest which is/are suspected to be methylated or unmethylated is added to run preferably, a real time PCR or conventional PCR. However, for a genome-wide profiling, it is preferred that, if amplification is deemed necessary, LM-PCT is applied so as to amplify the entire genome in a representative manner.

In accordance with the above described preferred embodiment of the present invention, the last method step comprises determining the methylation status of the DNA bound by the polypeptide capable of binding methylated DNA. The methylation status of the DNA bound by a polypeptide capable of binding methylated DNA may be analyzed by restriction enzyme digestion, sequencing such as bisulfite sequencing, pyrosequencing or Southern Blot.

For example, sequencing is preferably performed by methods known in the art, for example, automated dideoxy-sequencing using fluorescent dideoxy nucleotides according to the method of Sanger (Proc. Natl. Acad. Sci. 74 (1977), 5463-5467). For automated sequencing the DNA to be sequenced is prepared according to methods known in the art and preferably according to the instructions of the kit used for preparing said DNA for sequencing.

However, the determination of the methylation status of said DNA is not limited to the aforementioned methods but includes all other suitable methods known in the art for detecting methylated DNA such as RDA. Preferably, the methylation status of said DNA is analyzed by microarrays and the like.

Methods for creating microarrays by deposition and fixation of nucleic acids onto support substrates are well known in the art. Reviewed in DNA Microarrays : A Practical Approach (Practical Approach Series), Schena (ed. ), Oxford University Press (1999) (ISBN:0199637768) ; Nature Genet. 21(1) (suppl): 1-60 (1999); Microarray Biochip : Tools and Technology, Schena (ed.), Eaton Publishing Company/BioTechniques Books Division (2000) (ISBN: 1881299376).

If a microarray is used for determining the methylation status, a DNA microarray containing genomic DNA sequences is preferred. e.g. CpG Island microarrays as in Example 9 (sold by UHN Microarray Centre, Toronto, Ontario) or high-density DNA microarrays representing gene promoter regions such as enhancers, CpG islands, for example, located in exons and/or introns or in intergenomic regions, or large genomic regions, including the whole genome (corresponding microarrays are sold by Agilent, Nimblegene and Affymetrix). When applying a microarray, the DNA whose methylation status is to be determined is preferably labelled prior to bringing it into contact with said microarray.

Suitable labels include, by way of example and not limitation, radioisotopes, fluorophores, chromophores, chemiluminescent moieties, etc. Due to their case of detection, DNA labeled with fluorophores is preferred. Fluorophores suitable for labeling DNA are described, for example, in the Molecular Probes catalog (Molecular Probes, Inc., Eugene, OR), and the references cited therein. Methods for attaching fluorophore labels to DNA are well known, and can be found, for example, in Goodchild, Bioconjug Chem. 1: 165-187 (1990). A preferred fluorophore label is Cy5 dye, which is available from Amersham Biosciences.
Alternatively, the DNA may be labeled by any other technique known in the art. Preferred techniques include direct chemical labeling methods and enzymatic labeling methods, such as kinasing and nick-translation.
In general, the label can be attached to any part of the DNA, including the free terminus of one or more of the bases. In preferred embodiments, the label is attached to a terminus of the DNA. Preferably, the position of the label will not interfere with hybridization, ligation, cleavage or other post-hybridization modifications of the labeled DNA.
Some embodiments of the invention employ multiplexing, i. e. the use of a plurality of distinguishable labels (such as different fluorophores). Multiplexing allows the simultaneous detection of a plurality of sequences in one hybridization reaction. For example, a multiplex of four colors reduces the number of hybridizations required by an additional factor of four.

Of course, in the context of employing a microarray for determining the methylation status of a DNA, also the target nucleic acids attached to the solid support of the microarray may be labelled. Accordingly, the afore described embodiments for labelling DNAs apply to the target nucleic acids attached to the solid support of the microarray, mutatis mutandis.

In one preferred embodiment of the invention, a labeled DNA is bound by means of complementary base-pairing interactions to a detectably labeled target nucleic acid that is itself attached to a solid substrate as part of a microarray, thereby forming a duplex.
Nucleotide bases "match" or are "complementary" if they form a stable duplex or binding pair under specified conditions. The specificity of one base for another is dictated by the availability and orientation of hydrogen bond donors and acceptors on the bases. For example, under conditions commonly employed in hybridization assays, adenine ("A") matches thymine ("T"), but not guanine ("G") or cytosine ("C"). Similarly, G matches C, but not A or T. Other bases which interact in less specific fashion, such as inosine or the Universal Base ("M" base, Nichols et al., Nature 369: 492-493 (1994), or other modified bases, for example methylated bases, are complementary to those bases for which they form a stable duplex under specified conditions. Nucleotide bases which are not complementary to one another are termed "mismatches". A pair of nucleic acid molecules, e. g. a DNA and a target nucleic acid, are termed "complementary" or a "match" if, under specified conditions, the nucleic acids hybridize to one another in an interaction mediated by the pairing of complementary nucleotide bases, thereby forming a duplex. A duplex formed between two nucleic acid molecules may include one or more base mismatches. Such a duplex is termed a "mismatched duplex" or "heteroduplex". The less stringent the hybridization conditions are, the more likely it is that mismatches will be tolerated and relatively stable mismatched duplexes can be formed.
A subset of matched nucleic acid molecules, termed "perfectly complementary" or "perfectly matched" nucleic acid molecules, is composed of pairs of nucleotides containing continuous sequences of bases that are complementary to one another and wherein there are no mismatches (i.e. absent any surrounding sequence effects, the duplex formed has the maximal binding energy for the particular nucleotide sequences). "Perfectly complementary" and "perfect match" are also meant to encompass nucleic acid molecules and duplexes which have analogs or modified nucleotides. A "perfect match" for an analog or modified nucleotide is judged according to perfect match rule selected for that analog or modified nucleotide (e. g. the binding pair that has maximal binding energy for a particular analog or modified nucleotide).

In a particularly preferred embodiment of the invention, a microarray is provided wherein target DNA fragments are provided on a solid substrate under conditions which permit them to hybridize with at least one set of detectably labeled DNAs provided in solution. Both within the sets and between the sets the DNAs may be of the same length or of different lengths. Guidelines for determining appropriate hybridization conditions can be found, e.g. in WO 98/31836. For example, if the methylation status of any of the nucleic acid molecules having the nucleotide sequence of any one of SEQ ID NOs: 1-111 an allelic variant, a portion or the complementary strand of any of said nucleotide sequences as described herein above may be determined by employing a microarray, it is envisaged that the microarray contains one or more nucleic acid molecule having a nucleotide sequence which is/are capable of forming a duplex with one or more of the nucleotide sequences of SEQ ID NOs: 1-111 an allelic variant, a portion or the complementary strand of any of said nucleotide sequences as explained herein above.

The embodiments disclosed in connection with the method of the present invention apply, mutatis mutandis, to the kit for performing the method of the present invention. Preferably, said kit is a diagnostic kit.
Said kit comprises a polypeptide capable of binding methylated DNA as described herein and probes, primers and/or aptamers specific for at least one nucleic acid molecule having a nucleotide sequence of any one of SEQ ID NOs. 1, 2, 3 or 4, an allelic variant, a portion or the complementary strand of any of said nucleotide sequences. Preferably, said kit further comprises probes, primers and/or aptamers specific for at least one nucleic acid molecule having a nucleotide sequence of any one of SEQ ID NOs: 5-16, 17-48 or 49-111, an allelic variant, a portion or the complementary strand of any of said nucleotide sequences. Specificity of a primer, probe or aptamers is explained herein above with respect to the employment of a microarray and is thus applicable, mutatis mutandis, with respect to the specificity of a primer, probe or aptamers. The term "primer" encompasses also a pair of primers specific for at least one nucleic acid molecule of any one of SEQ ID NOs. 1-111.

Advantageously, the kit of the present invention further comprises, optionally (a) reaction buffer(s), storage solutions, wash solutions and/or remaining reagents or materials required for the conduction of scientific or diagnostic assays or the like as described herein. Furthermore, parts of the kit of the invention can be packaged individually in vials or bottles or in combination in containers or multicontainer units.

The kit of the present invention may be advantageously used, inter alia, for performing the methods as described herein and/or it could be employed in a variety of applications referred herein, e.g., as diagnostic kits, as research tools or therapeutic tools. Additionally, the kit of the invention may contain means for detection suitable for scientific, medical and/or diagnostic purposes. The manufacture of the kits follows preferably standard procedures which are known to the person skilled in the art. The kit of the present invention is preferably useful in a "single-tube" assay as provided herein. The kit of the present invention may further comprise instructions for use.

In another aspect, the present invention relates to a diagnostic composition comprising at least one nucleic acid molecule having a nucleotide sequence of any one of SEQ ID NOs: 1-4, 5-16, 17-48 or 49-111, an allelic variant, a portion or the complementary strand of any of said nucleotide sequences. Said diagnostic composition optionally further comprising suitable means for detection as described herein. The diagnostic composition may be used, inter alia, for the same purposes as the kit of the present invention as described herein.

A further aspect of the present invention is the use of at least one nucleic acid molecule having a nucleotide sequence of any one of SEQ ID NOs: 1, 2, 3 or 4, an allelic variant, a portion or the complementary strand of any of said nucleotide sequences for the preparation of a diagnostic composition for determining a predisposition of cancer or cancer by determining the methylation status of its corresponding genomic DNA in a sample from a subject. Preferably, said diagnostic composition further comprises at least one nucleic acid molecule having a nucleotide sequence of SEQ ID NOs: 5-16, 17-48 or 49-111, an allelic variant, a portion or the complementary strand of any of said nucleotide sequences.

**References**

Reference List

(1) Costello JF, Plass C. Methylation matters. J Med Genet 2001 May;38(5):285-303.

(2) Esteller M, Fraga MF, Paz MF, Campo E, Colomer D, Novo FJ, et al. Cancer epigenetics and methylation. Science 2002 Sep 13;297(5588):1807-8.

(3) Ng HH, Bird A. DNA methylation and chromatin modification. Curr Opin Genet Dev 1999 Apr;9(2):158-63.

(4) Razin A. CpG methylation, chromatin structure and gene silencing-a three-way connection. EMBO J 1998 Sep 1;17(17):4905-8.

(5) Robertson KD. DNA methylation and human disease. Nat Rev Genet 2005 Aug;6(8):597-610.

(6) Herman JG, Baylin SB. Gene silencing in cancer in association with promoter hypermethylation. N Engl J Med 2003 Nov 20;349(21):2042-54.

(7) Momparler RL. Cancer epigenetics. Oncogene 2003 Sep 29;22(42):6479-83.

(8) Plass C. Cancer epigenomics. Hum Mol Genet 2002 Oct 1;11(20):2479-88.

(9) Costello JF, Fruhwald MC, Smiraglia DJ, Rush LJ, Robertson GP, Gao X, et al. Aberrant CpG-island methylation has non-random and tumour-type-specific patterns. Nat Genet 2000 Feb;24(2):132-8.

(10) Esteller M, Corn PG, Baylin SB, Herman JG. A gene hypermethylation profile of human cancer. Cancer Res 2001 Apr 15;61(8):3225-9.

(11) Esteller M. Dormant hypermethylated tumour suppressor genes: questions and answers. J Pathol 2005 Jan; 205(2):172-80.

(12) Kalebic T. Epigenetic changes: potential therapeutic targets. Ann N Y Acad Sci 2003 Mar;983:278-85.

(13) Esteller M. Relevance of DNA methylation in the management of cancer. Lancet Oncol 2003 Jun;4(6):351-8.

(14) Dahl C, Guldberg P. DNA methylation analysis techniques. Biogerontology 2003;4(4):233-50.

(15) Krause SW, Rehli M, Kreutz M, Schwarzfischer L, Paulauskis JD, Andreesen R. Differential screening identifies genetic markers of monocyte to macrophage maturation. J Leukoc Biol 1996 Oct;60(4):540-5.

(16) Frommer M, McDonald LE, Millar DS, Collis CM, Watt F, Grigg GW, et al. A genomic sequencing protocol that yields a positive display of 5-methylcytosine residues in individual DNA strands. Proc Natl Acad Sci U S A 1992 Mar 1;89(5):1827-31.

(17) Haehnel V, Schwarzfischer L, Fenton MJ, Rehli M. Transcriptional regulation of the human toll-like receptor 2 gene in monocytes and macrophages. J Immunol 2002 Jun 1;168(11):5629-37.

(18) Cross SH, Charlton JA, Nan X, Bird AP. Purification of CpG islands using a methylated DNA binding column. Nat Genet 1994 Mar;6(3):236-44.

(19) Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet 1997 Mar;6(3):387-95.

(20) Chim CS, Wong AS, Kwong YL. Epigenetic inactivation of INK4/CDK/RB cell cycle pathway in acute leukemias. Ann Hematol 2003 Dec;82(12):738-42.

(21) Dodge JE, List AF, Futscher BW. Selective variegated methylation of the p15 CpG island in acute myeloid leukemia. Int J Cancer 1998 Nov 23;78(5):561-7.

(22) Dodge JE, Munson C, List AF. KG-1 and KG-1 a model the p15 CpG island methylation observed in acute myeloid leukemia patients. Leuk Res 2001 Oct;25(10):917-25.

(23) Issa JP, Zehnbauer BA, Civin CI, Collector MI, Sharkis SJ, Davidson NE, et al. The estrogen receptor CpG island is methylated in most hematopoietic neoplasms. Cancer Res 1996 Mar 1;56(5):973-7.

(24) Paz MF, Wei S, Cigudosa JC, Rodriguez-Perales S, Peinado MA, Huang TH, et al. Genetic unmasking of epigenetically silenced tumor suppressor genes in colon cancer cells deficient in DNA methyltransferases. Hum Mol Genet 2003 Sep 1;12(17):2209-19.

(25) Douglas DB, Akiyama Y, Carraway H, Belinsky SA, Esteller M, Gabrielson E, et al. Hypermethylation of a small CpGuanine-rich region correlates with loss of activator protein-2alpha expression during progression of breast cancer. Cancer Res 2004 Mar 1;64(5):1611-20.

(26) Schwab J, Illges H. Regulation of CD21 expression by DNA methylation and histone deacetylation. Int Immunol 2001 May;13(5):705-10.

(27) Sato K, Tamura G, Tsuchiya T, Endoh Y, Usuba O, Kimura W, et al. Frequent loss of expression without sequence mutations of the DCC gene in primary gastric cancer. Br J Cancer 2001 Jul 20;85(2):199-203.

(28) Jones PA, Wolkowicz MJ, Rideout WM, III, Gonzales FA, Marziasz CM, Coetzee GA, et al. De novo methylation of the MyoD1 CpG island during the establishment of immortal cell lines. Proc Natl Acad Sci U S A 1990 Aug;87 (16):6117-21.

(29) Yuan BZ, Durkin ME, Popescu NC. Promoter hypermethylation of DLC-1, a candidate tumor suppressor gene, in several common human cancers. Cancer Genet Cytogenet 2003 Jan 15;140(2):113-7.

(30) Choi MC, Jong HS, Kim TY, Song SH, Lee DS, Lee JW, et al. AKAP12/Gravin is inactivated by epigenetic mechanism in human gastric carcinoma and shows growth suppressor activity. Oncogene 2004 Sep 16;23(42):

7095-103.

(31) Weber M, Davies JJ, Wittig D, Oakeley EJ, Haase M, Lam WL, et al. Chromosome-wide and promoter-specific analyses identify sites of differential DNA methylation in normal and transformed human cells. Nat Genet 2005 Aug; 37(8):853-62.

(32) Costello JF, Smiraglia DJ, Plass C. Restriction landmark genome scanning. Methods 2002 Jun;27(2):144-9.

(33) Smith RJ, Dean W, Konfortova G, Kelsey G. Identification of novel imprinted genes in a genome-wide screen for maternal methylation. Genome Res 2003 Apr;13(4):558-69.

(34) Yan PS, Chen CM, Shi H, Rahmatpanah F, Wei SH, Caldwell CW, et al. Dissecting complex epigenetic alterations in breast cancer using CpG island microarrays. Cancer Res 2001 Dec 1;61(23):8375-80.

(35) Cross SH, Charlton JA, Nan X, Bird AP. Purification of CpG islands using a methylated DNA binding column. Nat Genet 1994 Mar;6(3):236-44.

(36) Brock GJ, Huang TH, Chen CM, Johnson KJ. A novel technique for the identification of CpG islands exhibiting altered methylation patterns (ICEAMP). Nucleic Acids Res 2001 Dec 15;29(24):E123.

(37) Shiraishi M, Chuu YH, Sekiya T. Isolation of DNA fragments associated with methylated CpG islands in human adenocarcinomas of the lung using a methylated DNA binding column and denaturing gradient gel electrophoresis. Proc Natl Acad Sci U S A 1999 Mar 16;96(6):2913-8.

(38) Klose RJ, Sarraf SA, Schmiedeberg L, McDermott SM, Stancheva I, Bird AP. DNA binding selectivity of MeCP2 due to a requirement for A/T sequences adjacent to methyl-CpG. Mol Cell 2005 Sep 2;19(5):667-78.

(39) Fraga MF, Ballestar E, Montoya G, Taysavang P, Wade PA, Esteller M. The affinity of different MBD proteins for a specific methylated locus depends on their intrinsic binding properties. Nucleic Acids Res 2003 Mar 15;31(6): 1765-74.

(40) Rush LJ, Dai Z, Smiraglia DJ, Gao X, Wright FA, Fruhwald M, et al. Novel methylation targets in de novo acute myeloid leukemia with prevalence of chromosome 11 loci. Blood 2001 May 15;97(10):3226-33.

(41) Kelly LM, Englmeier U, Lafon I, Sieweke MH, Graf T. MafB is an inducer of monocytic differentiation. EMBO J 2000 May 2;19(9):1987-97.

## Examples

A better understanding of the present invention and of its many advantages will be seen from the following examples, offered for illustrative purposes only, and are not intended to limit the scope of the present invention in any way. Accordingly, the Examples are included for purposes of illustration and the present invention is limited only by the claims.

### Example 1: Reagents

All chemical reagents used were purchased form Sigma-Aldrich (Taufkirchen, Germany) unless otherwise noted. Oligonucleotides were synthesized and HPLC-purified by Metabion (Planegg-Martinsried, Germany). DNA sequencing was done by Entelechon (Regensburg).

### Example 2: Cells

Human monocytes were isolated as previously described (15). The human myeloid leukemia cell lines THP-1, KG-1, and U937 were grown in RPMI 1640 medium supplemented with 10% fetal calf serum (Gibco BRL, Eggenstein, Germany). For demethylation experiments, U937 cells were grown in the presence of Decitabine (Sigma) and the medium was replaced every 24h. *Drosophila* S2 cells (ATTC) were cultured in Insect-Xpress medium (Bio Whittaker) containing 10% FCS (PAA) in an incubator at 21°C. Fresh peripheral blood samples from 12 patients with newly diagnosed and untreated de novo AML were used for the study. Patients were treated according to the protocol AMLCG-2000 of the German AML Cooperative Group. Written informed consent was obtained from each patient.

**Example 3: DNA preparation**

Genomic DNA from various cell types was prepared using the Blood and Cell Culture DNA Midi Kit from Qiagen. At least 1 μg genomic DNA was digested using *Mse*I or a combination of *Mse*I and *Csp*6I. Completion of the digest was controlled using agarose gel electrophoresis and digested DNA was quantified using PicoGreen dsDNA Quantitation Reagent (Molecular Probes).

**Example 4: Plasmid construction**

To enable an antibody-like detection of double-stranded CpG-methylated DNA, we constructed a vector encoding a fusion protein comprising the methyl-CpG binding domain (MBD) of human methyl-CpG-binding domain 2 (MBD2), a flexible linker polypeptide and the Fc-portion of human IgG1.
Specifically, a cDNA corresponding to the methyl-CpG binding domain (MBD) of human MBD2 (Genbank acc. no. NM_003927; AA 144-230) was PCR-amplified from reverse transcribed human macrophage total RNA using primers MBD2-Nhe_S (5'-AGA TGC TAG CAC GGA GAG CGG GAA GAG G-3') (SEQ ID NO: 115) and MBD2-Not_AS (5'-ATC ACG CGG CCG CCA GAG GAT CGT TTC GCA GTC TC-3') (SEQ ID NO: 116) and Herculase DNA Polymerase (Stratagene). The PCR-product was directly cloned into *Not*I/*Nhe*I-sites of Signal pIg plus vector (Ingenius, R&D Systems) and sequence verified. An *Apa*I/*Nhe*I-fragment of pIg/MBD-Fc, containing the MBD of human MBD2 fused to the Fc-tail of human IgG1, was subcloned into ApaI/SpeI-sites of pMTBiP/V5-His B (Invitrogen) resulting in pMTBip/MBD-Fc.

**Example 5: Protein expression**

The MBD-$F_c$ polypeptide was expressed under the control of a metal-inducible promoter in *Drosophila* S2 Schneider cells, and collected from the supernatant via Protein A affinity chromatography. The purified protein had the expected molecular weight of 40 kDa.
Specifically, 4 x $10^6$ *Drosophila* S2 cells/60 mm cell culture dish were stably transfected with a mixture of 1.5 μg pMTBip/MBD-Fc and 0.3 μg pCoHygro (Invitrogen) using Effectene transfection reagent (Qiagen) according to the manufacturers protocol. For large scale production, 1-5 x $10^8$ cells were cultured in 100-200 ml Insect-Xpress without FCS and hygromycin in 2000 ml roller bottles for two days before the addition of 0.5 mM $CuSO_4$. Culture medium was harvested every 4-7 days and cell culture supernatants were purified using a protein A sepharose column.

**Example 6: Reverse South-Western blot**

To test, whether MBD-$F_c$ was able to detect CpG-methylated DNA, we blotted *in vitro* methylated PCR-fragments with different CpG density onto a nylon membrane using a capillary transfer system equivalent to traditional Southern blotting, however, without denaturing the DNA prior to blotting. As shown in **Figure 1**, using standard immunoblot conditions and MBD-$F_c$ as equivalent to the primary antibody, methylated DNA could be detected on nylon membranes in a linear fashion.
Specifically, DNA fragments were separated by agarose gel electrophoresis and directly blotted (without prior denaturation) onto a Nylon-membrane using a capillary transfer system equivalent to traditional Southern blotting procedures. DNA was UV-crosslinked after transfer and blocked o/n with 5% fat-free powdered milk in TBST (20 mM Tris/HCl pH 7.4, 150 mM NaCl, 0.1% Tween). Blots were washed three times in TBST for 10 min at RT. Blots were then incubated with MBD-$F_c$ (dilution 1/20 000) in TBST with 5 % milk powder for 1 h at RT, washed three times (TBST, 10 min, RT ) and incubated with HRP-conjugated anti-human Fc (1/10000) in TBST with 5% milk powder for 1 h at RT. After three more washes (TBST, 10 min, RT), bands were detected using ECL (Amersham).

**Example 7: Methyl-CpG immunoprecipitation**

We next tested MBD-$F_c$ binding of *in vitro* generated and differentially methylated DNA-fragments in an immunoprecipitation-like approach. PCR fragments of human promoters with varying CpG-density were generated using PCR and *in vitro* CpG-methylated using *Sss*I or left un-methylated. The mixture of methylated and unmethylated DNA fragments was bound to MBD-$F_c$ Protein A sepharose beads and eluted using increasing concentrations of NaCl. Fractions were collected, spin-purified and subjected to agarose gel electrophoresis. As shown in **Figure 2**, the affinity of a methylated fragment increased with the density of methylated CpG-dinucleotide, with unmethylated DNA (CPM promoter fragment) eluting at relatively low salt concentrations and highly methylated DNA (TLR2 promoter fragment) eluting at high salt concentrations. Variation of the amount of input-DNA within the binding capacity of the MBD-$F_c$ polypeptide did not significantly change the elution profile. However, the salt-dependent affinity of methylated DNA was contingent on the density of the MBD-$F_c$ fusion protein on the protein A sepharose beads. To clarify whether this approach allows the detection of differential methylation of a single gene locus, a fragment of the CpG island promoter of CPM was subcloned into the CpG-free plasmid pCpG-mcs (Invivogen). The plasmid was linearized to generate a fragment of the CpG-island promoter flanked with CpG-less sequences on either side. The DNA fragment was then treated with *Sss*I (New England Biolabs) and decreasing amounts of the methyl-donor S-adenosylmethionine. Samples were combined to obtain a mixture of DNA fragments with varying density of CpG methylation, fractionated by MCIp and subjected to bisulfite sequencing. As shown in **Figure 3**, partially in vitro methylated DNA fragments were separable according to their methylation degree in a fractionating approach using increasing concentrations of NaCl.

Specifically, purified MBD-$F_c$ protein (usually 7.5 μg) was added to 50 μl Protein A Sepharose 4 Fast Flow beads (Amersham) in 1 ml TBS and rotated over night on a rotator at 4°C. On the next day, MBD-$F_c$ beads were transferred into SpinX-columns (Sigma-Aldrich) and spin-washed twice with buffer A (20 mM Tris-HCl pH 8.0, 2 mM $MgCl_2$, 0.5 mM EDTA, 300 mM NaCl, 0.1 % NP-40). Digested DNA (usually 300 ng) was added to the washed MBD-$F_c$ beads in 350 μl buffer A and rotated in sealed SpinX-columns for 3 h on a rotator at 4°C. Beads were centrifuged to recover unbound DNA (300 mM-fraction) and subsequently washed with increasing NaCl-concentrations (400 - 600 mM). Flow through of each wash step was either discarded or collected in separate tubes for further analyses. Highly CpG-methylated DNA was eluted with 350 μl buffer E (20 mM Tris-HCl pH 8.0, 2 mM $MgCl_2$, 0.5 mM EDTA, 1000 mM NaCl, 0.1 % NP-40) into a separate tube. Eluted DNA was desalted using Qiaquick PCR Purification Kit (Qiagen). In parallel, 300 ng digested DNA (IN) was resuspended in 350 μl buffer D and desalted using QIAquick PCR Purification Kit (Qiagen). DNA preparations were quantified using PicoGreen dsDNA Quantitation Reagent (Molecular Probes).

**Example 8: Combination of MCIp and real-time PCR to detect the methylation status of specific CpG island promoters**

We assumed that MCIp may be used to discriminate methylated and unmethylated DNA fragments from genomic DNA. To explore this type of application on single gene level, we used the above procedure to precipitate *M*seI-restricted genomic DNA of in vitro SssI-methylated and untreated normal DNA from monocytes of a healthy donor. *M*seI was chosen for DNA fragmentation, because it is known to preferentially cut in regions of low CpG content while leaving many CpG islands uncut (18).

The salt concentration-dependent enrichment of four different CpG-island promoters and a promoter with low CpG density was determined in SssI-methylated and untreated DNA relative to the input-DNA using LightCycler real-time PCR. As a positive control for DNA methylation, we used the *SNRPN* gene promoter that is subject to maternal imprinting with one of its two copies being methylated also in normal cells (19). In normal DNA the two differentially methylated allele-fragments of *SNRPN* were enriched in two separate fractions (s. **Figure 4A**). Only one enriched fraction was observed with SssI-methylated DNA. In the case of *CDKN2B* gene (also known as $p15^{INK4b}$) which is known to be frequently methylated in leukemia cells (20-22) (**Figure 4B**), the fragment was detected mainly in a low salt fraction from normal DNA and in the high salt fraction from SssI-methylated DNA. Similar results were obtained for the human estrogen receptor 1 (*ESR1*) gene and the human Toll-like receptor 2 gene (*TLR2*) (data not shown). The profiles of methylated and unmethylated DNA at the *CHI3L1* locus were significantly different from those of the above tested CpG island promoters (**Figure 4C**). Most of the untreated *CHI3L1*-fragment was recovered at lower NaCl concentrations and a shift was observed towards higher NaCl concentrations when the DNA was SssI-methylated. The average difference between SssI-treated and untreated monocyte DNA at the *CHI3L1* locus is approximately five to six (out of twelve) methylated CpG residues (data not shown), suggesting that the fractionated approach is able to discriminate relatively small differences in CpG methylation. Analysis of the above elution profiles suggests that:

a.) A two to three hundred-fold enrichment of stronger over less methylated genomic fragments can be obtained in either low or high salt fractions;

b.) Fragments with low CpG density are largely excluded from the high salt fraction.

c.) The fractionated MCIp approach may allow the resolution of relatively small differences in CpG methylation density.

To test whether MCIp can detect aberrant hypermethylation in tumor samples, DNA from three leukemia cell lines KG1 (acute myeloid leukemia), U937 (histiocytic malignancy, monocytic), THP-1 (acute monocytic leukemia) as well as from monocytes of a healthy donor were analyzed for *SNRNP, CDKN2B, ESR1* and *TLR2* promoter enrichment in the high salt fraction (s. **Figure 5A**). The *TLR2* gene promoter was enriched in KG-1 and U937 cells, but not in THP-1 or normal cells. The methylation pattern of *TLR*2 was confirmed by bisulfite sequencing (17) (data not shown). Results for *CDKN2B* (methylated in KG-1 and unmethylated in U937) and *ESR1* (methylated in KG-1) were in line with previously published studies (20;22;23). None of the three MseI fragments was significantly enriched in the DNA from normal cells. In concordance with its imprinting-related methylation status the *SNRPN* gene promoter was significantly enriched in all leukemia cell lines as well as in normal cells. These experiments established that the high salt MCIp fraction specifically enriches CpG-rich genomic DNA-fragments with a high degree of CpG methylation.
To test the sensitivity of our approach, decreasing amounts of U937 DNA were analyzed using the MCIp approach. The enrichment of *TLR2* (strong methylation) and *CDKN2B* gene fragments (no methylation) were determined by LightCycler real-time PCR. As shown in **Figure 5B**, a significant enrichment of the TLR2 fragment was achieved using as little as 1 ng of genomic DNA fragments (equivalent to approximately 150 tumor cells) for the MCIp procedure.
Samples derived from tumors may contain significant numbers of normal cells, that are expected to be unmethylated at most CpG islands. To test the linearity of methyl-CpG detection with respect to cell purity, MCIp was performed using mixtures of DNA from normal blood cells and the leukemia cell line KG-1 showing high levels of CpG island methylation at several promoters. As shown in **Figure 5C**, the *TLR2* promoter fragment was only detected in samples containing KG-1 DNA and the signal gradually increased with the proportion of methylated DNA in the sample. Similar results were obtained for the *ESR1* locus (data not shown).

**Real-time genomic PCR**

Enrichment of a specific MseI-fragment in the $MCI_P$ eluate or in $MCI_P$ amplicons was detected and quantified relative to the genomic input by real-time LightCycler-PCR using the Quantitect kit (Qiagen) according to the manufacturer's instructions. Primer sequences are given in the **Table 2**. Cycling parameters were: denaturation 95 °C, 15 min, amplification 95 °C, 15 s, 57 °C, 20 s, 72 °C, 25 s, for 42 cycles. The product size was initially controlled by agarose gel electrophoresis and melting curves were analyzed to control for specificity of the PCR reactions.

Table 2: Gene-specific oligonucleotide primers for real-time amplification of CpG-island fragments

| Gene | Primer sequence (sense & antisense) | Gene | Primer sequence (sense & antisense) |
|---|---|---|---|
| *CBX6* | 5'-AAG CTT CCG CCA TTG CTC TG-3' (SEQ ID NO: 117) | *MLF1* | 5'-AAA TCT GAT AGG CTT CAT CCC ATT TCC-3' (SEQ ID NO: 155) |
| | 5'-TCC GTT CCT GGA CAG CCC-3' (SEQ ID NO. 118) | | 5'-GTC CTG TAT CCG AAA CAT TCT CTG G-3' (SEQ ID NO: 156) |
| *CDKN2B* | 5'-GGC TCA GCT TCA TTA CCC TCC-3' (SEQ ID NO: 119) | *PAX9* | 5'-CTC TGC TTG TCA TAA CTG CAA CTC GG-3' (SEQ ID NO: 157) |
| | 5'-AAA GCC CGG AGC TAA CGA C-3' (SEQ ID NO: 120) | | 5'-TGA TGA CTG TGG ATG GGA GGA TAG G-3' (SEQ ID NO: 158) |
| *CHI3L1* | 5'- ATC ACC CTA GTG GCT CTT CTG C -3' (SEQ ID NO: 121) | *PDE4B* | 5'-CAG GAG GTC TGT GAG GTA GGT G-3' (SEQ ID NO: 159) |
| | 5'- CTT TTA TGG GAA CTG AGC TAT GTG TC -3' (SEQ ID NO: 122) | | 5'-TGT GTA GTA GGT TGT AAC TGC TGA GG-3' (SEQ ID NO: 160) |
| *COL14A1* | 5'-AGA CGC AAT GCA GTT CCA TGG-3' (SEQ ID NO: 123) | *PFC* | 5'-CGT TAC GGG TTT CCT GAT TGG C-3' (SEQ ID NO: 161) |
| | 5'-ATC TCC CTA CAC CGT GAA CCC-3' (SEQ ID NO: 124) | | 5'-GGA ATC TAG GGA GGT CCA GGA G-3' (SEQ ID NO: 162) |
| *CYP1B1* | 5'-TGT TGA ATC CGT GCT TAG TAG AGA CC-3' (SEQ ID NO: 125) | *PLA2G7* | 5'-GTG CTG GTG TCA TTT CTC CCT G-3' (SEQ ID NO: 163) |
| | 5'-CAG AGT AGC ATT CAG AAA GGC AGA TGG-3' (SEQ ID NO: 126) | | 5'-TCT AGC TCC ATT TCT CCT CAG ACC-3' (SEQ ID NO: 164) |
| *CYP27B1* | 5'-CAT CCG TTC TCT CTG GCT GTC C-3' (SEQ ID NO: 127) | *RAB3C* | 5'-TGA GGG ATC GGG CTA TTC GC-3' (SEQ ID NO: 165) |
| | 5'-CTG TCG AGG CTA CAC GAG CTG C-3' (SEQ ID NO: 128) | | 5'-GCC AAG AGA GGA GAT CAA TGC C-3' (SEQ ID NO: 166) |
| *DMRT2* | 5'-CAC GTT TTT GCT AGA GGT GAG GG-3' (SEQ ID NO: 129) | *RAX* | 5'-CAT GGA CAC CCG TGA ATT CCG AG-3' (SEQ ID NO: 167) |
| | 5'-TCC TCC ATC CGT ACT GAC ATA GGG-3' (SEQ ID NO: 130) | | 5'-AGG TAA AGC GCC CAG GTT GAG-3' (SEQ ID NO: 168) |
| *ESR1* | 5'-GAC TGC ACT TGC TCC CGT C-3' (SEQ ID NO: 131) | *RGMA* | 5'-AAA GAC CGT ATC GCA CTC CCT C-3' (SEQ ID NO: 169) |
| | 5'-AAG AGC ACA GCC CGA GGT TAG-3' (SEQ ID NO: 132) | | 5'-CGCAGAGACTGGAAAGAACCG-3' (SEQ ID NO: 170) |

(continued)

| Gene | Primer sequence (sense & antisense) | Gene | Primer sequence (sense & antisense) |
|---|---|---|---|
| *FARP1* | 5'-GCT CCG TAG AGT TCC CGA AAC C-3' (SEQ ID NO: 133) | *RPIB9* | 5'-AAA GAC TCT ACA CTG GCA CCA CG-3' (SEQ ID NO: 171) |
| | 5'-AGC GAA TCC CAT GAC AGT TCC C-3' (SEQ ID NO: 134) | | 5'-TAG TGC CGA CAT TTC TTG CCC-3' (SEQ ID NO: 172) |
| *FNBP1* | 5'-ATC CAA AGG TCT GCA CAA ATG TTC CTG-3' (SEQ ID NO: 135) | *RPP30* | 5'-AGC TTC TAA GTT ACT ATC AGC CCT TCC-3' (SEQ ID NO: 173) |
| | 5'-CGA GGG AGA AAG ATA AGC TGT GGG-3' (SEQ ID NO: 136) | | 5'-GTA TTG TTC CAA CAC TCC CAC GTC C-3' (SEQ ID NO: 174) |
| *HOXD10* | 5-TCT ATA GTG ACG CTA CCT TTC CCG-3' (SEQ ID NO: 137) | *SETBP1* | 5'-TGT GCG TTT CTA GAG GAG CCG-3' (SEQ ID NO: 175) |
| | 5'-CTT GAG AGG ACA ACG ACA TTT AGG G-3' (SEQ ID NO: 138) | | 5'-AAA TCG ATA CCG AAG GGT TCC C-3' (SEQ ID NO: 176) |
| *JUN* | 5'-AGG AGT TAG TGT GAC AGG GTC GC-3' (SEQ ID NO: 139) | *SLITRK3* | 5'-TAC CTC TTA CAA CAC CAG CGA GC-3' (SEQ ID NO: 177) |
| | 5'-CCA AAT CGC ACT CTT ATA TCC TGG C-3' (SEQ ID NO: 140) | | 5'-GGA TCA GTT AGG TGT AAG GAC GTT GG-3' (SEQ ID NO: 178) |
| *JUN (p)* | 5'-ATT GGC TCG CGT CGC TCT C-3' (SEQ ID NO: 141) | *SNRNP* | 5'-TAC ATC AGG GTG ATT GCA GTT CC-3' (SEQ ID NO: 179) |
| | 5'-GGA GCA TTA CCT CAT CCC GTG-3' (SEQ ID NO: 142) | | 5'-TAC CGA TCA CTT CAC GTA CCT TCG-3' (SEQ ID NO: 180) |
| *KLF5* | 5' -AGA CAC TTC ATT TAG TAG CTC TTT GGC G-3' (SEQ ID NO: 143) | *SSIAH2* | 5' -CTG AGA CAC TCC GCT CCA GC-3' (SEQ ID NO: 181) |
| | 5'-GCC CTC TCA CAG CAA GAC CC-3' (SEQ ID NO: 144) | | 5'-TGT TAT TGG CTG TCT CTG CAC CTC-3' (SEQ 1D NO: 182) |
| *KLF11* | 5'-GAC AGC GGG CTA GAT GTC TCC-3' (SEQ ID NO: | *TGIF* | 5'-GTC CGG GAA GGA ACT GTG CTC-3' (SEQ ID NO: |
| | 5'-GTC AGG GGA AGC CGA AAC G-3' (SEQ ID NO: 146) | | 5'-CTG CTC GGG ACA GAA GAG AAC AC-3' (SEQ ID NO: 184) |
| *KLF11 (p)* | 5'-GTT GAG GCC TCT AGG TGG GTC TC-3' (SEQ ID NO: 147) | *TLR2* | 5'-TGT GTT TCA GGT GAT GTG AGG TC-3' (SEQ ID NO: 185) |
| | 5'-CCA CGC TTA TAG GAA CCT CCT GC-3' (SEQ ID NO: 148) | | 5'-CGA ATC GAG ACG CTA GAG GC-3' (SEQ ID NO: 186) |
| *LDLR* | 5'-GGG TAC AAA TAA TCA CTC CAT CCC TG-3' (SEQ ID NO: 149) | *ZFP36L1* | 5'-AAA CAT TGT CCC GAG ACT CAC TTC C-3' (SEQ ID NO: 187) |
| | 5'-TAA ATC CCT CAG ACT CCT CCC G-3' (SEQ ID NO: 150) | | 5'-GTC TGT CCA GCG GCA TTA CC-3' (SEQ ID NO: 188) |
| *MAFB* | 5'-TGT GCA GAC TAT GTA TGG CTC CG-3' (SEQ ID NO: 151) | *ZNF516* | 5'-CAG GTG ATG ATG GAA CCC ACT C-3' (SEQ ID NO: 189) |
| | 5'-AAA CAC TCT GGG AGC CAC AGG-3' (SEQ ID NO: 152) | | 5'-TGC TGC CCT TCA CTT TTC TAC G-3' (SEQ ID NO: 190) |
| *MAFB (p)* | 5'-TCG AGG TGT GTC TTC TGT TCG G-3' (SEQ ID No: 153) | *ZNF516 (p)* | 5'-CCC TCA GTG TGG CAG AAC TTT G-3' (SEQ ID No: 191) |
| | 5'-GAC CTG CTC AAG TTC GAC GTG-3' (SEQ ID NO: 154) | | 5'-CCC AGC CTG GAA ATG GTC-3' (SEQ ID NO: 192) |

**Real-time RT-PCR**

Total RNA was prepared using the RNeasy Midi Kit (Qiagen). Contaminating genomic DNA was removed from the samples using TURBO DNA-free Kit (Ambion) before 1 μg of total RNA was reverse transcribed using M-MLV Reverse Transcriptase, RNase H Minus, Point Mutant (Promega). Real-time PCR was performed using the LightCycler (Roche) with the Quantitect kit (Qiagen) according to the manufacturer's instructions. Primers used are given in **Table 3**. Cycling parameters were as above..

Table 3: Gene-specific oligonucleotide primers for real-time RT-PCR

| Gene | Primer sequence (sense & antisense) |
|---|---|
| *MAFB* | 5'-GAG CCG GAG AGA GAG ACG-3 (SEQ ID NO: 193)' |
| | 5'-AGG AGT CTC CAG ATG GCC TTG-3' (SEQ ID NO: 194) |
| *JUN* | 5'-CGG CGG TAA AGA CCA GAA GG-3' (SEQ ID NO: 195) |
| | 5'-CGC CCA AGT TCA ACA ACC G-3' (SEQ ID NO: 196) |
| *KLF11* | 5'-ACC TAC TTC AAA AGT TCC CAC C-3' (SEQ ID NO: 197) |
| | 5'-CAT GAA ACG TCG GTC ACA CAC-3' (SEQ ID NO: 198) |
| *SSIAH2* | 5'-GTT TCA GCA CTA CAA GGC TAA ACG G-3' (SEQ ID NO: 199) |
| | 5'-AAG CTG CCT TGC TCT GGA GC-3' (SEQ ID NO: 200) |
| *ZNF516* | 5'-GTT CTG AAG TTC ATA CCA CCT CCG-3' (SEQ ID NO: 201) |
| | 5'-TCA GAG GCA CTG TCT GGA CGG-3 (SEQ ID NO: 202) |

**Example 9: CpG island microarray analysis**

To achieve a genome-wide analysis of CpG island DNA methylation, we combined MCIp with CpG island microarray hybridization. A small amount of *Mse*I-restricted DNA (300 ng) of three leukemia cell lines (KG1, U937, THP-1) and of normal human peripheral blood monocytes were subjected to MCIp. The isolated DNA was amplified using ligation-mediated PCR.

The resulting amplicons were directly labeled with Cy5 (normal DNA) and Cy3 (leukemia cell DNA) and each leukemia sample was co-hybridized with the normal control sample to CpG island microarrays (UHN Microarray Centre, Toronto, Canada) (s. **Figure 6**). This array contains 12,192 CpG-island clones from a *Mse*I-CpG DNA library that was originally prepared by MeCP2-column purification of non-methylated CpG island fragments (18). Representative scatter plots of microarray hybridizations are presented in **Figure 6C**. In hybridizations using the amplicons from tumor cell lines, signals corresponding to both hypo- and hypermethylated fragments were observed. Because we were interested in tumor-specific epigenetic silencing, we focused on the analysis of hypermethylated fragments. To identify CpG island fragments that were affected by hypermethylation, results of three independent MCIp experiments (using two different MBD-$F_c$ preparations and three independent DNA preparations) were analyzed in conjunction. Hybridization signals that were consistently different (more than two fold enriched in the leukemia sample) in at least one cell line were selected for further analysis.

In total, THP-1 cells showed 277, U937 cells showed 454 and KG-1 cells 330 differential hybridization signals. 191 out of 535 spots in total were unambiguously annotated and located in the close proximity (approximately +/- 3000bp) to predicted transcriptional start sites and were chosen for further analysis. Several sequences were represented more than once on the CpG island microarray. The final, non-redundant list of differentially represented CpG island DNA fragments contained 131 entries that were in the close proximity to 134 genes (shown in **Table 4**). At least nine of the listed genes (*LMX1A* (24), *TFAP2A* (25), CR2 (26), *DCC* (27), MYOD1 (28), DLEC1 (29), AKAP12 (30), *SSIAH2* (LOC283514) and *FOXF1* (31) have previously been identified as targets of hypermethylation in cancer.

The hypermethylated genes listed in **Table 4** are involved in many biological functions. Most strikingly, half of the genes with an assigned molecular function (46/89) are involved in DNA-binding and transcriptional regulation.

In particular, to generate fluorescently labeled DNA for CpG-island microarray hybridization, Msel-compatible uni-directional LMPCR-Linker (LMPCR_S-L 5'-GCG GTG ACC CGG GAG ATC TCT TAA G-3' and LMPCR_AS-L: 5'-TAC TTA AGA GAT C-3', 20 μM) was ligated to the MCIp-eluted-DNA and in a separate reaction to an equal amount of Input-DNA (0.5 μl Linker /ng DNA) in 60 μl reactions using 1200 u T4-Ligase (NEB) at 16°C o/n. Linker-ligated DNA was desalted using QIAquick PCR Purification Kit (Qiagen). Amplification of linker-ligated DNA preparations was performed using LMPCR-Primer (5'-GTG ACC CGG GAG ATC TCT TAA G-3') and Taq-Polymerase (Roche) in the presence of 1.3 M betaine. Amplicons were desalted using Qiaquick PCR Purification Kit (Qiagen) and quantified (PicoGreen dsDNA Quantitation Reagent, Molecular Probes). Labeling and hybridization of MClp amplicons was done by the KFB (Regensburg) according to the protocol provided by the CpG island microarray manufacturer (Microarray Centre UHN, Toronto, Canada) with modifications. Briefly, four microgram of normal and tumor MCIp-amplicons were directly labeled with Cy5- and Cy3-dUTP, respectively, using the BioPrime® Array CGH Genomic Labeling System (Invitrogen). Ten microgram of each fluorescently labeled and purified DNA amplicon) in 300 μl DIG Easy Hyp Solution (Roche) supplemented with 25 μg Cot-1 DNA (Invitrogen) and 30 μg Yeast tRNA were hybridized to Human CpG 12K Arrays (HCGI12K, Microarray Centre, UHN, Toronto, Canada) in 60x21 mm Gene Frames (ABgene) at 37°C for o/n. Slides were washed three times in 1x SSC, 0.1% SDS at 50°C for 10 min. After two more rinses with 0.1xSSC, slides were dried and scanned using the Affymetrix 428 Scanner. Images were analyzed using the ImaGene 5.6 and Gene Sight Lite software (BioDiscovery, Inc.). LOWESS normalization was used to normalize Cy3 and Cy5 signals. Clones that produced reproducible differential signals on the CpG island microarray were sequenced by the Microarray Centre UHN (Toronto, Canada).

# Table 4: Hypermethylated gene fragments in myeloid leukemia cell lines

| Gene | | | CpG-methylation | | | | mRNA expression | | | | |
|---|---|---|---|---|---|---|---|---|---|---|---|
| Name | Symbol | Location | KG1 | U937 | THP1 | Positon | KG1 | U937 | THP1 | N | Probe Set ID |
| hypothetical gene | LOC400027 | 12q12 | 1.82 | 1.16 | 1.19 | down | NC/P | NC/P | NC/P | A | 226413_at |
| branched chain aminotransferase 2, mitochondrial | BCAT2 | 19q13 | 1.24 | 1.18 | 1.29 | proximal | 1 | 1.8 | NC/P | P | 203576_at |
| chondrolectin | CHODL | 21q11.2 | 1.52 | 1.49 | 1.44 | down | NC/A | NC/A | NC/A | A | 219867_at |
| ***collagen, type XIV, alpha 1 (undulin)*** | ***COL14A1*** | 8q23 | 2.02 | 2.97 | 2.03 | down | NC/A | NC/A | NC/A | A | 1562189_at |
| ***cytochrome P450, family 27, subfamily B, polypeptide 1*** | ***CYP27B1*** | 12q13.1-q13.3 | 1.55 | 1.87 | 2.15 | down | NC/A | NC/P | 0.8 | A | 205676_at |
| v-erb-a erythroblastic leukemia viral oncogene homolog 4 | ERBB4 | 2q33.3-q34 | 1.63 | 1.34 | 1.36 | down | NC/A | NC/A | NC/A | A | 241581_at |
| family with sequence similarity 5, member B | FAM5B | 1q24.1-q25.3 | 2.55 | 1.32 | 1.92 | proximal | NC/A | NC/A | NC/A | A | 214822_at |
| fibroblast growth factor 12 | FGF12 | 3q28 | 1.20 | 1.76 | 1.52 | proximal/down | NC/A | NC/A | NC/A | A | 240067_at |
| hypothetical gene | FLJ13192 | 15q14 | 1.79 | 1.49 | 1.37 | down | NC/A | NC/A | NC/A | A | 233382_at |
| hypothetical gene | FLJ20366 | 8q23.2 | 1.90 | 1.39 | 1.45 | up/down | NC/A | NC/A | NC/A | A | 218692_at |
| hypothetical gene | FLJ36633 | | | | | up | ND | ND | ND | ND | NA |
| hypothetical protein | FLJ20972 | 1p34.2 | 1.46 | 1.29 | 1.82 | down | NC/M | NC/P | NC/P | P | 230897_at |
| hypothetical protein | FLJ35074 | 6p24 | 2.07 | 1.09 | 1.59 | down | NC/A | 1.6 | NC/A | A | 1560503_a_at |
| transcription factor AP-2 alpha | TFAP2A | | | | | up | NC/A | 2.4 | NC/A | A | 204653_at |
| laeverin | FLJ90650 | 5q23.1 | 2.14 | 3.00 | 2.77 | up/down | NC/A | NC/A | NC/A | A | 235382_at |
| *forkhead box F1* | *FOXF1* | 16q24 | 1.40 | 1.84 | 2.72 | down | 2.7 | NC/A | NC/A | A | 205935_at |
| glycoprotein M6A | GPM6A | 4q34 | 1.30 | 2.05 | 1.19 | down | NC/A | NC/A | NC/A | A | 209469_at |
| GS homeobox 2 | GSH2 | 4q11-q12 | 1.34 | 2.29 | 1.26 | down | NC/A | NC/A | NC/A | A | 230338_x_at |
| hypocretin (orexin) receptor 2 | HCRTR2 | 6p11-q11 | 1.08 | 1.46 | 1.62 | down | NC/A | NC/A | NC/A | A | 207393_at |
| Hey-like transcriptional repressor | HELT | 4q35.1 | 1.37 | 3.51 | 1.52 | up | ND | ND | ND | ND | NA |
| homeo box C10 | HOXC10 | 12q13.3 | 1.13 | 1.62 | 1.01 | up | NC/A | 0.8 | NC/P | A | 214562_at |
| iroquois homeobox protein 1 | IRX1 | 5p15.3 | 2.20 | 2.32 | 2.07 | down | NC/A | NC/A | NC/A | A | 230472_at |
| hypothetical protein | KIAA1024 | 15q25.1 | 1.04 | 2.06 | 1.73 | down | NC/A | NC/A | NC/A | A | 215081_at |
| LIM homeobox transcription factor 1, alpha | LMX1A | 1q22-q23 | 2.42 | 2.08 | 2.29 | down | NC/A | NC/A | NC/A | A | 1553541_at |
| ***similar to seven in absentia 2 (SSIAH2)*** | ***LOC283514*** | 13q14.13 | 2.43 | 2.73 | 1.54 | proximal | NC/A | NC/A | NC/A | A | 1560676_at |
| hypothetical protein | MGC12982 | 1p33 | 2.17 | 2.36 | 1.92 | up | NC/P | NC/A | NC/A | A | 207653_at |
| forkhead box D2 | FOXD2 | | | | | down | NC/P | NC/A | NC/A | A | 224457_at |
| hypothetical protein | MGC42090 | 7p21.1 | 2.62 | 4.03 | 1.97 | proximal | NC/A | NC/A | NC/A | A | 1552293_at |
| hypothetical protein | MGC4767 | 12q24.31 | 1.68 | 2.58 | 2.23 | proximal | 1 | 1.2 | NC/P | P | 223114_at |
| *myogenic differentiation 1* | *MYOD1* | 11p15.4 | 2.49 | 2.57 | 1.14 | down | NC/A | NC/A | NC/A | A | 206657_s_at |
| NK2 transcription factor related, locus 3 (Drosophila) | NKX2-3 | 10q24.2 | 2.96 | 4.06 | 1.30 | down | NC/A | NC/A | 3.3 | A | 1553808_a_at |
| one cut domain, family member 1 | ONECUT1 | 15q21.1-q21.2 | 1.58 | 2.64 | 2.49 | up | NC/A | NC/A | NC/A | P | 210745_at |
| protocadherin gamma subfamily B, 1 | PCDHGB1 | 5q31 | 1.12 | 1.69 | 3.19 | proximal | ND | ND | ND | ND | NA |
| ***phospholipase A2, group VII*** | ***PLA2G7*** | 6p21.2-p12 | 1.39 | 1.36 | 2.27 | proximal | -8.9 | -5.1 | -5.7 | P | 206214_at |
| phospholipase D family, member 5 | PLD5 | 1q43 | 1.43 | 1.69 | 1.92 | down | NC/A | NC/P | NC/A | A | 1563933_a_at |
| scinderin | SCIN | 7p21.3 | 1.52 | 1.42 | 1.15 | proximal/up | NC/A | NC/A | NC/A | A | 239365_at |
| ***SLIT and NTRK-like family, member 3*** | ***SLITRK3*** | 3q26.1 | 2.56 | 1.42 | 2.56 | down | NC/A | NC/A | NC/A | A | 206732_at |
| Sp5 transcription factor | SP5 | 2q31.1 | 1.30 | 1.89 | 1.00 | down | NC/A | NC/A | NC/A | A | 235845_at |
| transcription factor AP-2 gamma | TFAP2C | 20q13.2 | 1.60 | 1.02 | 1.07 | up | NC/P | NC/A | NC/A | A | 205286_at |
| transmembrane protein 39A | TMEM39A | 3q13.33 | 1.61 | 1.48 | 1.64 | prom | NC/P | NC/P | 0.6 | P | 222690_s_at |
| zinc finger protein 483 | ZNF483 | 9q31.3 | 1.98 | 1.71 | 2.43 | down | NC/A | NC/P | NC/P | A | 1570534_a_at |
| zinc finger protein 565 | ZNF565 | 19q13.12 | 2.08 | 1.74 | 1.80 | down | NC/P | NC/P | 0.9 | P | 228305_at |
| hypothetical gene | AF086288 | 9p24 | 1.75 | 1.34 | -0.52 | proximal | NC/A | NC/A | NC/A | A | 237421_at |
| hypothetical gene | AY358245 | 15q24 | 1.02 | 1.33 | 0.01 | proximal | ND | ND | ND | ND | NA |
| hypothetical gene | BC026095 | 11q12.1 | 1.40 | 1.26 | -0.11 | down | NC/A | NC/A | NC/A | A | 1570068_at |
| bone morphogenetic protein 4 | BMP4 | 14q22-q23 | 2.07 | 1.10 | 0.82 | down | NC/A | NC/A | NC/A | A | 211518_s_at |
| chromosome 16 open reading frame 45 | C16orf45 | 16p13.11 | 1.00 | 1.17 | -0.23 | proximal | NC/A | NC/A | NC/A | A | 239971_at |
| chromosome 1 open reading frame 126 | C1orf126 | 1p36.21 | 1.84 | 1.44 | -0.40 | proximal | ND | ND | ND | ND | NA |
| chromosome 20 open reading frame 39 | C20orf39 | 20p11.21 | 1.08 | 1.70 | 0.08 | down | NC/P | NC/A | NC/A | A | 231619_at |
| calponin 1, basic, smooth muscle | CNN1 | 19p13.2-p13.1 | 1.31 | 1.27 | -0.37 | proximal | NC/A | NC/A | NC/A | A | 203951_at |
| hypothetical gene | CR611340 | 6p22.1 | 2.15 | 1.70 | 0.38 | proximal | ND | ND | ND | ND | NA |
| chemokine (C-X-C motif) ligand 5 | CXCL5 | 4q12-q13 | 1.22 | 1.35 | 0.84 | proximal | NC/A | NC/A | NC/A | A | 207852_at |
| ***cytochrome P450, family 1, subfamily B, polypeptide 1*** | ***CYP1B1*** | 2p21 | 1.25 | 2.83 | 0.90 | down | 1.7 | -6.6 | -2.1 | P | 202435_s_at |
| fatty acid desaturase 3 | FADS3 | 11q12-q13.1 | 1.01 | 1.15 | -0.13 | up | NC/A | NC/A | NC/A | A | 204257_at |
| hypothetical protein | FLJ42262 | 8q12.3 | 2.67 | 2.22 | 0.96 | up | NC/A | NC/A | -3.4 | A | 242193_at |
| ***homeo box D10*** | ***HOXD10*** | 2q31.1 | 1.91 | 1.70 | 0.72 | up | -1.5 | -1.2 | NC/A | A | 229490_at |
| hypothetical protein | KIAA1465 | 15q24.1 | 1.34 | 1.49 | 0.30 | up/down | NC/A | NC/A | NC/A | A | 232208_at |
| ***Kruppel-like factor 5*** | ***KLF5*** | 13q22.1 | 1.82 | 2.09 | 0.29 | down | -3.7 | NC/A | NC/A | A | 209212_s_at |
| ladybird homeobox homolog 1 (Drosophila) | LBX1 | 10q24 | 1.03 | 1.64 | 0.97 | up | NC/A | NC/A | NC/A | A | 208380_at |
| LIM homeobox 9 | LHX9 | 1q31-q32 | 2.65 | 1.60 | 0.59 | up/down | NC/A | NC/A | NC/A | A | 1565407_at |
| hypothetical protein | LOC282992 | 10q24.32 | 1.52 | 2.34 | 0.60 | down | NC/A | NC/A | NC/A | A | 244209_at |
| ***myeloid leukemia factor 1*** | ***MLF1*** | 3q25.1 | 1.68 | 1.79 | 0.23 | proximal | -2.9 | -1.3 | 2 | A | 204784_s_at |
| 5'-nucleotidase, cytosolic IA | NT5C1A | 1p34.3-p33 | 1.53 | 1.57 | 0.80 | up | NC/A | NC/A | NC/A | A | 224529_s_at |
| ***phosphodiesterase 4B, cAMP-specific*** | ***PDE4B*** | 1p31 | 1.64 | 1.01 | -0.28 | proximal | -4.7 | -3.6 | -4.8 | P | 211302_s_at |
| ***properdin P factor, complement*** | ***PFC*** | Xp11.3-p11.23 | 1.31 | 1.79 | 0.44 | down | -5.9 | -7.8 | -8.2 | P | 206380_s_at |
| ***retina and anterior neural fold homeobox*** | ***RAX*** | 18q21.32 | 1.54 | 1.27 | -0.26 | down | NC/A | NC/A | NC/A | A | 208242_at |
| ***RGM domain family, member A*** | ***RGMA*** | 15q26.1 | 1.33 | 1.92 | 0.46 | up | -0.9 | -0.8 | NC/A | A | 223468_s_at |
| ***Rap2-binding protein 9*** | ***RPIB9*** | 7q21.12 | 1.31 | 1.68 | 0.20 | up | 6.7 | NC/A | NC/A | A | 215321_at |
| SHC (Src homology 2 domain containing) family, member 4 | SHC4 | 15q21.1-q21.2 | 1.45 | 1.48 | -0.11 | up | NC/A | NC/P | NC/A | A | 230538_at |
| ***SET binding protein 1*** | ***SETBP1*** | 18q21.1 | 1.47 | 1.75 | -0.07 | down | -0.9 | -0.7 | -2.8 | P | 205933_at |
| SRY (sex determining region Y)-box 9 | SOX9 | 17q24.3-q25.1 | 1.49 | 1.00 | 0.71 | up | NC/A | NC/A | NC/A | A | 202935_s_at |
| transcription factor 2, hepatic | TCF2 | 17cen-q21.3 | 1.99 | 2.92 | -0.35 | up | NC/A | NC/A | NC/A | A | 205313_at |
| ELAV (embryonic lethal, abnormal vision, Drosophila)-like 2 | ELAVL2 | 9p21 | 1.15 | 0.61 | 1.55 | up/down | NC/A | NC/A | NC/A | A | 1560905_at |
| forkhead box A1 | FOXA1 | 14q12-q13 | 1.03 | 0.92 | 1.47 | up | NC/A | NC/A | NC/A | A | 204667_at |
| potassium channel, subfamily T, member 2 | KCNT2 | 1q31.3 | 2.52 | 0.63 | 1.45 | up | NC/A | NC/A | NC/A | A | 244455_at |
| multiple PDZ domain protein | MPDZ | 9p24-p22 | 2.14 | 0.93 | 1.79 | down | NC/A | NC/A | NC/A | A | 213306_at |
| ***paired box gene 9*** | ***PAX9*** | 14q12-q13 | 1.41 | 0.59 | 1.48 | up | NC/M | NC/A | NC/A | A | 207059_at |
| serum deprivation response | SDPR | 2q32-q33 | 1.19 | 0.52 | 1.61 | down | 1.9 | -5.4 | -1.9 | P | 222717_at |
| *complement component (3d/Epstein Barr virus) receptor 2* | *CR2* | 1q32 | 1.45 | 0.55 | -0.17 | down | NC/A | NC/A | NC/A | A | 244097_at |
| hypothetical protein | FLJ40542 | 22q11.21 | 1.36 | 0.43 | 0.60 | down | -1.5 | -1 | -2.8 | P | 1556072_at |
| glial cell derived neurotrophic factor | GDNF | 5p13.1-p12 | 1.35 | 0.37 | 0.04 | up/down | NC/A | NC/A | NC/A | A | 221359_at |
| ***Kruppel-like factor 11*** | ***KLF11*** | 2p25 | 1.79 | 0.86 | -0.18 | up | -3.8 | -3.1 | -1.4 | P | 218486_at |
| LIM homeobox 4 | LHX4 | 1q25.2 | 2.26 | 0.89 | 0.51 | down | NC/A | NC/P | NC/A | A | 1553157_at |
| zinc finger protein 215 | ZNF215 | 11p15.4 | 1.82 | 0.78 | 0.43 | proximal | NC/A | NC/A | NC/A | A | 1555510_at |
| *A kinase (PRKA) anchor protein (gravin) 12* | *AKAP12* | 6q24-q25 | 0.90 | 1.50 | 1.66 | up | -3.3 | -6.1 | -5.4 | P | 210517_s_at |
| hypothetical gene | LOC389372 | 6p22.1 | 0.46 | 2.99 | 2.21 | proximal | NC/A | NC/A | NC/A | A | 1568826_at |
| hypothetical protein | FLJ10159 | 6q21 | 0.78 | 2.25 | 1.46 | up | NC/A | NC/A | NC/A | A | 1563906_at |
| ***formin binding protein 1*** | ***FNBP1*** | 9q34 | 0.47 | 1.18 | 1.09 | up | NC/P | -2.7 | -3 | P | 230389_at |
| homeo box A9 | HOXA9 | 7p15-p14 | -0.02 | 2.14 | 1.09 | up | 5.8 | 6.3 | 4.6 | A | 209905_at |
| zinc finger protein 312-like | LOC389549 | 7q31.32 | ND | 1.78 | 1.09 | up | ND | ND | ND | ND | NA |
| ***v-maf musculoaponeurotic fibrosarcoma oncogene B*** | ***MAFB*** | 20q11.2-q13.1 | 0.40 | 1.38 | 1.12 | up | -10.9 | -10.6 | -6.1 | P | 218559_s_at |
| hypothetical protein | MGC33530 | 7p11.2 | 0.99 | 1.06 | 1.84 | proximal | NC/A | NC/A | NC/A | A | 1554530_at |
| netrin 4 | NTN4 | 12q22-q23 | 0.91 | 2.82 | 2.43 | up | NC/A | NC/A | NC/A | A | 234202_at |
| orthopedia homolog (Drosophila) | OTP | 5q13.3 | 0.89 | 1.45 | 1.40 | down | NC/A | NC/A | NC/A | A | 237906_at |

| | | | | | | | | | | | |
|---|---|---|---|---|---|---|---|---|---|---|---|
| protocadherin 19 | PCDH19 | Xq13.3 | 0.45 | **1.45** | **1.51** | down | NC/A | NC/A | NC/A | A | 227282_at |
| protein tyrosine phosphatase, receptor type, K | PTPRK | 6q22.2-23.1 | 0.80 | **1.52** | **1.75** | down | NC/A | NC/A | NC/A | A | 233770_at |
| toll-IL 1 receptor (TIR) domain containing adaptor protein | TIRAP | 11q24.2 | 0.83 | **1.12** | **1.46** | up | NC/P | NC/P | NC/P | P | 1552360_a_at |
| zinc finger protein 37 homolog (mouse) | ZFP37 | 9q32 | 0.91 | **1.71** | **1.24** | proximal | NC/A | NC/A | NC/A | A | 207068_at |
| zinc finger protein 229 | ZNF229 | 19q13.31 | 0.23 | **2.64** | **1.37** | proximal | NC/P | NC/A | NC/A | A | 1562789_at |
| zinc finger protein 312 | ZNF312 | 3p14.2 | 0.22 | **2.02** | **1.05** | up | NC/A | NC/A | NC/A | A | 221086_s_at |
| zinc finger protein 629 | ZNF629 | 16p11.2 | 0.98 | **1.27** | **1.50** | down | -1.7 | NC/P | 1.2 | P | 213196_at |
| *deleted in colorectal carcinoma* | *DCC* | 18q21.3 | 0.71 | **1.30** | 0.34 | proximal | NC/A | NC/A | NC/A | A | 206939_at |
| *deleted in lung and esophageal cancer 1* | *DLEC1* | 3p22-p21.3 | 0.91 | **1.32** | -0.12 | proximal | NC/A | NC/A | NC/A | A | 207896_s_at |
| distal-less homeo box 3 | DLX3 | 17q21 | 0.21 | **1.22** | -0.03 | down | NC/A | NC/A | NC/A | A | 231778_at |
| dual oxidase 2 | DUOX2 | 15q15.3 | 0.82 | **1.97** | -0.25 | down | NC/A | NC/A | NC/A | A | 219727_at |
| endothelial PAS domain protein 1 | EPAS1 | 2p21-p16 | 0.21 | **2.57** | -0.46 | down | -3.9 | 1.8 | 1.6 | P | 200878_at |
| EPH receptor A10 | EPHA10 | 1p34.3 | 0.65 | **1.15** | 0.54 | up | NC/A | NC/A | NC/A | A | 243717_at |
| ES cell expressed Ras | ERAS | Xp11.23 | 0.99 | **1.00** | 0.62 | up | ND | ND | ND | ND | NA |
| ***FERM, RhoGEF and pleckstrin domain protein 1*** | ***FARP1*** | 13q32.2 | 0.44 | **2.57** | 0.44 | proximal/up | NC/A | NC/A | NC/A | A | 227996_at |
| hypothetical protein | FLJ42461 | 17p13.2 | 0.45 | **2.18** | 0.40 | proximal | NC/A | NC/A | NC/A | A | 229730_at |
| gamma-glutamyl hydrolase | GGH | 8q12.3 | 0.28 | **3.10** | -0.10 | proximal | 4.4 | -2.9 | 2.6 | P | 203560_at |
| glycoprotein V (platelet) | GP5 | 3q29 | 0.08 | **1.36** | -0.18 | down | NC/P | NC/P | NC/A | A | 207926_at |
| hyperpolarization activated cyclic nucleotide-gated K+4 | HCN4 | 15q24-q25 | 0.68 | **1.28** | 0.53 | up | NC/A | NC/A | NC/A | A | 206946_at |
| histone 1, H4I | HIST1H4L | 6p22-p21.3 | -0.63 | **3.21** | ND | proximal | NC/A | NC/A | NC/A | A | 214562_at |
| ***v-jun sarcoma virus 17 oncogene homolog (avian)*** | ***JUN*** | 1p32-p31 | 0.11 | **2.07** | -0.08 | up | -4.5 | -5.1 | -4.3 | P | 201466_s_at |
| potassium channel beta 3 chain | KCNAB1 | 3q26.1 | 0.66 | **1.48** | 0.98 | down/down | NC/A | 5.4 | NC/A | A | 210471_s_at |
| protocadherin 8 | PCDH8 | 13q14.3-q21.1 | 0.79 | **1.66** | -0.09 | up | NC/P | NC/P | NC/P | A | 206935_at |
| RAB38, member RAS oncogene family | RAB38 | 11q14 | 0.53 | **1.37** | 0.55 | down | NC/P | NC/P | NC/P | A | 234666_at |
| ***RAB3C, member RAS oncogene family*** | ***RAB3C*** | 5q13 | 0.00 | **1.57** | 0.19 | proximal | NC/A | NC/A | 1.7 | A | 242328_at |
| ***ribonuclease P/MRP 30kDa subunit*** | ***RPP30*** | 10q23.31 | -0.22 | **1.68** | -0.14 | up | 0.4 | -1 | NC/P | P | 203436_at |
| secretogranin III | SCG3 | 15q21 | 0.96 | **2.30** | 0.12 | down | NC/A | NC/A | NC/A | A | 219196_at |
| sonic hedgehog homolog (Drosophila) | SHH | 7q36 | 0.99 | **1.29** | 0.50 | down | NC/A | NC/A | NC/M | A | 207586_at |
| synaptojanin 2 | SYNJ2 | 6q25.3 | 0.01 | **1.15** | 0.07 | up | 2.3 | 2.2 | 1.3 | P | 212828_at |
| ***zinc finger protein 36, C3H type-like 1*** | ***ZFP36L1*** | 14q22-q24 | -0.02 | **2.07** | -0.03 | down | -1.2 | -5.5 | -3.8 | P | 211965_at |
| zinc finger protein 222 | ZNF222 | 19q13.2 | 0.06 | **1.56** | 0.00 | proximal | 2.2 | NC/A | 3.4 | A | 206175_x_at |
| ***zinc finger protein 516*** | ***ZNF516*** | 18q23 | -0.51 | **1.86** | -0.12 | down | NC/P | -1.6 | NC/P | P | 203604_at |
| zinc finger protein 582 | ZNF582 | 19q13.43 | 0.10 | **1.27** | 0.47 | proximal | NC/P | NC/P | NC/P | A | 1553221_at |
| zinc finger protein 610 | ZNF610 | 19q13.41 | 0.96 | **1.72** | 0.47 | up/down | 1.9 | NC/A | NC/P | A | 235953_at |
| hypothetical gene | AK055761 | 12q24.2 | 0.43 | 0.75 | **2.18** | down | ND | ND | ND | ND | NA |
| ***doublesex and mab-3 related transcription factor 2*** | ***DMRT2*** | 9p24.3 | 0.03 | 0.12 | **2.08** | up | NC/A | NC/A | 2.9 | A | 223704_s_at |
| NK6 transcription factor related, locus 1 (Drosophila) | NKX6-1 | 4q21.2-q22 | -0.09 | 0.86 | **2.02** | down | NC/A | NC/A | NC/A | A | 221366_at |

Hybridisation results of CpG island microarrays are presented as mean $\log_2$ ratios between normal and tumor cell lines of three independent microarray experiments ($\text{Log}_2$ ratios above 1 are boxed in black). Results of expression array analysis are presented as mean $\log_2$ ratios between normal and tumor cell lines if a significant change was detected (negative $\text{Log}_2$ ratios indicate lower expression in tumor cell lines and are boxed in black). P, present; A, absent; NC, no change (boxed in gray); ND, not detected. Genes that were independently analyzed by MCIp and real time PCR (s. Figure 4 and Supplementary Figure 2 online) are indicated in bold lettering. Confirmed targets of aberrant hypermethylation are indicated in bold italics. Genes that have been shown to be hypermethylated in other types of tumors are in italics.

**Example 10:Affymetrix microarray analysis**

RNA from KG-1, U937, and THP-1 cells as well as from freshly isolated human blood monocytes of a health donor were analyzed using Affymetrix HG-U133_Plus_2 arrays. Hybridization, cRNA labeling and data handling was done by the KFB (Regensburg).

Complementary mRNA expression data of the above cell lines was generated by microarray analysis using the human HG-133 Plus 2 Array (Affymetrix). A side by side comparison of CpG-methylation and mRNA expression data is presented in **Table 4**. Interestingly, more than half of the genes (69/125) were undetectable ('absent') by microarray analysis in all samples. In cases where significant signals were detected, the mRNA expression of genes that were found to be hypermethylated in tumor cell lines was often low relative to normal monocytes (e.g.

*PLA2G7, FNBP1, MAFB,* or *ZNF516*). In some cases, the degree of methylation showed no correlation with gene expression detected by microarray analysis (e.g. *HOXA10, EPAS1,* or *SYNJ2*), suggesting that CpG-methylation in those cases may target regions not relevant for transcription.
Quantitative Real-time PCR analysis of transcripts for five of the previously tested genes with hypermethylated CpG islands (*JUN, MAFB, KLF11, SSIAH2,* and *ZNF516*) confirmed their downregulation in leukemia cell lines relative to human blood monocytes (data not shown) and demonstrated a significant derepression by treatment with Decitabine (5-aza-2'-deoxycytidine) in U937 cells (s. **Figure 7**). The effect of demethylation was most striking for *MAFB* and *SSIAH2* (data not shown) that were induced up to a hundred fold in treated cells.

**Example 11: Experimental validation of microarray results**

A representative number of gene fragments that were identified using combined MCIp-chip analyses was selected for further validation. LightCycler real time PCR was utilized to measure the MCIp enrichment of twenty nine candidate genes (s. Figure 8). Out of these, twenty six gene fragments were enriched in a manner comparable to the results obtained by microarray analysis. To validate MCIp detected methylation differences using an independent approach, the methylation status of six CpG island fragments (*JUN, RAB3C, MAFB, KLF11, ZNF516* and *SSIAH2* (LOC283514)) was additionally determined using bisulfite sequencing. As shown in **Figure 9**, the degree of methylation as determined by bisulfite sequencing correlated well with the results obtained by MCIp. In several cases, the MseI fragment represented on the CpG island microarray did not include the proximal promoter. Since transcription factors may have a particular role in leukemogenesis, DNA fragments that included transcriptional start sites of the four transcription factor genes (*MAFB, KLF11, JUN* and *ZNF516*) were additionally analyzed by MCIp. Whereas *JUN* promoter fragments were not significantly methylated in any of the samples (data not shown), *MAFB, KLF11,* and *ZNF516* promoter fragments also showed significant methylation (s. **Figure 8**).

**Example 12: Databases for sequence analysis**

Mapping of CpG island clones was done using the UCSC Genome Browser (http://genome.ucsc.edu/). Expression profiles for myeloid cells types other than monocytes were obtained from GNF SymAtlas (http://symatlas.gnf.org/SymAtlas/)

**Example 13:Sodium bisulfite sequencing**

Modification of DNA with sodium bisulfite was performed as previously described (16) with modifications (17). Bisulfite-treated DNA was amplified in individual PCR reactions using the primers given in **Table 5**. PCR-products (representing the sense strand) were cloned using the TOPO-cloning kit (Invitrogen) and several individual clones were sequenced.

Table 5: Gene-specific oligonucleotide primers for nested PCR amplification of bisulfite-treated DNA

| **Gene** | | **Primer sequence (sense & antisense)** |
|---|---|---|
| *RAB3C* | *outer* | 5'-ATT GGG AGA GGT AAT TTA GGA G-3' (SEQ ID NO: 202) |
| | | 5'-ATT TTA AAC AAA CAC TCT TAT CCT C-3' (SEQ ID NO: 203) |
| | *inner* | 5'-TTT GGA AAG GAG TAG GGA GG-3' (SEQ ID NO: 204) |
| | | 5'-ATC CCT CAT CAA AAC AAC CC-3' (SEQ ID No: 205) |
| *MAFB* | *outer* | 5'-GGG TAY GGY GTG GTA TTG GG-3' (SEQ ID NO: 206) |
| | | 5'-TAA AAT AAA TCA CAA CTT AAC CTA TCC ATC-3' SEQ ID NO: 207) |
| | *inner* | 5'-TTT AAT TTA ATT TTG TGG GGT GGT-3' (SEQ ID NO: 208) |
| | | 5'-CTC TAA AAA CCA CAA ATC TCT TAA AAC C-3' (SEQ ID NO: 209) |
| *JUN* | *outer* | 5'-TTT AGA TGG GAA TAA GYG TGT AGG-3 (SEQ ID NO: 210) |
| | | 5'-TAC TAC AAA TCC AAC TTC AAA CC-3'(SEQ ID NO: 211) |
| | *inner* | 5'-TYG GGA AAA TAA GTT TAG AAG G-3 (SEQ ID NO: 212) |
| | | 5'-ACT CTT ATA TCC TAA CAT CCT ATC C-3' (SEQ ID No: 213) |
| *SSIAH2* | *outer* | 5'-TTT AAT ATA TGG GAT AGA GAG AAT TTG G-3' (SEQ ID NO: 214) |
| | | 5'-TTC TAT CCT TTT AAT TAA CCR CCT CAC-3' (SEQ ID NO: 215) |
| | *inner* | 5'-AAA TAG TAG GGG GAG TGA TGG G-3' (SEQ ID NO: 216) |
| | | 5'-AAA CCC AAA AAC TCA CAA CTT CC-3' (SEQ ID NO: 217) |

(continued)

| Gene | | Primer sequence (sense & antisense) |
|---|---|---|
| *KLF11* | *outer* | 5'-TGT TTA TGT GAG TGG TGG GG-3' (SEQ ID NO: 218) |
| | | 5'-ACC CAC CTA AAA ACC TCA ACC-3' (SEQ ID NO: 219) |
| | *inner* | 5'-TTG TTT TYG TTT TTT GGA TGG AG-3' (SEQ ID NO: 220) |
| | | 5'-TAT TTT TAA CTT CTA TCA TTC TCC C-3' (SEQ ID NO: 221) |
| *ZNF516* | *outer* | 5'-CAC CCA ATT CTA CCC CTC C-3' (SEQ ID NO: 222) |
| | | 5'-ATT TTT TTA TTG GGA GTT GAT G-3' (SEQ ID NO: 223) |
| | *inner* | 5'-ACC TCT CCA TTA CAT CAT CCC-3' (SEQ ID NO: 224) |
| | | 5'-GTT TTT GGT AAA TTT TAG AAG GTG-3' (SEQ ID NO: 225) |

**Example 14:GEO accession numbers**

Microarray data has been deposited with the GEO-database (accession numbers for Affymetrix gene arrays: GSM73641, GSM73642, GSM73644, GSM73645; accession numbers for CpG island microarrays: GSM91623-30)

**Example 15:Aberrant hypermethylation in acute myeloid leukemia**

Tumor cell lines represent *in vitro* models of primary tumors which may have acquired additional alterations both on genetic and on epigenetic levels. To test whether genes that were found to be hypermethylated in the leukemia cell lines are also affected in primary tumors, we analyzed DNA of blast cells obtained from twelve AML patients for hypermethylation at 22 promoter fragments including e.g. *CDKN2B, MAFB* (promoter and upstream fragment), *KLF11, ZNF516, KLF5, PFC and SSIAH2.* With the exception of ZNF516 (data not shown), a significant number of patients (ranging from 2 to 9) were markedly hypermethylated at each of the above loci (s. **Figure 10**). These results suggest that the CpG island fragments identified in tumor cell lines can also be subject to hypermethylation in primary tumor cells and may represent novel biological markers for leukemia. Notably, the youngest patient (P20) was hypermethylated at all loci tested, whereas the oldest patient (P07) was only significantly methylated at the PFC CpG island, indicating that methylation of the above tested CpG fragments does not correlate with aging.

**Figures**

The figures show:

**Figure 1:** **Detection of CpG methylated DNA on nylon membrane using the recombinant MBD-Fc protein**. A PCR-fragment of a CpG island promoter (*ICSBP* gene) was methylated using SssI, subjected to agarose gel electrophoresis (ethidium bromide staining is shown as control) and directly blotted onto a nylon membrane. Membranes were stained using MBD-$F_c$, HRP-conjugated anti-human Fc and ECL reagent (Amersham-Pharmacia).

**Figure 2:** **Salt concentration-dependent binding of CpG-methylated DNA to immobilized MBD-$F_c$.** (A) Schematic presentation of human promoter fragments. Circles mark the position of CpG-dinucleotides (o: unmethylated - *CPM*; • SssI methylated - *CCL13, TLR2, CHI3L1*). Promoter fragments were generated from previously constructed pGL3 reporter gene plasmids by PCR using GL2 and RV3 primers. Primer sequences used to amplify the promoter fragments may be obtained on request. (B, C) Fragments were purified and treated with SssI (New England Biolabs) according to the manufacturers instructions. A mixture of methylated and un-methylated promoter fragments were bound to MBD-$F_c$-sepharose (amount of MBD-$F_c$/50 μl protein A-sepharose is given), eluted using increasing salt concentrations, purified and separated using agarose gel electrophoresis (along with 1/5 of the input DNA mixture). Bands were visualized with ethidium bromide and scanned using a Typhoon 9200 Imager (Pharmacia-Amersham).

**Figure 3: Bisulfite sequences of an in vitro partially methylated gene locus after MCIp.**
(A) A partially SssI-methylated CpG island fragment (CPM gene) was subjected to a salt concentration-dependent, fractionated methyl-CpG immunoprecipitation. Fragments were recovered from each fraction, treated with bisulfite. The entire CpG island, represented on top of the figure was amplified from bisulfite-treated DNA and cloned. Several independent inserts were sequenced and results are presented schematically. Squares mark the position of CpG-dinucleotides (empty: unmethylated; filled: methylated). (B) Results are presented as a graph (•, number of methylated CpGs). Horizontal bars represent the median number of methylated CpG dinucleotides.

**Figure 4: Detection of CpG methylated DNA using MCIp and gene-specific Real time PCR.**
(A-C) Fractionated Methyl-CpG immunoprecipitation (MCIp) was used in combination with real-time LightCycler PCR to detect the methylation status of the indicated genes from untreated (gray bars) and SssI-methylated and *Mse*I-restricted genomic DNA fragments (black bars). Gene fragments recovered from MCIp-eluates (NaCl-concentrations (in mM) are indicated) and an equivalent amount of genomic input-DNA (gDNA) were amplified by LightCycler-PCR.. A standard curve (3 log scales) was used to obtain the concentration of a specific gene fragment in the MCIp eluate relative to its concentration in the gDNA. Values (mean ± SD, n=4 using at least two different preparations of MBD-$F_c$, log (10) scale) of individual fractions represent the percentage of recovery and are calculated relative to the amount of PCR-product generated from the respective gDNA (100%). Above each figure a 3 kB region of the corresponding CpG island is schematically presented. Each CpG dinucleotide is represented by a vertical line. The positions of exons are indicated as grey boxes in the bottom and transcription start sites by an arrow. The white box represents 100 bp. Black boxes on top indicate the positions of the *Mse*I-fragments that are detected. Numbers refers to the number of CpG dinucleotides within the MseI-fragment.

**Figure 5: MCIp detection of CpG island methylation in leukaemia cell lines using real-time PCR.**
(A) *SNRPN, TLR2, ESR1* and *CDKN2B* gene fragments in the high salt (1000mM after 600 mM) $MCI_P$ fraction of three human myeloid leukemia cell lines (KG-1, U937 and THP-1) as well as normal human blood monocytes (N) were analyzed by real time PCR as described in Figure 1. (B) Decreasing amounts of MseI-treated U937 DNA were subjected to MCIp. *CDKN2B* and *TLR2* gene fragments were quantified as above. (C). MseI-treated DNA of normal human blood monocytes (N) and KG-1 cells was mixed at the indicated ratios, the mixture was subjected to MCIp and the TLR2 gene fragment was quantified in the 1000mM fraction using LightCycler-PCR as above.

**Figure 6: DNA methylation profiling using CpG-island microarrays.**
(A) Global methylation levels were determined using $MCI_P$ (steps 1-3) and subsequent co-hybridization of amplified tumor and normal DNA labeled with two different dyes (steps 4-6). (B) Methylated DNA shows a higher fluorescence for Cy3 (blue color) and normal DNA for Cy3 (yellow color). (C) Representative 2-dimensional scatter plots are shown for a control hybridization experiment of human blood monocytes DNA (normal/normal) and a hybridization displaying the differentially methylated CpG fragments between the U937 cell line (tumor) and human blood monocytes (normal).

**Figure 7: Derepression of hypermethylated target genes by Decitabine.**
*MAFB, JUN, KLF11,* and *ZNF516* mRNA expression levels were quantified in U937 cells after Decitabine treatment (5 μM) using real time PCR. The relative units were calculated from a standard curve with 4 different concentrations of log dilutions to the PCR cycle number at which the measured fluorescence intensity reaches a fixed value. Expression levels are relative to *HPRT* expression. Values are mean ± SE of two experiments.

**Figure 8: Validation of CpG island microarray results by single gene MCIp.**
Real-time LightCycler PCR was used to detect the methylation status of indicated human genes in three human myeloid leukemia cell lines (KG-1, U937 and THP-1) as well as normal human blood monocytes (N) after MCIp. No significant differences were detected at the LDLR, TGIF and CBX6 gene fragments (data not shown). Results are shown relative to the amount of PCR-product generated from the genomic input DNA (gDNA, 100%) of each cell type. Values are mean ± SD (n=3-4) using at least two different preparations of MBD-$F_c$. The corresponding CpG island is represented on top of each figure as described in **Figure 4**.

**Figure 9:** **Bisulfite sequences of six differentially methylated gene loci.**
Genomic DNA from the three human myeloid leukemia cell lines (KG-1, U937 and THP-1) as well as normal human blood monocytes (N) was analyzed for CpG methylation at *JUN, MAFB, RAB3C, KLF11, ZNF516* and *SSIAH2* loci by bisulfite sequencing. The CpG islands are represented on top of each figure as described in **Figure 4**. The indicated regions (boxed in grey) were amplified from bisulfite-treated DNA and cloned. Several independent inserts were sequenced and results are presented schematically. Squares mark the position of CpG-dinucleotides (empty: unmethylated; filled: methylated).

**Figure 10:** **Methylation profiles of AML patients.**
Schematic representation of the MCIp enrichment detected by single gene real-time LightCycler PCR for the indicated genes in *Mse*I- or *Mse*I/*Csp*6I-restricted DNA in the twelve AML samples (AML-P no. (Age in years)) as well as two normal human blood monocytes samples (N1, N2). Results are shown relative to the amount of PCR-product generated from the genomic input DNA (gDNA, 100%) of each cell type. Values are mean ± SE of at least two LightCycler amplifications using at least two different preparations of MBD-$F_c$.

**Figure 11:** Figure 11 shows the nucleotide sequence of plasmid pMTBip/MBD2-Fc and the protein sequence (in bold) of the MBD2-Fc bifunctional protein which is encoded by plasmid pMTBip/MBD2-Fc.
The amino acid sequence of the MBD2-Fc bifunctional protein has the following features.

| | |
|---|---|
| AA 1-28 (nt 851-934): | *Drosophila* BiP secretion signal (leader peptide from pMT/Bip/V5-His vector): |
| AA 29-115 (nt 935-1196): | AA 144-230 of human MBD2 |
| AA 116-129 (nt 1196-1237): | Flexible Linker (AAADPIEGRGGGGG) |
| AA 130-361 (1238-1933) : | AA99-330 of human IGHG1 |

## Claims

1. An in vitro method of diagnosing a predisposition of cancer or cancer comprising determining the methylation status of at least one nucleic acid molecule having a nucleotide sequence selected from any one of KLF11 (SEQ ID NO: 1), PLA2G7 (SEQ ID NO: 2), PFC (SEQ ID NO: 3) and MAFB (SEQ ID NO: 4), an allelic variant, a portion or the complementary strand of any of said nucleotide sequences in a sample obtained from a subject.

2. The method of claim 1, further comprising determining the methylation status of at least one nucleic acid molecule having a nucleotide sequence selected from any one of SEQ ID NOs: 5 - 16, an allelic variant, a portion or the complementary strand of any of said nucleotide sequences.

3. The method of claim 1 or 2, further comprising determining the methylation status of at least one nucleic acid molecule having a nucleotide sequence selected from any one of SEQ ID NOs: 17-48, an allelic variant, a portion or the complementary strand of any of said nucleotide sequences.

4. The method of claim 1, 2 or 3, further comprising determining the methylation status of at least one nucleic acid molecule having a nucleotide sequence selected from any one of SEQ ID NOs: 49 - 111, an allelic variant, a portion or the complementary strand of any of said nucleotide sequences.

5. The method of any one of claims 1-4, wherein said cancer is leukemia.

6. The method of any one of claims 1 to 5, comprising the steps of

   (a) isolating DNA from a sample obtained from a subject;
   (b) contacting said DNA with a polypeptide capable of binding methylated DNA;
   (c) optionally amplifying said DNA bound by said polypeptide; and
   (d) determining the methylation status of said DNA.

7. A kit for performing the method of any one of claims 1 to 6, comprising a polypeptide capable of binding methylated DNA and probes, primers and/or aptamers specific for at least one nucleic acid molecule having a nucleotide sequence as defined in claim 1, an allelic variant, a portion or the complementary strand of any of said nucleotide sequences.

8. The kit of claim 7, further comprising probes, primers and/or aptamers specific for at least one nucleic acid molecule having a nucleotide sequence as defined in claim 2, 3 or 4, an allelic variant, a portion or the complementary strand of any of said nucleotide sequences.

9. The kit of claim 7 or 8 which is a diagnostic kit.

10. Use of at least one nucleic acid molecule having a nucleotide sequence as defined in claim 1, an allelic variant, a portion or the complementary strand of any of said nucleotide sequences for the preparation of a diagnostic composition for determining predisposition of cancer or cancer by determining the methylation status of its corresponding genomic DNA in a sample obtained from a subject.

11. The use of claim 10, wherein said diagnostic composition further comprises at least one nucleic acid molecule having a nucleotide sequence as defined in claim 2, an allelic variant, a portion or the complementary strand of any of said nucleotide sequences.

12. The use of claim 10 or 11, wherein said diagnostic composition further comprises at least one nucleic acid molecule having a nucleotide sequence as defined in claim 3, an allelic variant, a portion or the complementary strand of any of said nucleotide sequences.

13. The use of claim 10, 11 or 12, wherein said diagnostic composition further comprises at least one nucleic acid molecule having a nucleotide sequence as defined in claim 4, an allelic variant, a portion or the complementary strand of any of said nucleotide sequences.

14. A diagnostic composition comprising at least one nucleic acid molecule having a nucleotide sequence as defined in claim 1, 2, 3 or 4, an allelic variant, a portion or the complementary strand of any of said nucleotide sequences.

Figure 1

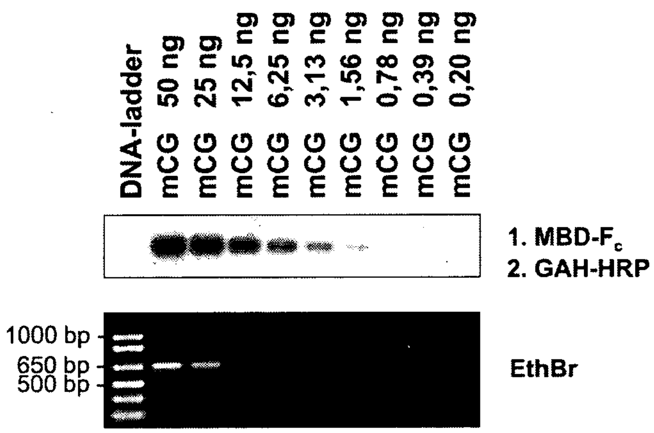
DNA-ladder
mCG 50 ng
mCG 25 ng
mCG 12,5 ng
mCG 6,25 ng
mCG 3,13 ng
mCG 1,56 ng
mCG 0,78 ng
mCG 0,39 ng
mCG 0,20 ng
1. MBD-$F_c$
2. GAH-HRP
1000 bp
650 bp
500 bp
EthBr

Figure 2

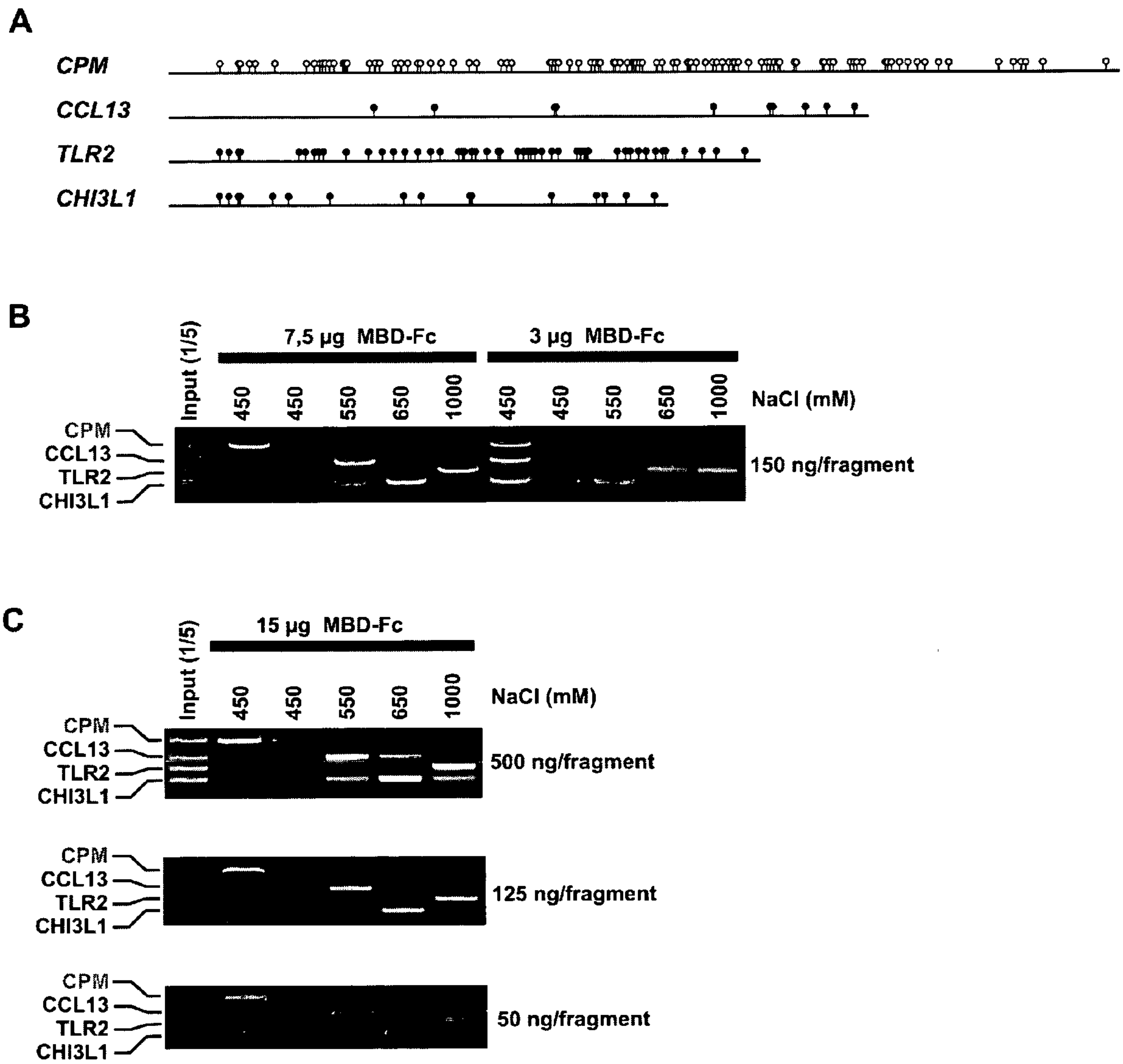
A
CPM
CCL13
TLR2
CHI3L1
B
Input (1/5)
7,5 µg MBD-Fc
3 µg MBD-Fc
450 450 550 650 1000 450 450 550 650 1000
NaCl (mM)
CPM
CCL13
TLR2
CHI3L1
150 ng/fragment
C
Input (1/5)
15 µg MBD-Fc
450 450 550 650 1000
NaCl (mM)
CPM
CCL13
TLR2
CHI3L1
500 ng/fragment
CPM
CCL13
TLR2
CHI3L1
125 ng/fragment
CPM
CCL13
TLR2
CHI3L1
50 ng/fragment

# Figure 3

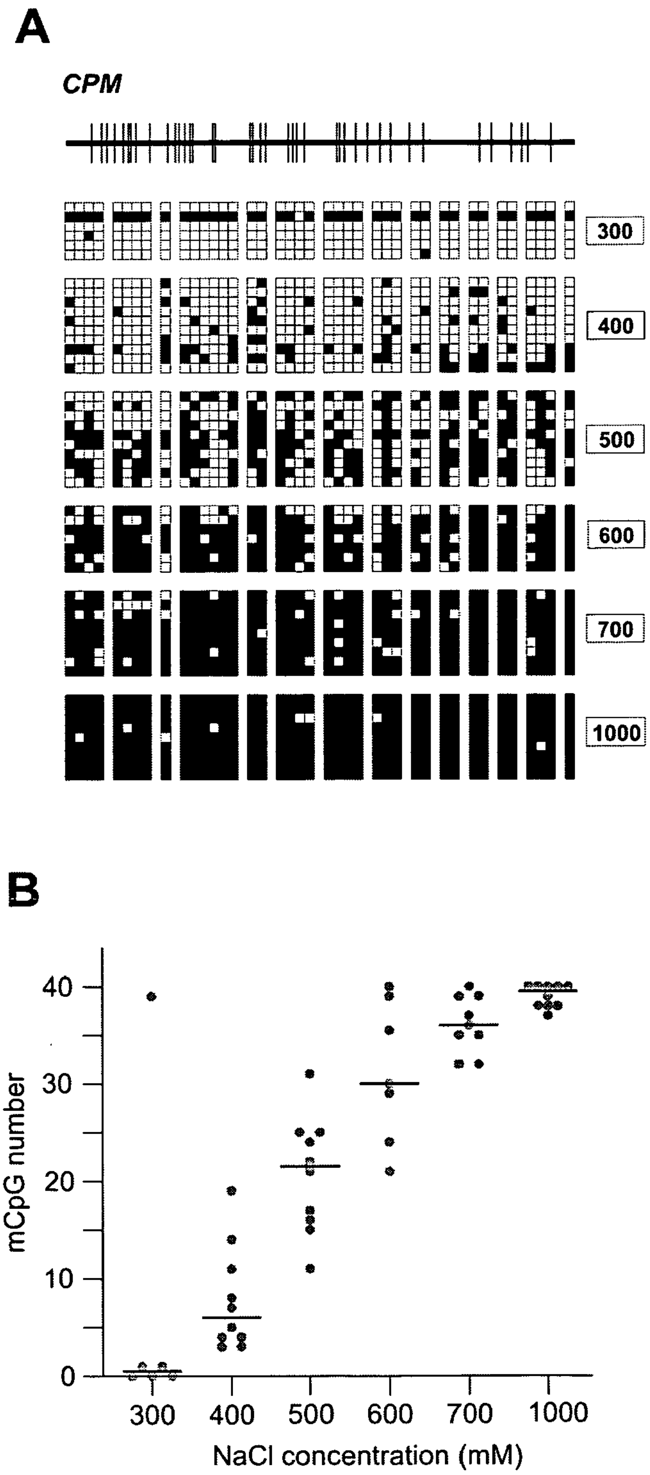
A
CPM
300
400
500
600
700
1000
B
mCpG number
0
10
20
30
40
300
400
500
600
700
1000
NaCl concentration (mM)

# Figure 4

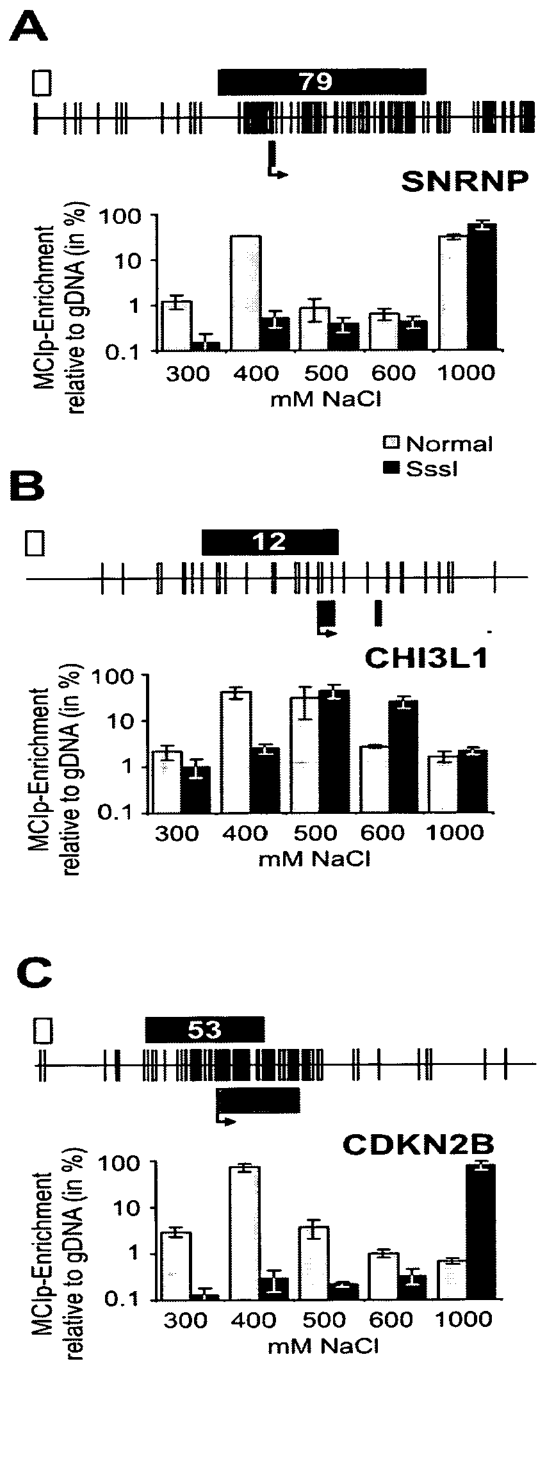
A
79
SNRNP
MCIp-Enrichment relative to gDNA (in %)
100
10
1
0.1
300 400 500 600 1000
mM NaCl
Normal
SssI
B
12
CHI3L1
MCIp-Enrichment relative to gDNA (in %)
100
10
1
0.1
300 400 500 600 1000
mM NaCl
C
53
CDKN2B
MCIp-Enrichment relative to gDNA (in %)
100
10
1
0.1
300 400 500 600 1000
mM NaCl

# Figure 5

A

B

C

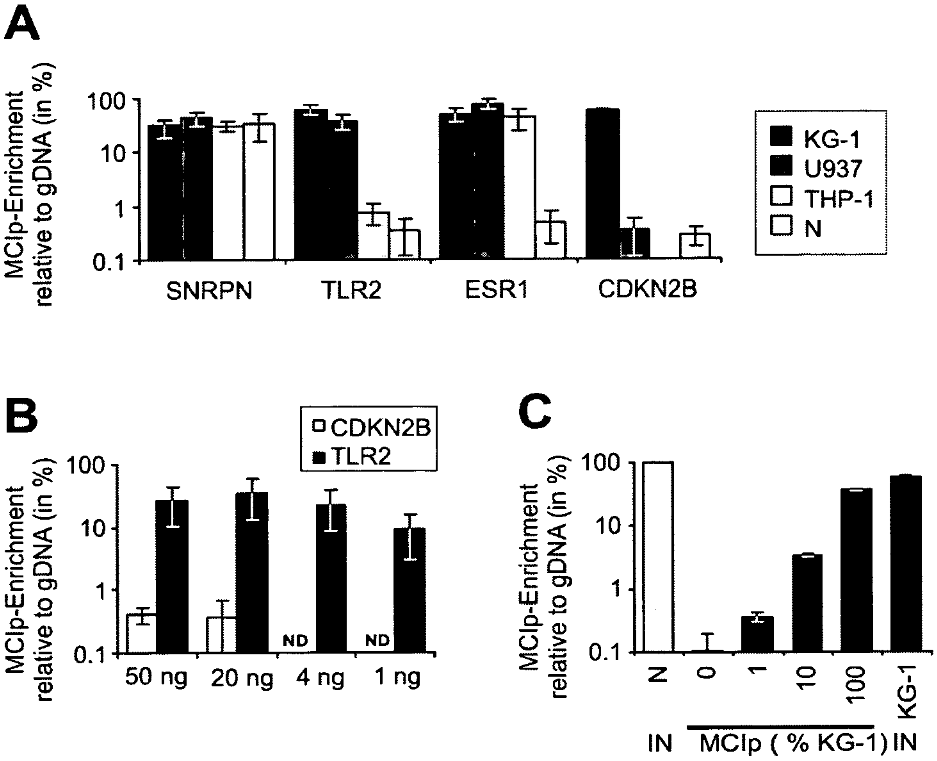
MCIp-Enrichment relative to gDNA (in %)
100
10
1
0.1
SNRPN
TLR2
ESR1
CDKN2B
KG-1
U937
THP-1
N
CDKN2B
TLR2
ND
ND
50 ng
20 ng
4 ng
1 ng
N
0
1
10
100
KG-1
IN
MCIp ( % KG-1)
IN

# Figure 6

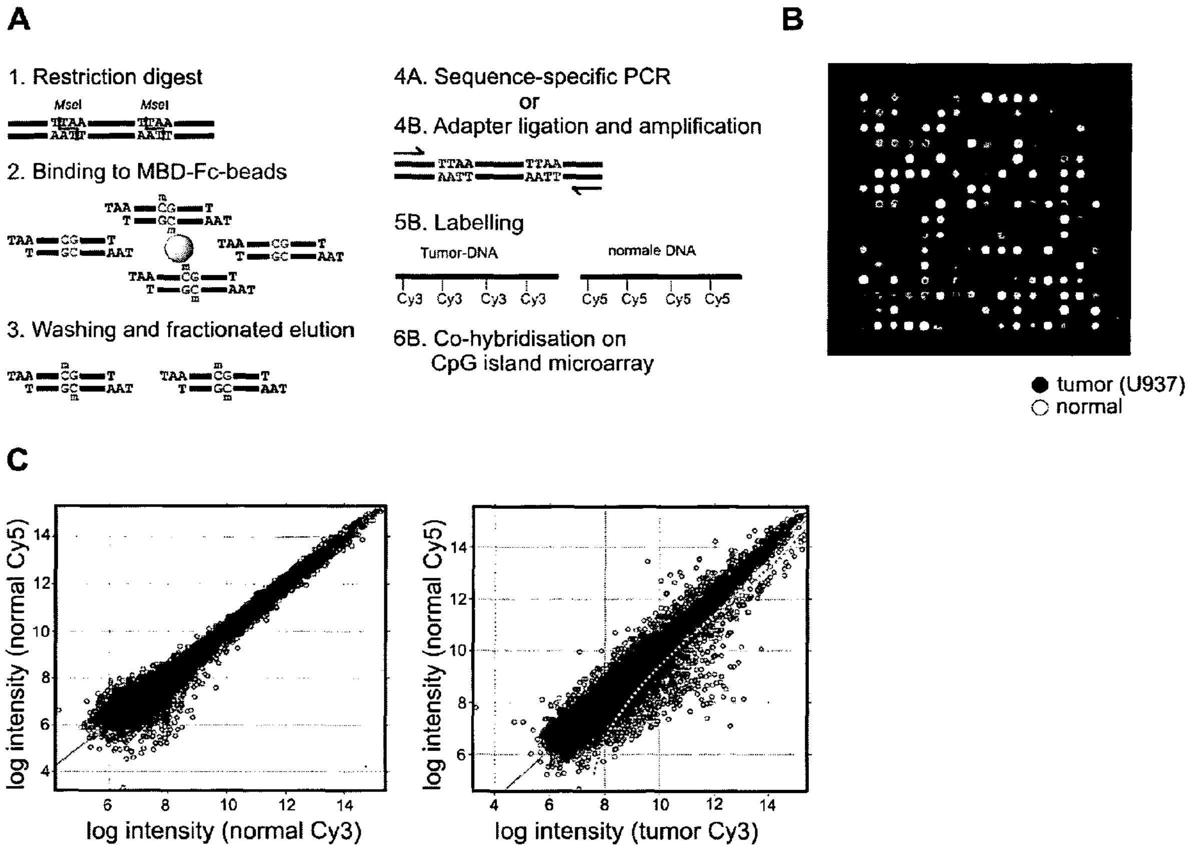
A
1. Restriction digest
MseI
MseI
2. Binding to MBD-Fc-beads
3. Washing and fractionated elution
4A. Sequence-specific PCR
or
4B. Adapter ligation and amplification
5B. Labelling
Tumor-DNA
normale DNA
Cy3 Cy3 Cy3 Cy3
Cy5 Cy5 Cy5 Cy5
6B. Co-hybridisation on
CpG island microarray
B
tumor (U937)
normal
C
log intensity (normal Cy5)
log intensity (normal Cy3)
log intensity (normal Cy5)
log intensity (tumor Cy3)

# Figure 7

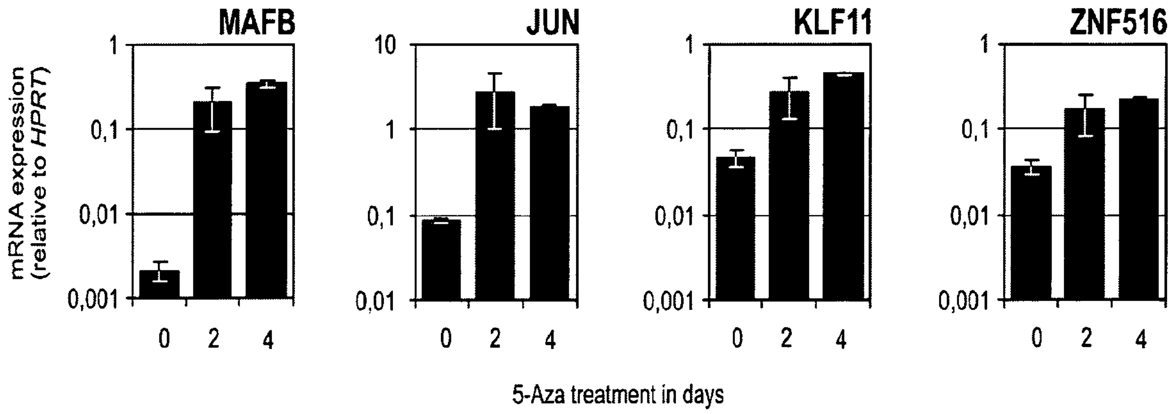
MAFB
JUN
KLF11
ZNF516
mRNA expression (relative to HPRT)
1
0,1
0,01
0,001
10
1
0,1
0,01
0 2 4
5-Aza treatment in days

# Figure 8 (I)

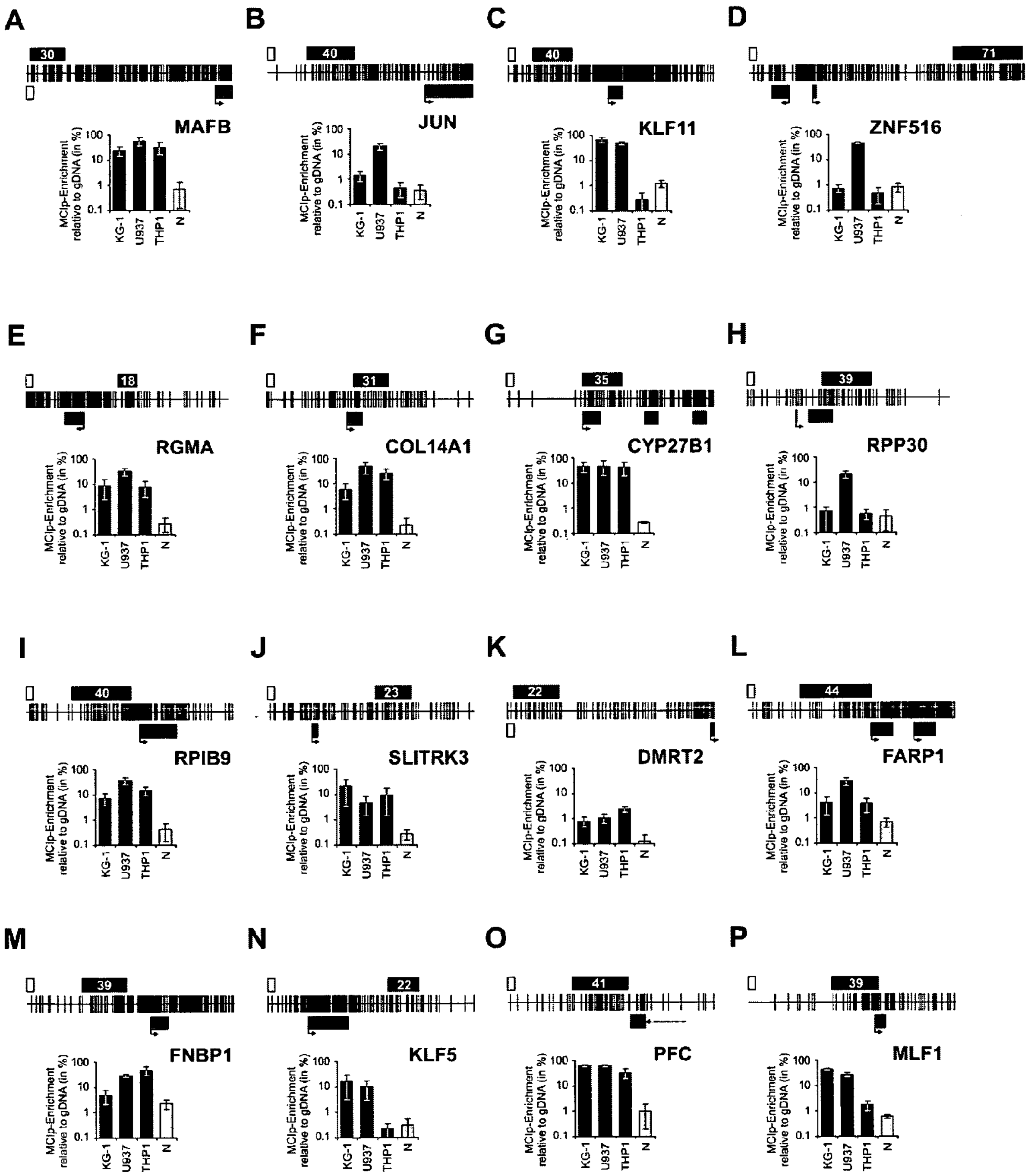
A
B
C
D
MAFB
JUN
KLF11
ZNF516
E
F
G
H
RGMA
COL14A1
CYP27B1
RPP30
I
J
K
L
RPIB9
SLITRK3
DMRT2
FARP1
M
N
O
P
FNBP1
KLF5
PFC
MLF1
MCIp-Enrichment relative to gDNA (in %)
KG-1
U937
THP1
N

# Figure 8 (II)

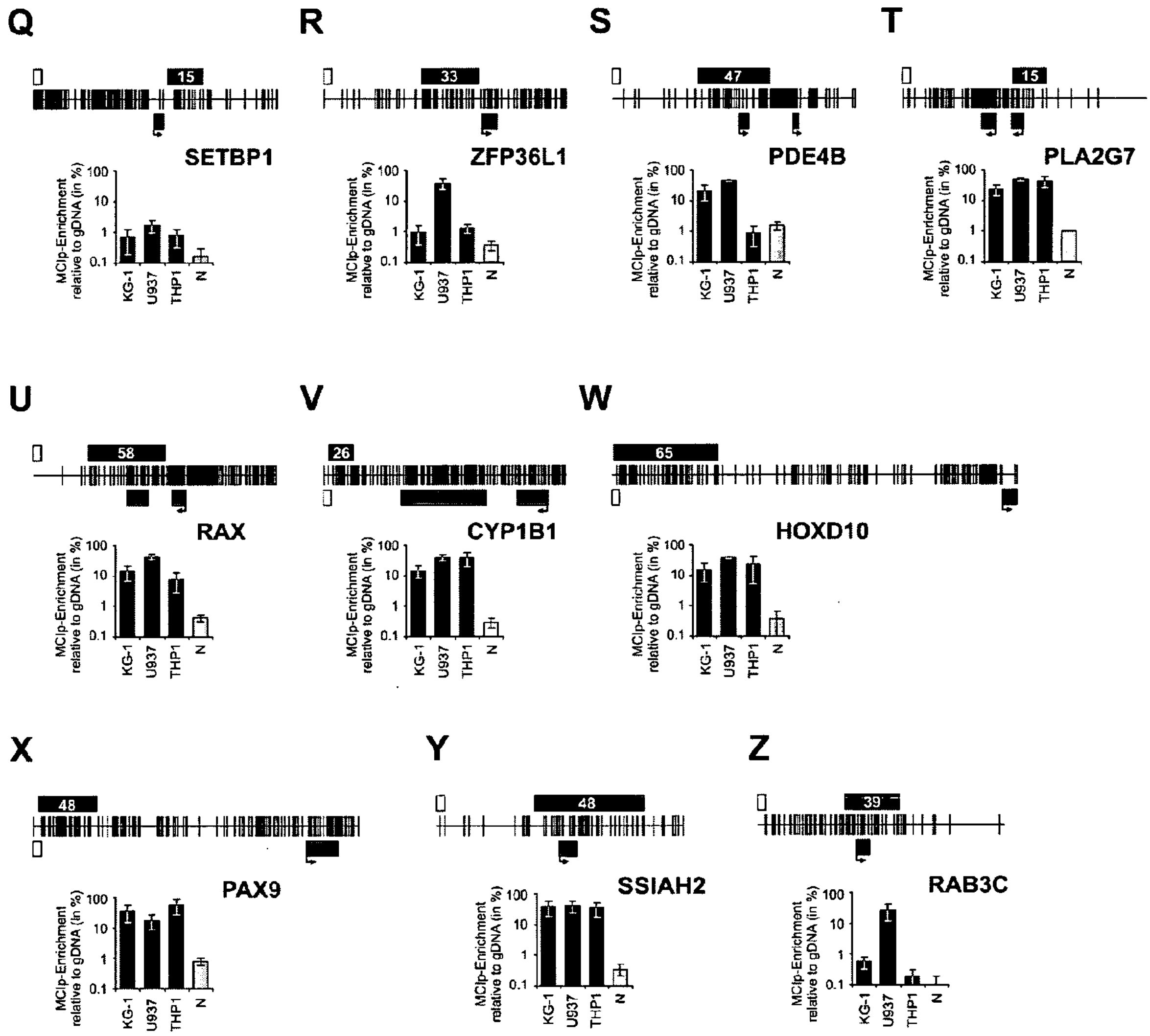
Q
R
S
T
SETBP1
ZFP36L1
PDE4B
PLA2G7
U
V
W
RAX
CYP1B1
HOXD10
X
Y
Z
PAX9
SSIAH2
RAB3C
15
33
47
15
58
26
65
48
48
39
MCIp-Enrichment relative to gDNA (in %)
100
10
1
0.1
KG-1
U937
THP1
N

# Figure 9

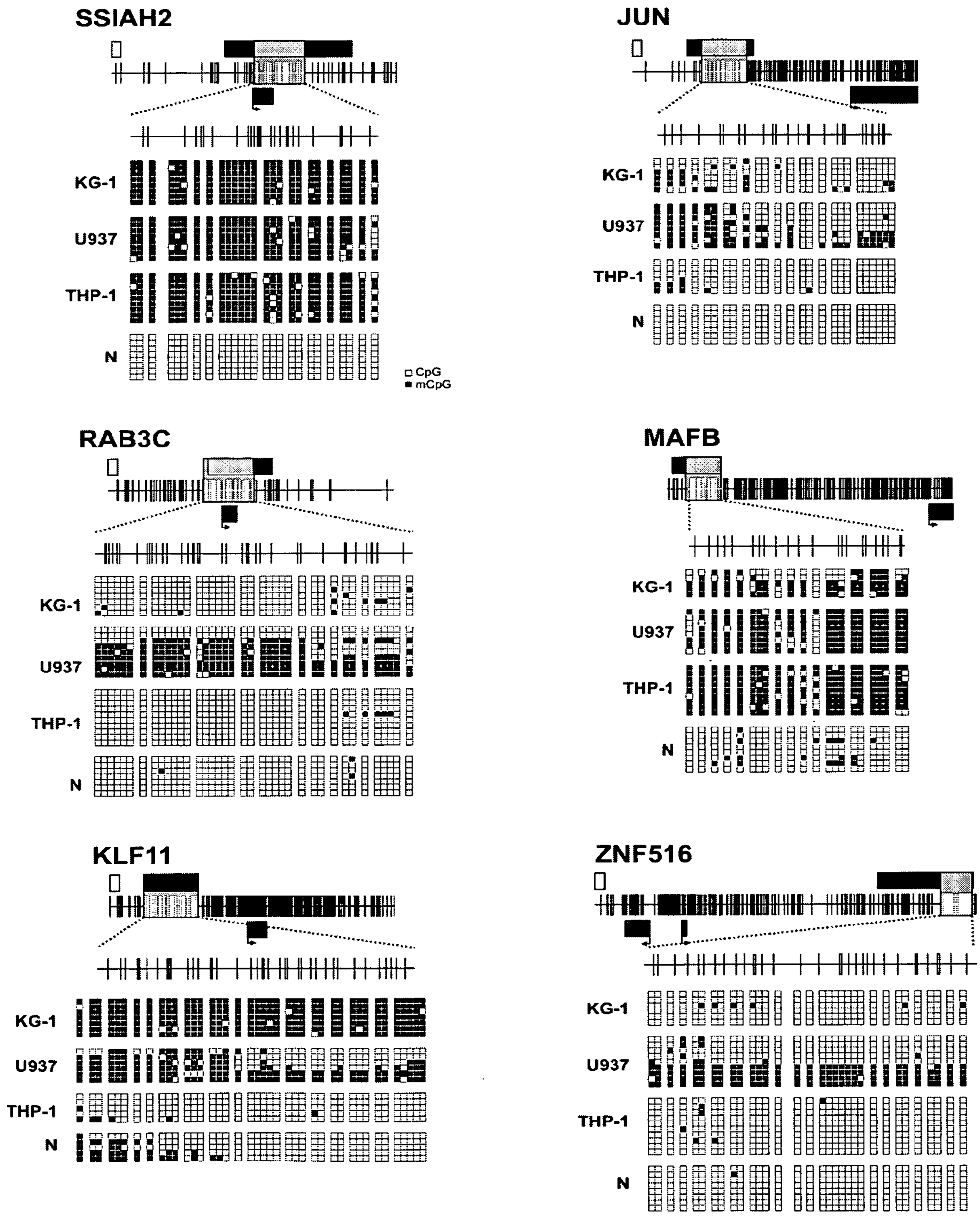

# Figure 10

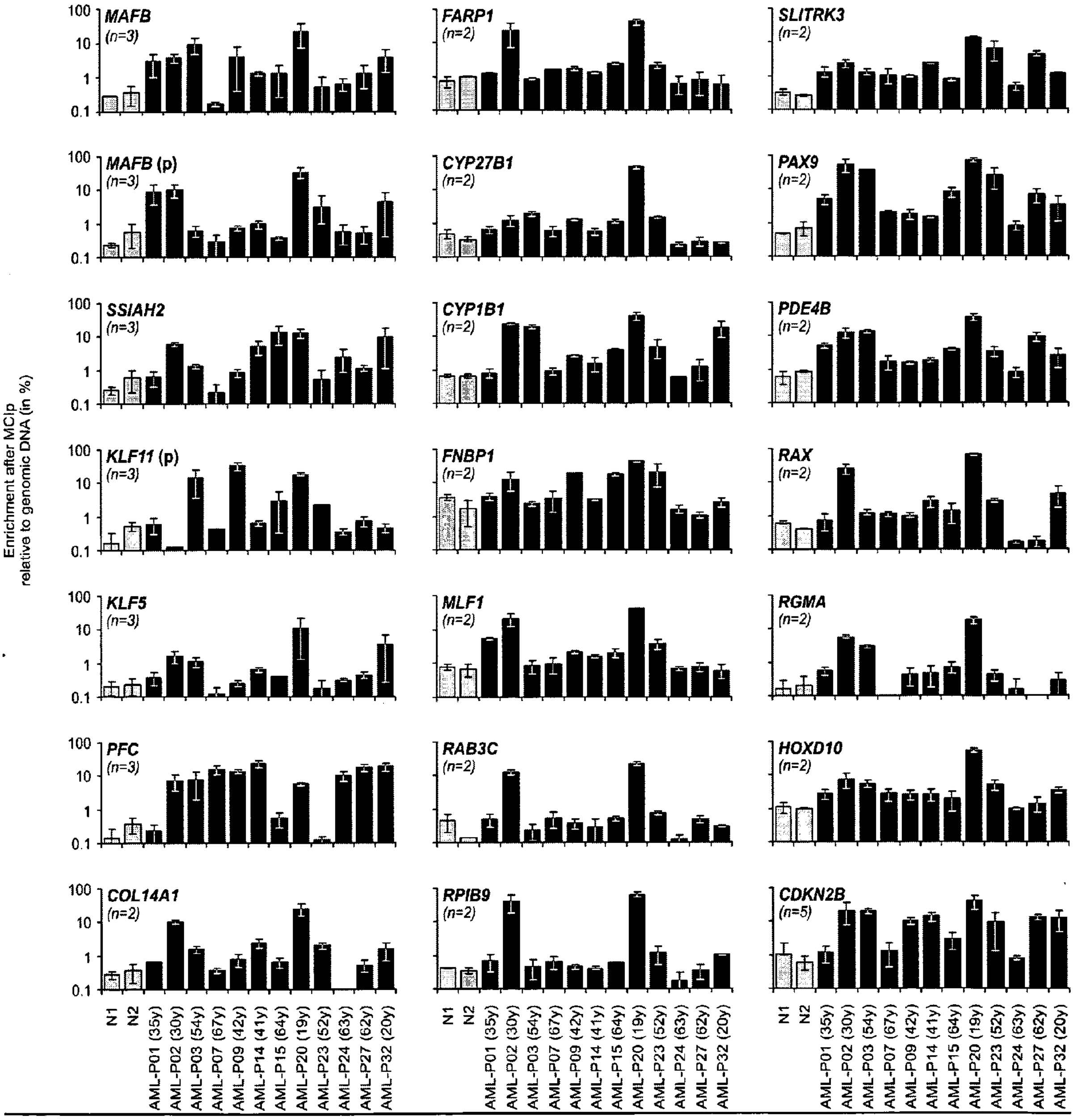
Enrichment after MCIp relative to genomic DNA (in %)
MAFB (n=3)
FARP1 (n=2)
SLITRK3 (n=2)
MAFB (p) (n=3)
CYP27B1 (n=2)
PAX9 (n=2)
SSIAH2 (n=3)
CYP1B1 (n=2)
PDE4B (n=2)
KLF11 (p) (n=3)
FNBP1 (n=2)
RAX (n=2)
KLF5 (n=3)
MLF1 (n=2)
RGMA (n=2)
PFC (n=3)
RAB3C (n=2)
HOXD10 (n=2)
COL14A1 (n=2)
RPIB9 (n=2)
CDKN2B (n=5)
100
10
1
0.1
N1
N2
AML-P01 (35y)
AML-P02 (30y)
AML-P03 (54y)
AML-P07 (67y)
AML-P09 (42y)
AML-P14 (41y)
AML-P15 (64y)
AML-P20 (19y)
AML-P23 (52y)
AML-P24 (63y)
AML-P27 (62y)
AML-P32 (20y)

# Figure 11 (I)

```
tcgcgcgttt cggtgatgac ggtgaaaacc tctgacacat gcagctcccg gagacggtca     60
cagcttgtct gtaagcggat gccgggagca gacaagcccg tcagggcgcg tcagcgggtg    120
ttggcgggtg tcggggctgg cttaactatg cggcatcaga gcagattgta ctgagagtgc    180
accatatgcg gtgtgaaata ccgcacagat gcgtaaggag aaaataccgc atcaggcgcc    240
attcgccatt caggctgcgc aactgttggg aagggcgatc ggtgcgggcc tcttcgctat    300
tacgccagct ggcgaaaggg ggatgtgctg caaggcgatt aagttgggta acgccagggt    360
tttcccagtc acgacgttgt aaaacgacgg ccagtgccag tgaattttaa cgttgcagga    420
caggatgtgg tgcccgatgt gactagctct ttgctgcagg ccgtcctatc ctctggttcc    480
gataagagac ccagaactcc ggccccccac cgcccaccgc cacccccata catatgtggt    540
acgcaagtaa gagtgcctgc gcatgcccca tgtgccccac caagagtttt gcatcccata    600
caagtcccca aagtggagaa ccgaaccaat tcttcgcggg cagaacaaaa gcttctgcac    660
acgtctccac tcgaatttgg agccggccgg cgtgtgcaaa agaggtgaat cgaacgaaag    720
acccgtgtgt aaagccgcgt ttccaaaatg tataaaaccg agagcatctg gccaatgtgc    780
atcagttgtg gtcagcagca aaatcaagtg aatcatctca gtgcaactaa aggggggatc    840
cgatctcaat atg aag tta tgc ata tta ctg gcc gtc gtg gcc ttt gtt        889
           Met Lys Leu Cys Ile Leu Leu Ala Val Val Ala Phe Val
           1               5                   10

ggc ctc tcg ctc ggg aga tct cca tgg ccc ggg gta cct act agc acg       937
Gly Leu Ser Leu Gly Arg Ser Pro Trp Pro Gly Val Pro Thr Ser Thr
    15                  20                  25

gag agc ggg aag agg atg gat tgc ccg gcc ctc ccc ccc gga tgg aag       985
Glu Ser Gly Lys Arg Met Asp Cys Pro Ala Leu Pro Pro Gly Trp Lys
30                  35                  40                  45

aag gag gaa gtg atc cga aaa tct ggg cta agt gct ggc aag agc gat      1033
Lys Glu Glu Val Ile Arg Lys Ser Gly Leu Ser Ala Gly Lys Ser Asp
                50                  55                  60

gtc tac tac ttc agt cca agt ggt aag aag ttc aga agc aag cct cag      1081
Val Tyr Tyr Phe Ser Pro Ser Gly Lys Lys Phe Arg Ser Lys Pro Gln
            65                  70                  75

ttg gca agg tac ctg gga aat act gtt gat ctc agc agt ttt gac ttc      1129
Leu Ala Arg Tyr Leu Gly Asn Thr Val Asp Leu Ser Ser Phe Asp Phe
        80                  85                  90

aga act gga aag atg atg cct agt aaa tta cag aag aac aaa cag aga      1177
Arg Thr Gly Lys Met Met Pro Ser Lys Leu Gln Lys Asn Lys Gln Arg
    95                  100                 105

ctg cga aac gat cct ctg gcg gcc gcg gat ccc atc gaa ggt cgt ggt      1225
Leu Arg Asn Asp Pro Leu Ala Ala Ala Asp Pro Ile Glu Gly Arg Gly
110                 115                 120                 125

ggt ggt ggt ggt gat ccc aaa tct tgt gac aaa cct cac aca tgc cca      1273
Gly Gly Gly Gly Asp Pro Lys Ser Cys Asp Lys Pro His Thr Cys Pro
                130                 135                 140

ctg tgc cca gca cct gaa ctc ctg ggg gga ccg tca gtc ttc ctc ttc      1321
Leu Cys Pro Ala Pro Glu Leu Leu Gly Gly Pro Ser Val Phe Leu Phe
            145                 150                 155

ccc cca aaa ccc aag gac acc ctc atg atc tcc cgg acc cct gag gtc      1369
Pro Pro Lys Pro Lys Asp Thr Leu Met Ile Ser Arg Thr Pro Glu Val
        160                 165                 170

aca tgc gtg gtg gtg gac gtg agc cac gaa gac cct gag gtc aag ttc      1417
Thr Cys Val Val Val Asp Val Ser His Glu Asp Pro Glu Val Lys Phe
    175                 180                 185

aac tgg tac gtg gac ggc gtg gag gtg cat aat gcc aag aca aag ccg      1465
```

# Figure 11 (II)

```
Asn Trp Tyr Val Asp Gly Val Glu Val His Asn Ala Lys Thr Lys Pro
190                 195                 200                 205

cgg gag gag cag tac aac agc acg tac cgt gtg gtc agc gtc ctc acc      1513
Arg Glu Glu Gln Tyr Asn Ser Thr Tyr Arg Val Val Ser Val Leu Thr
                210                 215                 220

gtc ctg cac cag gac tgg ctg aat ggc aag gag tac aag tgc aag gtc      1561
Val Leu His Gln Asp Trp Leu Asn Gly Lys Glu Tyr Lys Cys Lys Val
            225                 230                 235

tcc aac aaa gcc ctc cca gcc ccc atc gag aaa acc atc tcc aaa gcc      1609
Ser Asn Lys Ala Leu Pro Ala Pro Ile Glu Lys Thr Ile Ser Lys Ala
        240                 245                 250

aaa ggg cag ccc cga gaa cca cag gtg tac acc ctg ccc cca tcc cgg      1657
Lys Gly Gln Pro Arg Glu Pro Gln Val Tyr Thr Leu Pro Pro Ser Arg
    255                 260                 265

gat gag ctg acc aag aac cag gtc agc ctg acc tgc cta gtc aaa ggc      1705
Asp Glu Leu Thr Lys Asn Gln Val Ser Leu Thr Cys Leu Val Lys Gly
270                 275                 280                 285

ttc tat ccc agc gac atc gcc gtg gag tgg gag agc aat ggg cag ccg      1753
Phe Tyr Pro Ser Asp Ile Ala Val Glu Trp Glu Ser Asn Gly Gln Pro
                290                 295                 300

gag aac aac tac aag gcc acg cct ccc gtg ctg gac tcc gac ggc tcc      1801
Glu Asn Asn Tyr Lys Ala Thr Pro Pro Val Leu Asp Ser Asp Gly Ser
            305                 310                 315

ttc ttc ctc tac agc aag ctc acc gtg gac aag agc agg tgg cag cag      1849
Phe Phe Leu Tyr Ser Lys Leu Thr Val Asp Lys Ser Arg Trp Gln Gln
        320                 325                 330

ggg aac gtc ttc tca tgc tcc gtg atg cat gag gct ctg cac aac cac      1897
Gly Asn Val Phe Ser Cys Ser Val Met His Glu Ala Leu His Asn His
    335                 340                 345

tac acg cag aag agc ctc tcc ctg tct ccg ggt aaa tgagctagag           1943
Tyr Thr Gln Lys Ser Leu Ser Leu Ser Pro Gly Lys
350                 355                 360

ggcccgcggt tcgaaggtaa gcctatccct aaccctctcc tcggtctcga ttctacgcgt    2003
accggtcatc atcaccatca ccattgagtt taaacccgct gatcagcctc gactgtgcct    2063
tctaaggcct gagctcgctg atcagcctcg atcgaggatc cagacatgat aagatacatt    2123
gatgagtttg gacaaaccac aactagaatg cagtgaaaaa aatgctttat ttgtgaaatt    2183
tgtgatgcta ttgctttatt tgtaaccatt ataagctgca ataaacaagt taacaacaac    2243
aattgcattc attttatgtt tcaggttcag ggggaggtgt gggaggtttt ttaaagcaag    2303
taaaacctct acaaatgtgg tatggctgat tatgatcagt cgacctgcag gcatgcaagc    2363
ttggcgtaat catggtcata gctgtttcct gtgtgaaatt gttatccgct cacaattcca    2423
cacaacatac gagccggaag cataaagtgt aaagcctggg gtgcctaatg agtgagctaa    2483
ctcacattaa ttgcgttgcg ctcactgccc gctttccagt cgggaaacct gtcgtgccag    2543
ctgcattaat gaatcggcca acgcgcgggg agaggcggtt tgcgtattgg gcgctcttcc    2603
gcttcctcgc tcactgactc gctgcgctcg gtcgttcggc tgcggcgagc ggtatcagct    2663
cactcaaagg cggtaatacg gttatccaca gaatcagggg ataacgcagg aaagaacatg    2723
tgagcaaaag gccagcaaaa ggccaggaac cgtaaaaagg ccgcgttgct ggcgtttttc    2783
cataggctcc gcccccctga cgagcatcac aaaaatcgac gctcaagtca gaggtggcga    2843
aacccgacag gactataaag ataccaggcg tttccccctg gaagctccct cgtgcgctct    2903
cctgttccga ccctgccgct taccggatac ctgtccgcct ttctcccttc gggaagcgtg    2963
gcgctttctc atagctcacg ctgtaggtat ctcagttcgg tgtaggtcgt tcgctccaag    3023
ctgggctgtg tgcacgaacc ccccgttcag cccgaccgct gcgccttatc cggtaactat    3083
cgtcttgagt ccaacccggt aagacacgac ttatcgccac tggcagcagc cactggtaac    3143
```

# Figure 11 (III)

```
aggattagca gagcgaggta tgtaggcggt gctacagagt tcttgaagtg gtggcctaac   3203
tacggctaca ctagaaggac agtatttggt atctgcgctc tgctgaagcc agttaccttc   3263
ggaaaaagag ttggtagctc ttgatccggc aaacaaacca ccgctggtag cggtggtttt   3323
tttgtttgca agcagcagat tacgcgcaga aaaaaaggat ctcaagaaga tcctttgatc   3383
ttttctacgg ggtctgacgc tcagtggaac gaaaactcac gttaagggat tttggtcatg   3443
agattatcaa aaaggatctt cacctagatc cttttaaatt aaaaatgaag ttttaaatca   3503
atctaaagta tatatgagta aacttggtct gacagttacc aatgcttaat cagtgaggca   3563
cctatctcag cgatctgtct atttcgttca tccatagttg cctgactccc cgtcgtgtag   3623
ataactacga tacgggaggg cttaccatct ggccccagtg ctgcaatgat accgcgagac   3683
ccacgctcac cggctccaga tttatcagca ataaaccagc cagccggaag ggccgagcgc   3743
agaagtggtc ctgcaacttt atccgcctcc atccagtcta ttaattgttg ccgggaagct   3803
agagtaagta gttcgccagt taatagtttg cgcaacgttg ttgccattgc tacaggcatc   3863
gtggtgtcac gctcgtcgtt tggtatggct tcattcagct ccggttccca acgatcaagg   3923
cgagttacat gatcccccat gttgtgcaaa aaagcggtta gctccttcgg tcctccgatc   3983
gttgtcagaa gtaagttggc cgcagtgtta tcactcatgg ttatggcagc actgcataat   4043
tctcttactg tcatgccatc cgtaagatgc ttttctgtga ctggtgagta ctcaaccaag   4103
tcattctgag aatagtgtat gcggcgaccg agttgctctt gcccggcgtc aatacgggat   4163
aataccgcgc cacatagcag aactttaaaa gtgctcatca ttggaaaacg ttcttcgggg   4223
cgaaaactct caaggatctt accgctgttg agatccagtt cgatgtaacc cactcgtgca   4283
cccaactgat cttcagcatc ttttactttc accagcgttt ctgggtgagc aaaaacagga   4343
aggcaaaatg ccgcaaaaaa gggaataagg gcgacacgga aatgttgaat actcatactc   4403
ttcctttttc aatattattg aagcatttat cagggttatt gtctcatgag cggatacata   4463
tttgaatgta tttagaaaaa taaacaaata ggggttccgc gcacatttcc ccgaaaagtg   4523
ccacctgacg tctaagaaac cattattatc atgacattaa cctataaaaa taggcgtatc   4583
acgaggccct ttcgt                                                    4598
```

European Patent Office

# EUROPEAN SEARCH REPORT

Application Number
EP 06 01 1026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/123942 A2 (NOVARTIS FORSCHUNGSSTIFTUNG [CH]; SCHUEBELER DIRK [CH]; WEBER MICHAEL) 29 December 2005 (2005-12-29) | 1-14 | INV. C12Q1/68 C12N15/10 |
| X | * page 32, lines 17-27 - page 33, lines 1-5 * | 1-14 | |
| Y | * page 17 - page 19; figure 1 * ----- | 1-14 | |
| Y | MOLINA JULIAN R ET AL: "Detection of pancreatic cancer by assay of DNA alterations in stool" GASTROENTEROLOGY, vol. 126, no. 4, Suppl. 2, April 2004 (2004-04), page A24, XP008071674 & DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085 * the whole document * ----- | 1-14 | |
| | | | TECHNICAL FIELDS SEARCHED (IPC) |
| A | RAUCH T ET AL: "Methylated-CpG island recovery assay: a new technique for the rapid detection of methylated-CpG islands in cancer" LABORATORY INVESTIGATION, UNITED STATES AND CANADIAN ACADEMY OF PATHOLOGY, BALTIMORE,, US, vol. 85, no. 9, 18 July 2005 (2005-07-18), pages 1172-1180, XP002375686 ISSN: 0023-6837 * the whole document * ----- | 1-14 | C12Q C12N |
| A | WO 2006/008650 A (KAROLINSKA INNOVATIONS AB [SE]; CORCORAN MARTIN MATTHEW [SE]) 26 January 2006 (2006-01-26) * the whole document * ----- -/-- | 1-14 | |

~~The present search report has been drawn up for all claims~~

| Place of search | Date of completion of the search | Examiner |
|---|---|---|
| The Hague | 22 November 2006 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 01 1026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PLASS C ET AL: "RESTRICTION LANDMARK GENOME SCANNING FOR ABERRANT METHYLATION IN PRIMARY REFRACTORY AND RELAPSED ACUTE MYELOID LEUKEMIA" ONCOGENE, BASINGSTOKE, HANTS, GB, vol. 18, no. 20, 20 May 1999 (1999-05-20), pages 3159-3165, XP009010444 ISSN: 0950-9232 * the whole document * ----- | 1-14 | |
| A | WO 02/101353 A2 (U S GENOMICS INC [US]) 19 December 2002 (2002-12-19) * the whole document * ----- | 1-14 | |
| | | | TECHNICAL FIELDS SEARCHED (IPC) |

~~The present search report has been drawn up for all claims~~

| Place of search | Date of completion of the search | Examiner |
|---|---|---|
| The Hague | 22 November 2006 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 06 01 1026

## CLAIMS INCURRING FEES

The present European patent application comprised at the time of filing more than ten claims.

- [ ] Only part of the claims have been paid within the prescribed time limit. The present European search report has been drawn up for the first ten claims and for those claims for which claims fees have been paid, namely claim(s):

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-14 (all partially)

European Patent Office

# LACK OF UNITY OF INVENTION SHEET B

Application Number
EP 06 01 1026

The Search Division considers that the present European patent application does not comply with the requirements of unity of invention and relates to several inventions or groups of inventions, namely:

Invention 1 (claims 1-14 all partially):

Methods and for diagnosing a predispostion of cancer or cancer comprising determining the methylation status of KLF11 (SEQ ID NO:1) alone or in combination with any of SEQ ID NOs: 2-111; a kit for performing such method; the use of the sequences above for diagnosing a predispostion of cancer or cancer; a diagnostic composition comprising sequence KLF11 (SEQ ID NO:1) alone or in combination with any SEQ ID NO: 2-111.

---

Inventions 2-4 (claims 1-14 all partially)

Same as above where the sequences whose methylation status is determined (taken alone or in combination with any of SEQ ID NO: 2-111) are: PLA2G7 (SEQ ID NO:2) for invention 2; PFC (SEQ ID NO: 3) for invention 3; MAFB (SEQ ID NO: 4) for invention 4.

---

Invention 5 (claim 14 partially)

A diagnostic composition comprising SEQ ID NO:5 taken alone or in combination with any of SEQ ID NOs: 2-111.

---

Invention 6-111 (claim 14 partially)

Same as above for each of SEQ ID Nos: 6-111 taken alone or in combination with any of SEQ ID Nos: 2-111.

---

## ANNEX TO THE EUROPEAN SEARCH REPORT ON EUROPEAN PATENT APPLICATION NO. EP 06 01 1026

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2006

| Patent document cited in search report | Publication date | Patent family member(s) | Publication date |
|---|---|---|---|
| WO 2005123942 A2 | 29-12-2005 | AU 2005254616 A1 | 29-12-2005 |
| | | CA 2568575 A1 | 29-12-2005 |
| WO 2006008650 A | 26-01-2006 | NONE | |
| WO 02101353 A2 | 19-12-2002 | AU 2002318336 A1 | 23-12-2002 |
| | | EP 1402071 A2 | 31-03-2004 |
| | | JP 2004536596 T | 09-12-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4946778 A
- US 4741900 A
- WO 9831836 A

### Non-patent literature cited in the description


- A multilingual glossary of biotechnological terms: (IUPAC Recommendations. Helvetica Chimica Acta. 1995
- **SINGAL.** *Blood,* 1999, vol. 93, 4059-4070
- **ROBERTSON.** *Nat. Rev. Genet.,* 2000, vol. 1, 11-19
- *Curr. Opin. Genet. Dev.,* 2000, 158-163
- **RAZIN.** *EMBO J.,* 1998, vol. 17, 4905-4908
- Genes II. John Wiley & Sons, 1985
- **RODAK.** Haematology: Clinical Principles & Applications. WB Saunders Co, 2002
- **BRUNZEL.** Fundamentals of Urine and Body Fluids Analysis. WB Saunders Co, 1994
- Cerebrospinal Fluid. Kluwer Academic Pub, 1989
- **SAMBROOK et al.** Molecular Cloning: A Laboratory Manual. Cold Spring Harbor Laboratory, 2001
- Current Protocols in Molecular Biology. John Wiley and Sons, Inc, 1999
- PROTEINS - STRUCTURE AND MOLECULAR PROPERTIES. W. H. Freeman and Company, 1993
- POST-TRANSLATIONAL COVALENT MODIFICATION OF PROTEINS. Academic Press, 1983, 1-12
- **SEIFTER.** *Meth. Enzymol.,* 1990, vol. 182, 626-646
- **RATTAN.** *Ann. NY Acad. Sci.,* 1992, vol. 663, 48-62
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **NIELSEN et al.** *Science,* 1991, vol. 254, 1497
- **EGHOLM et al.** *Nature,* 1993, vol. 365, 666
- **NIELSEN.** *Science,* 1991, vol. 254, 1497-1500
- **HOUGHTON.** *Proc. Natl. Acad. Sci. USA,* 1985, (82), 5131-5135
- **NEEDLEMAN.** *J. Mol Biol.,* 1970, vol. 48, 443-453
- accepted point mutation. **DAYHOFF et al.** Atlas of Protein Sequence and Structure. Nat. Biomed. Res. Foundation, 1978, vol. 5, 354-352
- **BALLESTER ; WOLFFE.** *Eur. J. Biochem.,* 2001, vol. 268, 1-6
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1977, vol. 25, 3389-3402
- **HENIKOFF.** *Proc. Natl. Acad. Sci.,* 1989, vol. 89, 10915
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENDRICH ; TWEEDY.** *Trends Genet.,* 2003, vol. 19, 269-77
- **BOWIE.** *Science,* 1990, vol. 247, 1306-1310
- **CUNNINGHAM ; WELLS.** *Science,* 1989, vol. 244, 1081-1085
- Computational Molecular Biology. Oxford University Press, 1988
- Biocomputing: Infoliuaties and Genome Projects. Academic Press, 1993
- Informafies Computer Analyzis of Sequence Data. Humana Press, 1994
- **VON HEINJE, G.** Sequence Analyzis in Molecular Biology. Academie Press, 1987
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96

- Monoclonal Antibodies For Immunotargeting Of Drugs In Cancer Therapy. **ARNON et al.** Monoclonal Antibodies And Cancer Therapy. Alan R. Liss, Inc, 1985, 243-56
- Antibodies For Drug Delivery. **HELLSTROM et al.** Controlled Drug Delivery. Marcel Dekker, Inc, 1987, 623-53
- Antibody Carriers Of Cytotoxic Agents In Cancer Therapy: A Review. Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **THORPE.** *Immunol. Rev.,* 119-158
- **DING.** *J. Biochem. Mol. Biol.,* 2004, vol. 37, 1-10
- **GILLAM.** *Gene,* 1979, vol. 8, 81-97
- **INNIS.** PCR Protocols: A guide to methods and applications. Academic Press, 1990
- **GOULIAN.** *Biochemistry,* 1973, vol. 12, 2893-2901
- **SANGER.** *Proc. Natl. Acad. Sci.,* 1977, vol. 74, 5463-5467
- DNA Microarrays : A Practical Approach (Practical Approach Series. Oxford University Press, 1999
- *Nature Genet.,* 1999, vol. 21 (1), 1-60
- Microarray Biochip : Tools and Technology. Eaton Publishing Company/BioTechniques Books Division, 2000
- **GOODCHILD.** *Bioconjug Chem.,* 1990, vol. 1, 165-187
- **NICHOLS et al.** *Nature,* 1994, vol. 369, 492-493
- **COSTELLO JF ; PLASS C.** Methylation matters. *J Med Genet,* May 2001, vol. 38 (5), 285-303
- **ESTELLER M ; FRAGA MF ; PAZ MF ; CAMPO E ; COLOMER D ; NOVO FJ et al.** Cancer epigenetics and methylation. *Science,* 13 September 2002, vol. 297 (5588), 1807-8
- **NG HH ; BIRD A.** DNA methylation and chromatin modification. *Curr Opin Genet Dev,* April 1999, vol. 9 (2), 158-63
- **RAZIN A.** CpG methylation, chromatin structure and gene silencing-a three-way connection. *EMBO J,* 01 September 1998, vol. 17 (17), 4905-8
- **ROBERTSON KD.** DNA methylation and human disease. *Nat Rev Genet,* August 2005, vol. 6 (8), 597-610
- **HERMAN JG ; BAYLIN SB.** Gene silencing in cancer in association with promoter hypermethylation. *N Engl J Med,* 20 November 2003, vol. 349 (21), 2042-54
- **MOMPARLER RL.** Cancer epigenetics. *Oncogene,* 29 September 2003, vol. 22 (42), 6479-83
- **PLASS C.** Cancer epigenomics. *Hum Mol Genet,* 01 October 2002, vol. 11 (20), 2479-88
- **COSTELLO JF ; FRUHWALD MC ; SMIRAGLIA DJ ; RUSH LJ ; ROBERTSON GP ; GAO X et al.** Aberrant CpG-island methylation has non-random and tumour-type-specific patterns. *Nat Genet,* February 2000, vol. 24 (2), 132-8
- **ESTELLER M ; CORN PG ; BAYLIN SB ; HERMAN JG.** A gene hypermethylation profile of human cancer. *Cancer Res,* 15 April 2001, vol. 61 (8), 3225-9
- **ESTELLER M.** Dormant hypermethylated tumour suppressor genes: questions and answers. *J Pathol,* January 2005, vol. 205 (2), 172-80
- **KALEBIC T.** Epigenetic changes: potential therapeutic targets. *Ann N Y Acad Sci,* March 2003, vol. 983, 278-85
- **ESTELLER M.** Relevance of DNA methylation in the management of cancer. *Lancet Oncol,* June 2003, vol. 4 (6), 351-8
- **DAHL C ; GULDBERG P.** DNA methylation analysis techniques. *Biogerontology,* 2003, vol. 4 (4), 233-50
- **KRAUSE SW ; REHLI M ; KREUTZ M ; SCHWARZFISCHER L ; PAULAUSKIS JD ; ANDREESEN R.** Differential screening identifies genetic markers of monocyte to macrophage maturation. *J Leukoc Biol,* October 1996, vol. 60 (4), 540-5
- **FROMMER M ; MCDONALD LE ; MILLAR DS ; COLLIS CM ; WATT F ; GRIGG GW et al.** A genomic sequencing protocol that yields a positive display of 5-methylcytosine residues in individual DNA strands. *Proc Natl Acad Sci U S A,* 01 March 1992, vol. 89 (5), 1827-31
- **HAEHNEL V ; SCHWARZFISCHER L ; FENTON MJ ; REHLI M.** Transcriptional regulation of the human toll-like receptor 2 gene in monocytes and macrophages. *J Immunol,* 01 June 2002, vol. 168 (11), 5629-37
- **CROSS SH ; CHARLTON JA ; NAN X ; BIRD AP.** Purification of CpG islands using a methylated DNA binding column. *Nat Genet,* March 1994, vol. 6 (3), 236-44
- **ZESCHNIGK M ; SCHMITZ B ; DITTRICH B ; BUITING K ; HORSTHEMKE B ; DOERFLER W.** Imprinted segments in the human genome: different DNA methylation patterns in the Prader-Willi/Angelman syndrome region as determined by the genomic sequencing method. *Hum Mol Genet,* March 1997, vol. 6 (3), 387-95
- **CHIM CS ; WONG AS ; KWONG YL.** Epigenetic inactivation of INK4/CDK/RB cell cycle pathway in acute leukemias. *Ann Hematol,* December 2003, vol. 82 (12), 738-42
- **DODGE JE ; LIST AF ; FUTSCHER BW.** Selective variegated methylation of the p15 CpG island in acute myeloid leukemia. *Int J Cancer,* 23 November 1998, vol. 78 (5), 561-7

- **DODGE JE ; MUNSON C.** List AF. KG-1 and KG-1 a model the p15 CpG island methylation observed in acute myeloid leukemia patients. *Leuk Res,* October 2001, vol. 25 (10), 917-25
- **ISSA JP ; ZEHNBAUER BA ; CIVIN CL ; COLLECTOR ML ; SHARKIS SJ ; DAVIDSON NE et al.** The estrogen receptor CpG island is methylated in most hematopoietic neoplasms. *Cancer Res,* 01 March 1996, vol. 56 (5), 973-7
- **PAZ MF ; WEI S ; CIGUDOSA JC ; RODRIGUEZ-PERALES S ; PEINADO MA ; HUANG TH et al.** Genetic unmasking of epigenetically silenced tumor suppressor genes in colon cancer cells deficient in DNA methyltransferases. *Hum Mol Genet,* 01 September 2003, vol. 12 (17), 2209-19
- **DOUGLAS DB ; AKIYAMA Y ; CARRAWAY H ; BELINSKY SA ; ESTELLER M ; GABRIELSON E et al.** Hypermethylation of a small CpGuanine-rich region correlates with loss of activator protein-2alpha expression during progression of breast cancer. *Cancer Res,* 01 March 2004, vol. 64 (5), 1611-20
- **SCHWAB J ; LLLGES H.** Regulation of CD21 expression by DNA methylation and histone deacetylation. *Int Immunol,* May 2001, vol. 13 (5), 705-10
- **SATO K ; TAMURA G ; TSUCHIYA T ; ENDOH Y ; USUBA O ; KIMURA W et al.** Frequent loss of expression without sequence mutations of the DCC gene in primary gastric cancer. *Br J Cancer,* 20 July 2001, vol. 85 (2), 199-203
- **JONES PA ; WOLKOWICZ MJ ; RIDEOUT WM ; GONZALES FA ; MARZIASZ CM ; COETZEE GA et al.** De novo methylation of the MyoD1 CpG island during the establishment of immortal cell lines. *Proc Natl Acad Sci U S A,* August 1990, vol. 87 (16), 6117-21
- **YUAN BZ ; DURKIN ME ; POPESCU NC.** Promoter hypermethylation of DLC-1, a candidate tumor suppressor gene, in several common human cancers. *Cancer Genet Cytogenet,* 15 January 2003, vol. 140 (2), 113-7
- **CHOI MC ; JONG HS ; KIM TY ; SONG SH ; LEE DS ; LEE JW et al.** AKAP12/Gravin is inactivated by epigenetic mechanism in human gastric carcinoma and shows growth suppressor activity. *Oncogene,* 16 September 2004, vol. 23 (42), 7095-103
- **WEBER M ; DAVIES JJ ; WITTIG D ; OAKELEY EJ ; HAASE M ; LAM WL et al.** Chromosome-wide and promoter-specific analyses identify sites of differential DNA methylation in normal and transformed human cells. *Nat Genet,* August 2005, vol. 37 (8), 853-62
- **COSTELLO JF ; SMIRAGLIA DJ ; PLASS C.** Restriction landmark genome scanning. *Methods,* June 2002, vol. 27 (2), 144-9
- **SMITH RJ ; DEAN W ; KONFORTOVA G ; KELSEY G.** Identification of novel imprinted genes in a genome-wide screen for maternal methylation. *Genome Res,* April 2003, vol. 13 (4), 558-69
- **YAN PS ; CHEN CM ; SHI H ; RAHMATPANAH F ; WEI SH ; CALDWELL CW et al.** Dissecting complex epigenetic alterations in breast cancer using CpG island microarrays. *Cancer Res,* 01 December 2001, vol. 61 (23), 8375-80
- **BROCK GJ ; HUANG TH ; CHEN CM ; JOHNSON KJ.** A novel technique for the identification of CpG islands exhibiting altered methylation patterns (ICEAMP). *Nucleic Acids Res,* 15 December 2001, vol. 29 (24), E123
- **SHIRAISHI M ; CHUU YH ; SEKIYA T.** Isolation of DNA fragments associated with methylated CpG islands in human adenocarcinomas of the lung using a methylated DNA binding column and denaturing gradient gel electrophoresis. *Proc Natl Acad Sci U S A,* 16 March 1999, vol. 96 (6), 2913-8
- **KLOSE RJ ; SARRAF SA ; SCHMIEDEBERG L ; MCDERMOTT SM ; STANCHEVA I ; BIRD AP.** DNA binding selectivity of MeCP2 due to a requirement for A/T sequences adjacent to methyl-CpG. *Mol Cell,* 02 September 2005, vol. 19 (5), 667-78
- **FRAGA MF ; BALLESTAR E ; MONTOYA G ; TAYSAVANG P ; WADE PA ; ESTELLER M.** The affinity of different MBD proteins for a specific methylated locus depends on their intrinsic binding properties. *Nucleic Acids Res,* 15 March 2003, vol. 31 (6), 1765-74
- **RUSH LJ ; DAI Z ; SMIRAGLIA DJ ; GAO X ; WRIGHT FA ; FRUHWALD M et al.** Novel methylation targets in de novo acute myeloid leukemia with prevalence of chromosome 11 loci. *Blood,* 15 May 2001, vol. 97 (10), 3226-33
- **KELLY LM ; ENGLMEIER U ; LAFON I ; SIEWEKE MH ; GRAF T.** MafB is an inducer of monocytic differentiation. *EMBO J,* 02 May 2000, vol. 19 (9), 1987-97